# EUROPEAN PATENT APPLICATION

(11) **EP 4 759 810 A1**
(43) Date of publication of application: **17.06.2026**
(21) Application number: 24853531.2
(22) Date of filing: 26.07.2024
(51) Int. Cl.: C07D 417/14, C07D 277/62, A61K 31/4725, A61K 31/425, A61K 31/427, A61P 43/00

(54) **PROTAC TARGETING BCL-XL PROTEIN AND USE THEREOF**

(30) Priority: 11.08.2023 CN 202311013468
(71) Applicant: BEIJING SUNCELL BIO-MEDICAL CO., LTD., Beijing 100085 (CN)
(72) Inventor: DENG, Hongkui, Beijing 100085 (CN); DONG, Jiebin, Beijing 100085 (CN); LI, Honggang, Beijing 100085 (CN); ZHAO, Jingjing, Beijing 100085 (CN)
(74) Representative: Altmann Stößel Dick Patentanwälte PartG mbB
(86) International application number: PCT/CN2024/107857
(87) International publication number: WO 2025/036119

(57) **Abstract**

The present application relates to PROTACs targeting the Bcl-xL protein and use thereof. In particular, the present invention provides a compound of formula I or pharmaceutically acceptable salts, esters, prodrugs, solvates, stereoisomers or deuterated compounds thereof: A-L-E (I), wherein A is a small molecule ligand moiety targeting the Bcl-xL protein; E is a small molecule ligand moiety targeting an E3 ubiquitin ligase complex; and L is a linker. The compound or composition of the present invention can be used for selectively killing one or more senescent cells or treating aging-related diseases or disorders in a subject.

## Description

### Technical Field of Invention

The present invention relates to PROTACs targeting the Bcl-xL protein, and use thereof.

### Background of Invention

The BCL-2 (B-cell lymphoma 2) gene family members play a central role in regulating programmed cell death by controlling the pro-apoptotic and anti-apoptotic intracellular signals (Czabotar, P. E et al., Nat Rev Mol Cell Biol 15, 49-63 (2014)). Anti-apoptotic BCL-2 family proteins such as BCL-2, BCL-XL, BCL-w, and Mcl-1 have been demonstrated to be highly expressed in various diseases, and in particular the BCL-xL protein is specifically highly expressed in various senescent cells (He et al., Nat Commun 11, 1996 (2020)). Currently, although the anti-aging effect of BCL-2 family inhibitors has been validated in various preclinical models, they also have significant intra target hematological toxicity, including thrombocytopenia (Cang et al., Journal of Hematology & Oncology 8, 129 (2015)). Thus, there is need for developing more refined and novel strategies to improve the efficacy and specificity against senescent cells and to reduce toxicity.

The concept of PROTAC (Proteolysis Targeting Chimeras) was first proposed in 2001 by Professor Craig Crews' team at Yale University. A PROTAC molecule consists of three parts: a target protein (POI) ligand, a linker, and an E3 ubiquitin ligase ligand. The PROTAC is a heterobifunctional chimeric molecule that targets the POI at one end and recruit the E3 ubiquitin ligase at the other end, forming a dumbbell-shaped ternary complex. The PROTAC molecules mediate the transient interaction between the E3 ligase and the POI. The E3 ubiquitin ligase attaches ubiquitin onto the POI, marking it as a defective or damaged protein. Then, the proteasome recognizes and degrades the marked POI. The main advantages of the PROTAC technology include event-driven activity (rather than the occupancy-driven activity of typical small molecule inhibitors), targeting undruggable proteins, overcoming drug resistance, low dosage, and the like, and thus it is potentially to ameliorate many limitations of traditional inhibitors. Because peptides were utilized initially to validate the artificially induced intracellular protein degradation, while such peptides have too large molecular weights, cell-penetrating peptides were needed to improve cell permeability. Thus, the PROTAC technology developed slowly during the decade from 2001 to 2011. With the continuous discovery of small molecule E3 ligase ligands, the PROTAC technology has developed rapidly. For example, mouse double minute 2 (MDM2) protein, cellular inhibitor of apoptosis protein (cIAP), von Hippel-Lindau protein (VHL), and CRBN (cereblon) have been successfully used for small molecule PROTACs, and CRBN-based ARV-110 and ARV-471 have been used in clinical trials. However, because PROTAC molecules typically have large molecular weights and do not meet the "Lipinski's Rule of Five", their druggability is poor. So, drug development based on this technology often suffers from poor pharmacokinetic parameters in vivo, presenting challenges in terms of low cell permeability and oral bioavailability, which necessitates screening different E3 ligase ligands and carefully optimizing the structure. At the same time, the overall degradation efficiency of PROTACs depends not only on the affinity of the target protein ligands and the type of E3 ligands, but also on the appropriate linkers. Extensive data show that the length, constitution, rigidity/flexibility, and linking sites of the linkers are crucial for the formation, degradation activity, and target selectivity of an active PROTAC ternary complex. The design of linkers may further enhance potency by lowering free energy or restricting bioactive conformation. Optimizing the structure-activity relationship (SAR) within a linker will provide opportunities to adjust the pharmacokinetic properties of protein degraders. However, there is no a universally applicable design strategy for the design and optimization of linkers, which often starts with a short, simple alkane or polyethylene glycol (PEG) chain, and then involves iterative trial and error to adjust the relevant properties thereof. In summary, the PROTAC is a powerful approach cross-overing current drug development obstacles, but in future there is still need for further molecular design and optimization and gradually summarization of principles to improve physicochemical properties, and further explore the efficacy and safety of PROTACs in clinical. PROTACs have a wide range of potential targets and a huge market, and with the continuous advancement and improvement of this technology, PROTACs can become a successful therapy like small molecule inhibitors, monoclonal antibodies, immunotherapy, and the like, benefiting more patients with various diseases.

Aging is a major risk factor for physiological degeneration, increased incidence of chronic diseases, and age-related mortality (Lopez-Otin et al., Cell 153, 1194-1217 (2013)). During the aging, senescent cells accumulate in multiple tissues and cause tissue dysfunction (van Deursen et al., Nature 509, 439-446 (2014); McHugh et al., J Cell Biol 217, 65-77 (2018)). Senescent cells also secrete various pro-inflammatory factors, known as the senescence-associated secretory phenotype (SASP), which contributes to the age-related physical decline (Coppe et al., PLoS Biol 6, 2853-2868 (2008); Coppe et al., Annu Rev Pathol 5, 99-118 (2010)). Clearing senescent cells has become an attractive potential approach to ameliorate age-related diseases and improve health (Xu et al., Nat Med 24, 1246-1256 (2018); Baker et al., Nature 479, 232-236 (2011); Baker et al., Nature 530, 184-189 (2016)). However, due to the complexity and heterogeneity of senescent cells, specifically and effectively clearing various types of senescent cells remains challenging (Kirkland et al. J Am Geriatr Soc 65, 2297-2301 (2017); Lozano-Torres et al., Nature Reviews Chemistry 3, 426-441 (2019)). Existing studies have shown that the Bcl-2 anti-apoptotic pathway plays a crucial role in maintaining the self-survival of senescent cells (Zhu et al., Aging Cell 14, 644-658 (2015), Chang et al., Nat Med 22, 78-83 (2016)). Compounds that selectively kill senescent cells, known as "senolytics", have attracted considerable interests. BCL-xL inhibitors can effectively kill senescent cells as anti-aging drugs (Zhu et al., Aging Cell 15, 428-435 (2016)). The Bcl-xL inhibitors induce apoptosis in senescent cells by disrupting the key anti-apoptotic mechanisms for maintaining the survival of senescent cells, thereby achieving the clearance of senescent cells both in vitro and in vivo, which show great potential in the treatment of various aging-related diseases such as osteoarthritis, age-related macular degeneration, diabetic nephropathy, or the like.

By structurally modifying the BCL-xL protein inhibitors and combining them with E3 ligase ligands to form PROTACs, their advantages can be effectively combined. This on one hand can specifically induce apoptosis in senescent cells, and on the other hand can also effectively reduce the hematological toxicity of BCL-xL protein inhibitors. Meanwhile, this optimizes the overall molecular structure, which ensures the efficacy and safety for clinical applications, providing a new concept for the treatment of a range of age- and aging-related diseases.

### Summary of Invention

One object of the present invention is to provide a compound of formula I, or pharmaceutically acceptable salts, esters, prodrugs, solvates, stereoisomers, or deuterated compounds thereof:

A-L-E (I)

wherein A is a small molecule ligand moiety targeting the Bcl-xL protein; E is a small molecule ligand moiety targeting the E3 ubiquitin ligase complex; and L is a linker.

### Small molecule ligand moiety A targeting the Bcl-xL protein

The small molecule ligand moiety A targeting the Bcl-xL protein has the structure of formula 1 or 2: 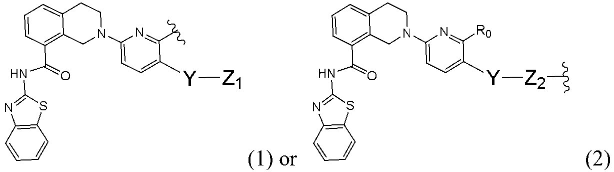 wherein R₀ is absent or selected from -COOR^{a}, -CONR^{a}R^{b}, -SO₃R^{a}, -SO₂NR^{a}R^{b}, -SO₂NR^{a}COR^{b}, - CONR^{a}SO₂R^{b}, -COSO₂NR^{a}R^{b}, 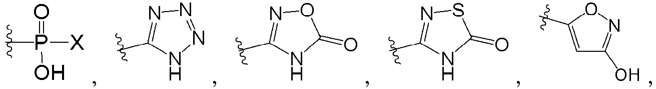 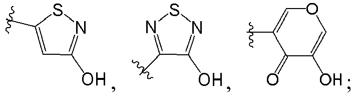
wherein X is selected from hydrogen, hydroxyl, amino, or CH(OR^{a})₂;
wherein R^{a} and R^{b} are independently selected from hydrogen, cyano, substituted or unsubstituted alkyl, or substituted or unsubstituted alkoxy;
Y is absent or selected from substituted or unsubstituted alkylene, substituted or unsubstituted alkyleneoxy, substituted or unsubstituted alkenylene, substituted or unsubstituted alkenyleneoxy, substituted or unsubstituted arylene, substituted or unsubstituted heteroarylene, substituted or unsubstituted cycloalkylene, or substituted or unsubstituted heterocyclylene;
Z₁ and Z₂ is absent or selected from substituted or unsubstituted -alkylene-cyclic group, substituted or unsubstituted cycloalkyl, substituted or unsubstituted heterocyclyl, substituted or unsubstituted aryl, or substituted or unsubstituted heteroaryl.

In some embodiments, the small molecule ligand moiety A targeting the Bcl-xL protein is a deuterated moiety, which has the structure of Formula 1' or 2': 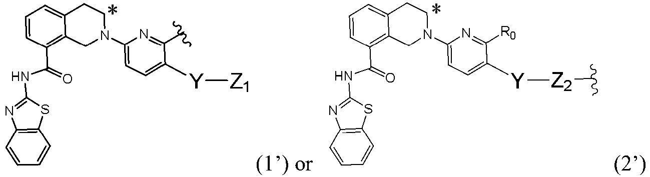 wherein one or more hydrogen atoms at position * are replaced by deuterium. In some embodiments, two hydrogen atoms at position * are replaced by deuterium.

In some embodiments, R^{a} and R^{b} are independently selected from hydrogen, cyano, substituted or unsubstituted C1-C6 alkyl, or substituted or unsubstituted C1-C6 alkoxy.

In some embodiments, R^{a} and R^{b} are independently selected from hydrogen, cyano, alkyl (e.g., C1-C6 alkyl), alkoxy (e.g., C1-C6 alkoxy), halogen-substituted alkyl (e.g., C1-C6 alkyl), or halogen-substituted alkoxy (e.g., C1-C6 alkoxy).

In some embodiments, Ro is absent or selected from: -COOR^{a}, -CONHR^{b}, -SO₃R^{a}, -SO₂NHR^{b}, - SO₂NHCOR^{b}, -CONHSO₂R^{b}, -COSO₂NHR^{b}, wherein R^{a} and R^{b} are independently selected from hydrogen, cyano, or alkyl (e.g., C1-C6 alkyl).

In some embodiments, R₀ is selected from:

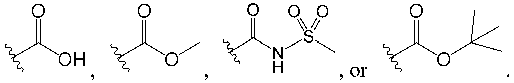

In some embodiments, Y is absent or selected from substituted or unsubstituted C1-6 alkylene, substituted or unsubstituted C1-6 alkyleneoxy, substituted or unsubstituted C2-6 alkenylene, substituted or unsubstituted C2-6 alkenyleneoxy, substituted or unsubstituted phenylene, substituted or unsubstituted 5- or 6-membered heteroarylene, substituted or unsubstituted C3-6 cycloalkylene, or substituted or unsubstituted 3 to 6-membered heterocyclylene.

In some embodiments, Y is selected from alkylene, alkyleneoxy, alkenylene, alkenyleneoxy, arylene, heteroarylene, cycloalkylene, or heterocyclylene, which are optionally substituted with one or more substituents selected from halogen or alkyl (e.g., C1-6 alkyl).

In some embodiments, Y is selected from C1-6 alkylene, C1-6 alkyleneoxy, C2-6 alkenylene, or C2-6 alkenyleneoxy.

In some embodiments, Y is selected from the following structures:

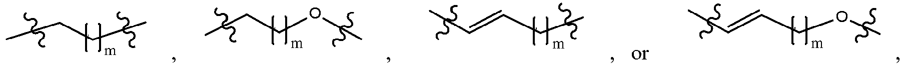

wherein m is 0, 1, 2, or 3.

In some embodiments, Y is selected from phenylene, or 5- or 6-membered heteroarylene, which are optionally substituted with one or more substituents selected from halogen or alkyl.

In some embodiments, Y is selected from pyrazolylene optionally substituted with one or more substituents selected from halogen or alkyl.

In some embodiments, Y is selected from the following structures: 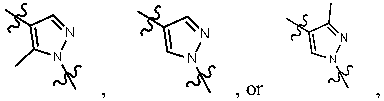 wherein Y is linked to the group Z₁ or Z₂ at the nitrogen atom indicated in the ring.

In some embodiments, Y is selected from the following structures: 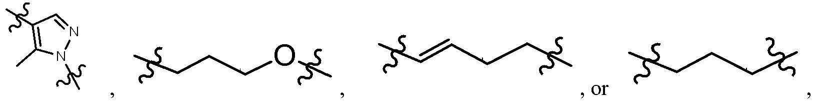 wherein Y is linked to the group Z₁ or Z₂ at the nitrogen atom indicated in the ring.

In some embodiments, Z₁ and Z₂ are independently selected from substituted or unsubstituted -C1-6 alkylene-cyclic group, substituted or unsubstituted C5-8 cycloalkyl, substituted or unsubstituted 5 to 8-membered heterocyclyl, substituted or unsubstituted phenyl, or substituted or unsubstituted 5 to 6-membered heteroaryl.

In some embodiments, Z₁ and Z₂ are independently selected from -alkylene-cyclic group, cycloalkyl-(R₂')ₚ, heterocyclyl-(R₂')ₚ, aryl-(R₂')ₚ, heteroaryl-(R₂')ₚ, aryl-propargylene, heteroaryl-propargylene, aryl-propargylene-N(R₂')₂, heteroaryl-propargylene-N(R₂')₂, aryl-propargylene-cycloalkyl-(R₂')ₚ, heteroaryl-propargylene-cycloalkyl-(R₂')ₚ, aryl-propargylene-heterocyclyl-(R₂')ₚ, or heteroaryl-propargylene-heterocyclyl-(R₂')ₚ, which are optionally substituted with one or more R₁.

In some embodiments, R₁' is deuterium, halogen, amino, hydroxyl, nitro, thiol, cyano, isocyano, substituted or unsubstituted alkyl (e.g., alkyl substituted with halogen, amino, hydroxyl, nitro, carboxyl, or the like), substituted or unsubstituted alkoxy (e.g., alkoxy substituted with halogen, amino, hydroxyl, nitro, carboxyl, or the like), alkylamino, dialkylamino, carboxyl, carbonyl, amido, sulfonyl, sulfonic acid group, phosphoryl, or phosphonyl.

In some embodiments, R₂' is hydrogen, alkyl, aryl, heteroaryl, cycloalkyl, or heterocyclyl.

In some embodiments, p is 0, 1, 2, 3 or 4.

In some embodiments, the cyclic group is

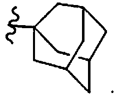

In some embodiments, Z₁ and Z₂ are independently selected from C5-8 cycloalkyl-(R₂')ₚ, heterocyclyl-(R₂')ₚ, phenyl-(R₂')ₚ, heteroaryl-(R₂')ₚ, phenyl-propargylene, heteroaryl-propargylene, phenyl-propargylene-N(R₂')₂, heteroaryl-propargylene-N(R₂')₂, aryl-propargylene-cycloalkyl-(R₂')ₚ, heteroaryl-propargylene-cycloalkyl-(R₂')ₚ, aryl-propargylene-heterocyclyl-(R₂')ₚ, or heteroaryl-propargylene-heterocyclyl-(R₂')ₚ, which are optionally substituted with one or more R_{1'}, wherein the heterocyclyl is 5- or 6-membered heterocyclyl containing one or two heteroatoms (e.g., piperidinyl, piperazinyl or morpholinyl) or 7 to 11-membered heterospirocyclyl (e.g., 3,9-diazaspiro[5.5]undecyl), the heteroaryl is 5- or 6-membered heteroaryl containing one or two heteroatoms (e.g., pyridinyl), and the heteroatoms are independently selected from nitrogen, oxygen or sulfur.

In some embodiments, p is 0 or 1.

In some embodiments, R_{1'} is selected from halogen (e.g., fluorine).

In some embodiments, R_{2'} is hydrogen or alkyl (e.g., methyl).

In some embodiments, Z₁ is selected from the following groups:

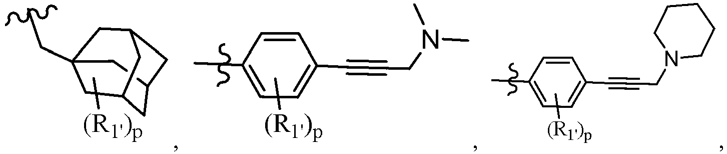

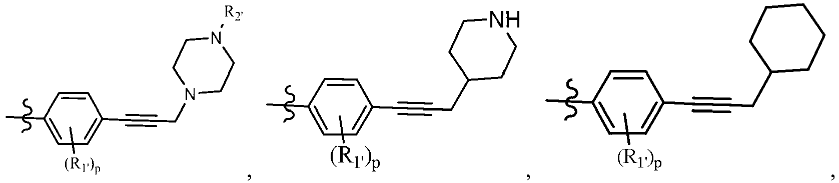

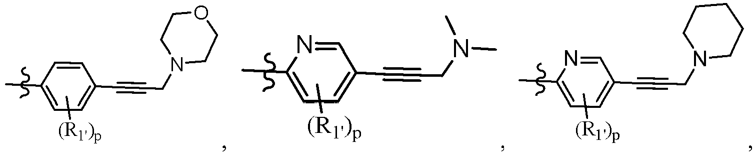

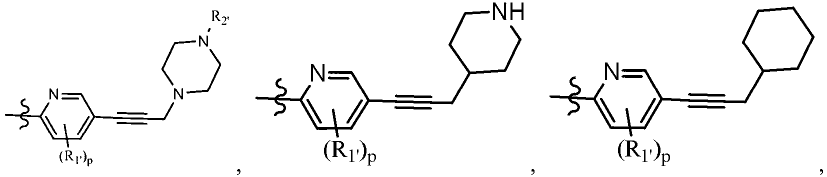

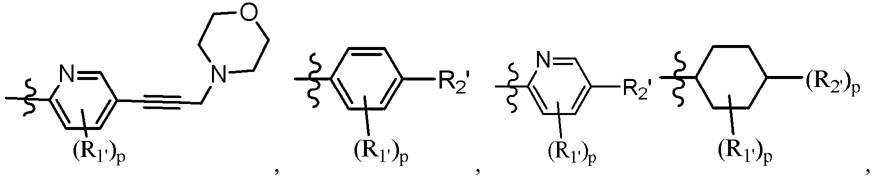

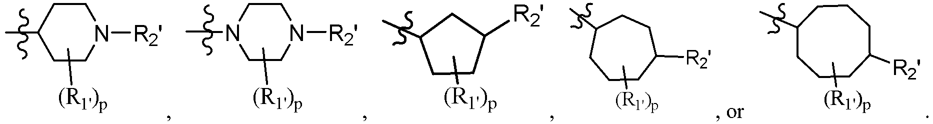

In some embodiments, Z₁ may be selected from the following groups:

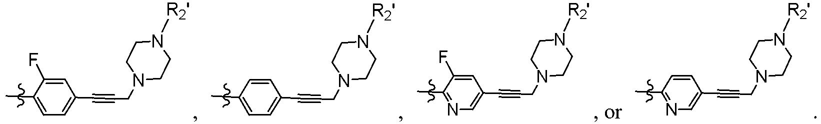

In some embodiments, Z₁ may be selected from the following groups:

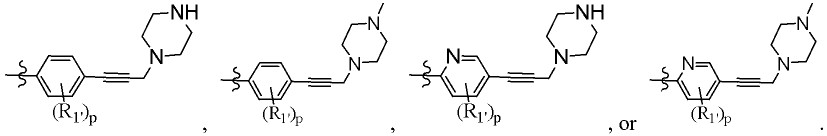

In some embodiments, Z₁ is selected from the following groups:

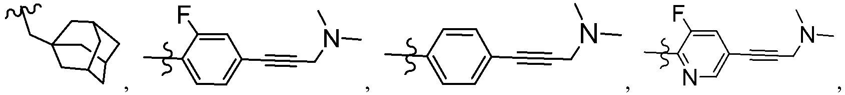

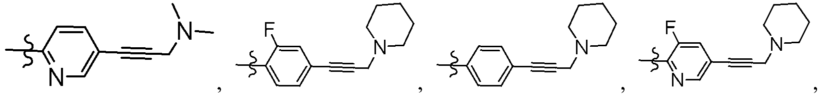

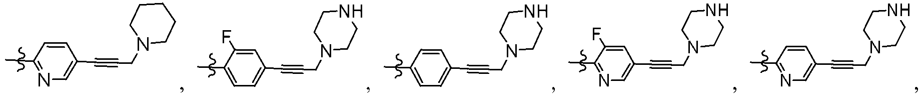

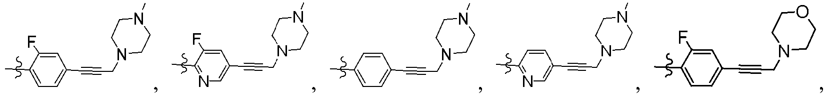

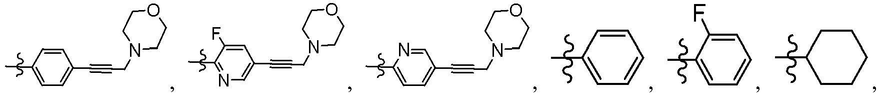

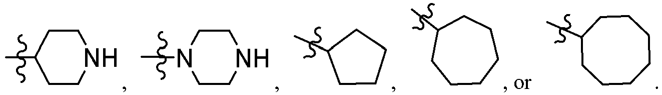

In some embodiments, Z₂ is selected from the following groups: 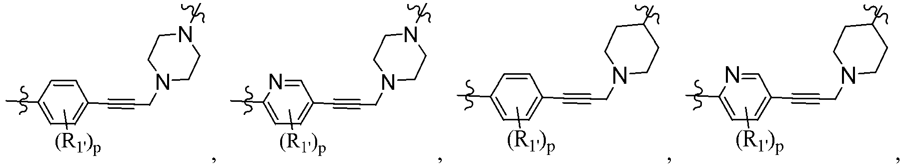 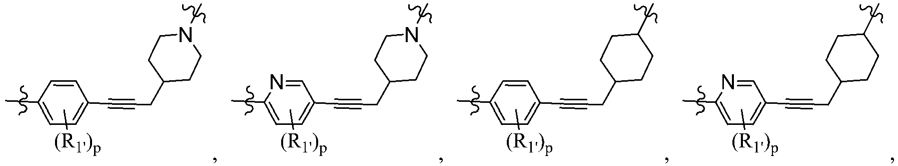 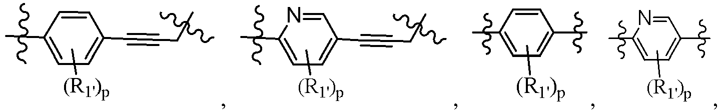 or

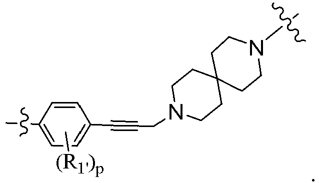

In some embodiments, Z₂ is selected from:

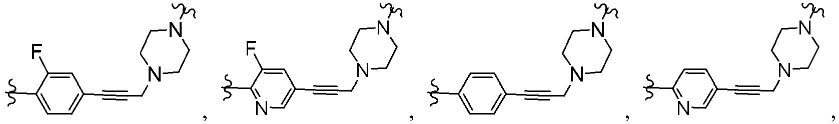

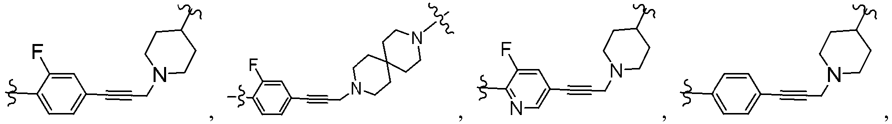

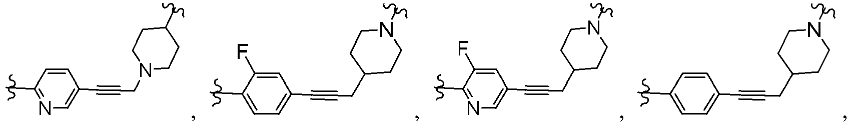

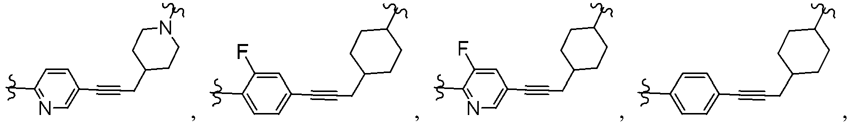

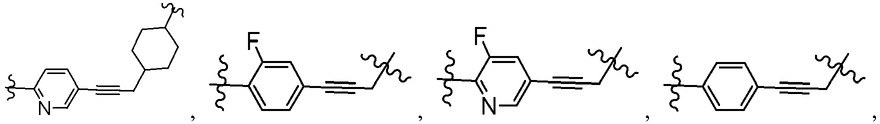

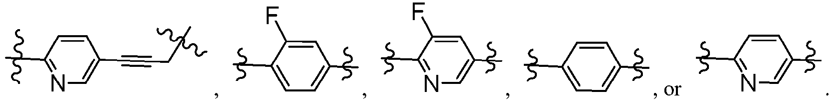

In some embodiments, the small molecule ligand moiety A targeting the Bcl-xL protein is selected from the following structures:

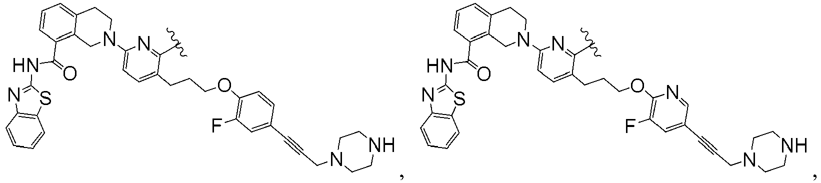

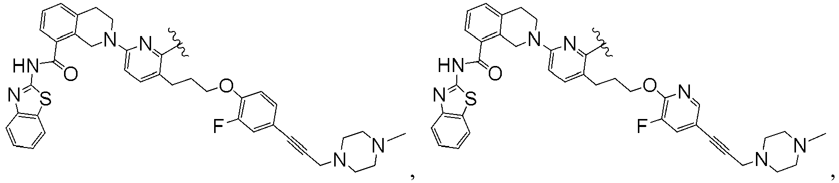

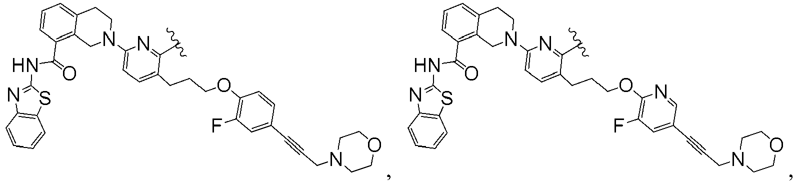

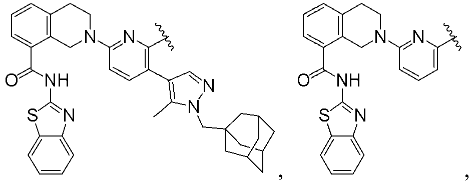

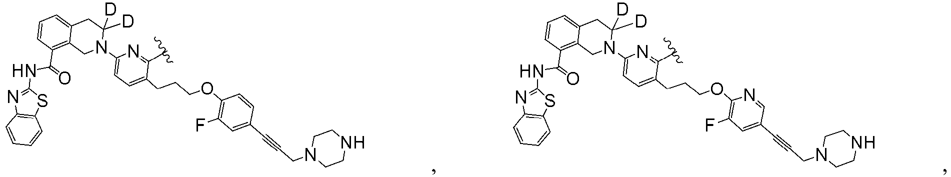

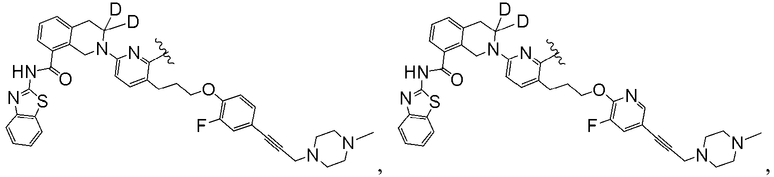

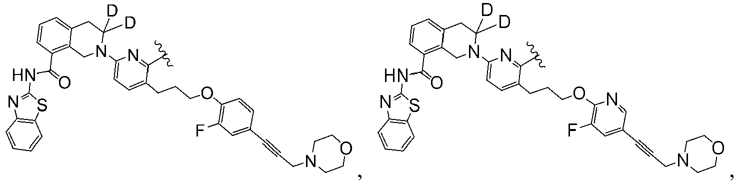

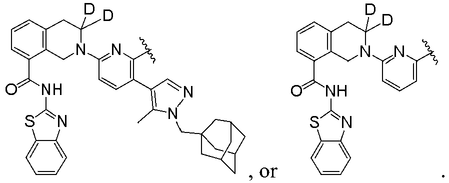

In some embodiments, the small molecule ligand moiety A targeting the Bcl-xL protein is selected from the following structures:

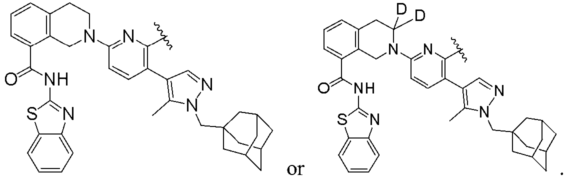

In some embodiments, the small molecule ligand moiety A targeting the Bcl-xL protein is selected from the following structures:

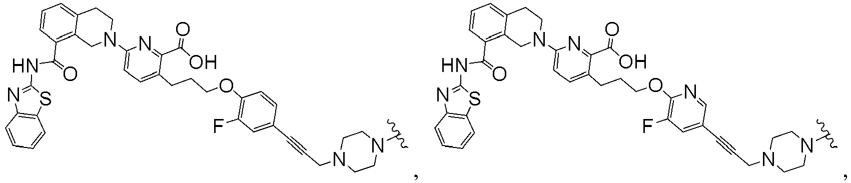

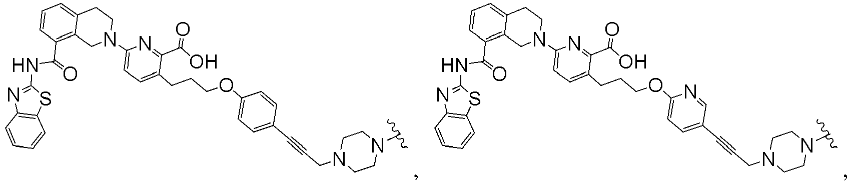

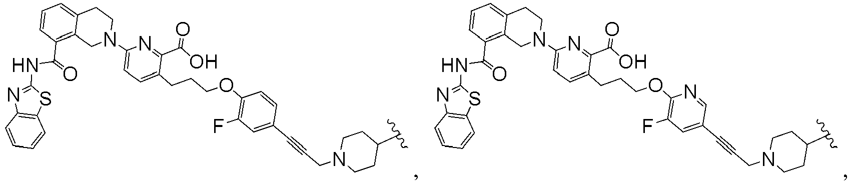

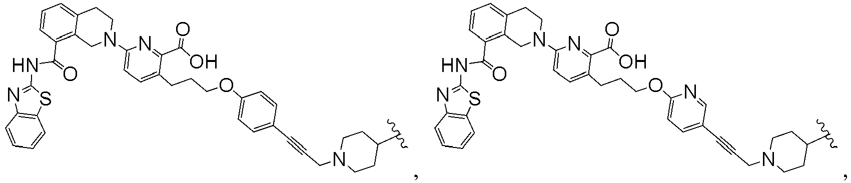

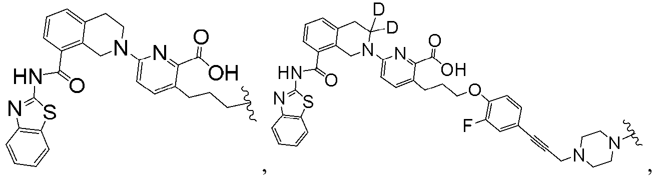

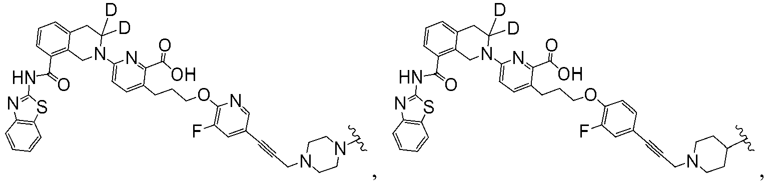

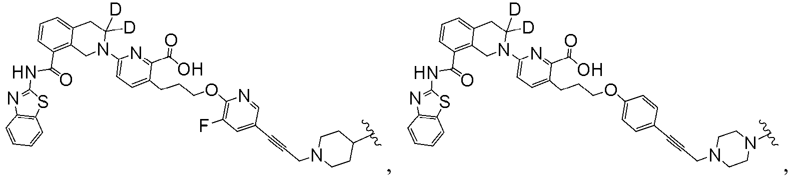

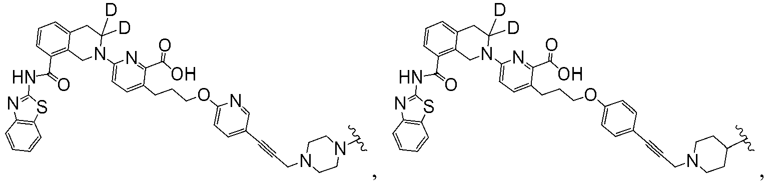

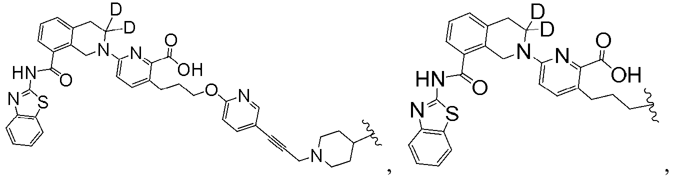

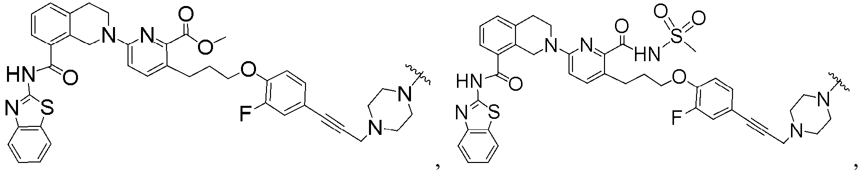

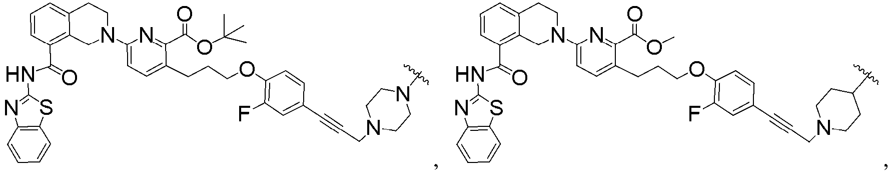

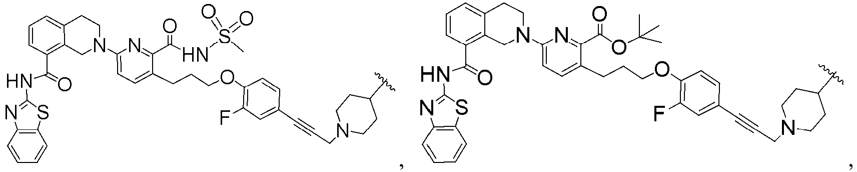

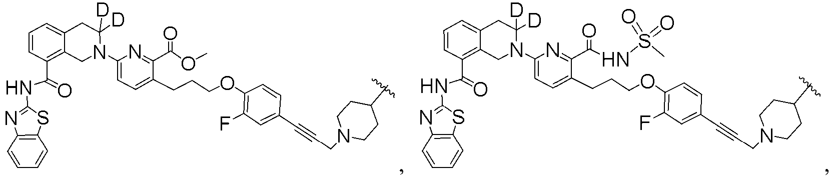

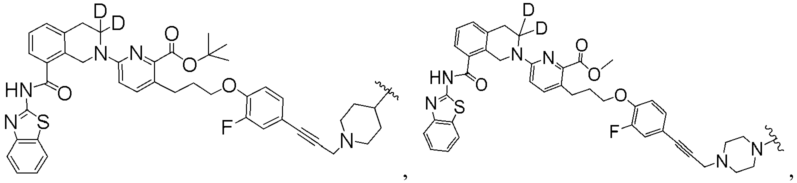

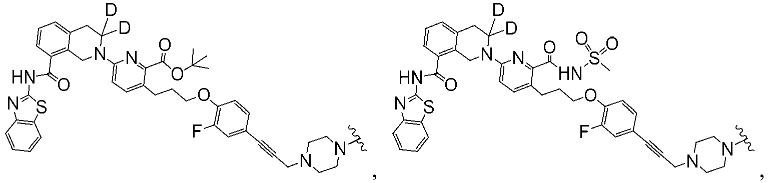

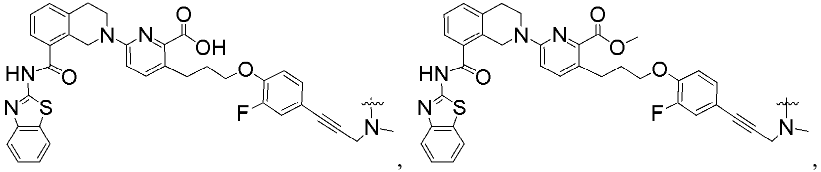

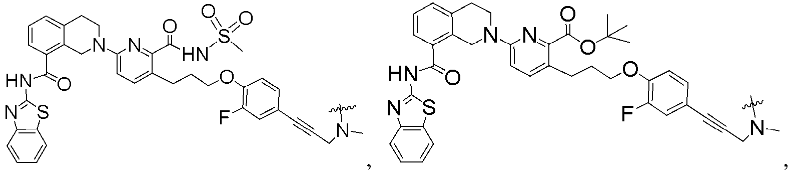

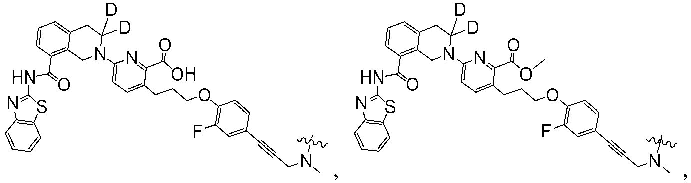

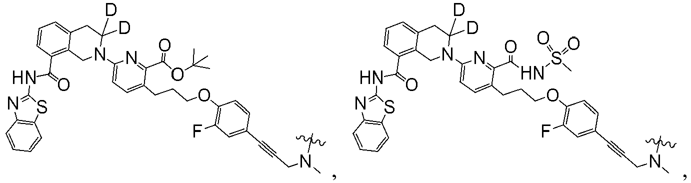

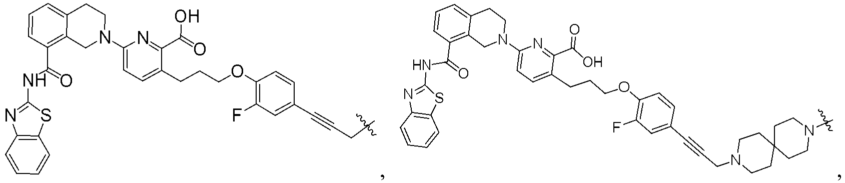

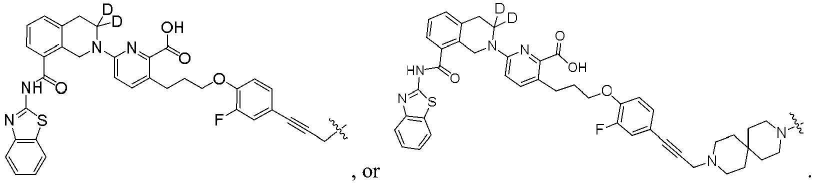

In some embodiments, the small molecule ligand moiety A targeting the Bcl-xL protein is selected from the following structures:

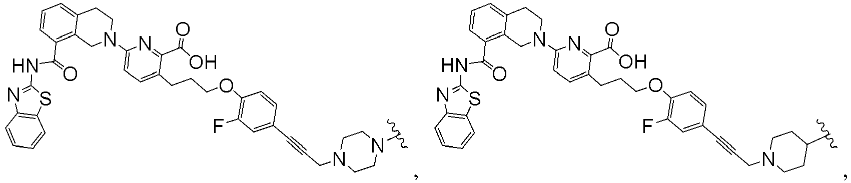

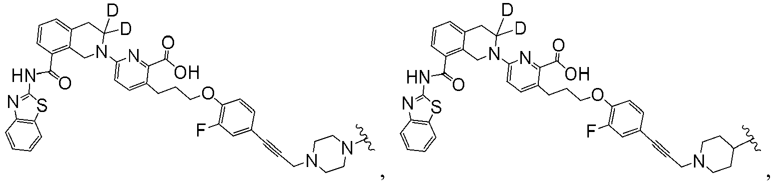

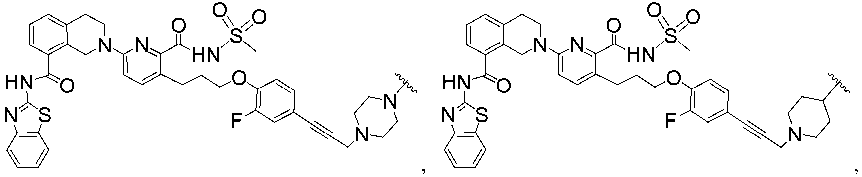

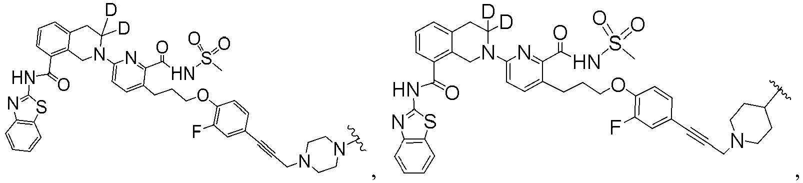

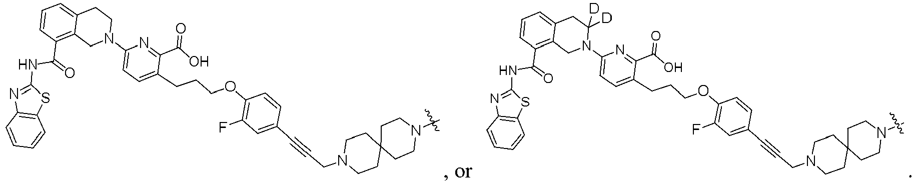

### Small molecule ligand moiety E targeting the E3 ubiquitin ligase complex

In some embodiments, the small molecule ligand moiety E targeting the E3 ubiquitin ligase complex is selected from the following structures: 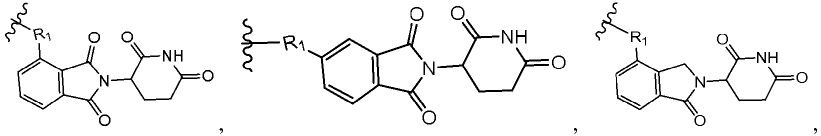 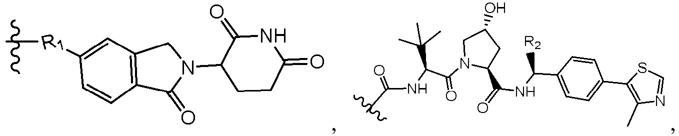 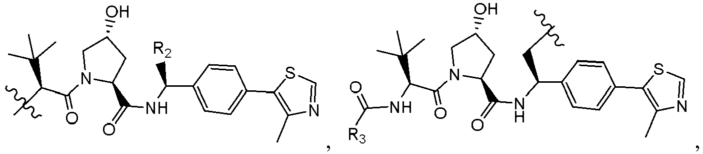 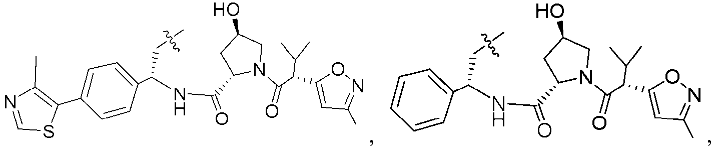 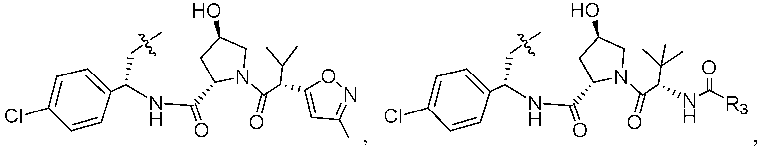 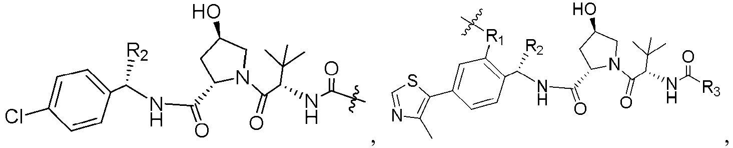 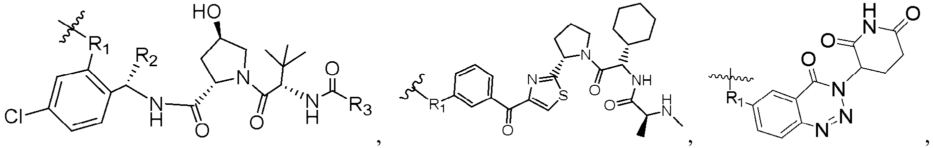 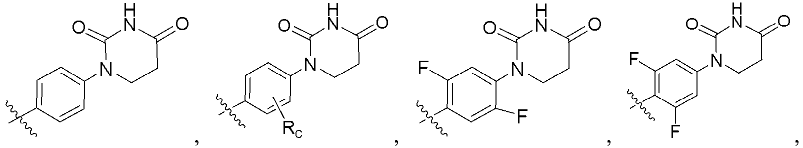 or 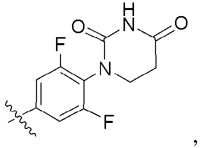 wherein R₁ is O, NH or absent; R₂ is H or methyl; R₃ is 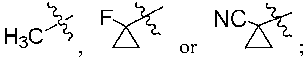 R_{c} is F, methoxy, Cl, or cyano.

In some embodiments, the small molecule ligand moiety E targeting the E3 ubiquitin ligase complex is selected from the following structures:

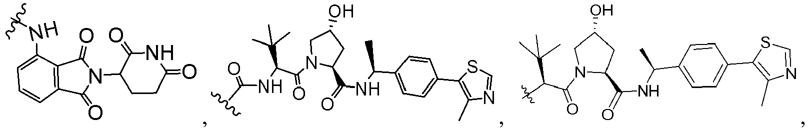

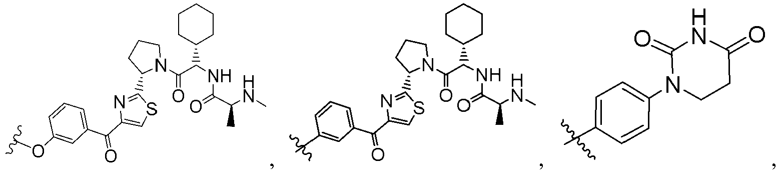

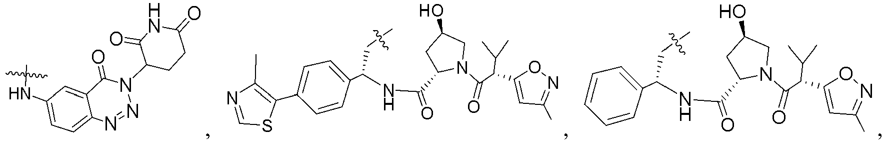

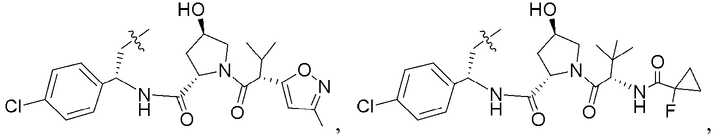

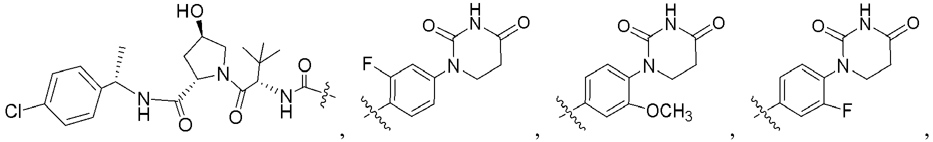

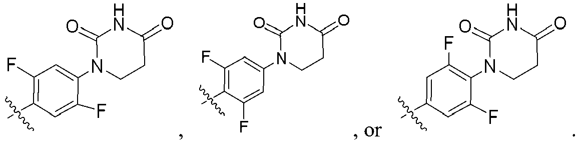

In some embodiments, the small molecule ligand moiety E targeting the E3 ubiquitin ligase complex is selected from the following structures:

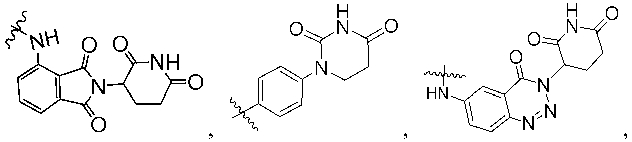

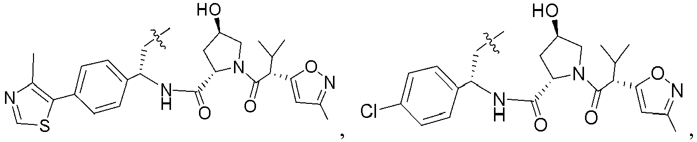

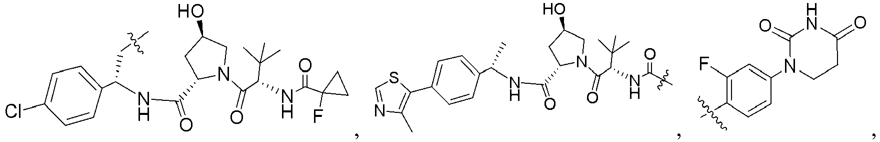

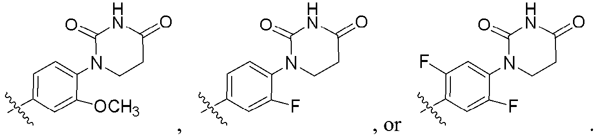

### Linker L

The linker L has the following structure:

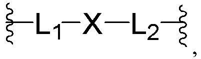

The linker L is linked to the small molecule ligand moiety A targeting the Bcl-xL protein at L1, and is linked to the small molecule ligand moiety E targeting the E3 ubiquitin ligase complex at L2,
wherein,
L₁ and L₂ are independently absent or selected from: 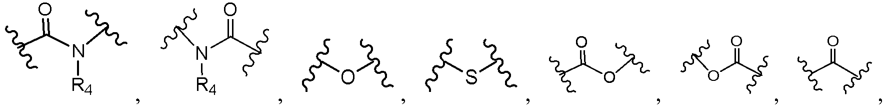 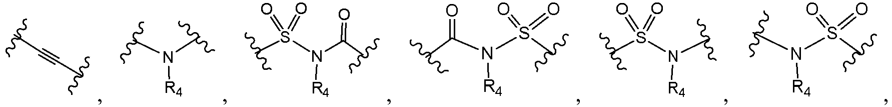 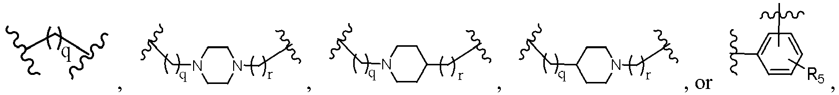
R₄ is independently selected from hydrogen, substituted or unsubstituted alkyl, substituted or unsubstituted cycloalkyl, or substituted or unsubstituted heterocyclyl;
R₅ is independently selected from hydrogen, nitro, cyano, isocyano, amino, hydroxyl, thiol, halogen, substituted or unsubstituted alkyl, substituted or unsubstituted alkoxy, alkylamino, dialkylamino, carboxyl, carbonyl, amido, sulfonyl, sulfonic acid group, phosphoryl, or phosphonyl;
q and r are independently selected from the integers from 0 to 10 (i.e., 0, 1, 2, 3, 4, 5, 6, 7, 8, 9, or 10);
X is absent or has the following structure: 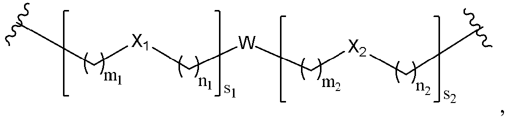
X₁ and X₂ are independently absent or selected from: 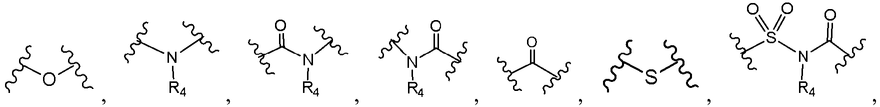 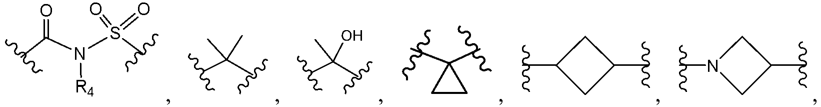 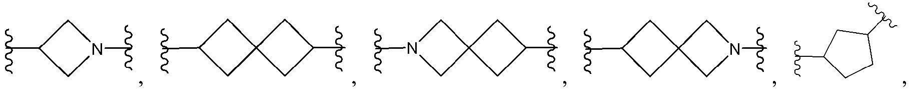 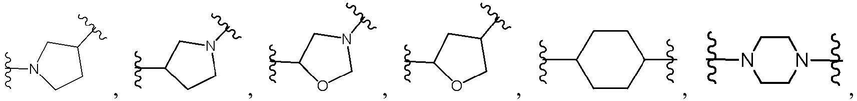 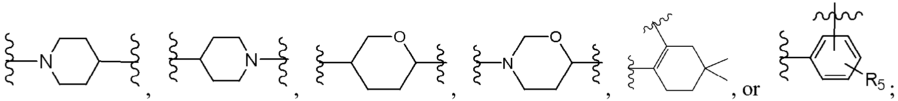
W is absent or selected from: 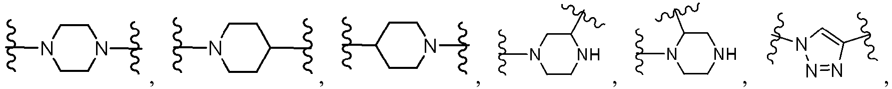 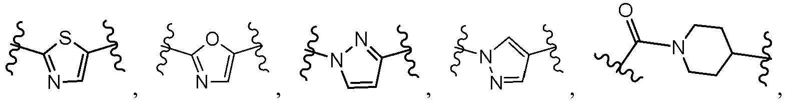 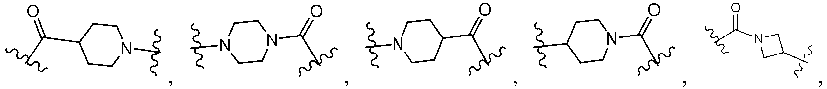 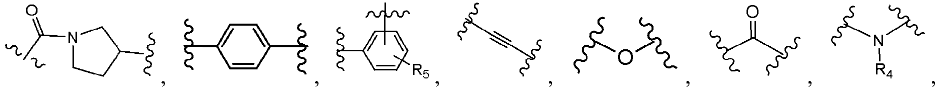 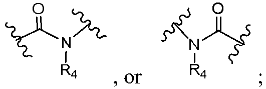
wherein m₁, m₂, n₁, n₂, s₁ and s₂ are independently selected from the integers from 0 to 10 (i.e., 0, 1, 2, 3, 4, 5, 6, 7, 8, 9, or 10);

In some embodiments, R₄ is independently selected from hydrogen, substituted or unsubstituted C1-6 alkyl, substituted or unsubstituted C3-8 cycloalkyl, or substituted or unsubstituted 3-8 membered heterocyclyl, wherein the alkyl, cycloalkyl, and heterocyclyl may be substituted with halogen, amino, hydroxyl, nitro, and/or carboxyl.

In some embodiments, R₅ is independently selected from hydrogen, nitro, cyano, isocyano, amino, hydroxyl, thiol, halogen, substituted or unsubstituted alkyl (e.g., alkyl substituted with halogen, amino, hydroxyl, nitro and/or carboxyl), substituted or unsubstituted alkoxy (e.g., alkoxy substituted with halogen, amino, hydroxyl, nitro and/or carboxyl), alkylamino, dialkylamino, carboxyl, substituted or unsubstituted carbonyl, amido, sulfonyl, sulfonic acid group, phosphoryl, or phosphonyl.

In some embodiments, L₁ is absent or selected from

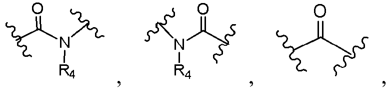

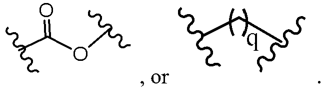

In some embodiments, L₂ is absent or selected from

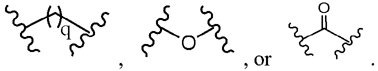

In some embodiments, the group X may be selected from the following structures: 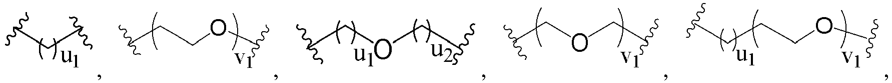 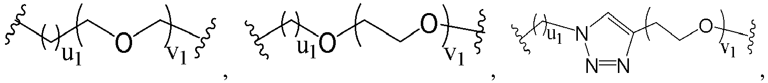 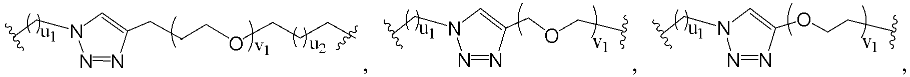 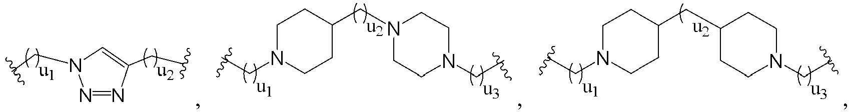 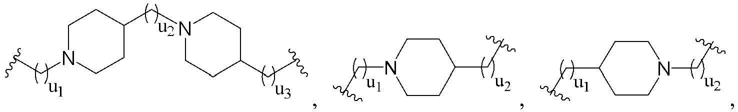 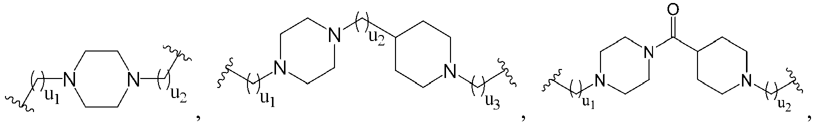 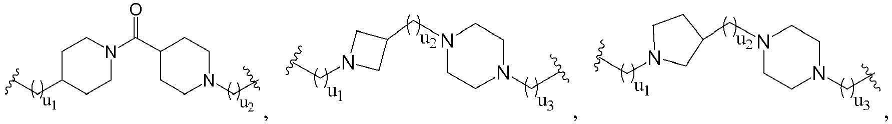 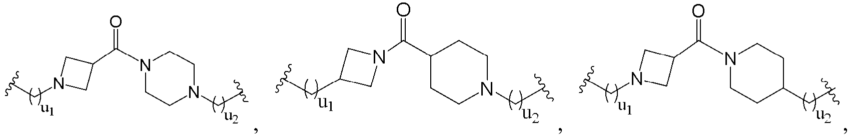 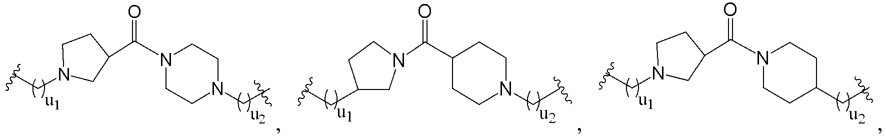 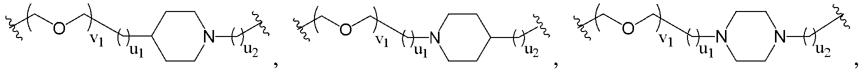 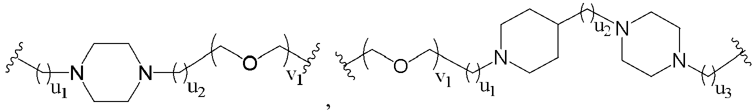 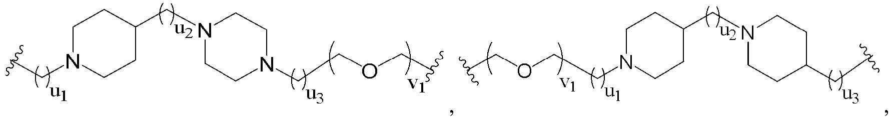 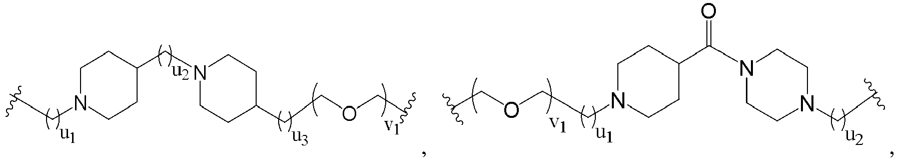 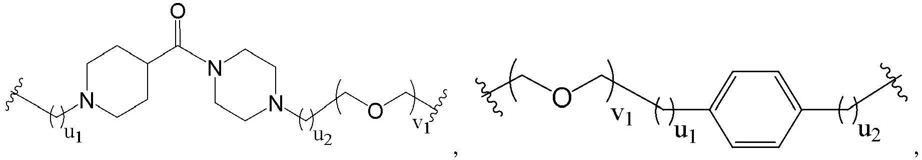 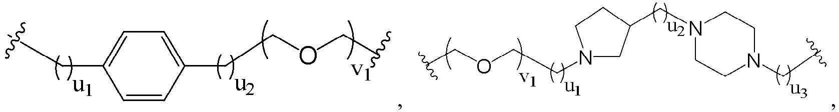 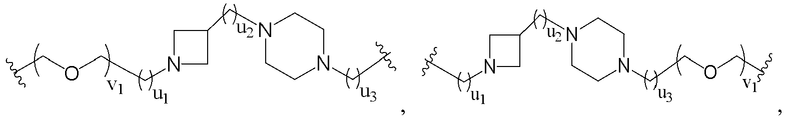 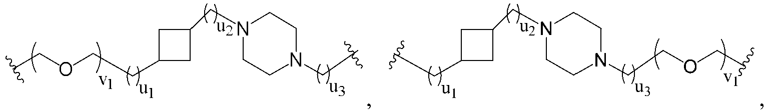 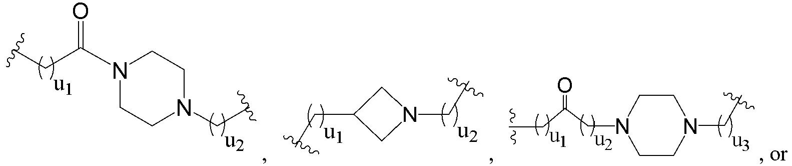 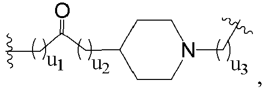 wherein u₁, u₂, u₃ and v₁ are independently selected from the integers from 0 to 10 (i.e., 0, 1, 2, 3, 4, 5, 6, 7, 8, 9, or 10).

In some embodiments, the linker L is selected from the following structures:

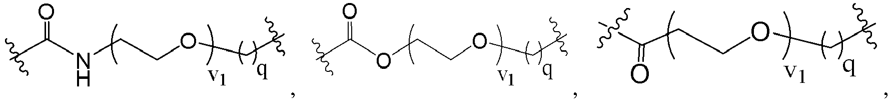

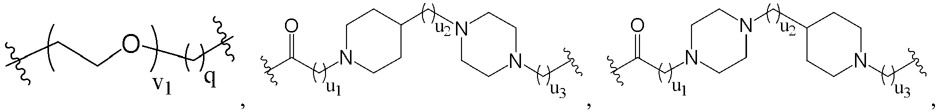

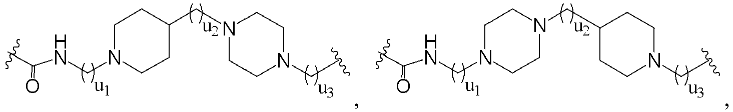

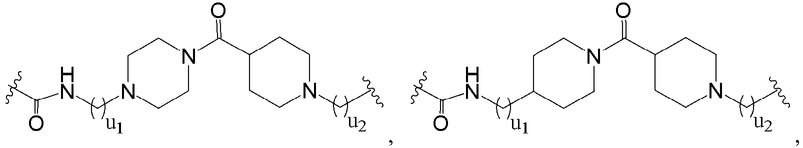

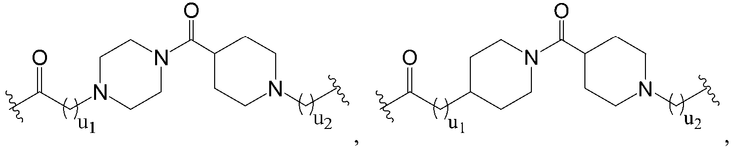

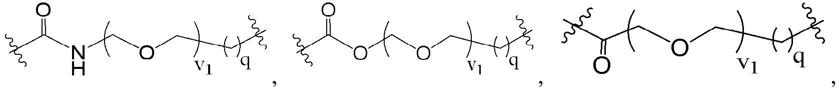

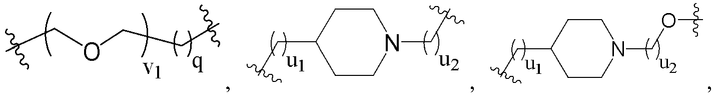

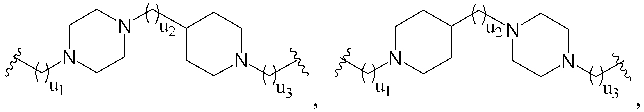

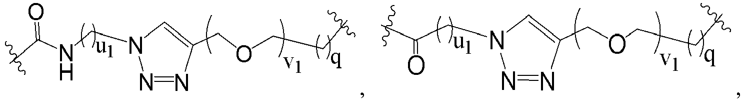

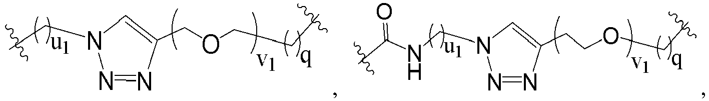

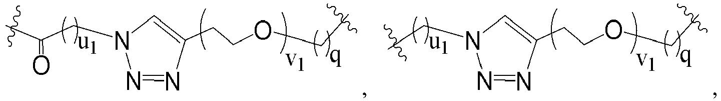

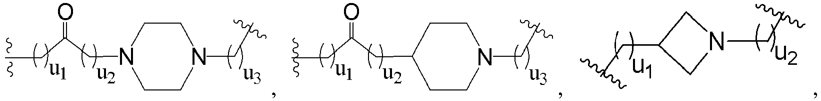

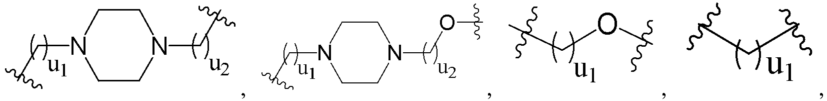

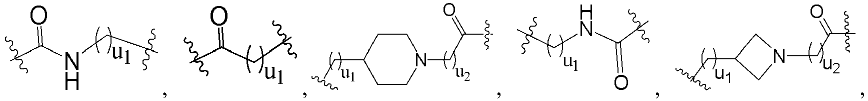

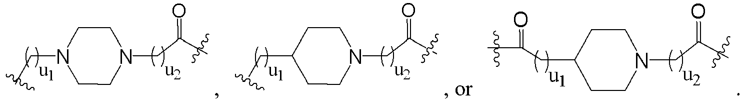

In some embodiments, q, u₁, u₂, u₃ and v₁ are independently selected from the integers from 0 to 10 (i.e., 0, 1, 2, 3, 4, 5, 6, 7, 8, 9, or 10).

In some embodiments, L is absent.

In some embodiments, the linker L is selected from the following structures:

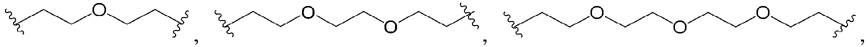

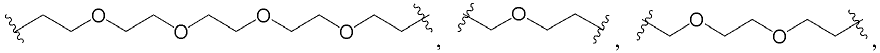

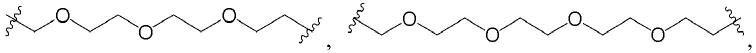

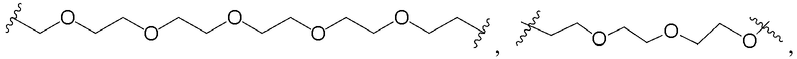

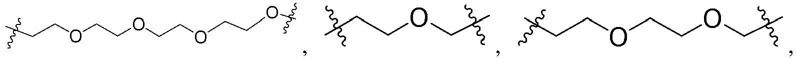

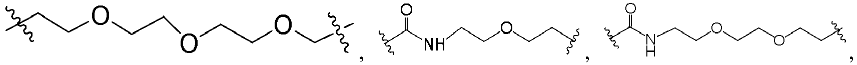

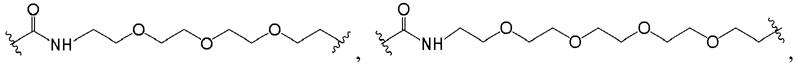

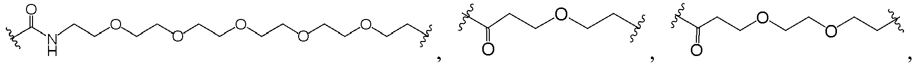

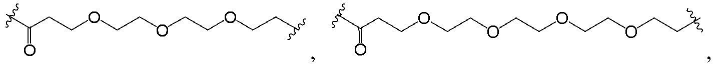

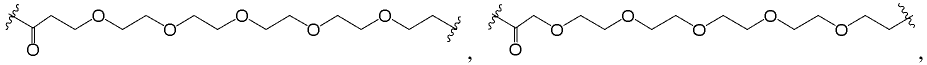

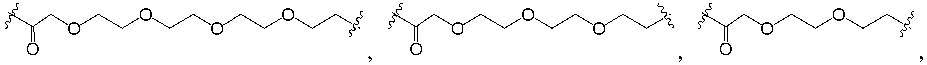

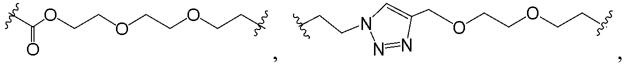

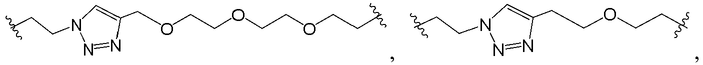

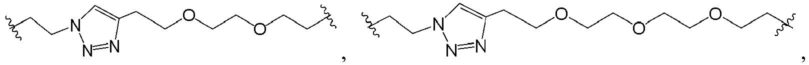

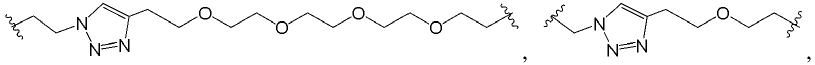

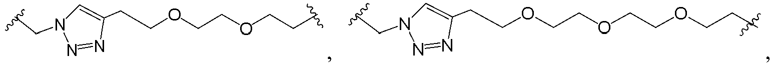

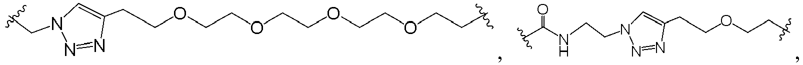

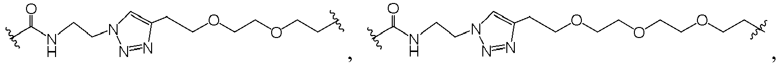

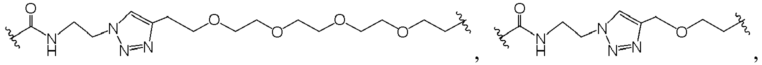

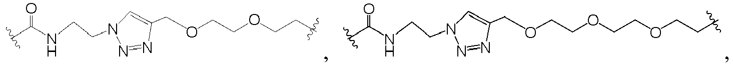

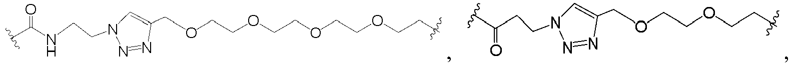

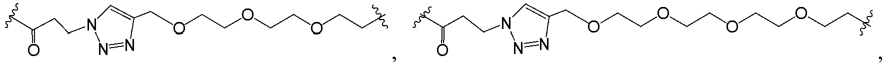

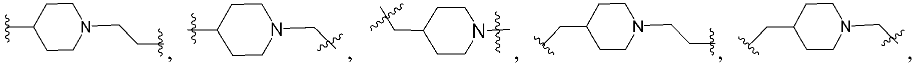

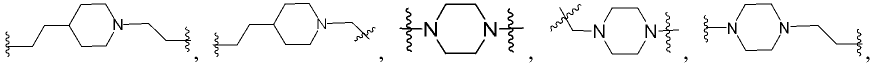

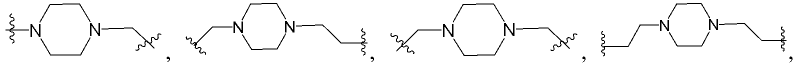

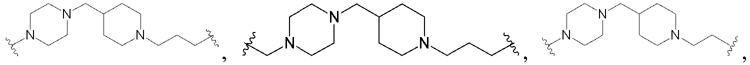

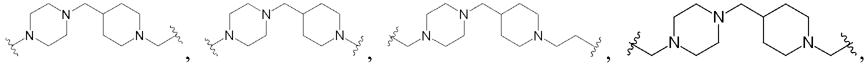

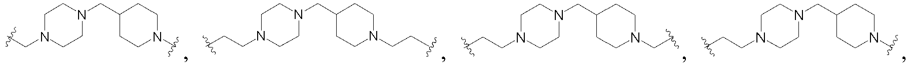

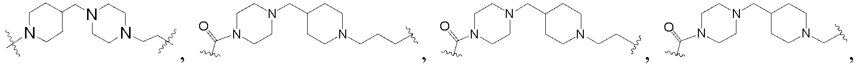

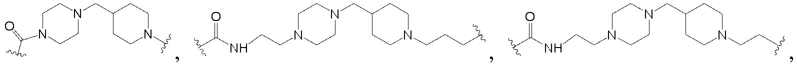

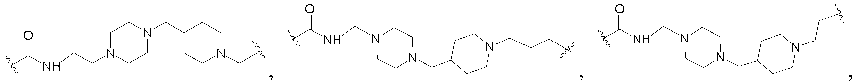

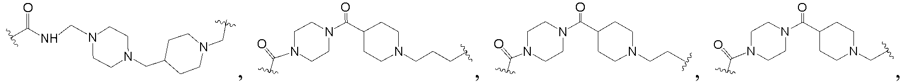

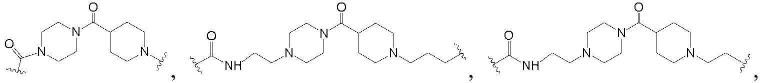

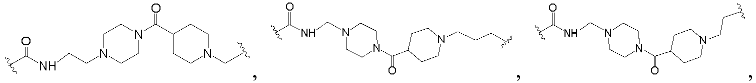

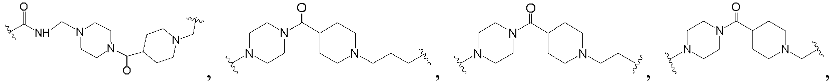

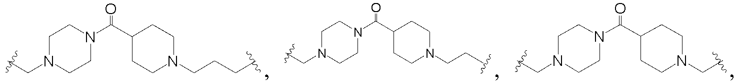

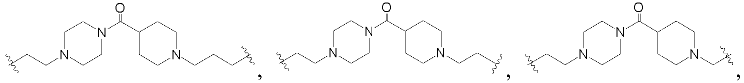

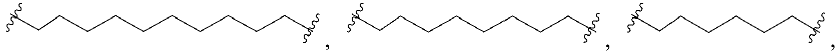

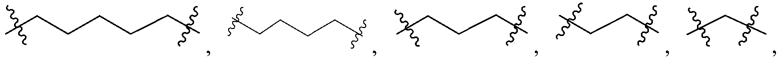

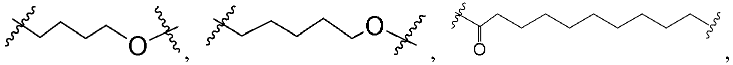

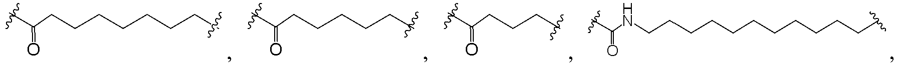

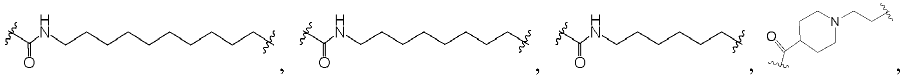

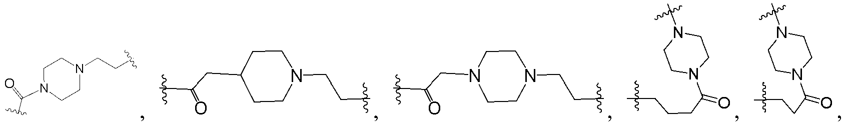

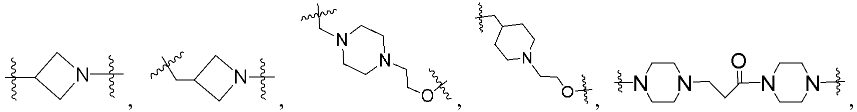

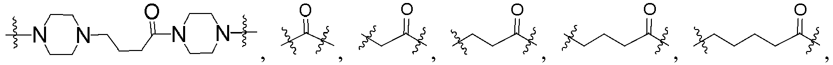

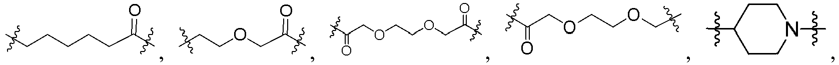

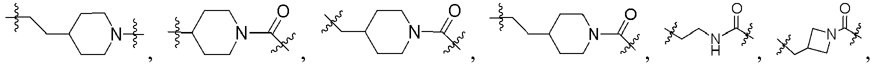

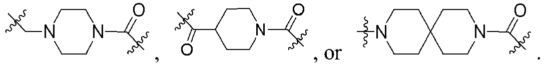

In some embodiments, the linker L is absent or selected from the following structures:

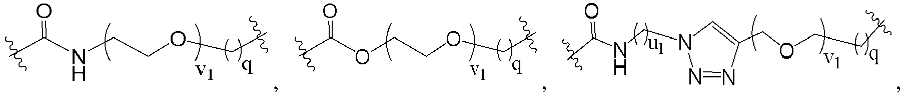

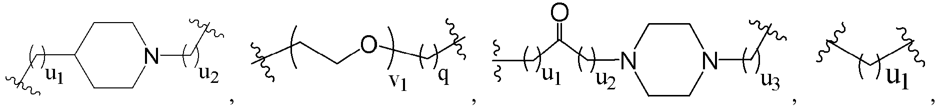

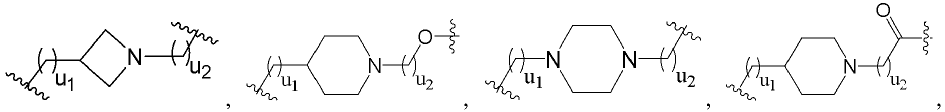

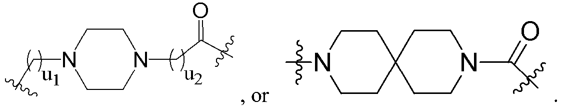

In some embodiments, the linker L is absent or selected from the following structures: 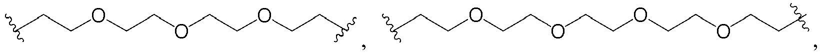 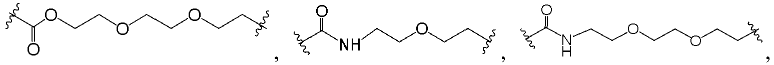 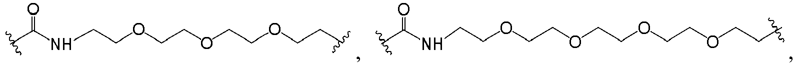 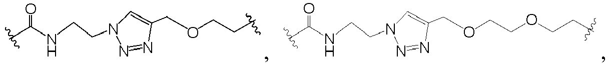 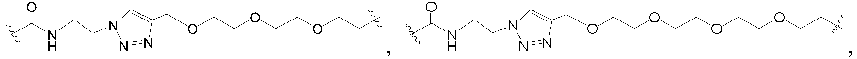 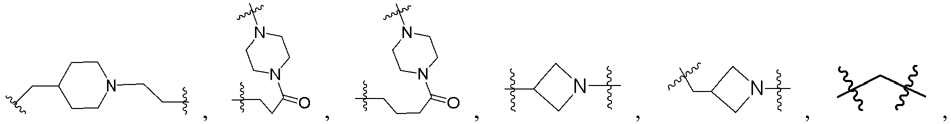 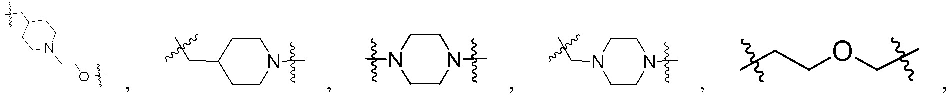 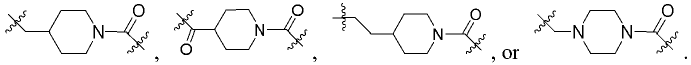 In some embodiments, when the small molecule ligand moiety A targeting the Bcl-xL protein has the structure of formula 1, the linker L is selected from the following structures: 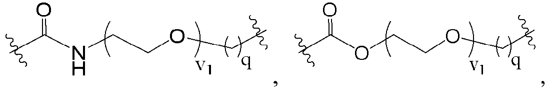 or 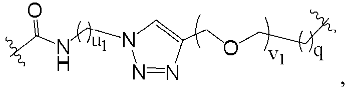 and
the small molecule ligand moiety E targeting the E3 ubiquitin ligase complex is selected from

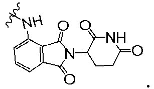

In some embodiments, when the small molecule ligand moiety A targeting the Bcl-xL protein has the structure of formula 2, the linker L is absent or selected from the following structures: 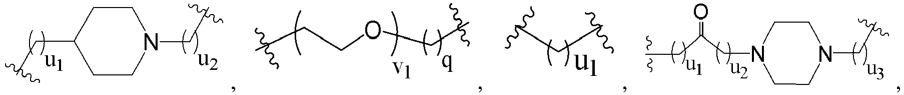 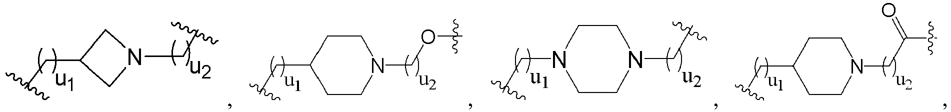 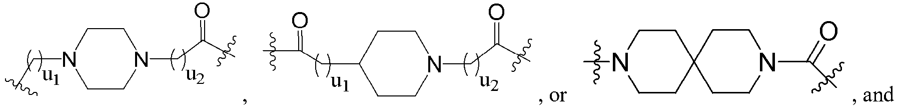 the small molecule ligand moiety E targeting the E3 ubiquitin ligase complex is selected from:

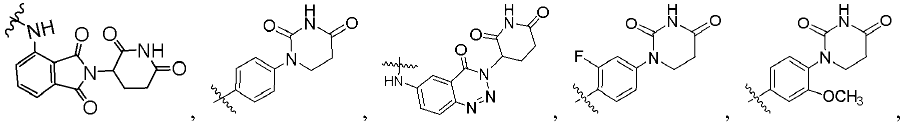

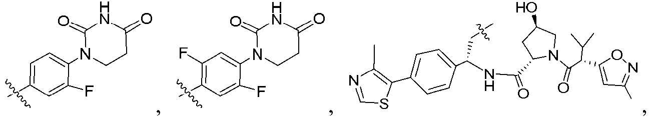

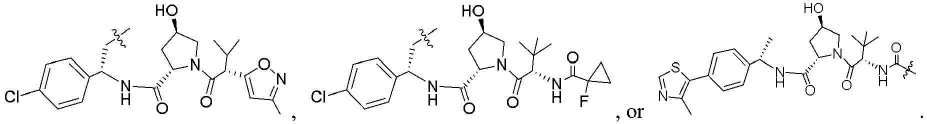

In some embodiments, the PROTAC molecule of Formula I has the following structure: 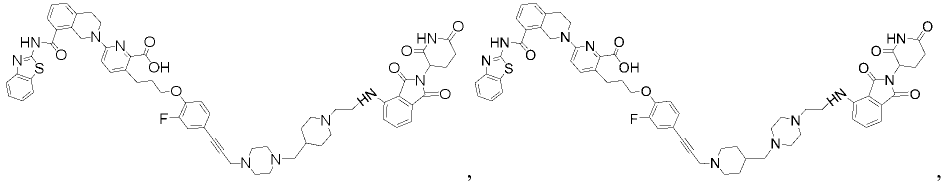 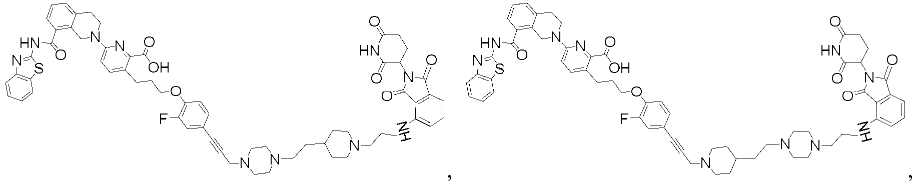 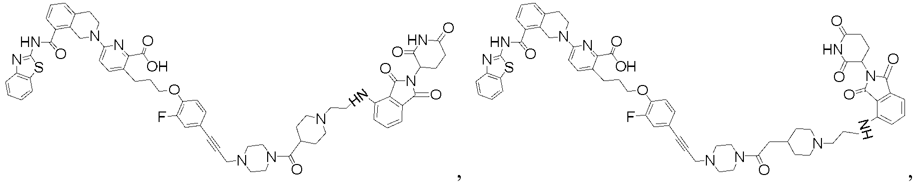 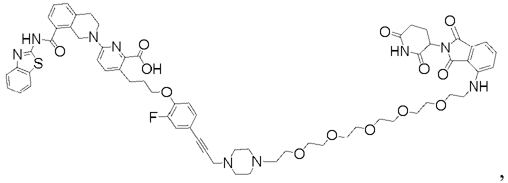 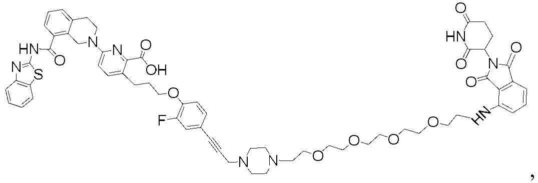 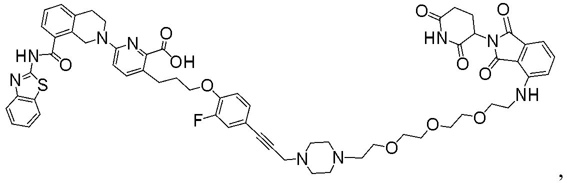 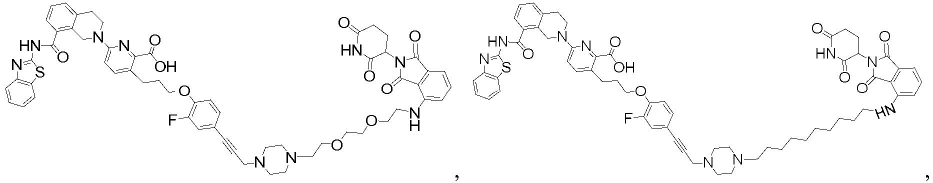 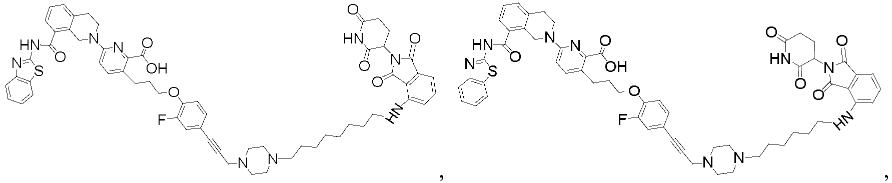 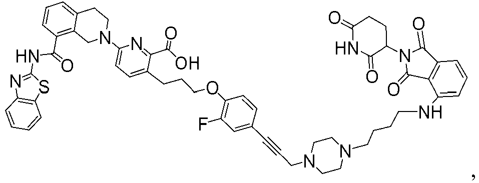 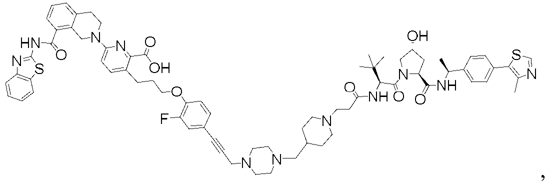 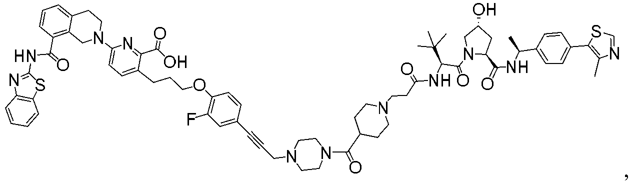 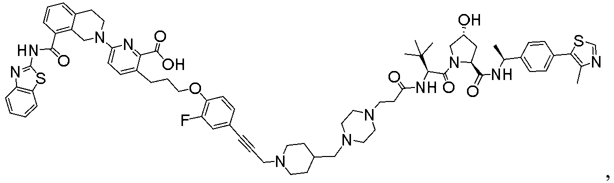 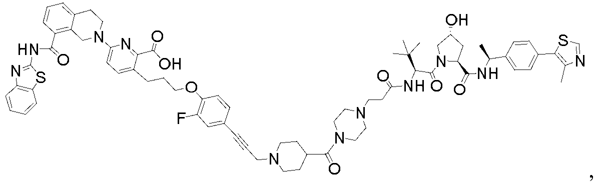 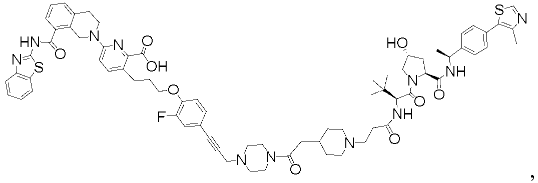 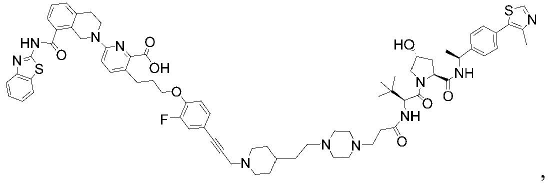 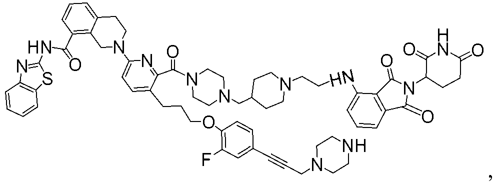 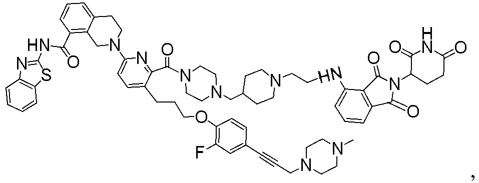 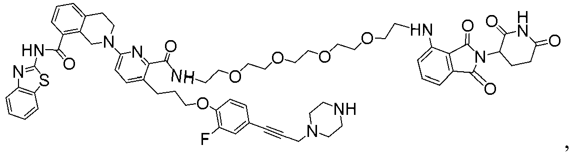 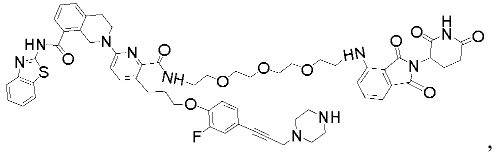 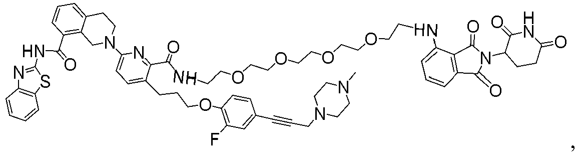 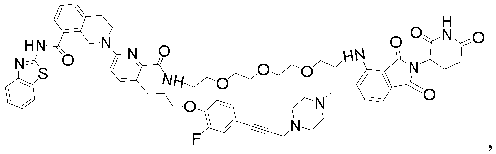 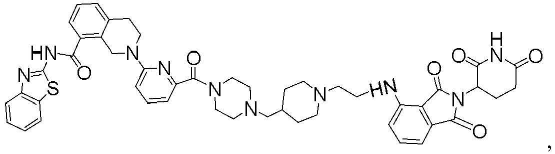 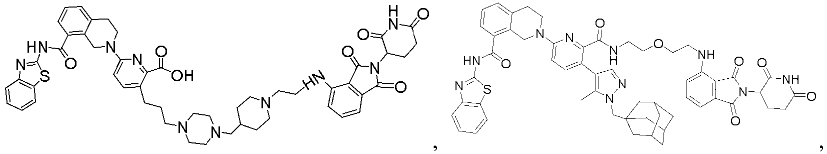 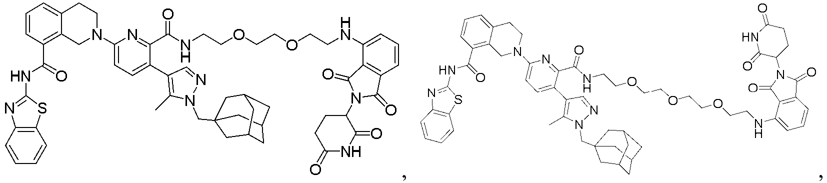 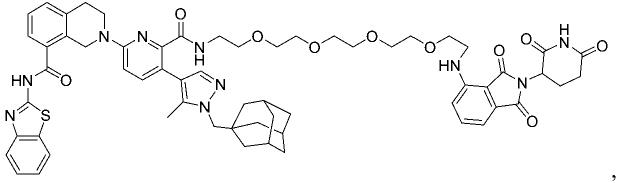 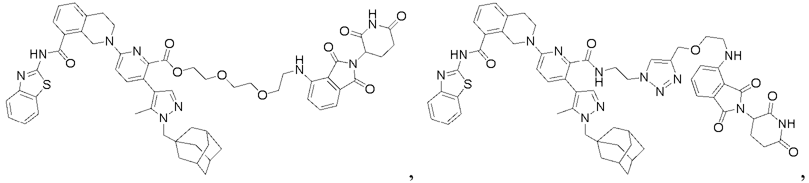 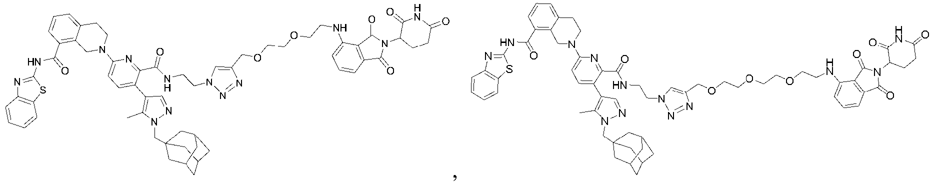 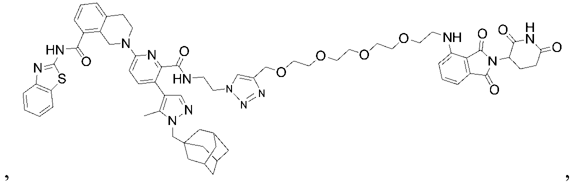 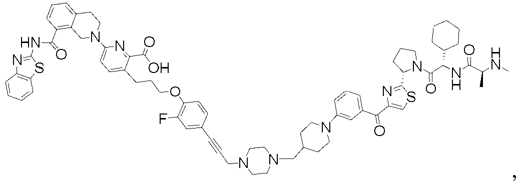 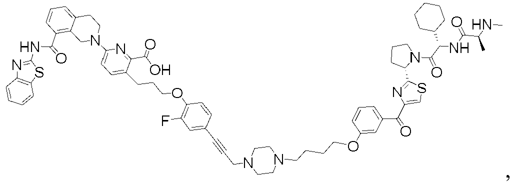 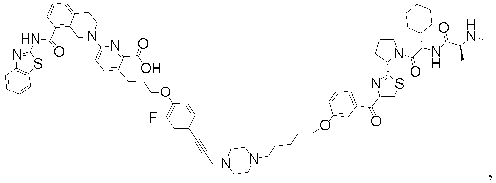 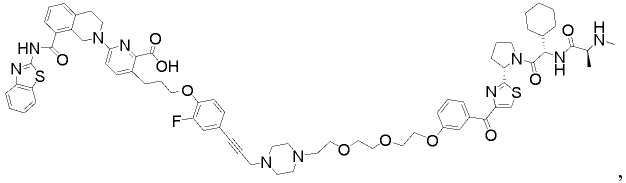 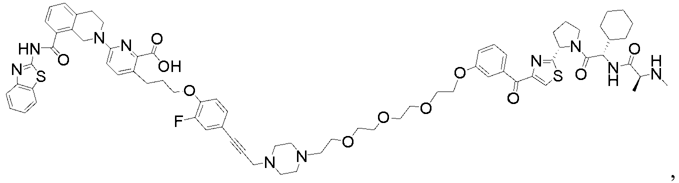 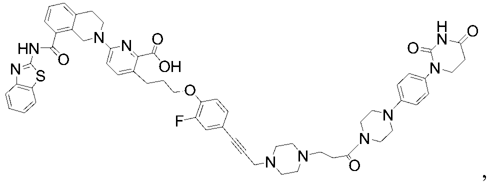 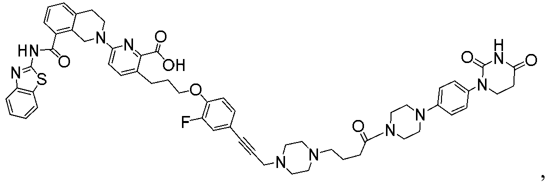 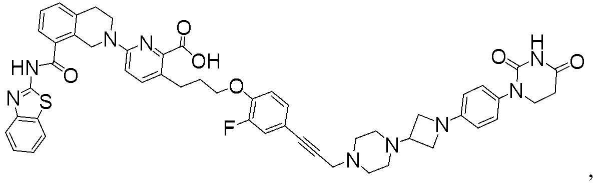 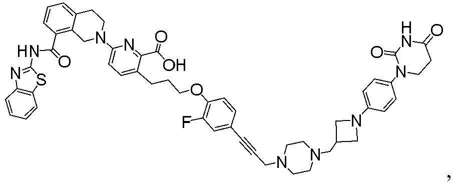 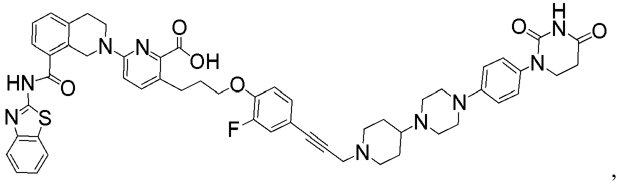 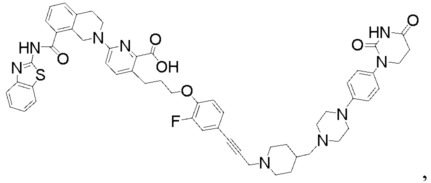 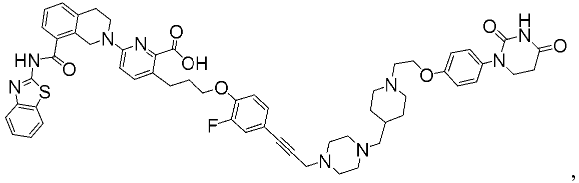 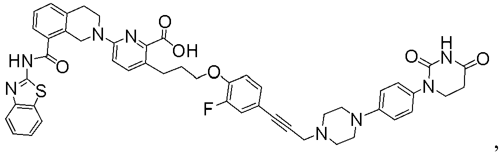 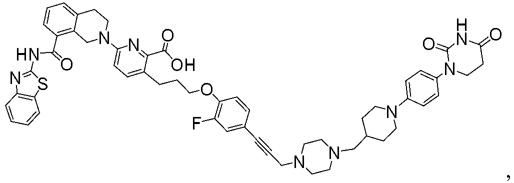 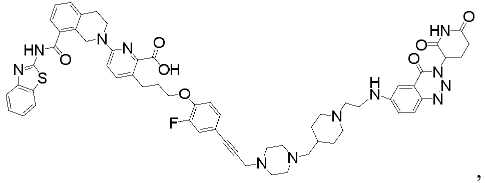 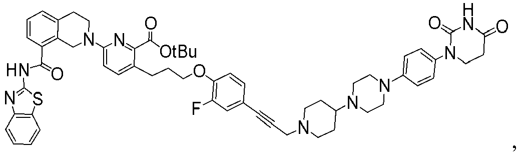 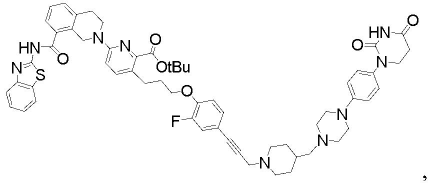 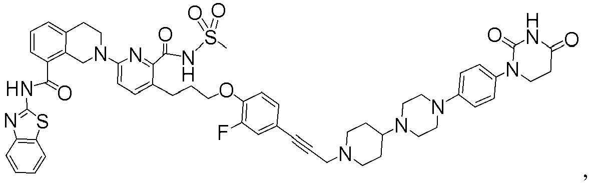 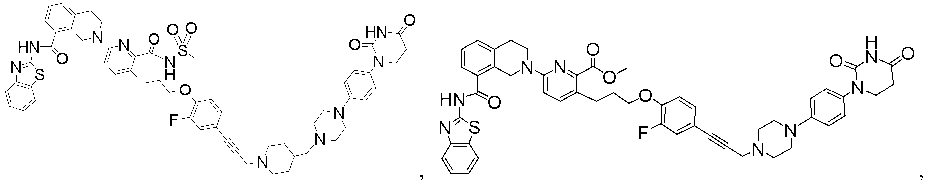 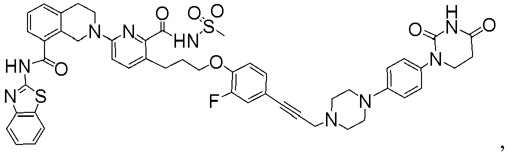 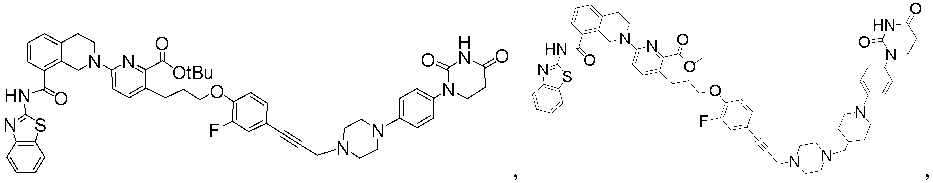 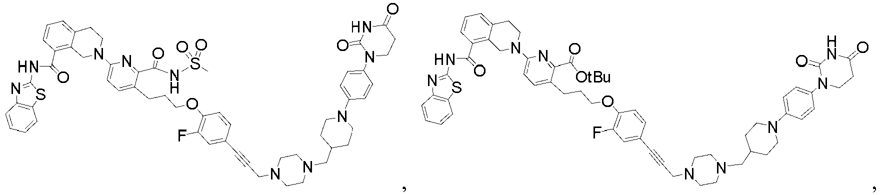 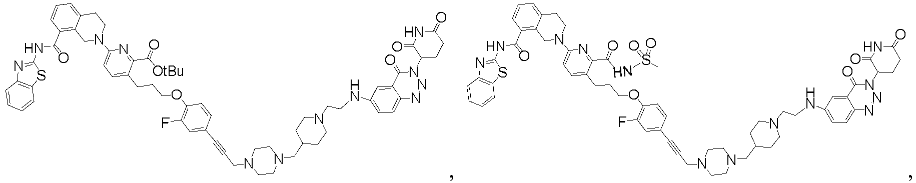 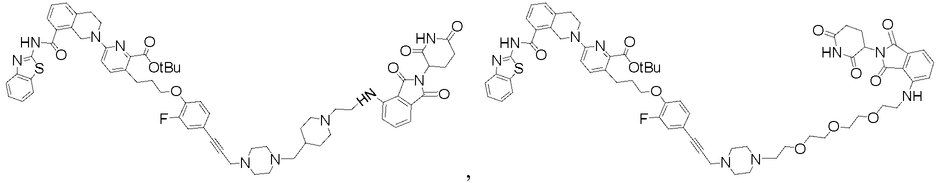 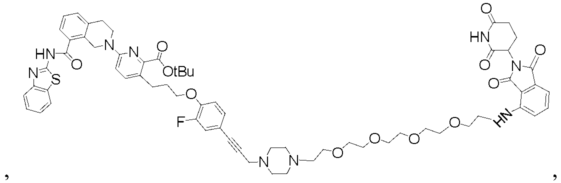 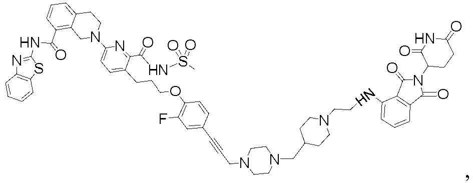 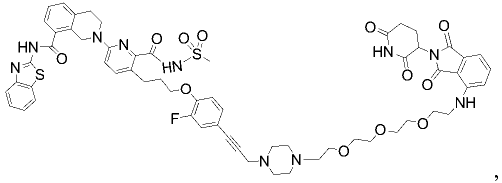 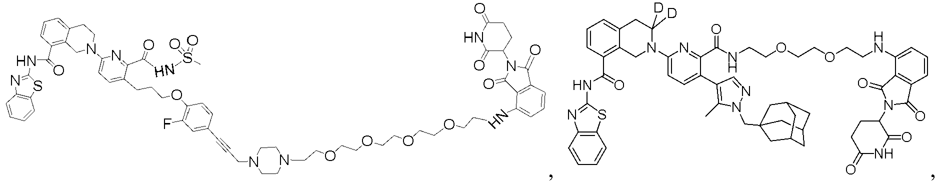 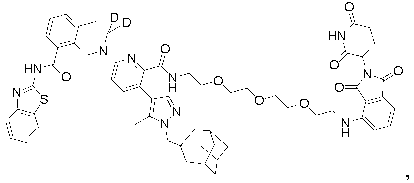 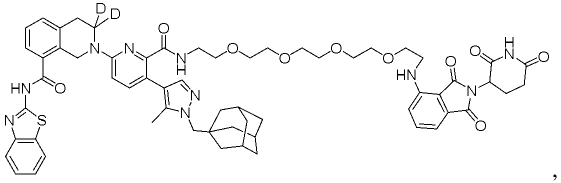 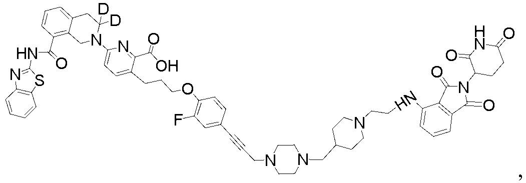 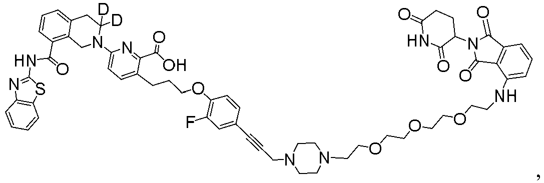 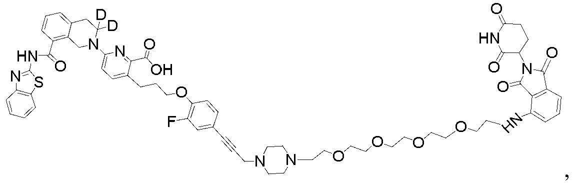 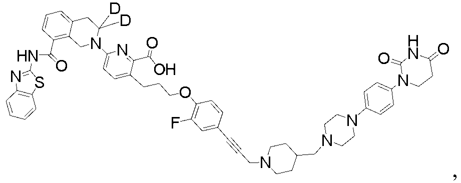 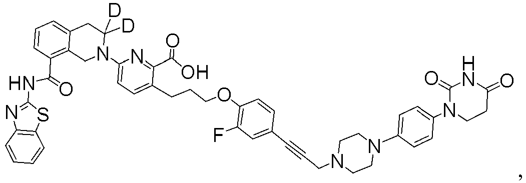 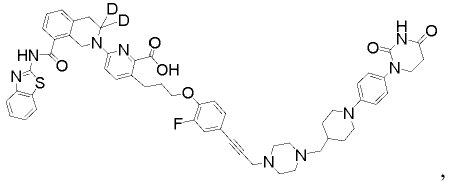 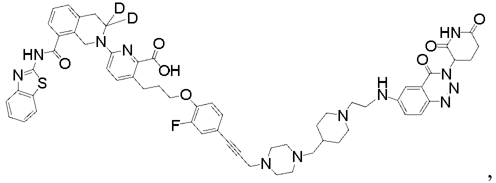 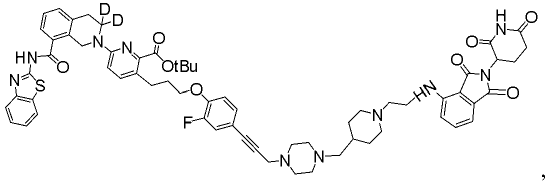 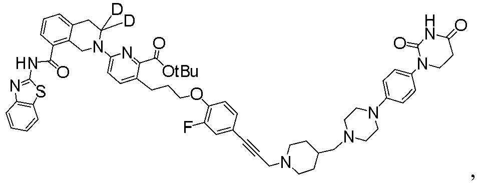 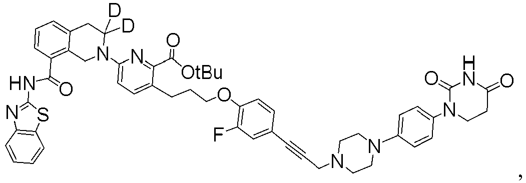 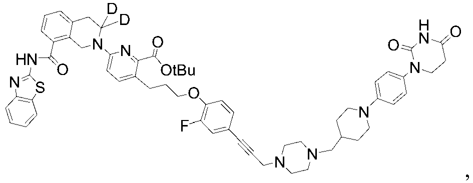 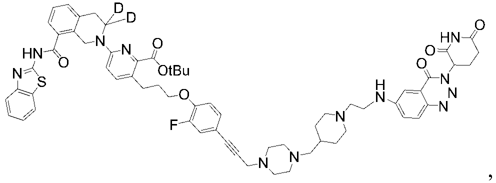 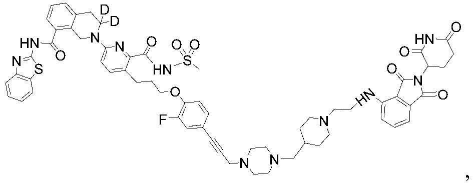 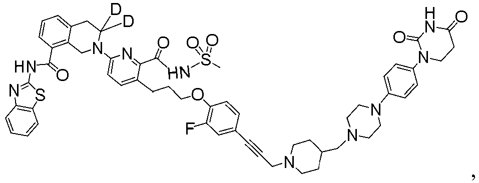 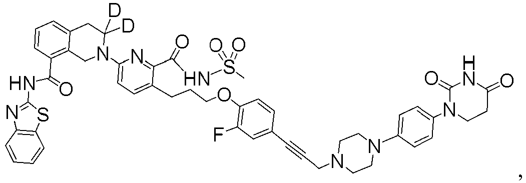 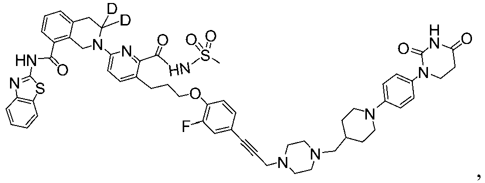 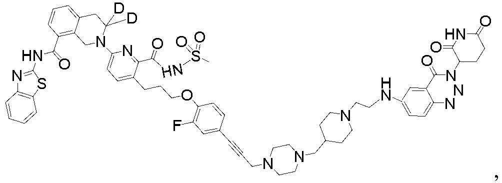 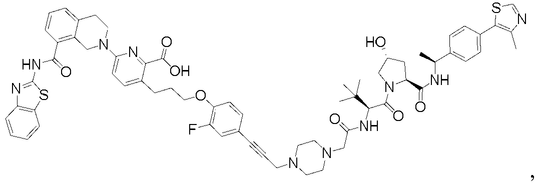 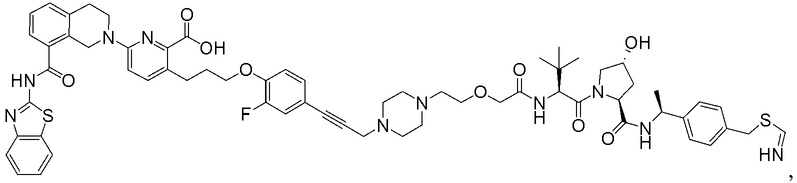 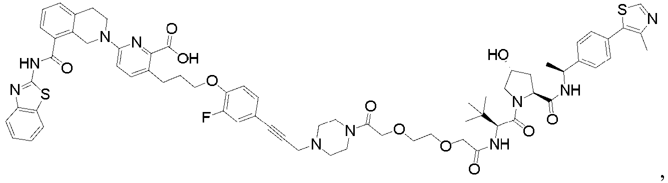 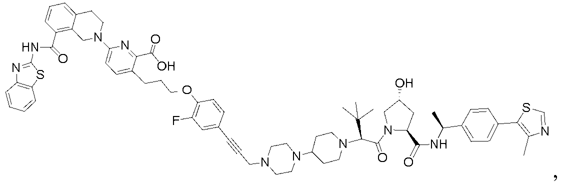 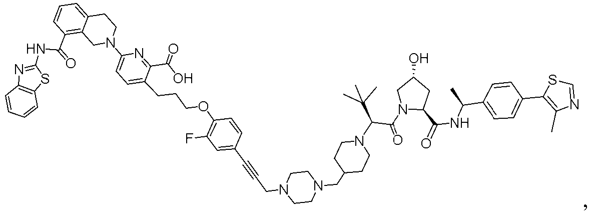 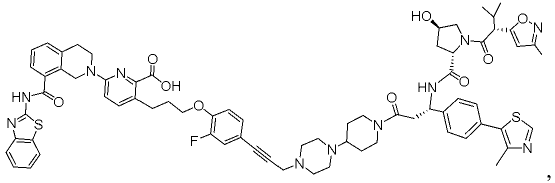 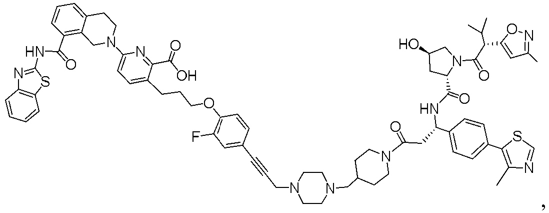 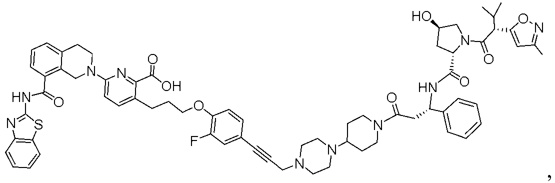 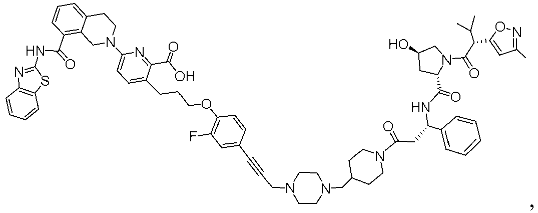 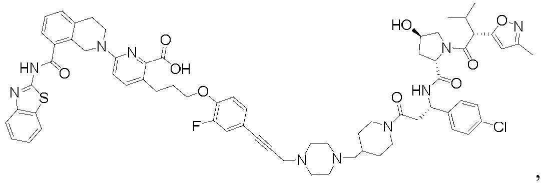 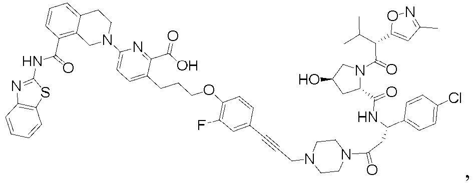 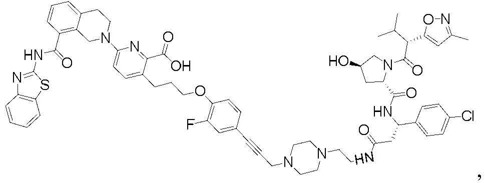 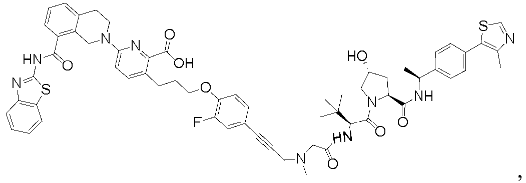 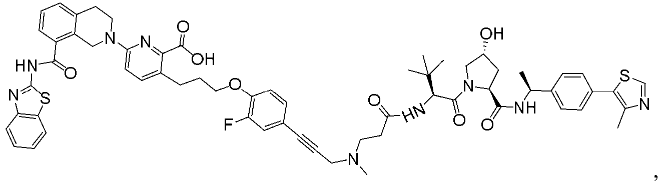 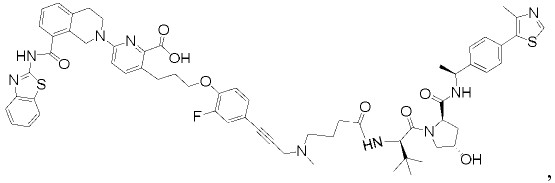 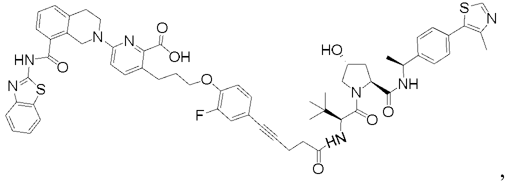 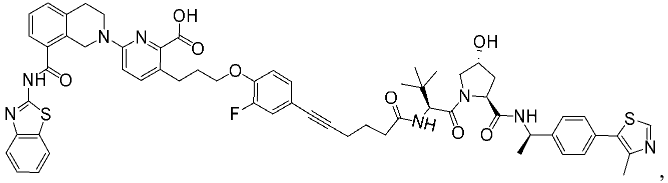 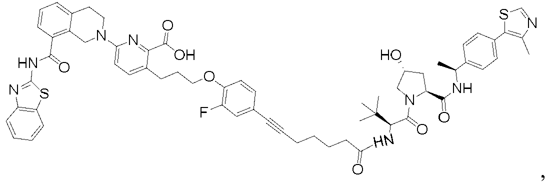 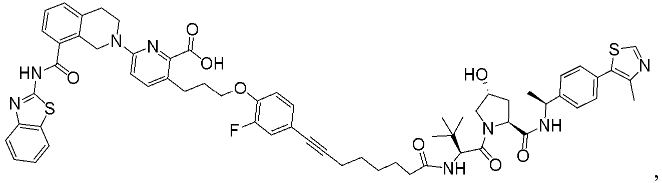 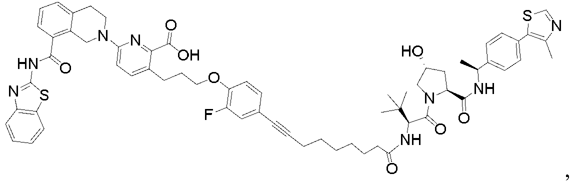 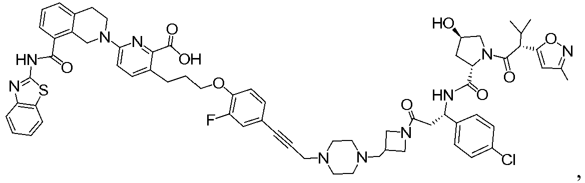 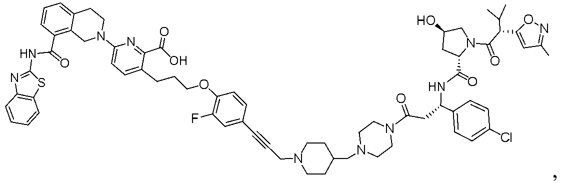 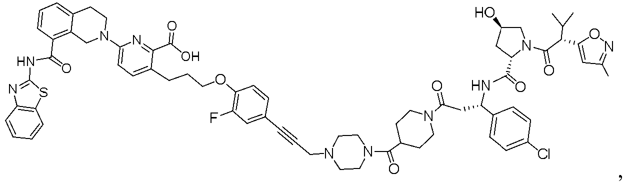 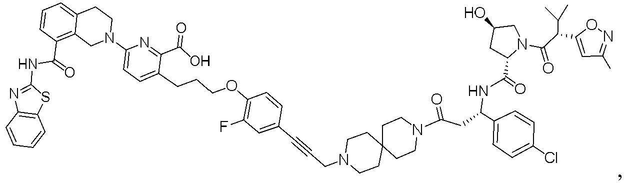 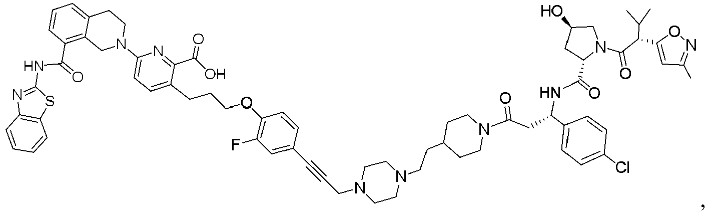 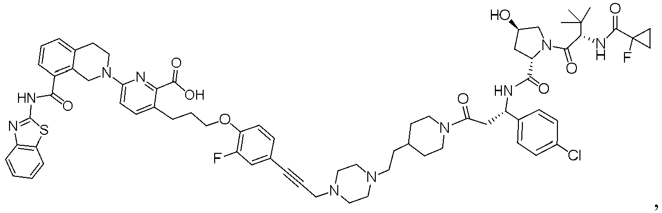 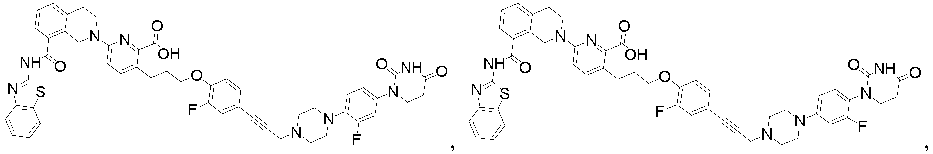 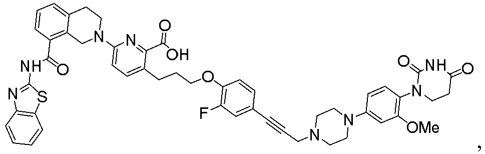 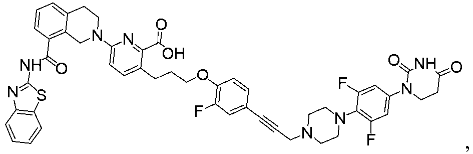 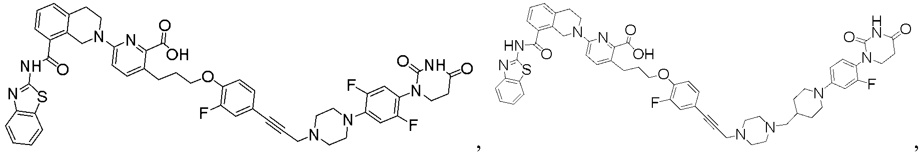 or

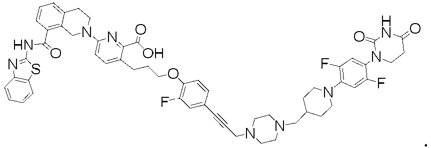

In an aspect, the present invention relates to a composition comprising an effective amount of a compound of formula (I) and a pharmaceutically acceptable carrier.

In an aspect, the present invention relates to a method for selectively killing one or more senescent cells or treating an aging-related disease or disorder in a subject, comprising administering to the subject a therapeutically effective amount of the compound or composition of the invention.

In an aspect, the present invention relates to use of the compound of formula (I) of the invention or the composition of the invention in the manufacture of a medicament for selectively killing one or more senescent cells or treating an aging-related disease or disorder in a subject.

In some embodiments, the aging-related disease or disorder is a metabolic disease, an inflammatory disease or disorder, a pulmonary disease or disorder, a neurological disease or disorder, a proliferative disorder, a renal disorder or disease, an ocular disease or disorder, or a dermatological disorder or disease.

In some embodiments, the aging-related disease or disorder is selected from:
(i) an inflammatory or autoimmune disease or disorder selected from oral mucositis, inflammatory bowel disease, kyphosis, or herniated disc;
(ii) a neurological disease or disorder selected from Alzheimer's disease, Parkinson's disease, Huntington's disease, dementia, mild cognitive impairment, macular degeneration, or motor neuron dysfunction;
(iii) a metabolic disease selected from diabetic ulcer, metabolic syndrome, or obesity;
(iv) a pulmonary disease selected from pulmonary fibrosis, chronic obstructive pulmonary disease, asthma, cystic fibrosis, emphysema, bronchiectasis, or age-related loss of lung function;
(v) an ocular disease or disorder selected from macular degeneration, glaucoma, cataracts, presbyopia, or vision loss;
(vi) an age-related disorder selected from renal failure, frailty, hearing loss, muscle fatigue, skin conditions, skin wound healing, liver fibrosis, pancreatic fibrosis, oral submucous fibrosis, or sarcopenia; and
(vii) a skin disease or disorder selected from eczema, psoriasis, hyperpigmentation, moles, rashes, atopic dermatitis, urticaria, diseases and disorders associated with photosensitivity or photoaging, wrinkles, pruritus, hypoesthesia, eczematous rashes, eosinophilic dermatoses, reactive neutrophilic dermatoses, pemphigus, pemphigoid, immunobullous skin diseases, cutaneous fibrohistiocytosis, cutaneous lymphoma, or cutaneous lupus.

In some embodiments, the subject has been diagnosed with cancer, and optionally is undergoing a cancer treatment selected from chemotherapy or radiotherapy.

In some embodiments, the compounds of formula (I) of the invention or the compositions of the invention
(i) reduces cells that have been pushed towards senescence or cancer cells;
(ii) reduces one or more side effects produced by senescent cells, wherein the side effects include inflammation, promotion of cancer growth, or promotion of metastasis;
(iii) reduces one or more side effects of chemotherapy; or
(iv) reduces one or more side effects of radiotherapy.

In some embodiments, the subject has a viral infection, optionally wherein the viral infection causes the overproduction of activated macrophages in the subject.

In some embodiments, the viral infection is a coronavirus infection caused by a virus selected from coronavirus, severe acute respiratory syndrome (SARS) coronavirus (CoV), SARS-CoV-2 (which causes COVID-19 (Coronavirus Disease 2019)), or Middle East respiratory syndrome (MERS)-CoV

In some embodiments, the subject has a SARS-CoV-2 infection and has been diagnosed with Coronavirus Disease 2019.

In some embodiments, a compound having the following structure is obtained in an effective amount for killing one or more senescent cells after in vivo administrating the compound or composition of the invention: 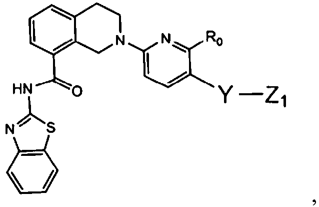 wherein R₀, Y and Z₁ are as defined herein.

### Brief Description of Drawings

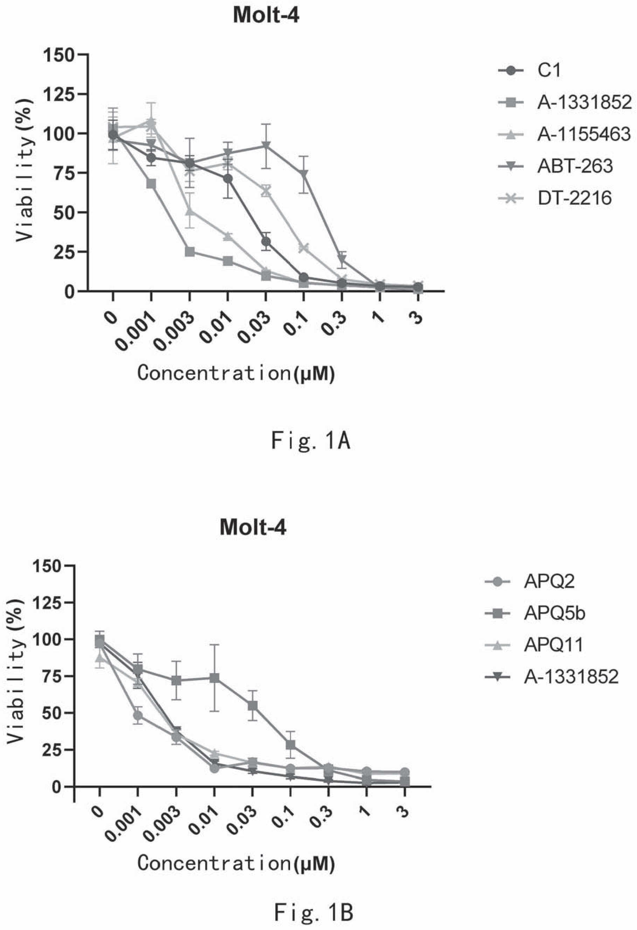

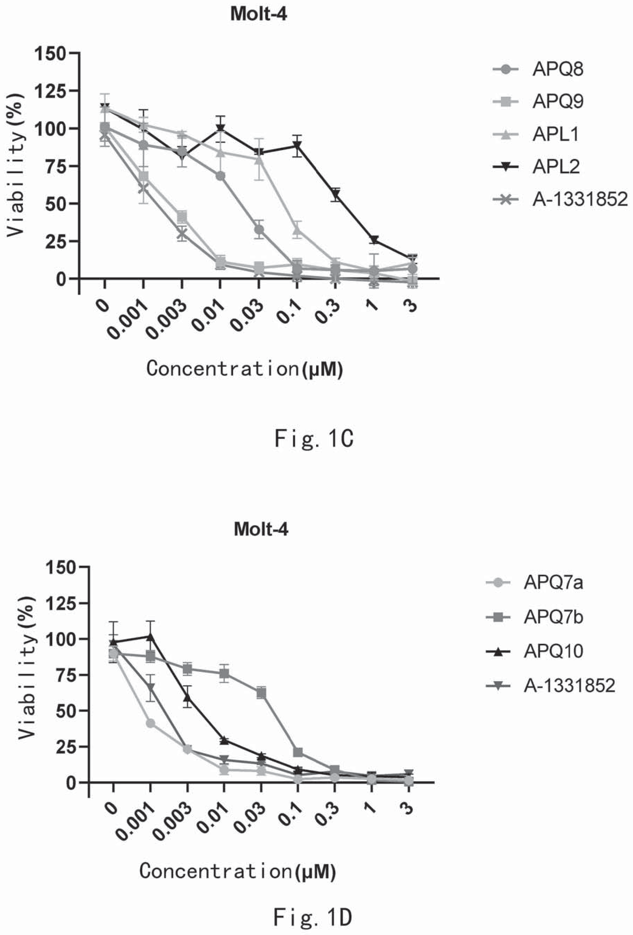

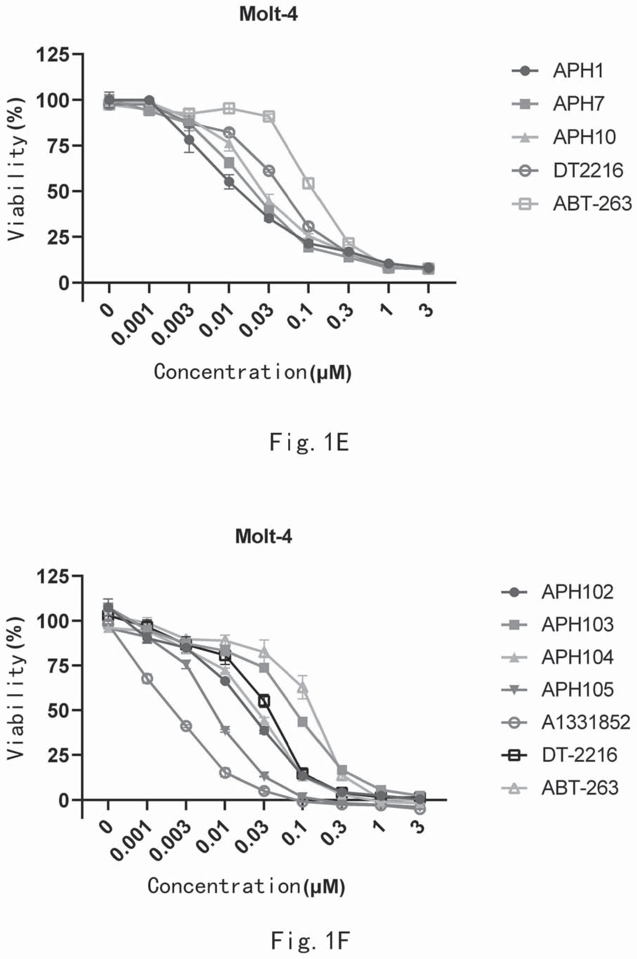

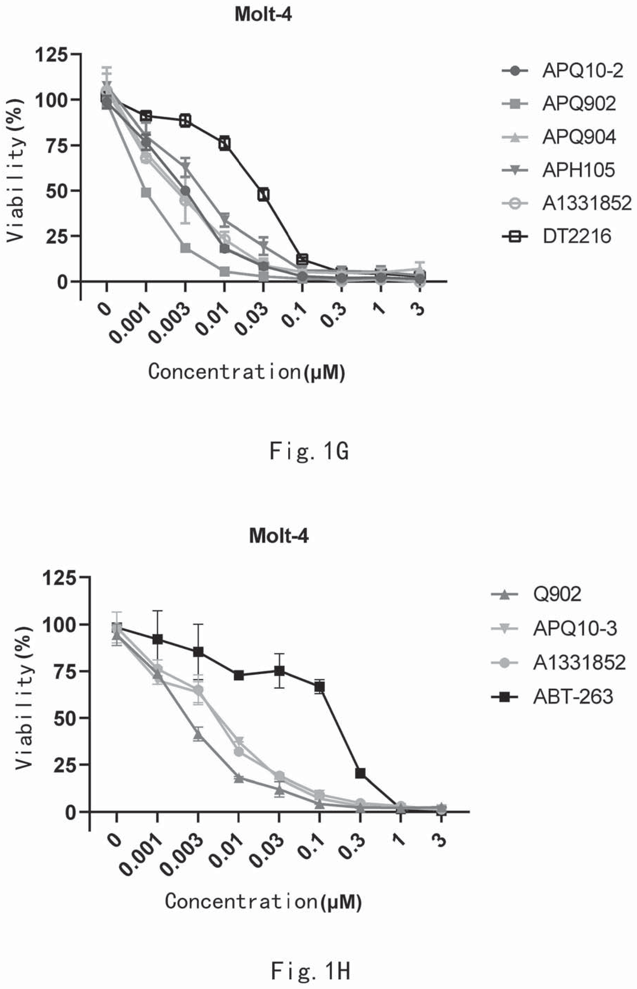

Figure 1 are graphs showing the tested compounds at different concentrations on killing Molt-4 cells. Figure 1A are graphs showing Compound C1 and other known compounds at different drug concentrations on killing Molt-4 cells. Figure 1B are graphs showing the PROTAC molecules APQ2, APQ5b, and APQ11 at different drug concentrations on killing Molt-4 cells. Figure 1C are graphs showing the PROTAC molecules APQ8, APQ9, APL-1 and APL-2 at different drug concentrations on killing Molt-4 cells. Figure 1D are graphs showing the PROTAC molecules APQ7a, APQ7b and APQ10 at different drug concentrations on killing Molt-4 cells. Figure 1E are graphs showing the PROTAC molecules APH1, APH7 and APH10 as well as other known compounds at different drug concentrations on killing Molt-4 cells. Figure 1F are graphs showing the PROTAC molecules APH102, APH103, APH104 and APH105 as well as other known compounds at different drug concentrations on killing Molt-4 cells. Figure 1G are graphs showing the PROTAC molecules APQ10-2, APQ902, APQ904 and APH105 as well as other known compounds at different drug concentrations on killing Molt-4 cells. Figure 1H are graphs showing the PROTAC molecules APQ10-3 and APQ902 as well as other known compounds at different drug concentrations on killing Molt-4 cells.

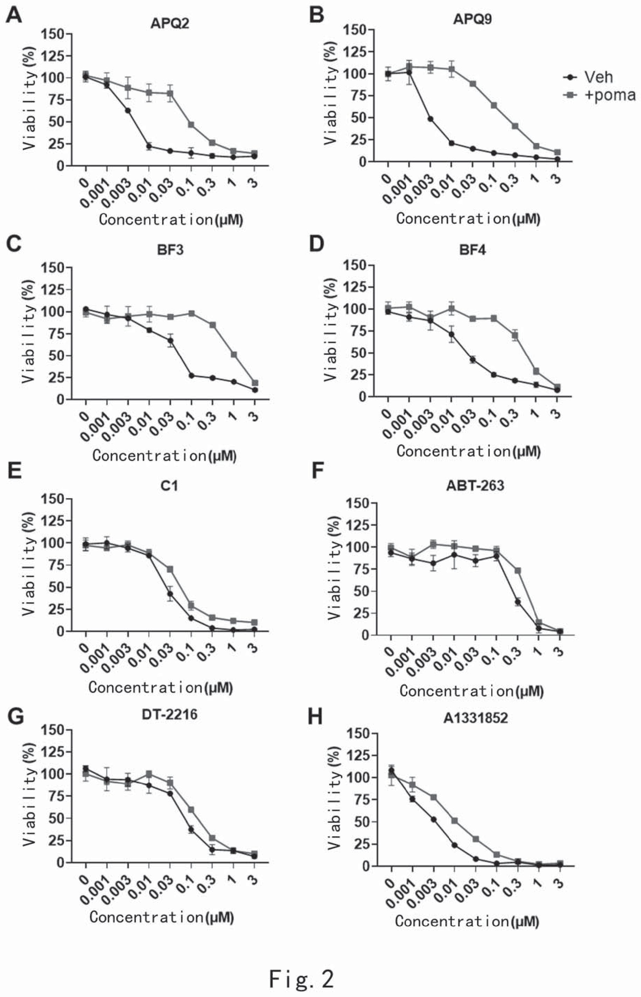

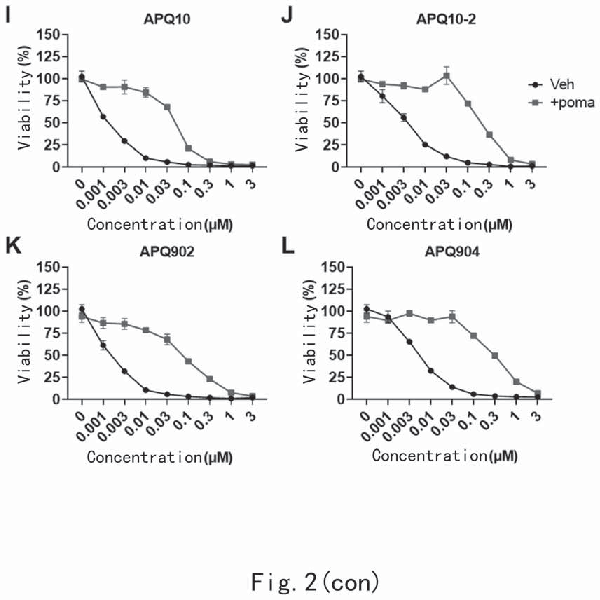

Figure 2 shows the results of the tested compounds at different concentrations on killing Molt-4 cells in the presence or absence of pomalidomide. Figures 2A, 2B, 2C, and 2D are graphs showing the CRBN-E3 enzyme-based PROTAC molecules APQ2, APQ9, BF3, and BF4 at different concentrations on killing Molt-4 cells respectively. Figures 2E, 2F, 2G, and 2H are graphs showing Compound Cl, control compound ABT-263, known VHL-E3 enzyme-based control PROTAC molecule DT-2216, and known Compound A1331852 at different concentrations on killing Molt-4 cells respectively. Figures 2I, 2J, 2K, and 2L are graphs showing the CRBN-E3 enzyme-based PROTAC molecules APQ10, APQ10-2, APQ902 and APQ904 at different concentrations on killing Molt-4 cells respectively. Veh: blank control group; + Poma: pomalidomide group.

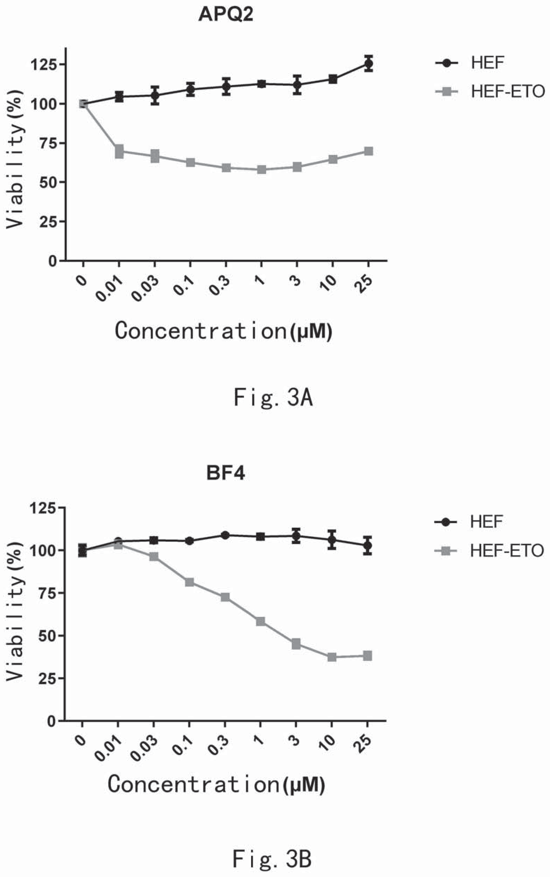

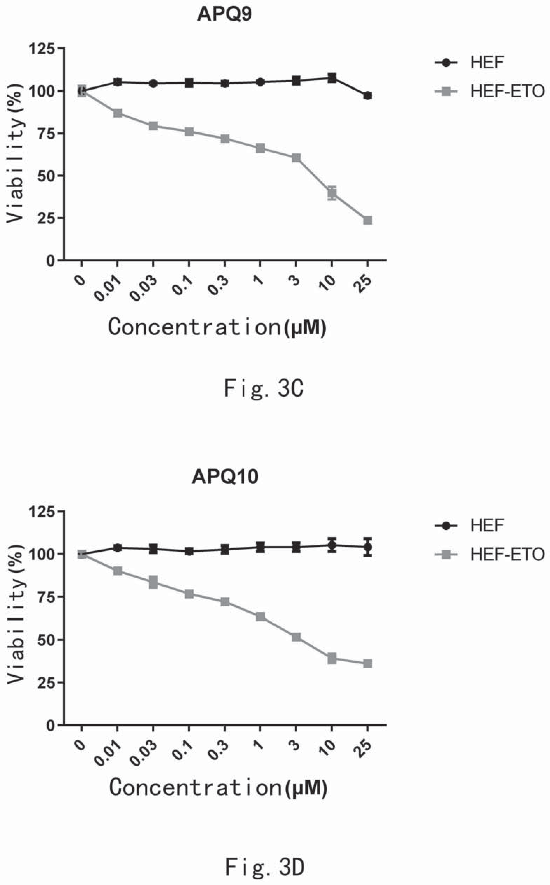

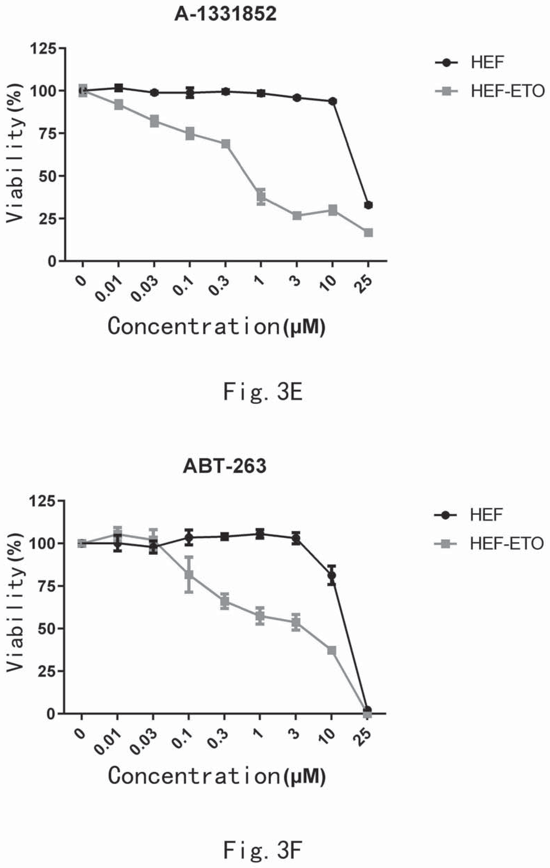

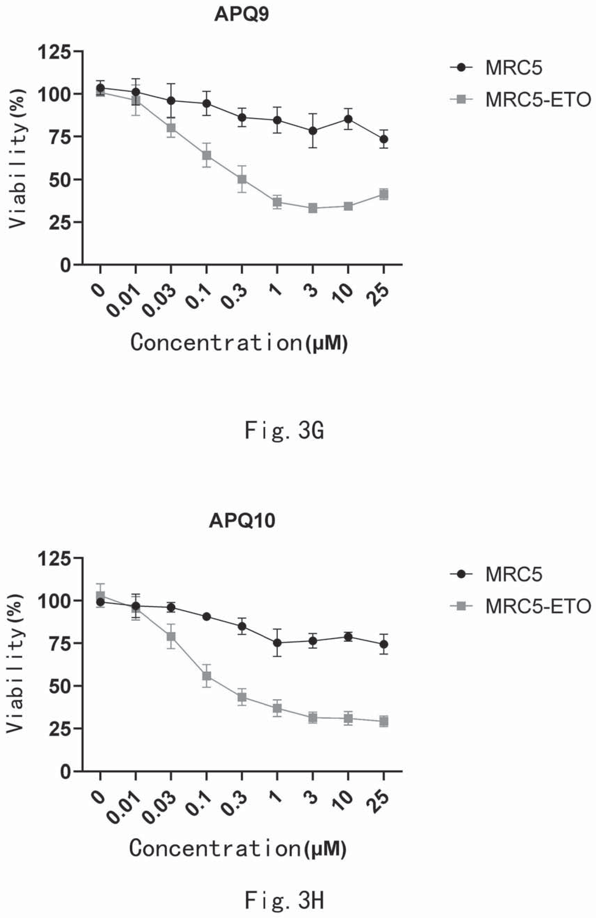

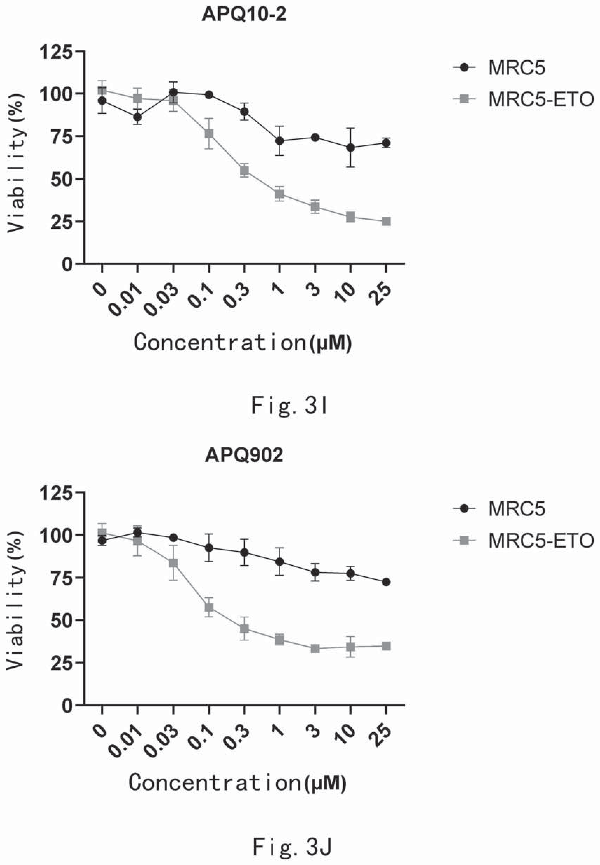

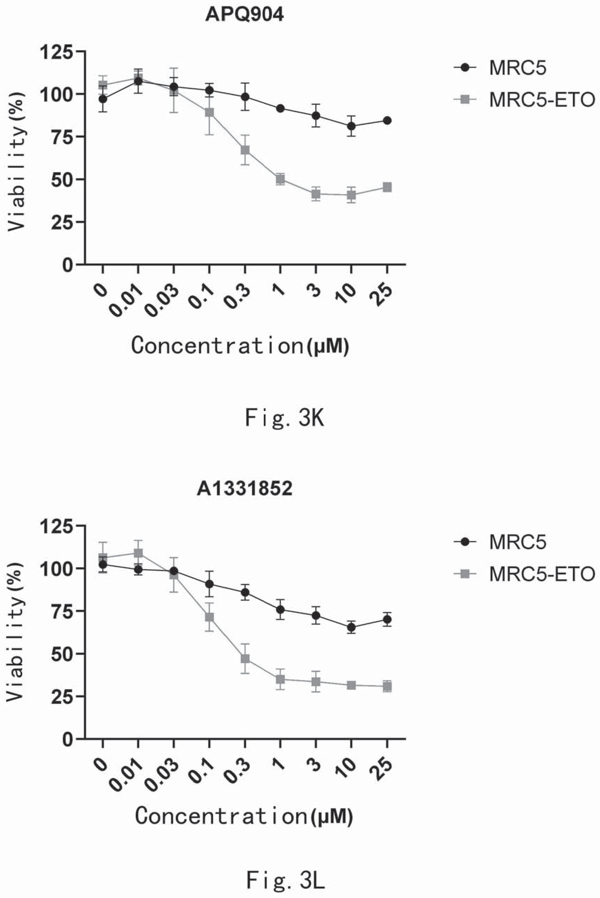

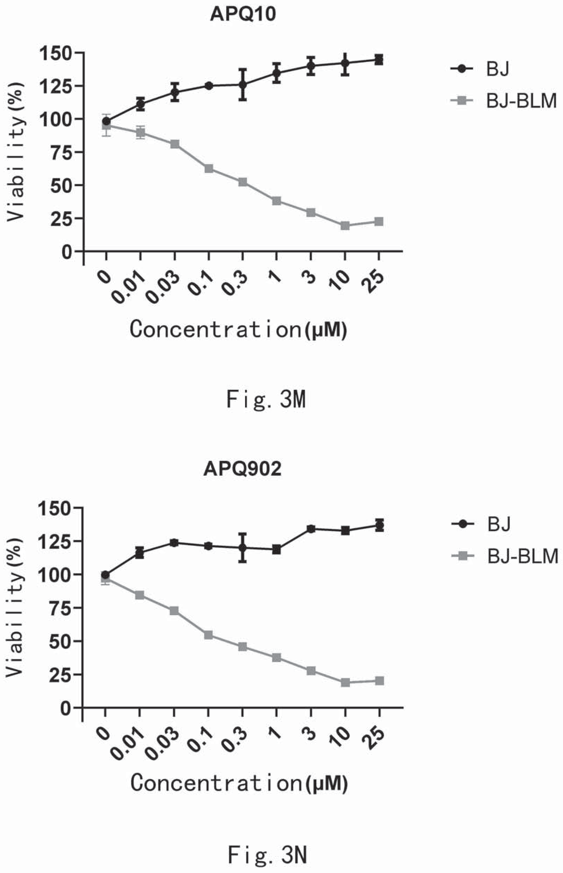

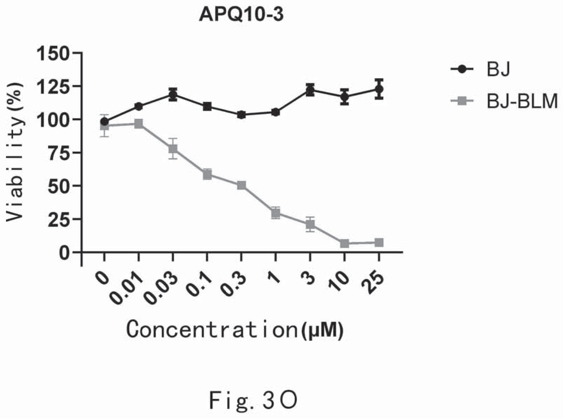

Figure 3 shows the results of the tested compounds at different concentrations on killing human embryonic fibroblasts (HEF cells), etoposide-induced senescent human HEF cells, human embryonic lung fibroblasts MRC5, etoposide-induced senescent human embryonic lung fibroblasts MRC5, human skin-derived BJ cells, and bleomycin-induced senescent human skin-derived BJ cells. Figures 3A, 3B, 3C and 3D are graphs corresponding to the PROTAC molecules APQ2, BF4, APQ9, and APQ10 for killing in a HEF cell model respectively. Figures 3E and 3F are graphs corresponding to the commercially available control molecules A-1331852 and ABT-263 for killing in a HEF cell model respectively. Figures 3G, 3H, 3I, 3J, 3K, and 3L are graphs corresponding to the PROTAC molecules APQ9, APQ10, APQ10-2, APQ902, APQ904, and the commercially available control molecule A-1331852 for killing in a MRC5 cell model respectively. Figures 3M, 3N, and 3O are graphs corresponding to the PROTAC molecules APQ10, APQ902, and APQ10-3 in a BJ cell model respectively. HEF: normally cultured HEF cell group; HEF-ETO: etoposide-induced senescent HEF cell group. MRC5: normally cultured MRC5 cell group; MRC5-ETO: etoposide-induced senescent MRC5 cell group. BJ: normally cultured BJ cell group; BJ-BLM: bleomycin-induced senescent BJ cell group.

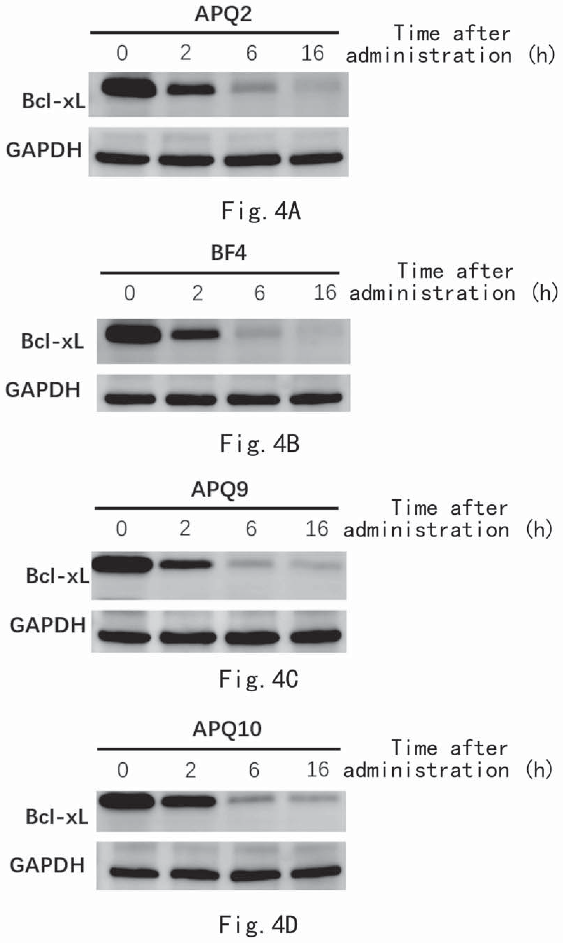

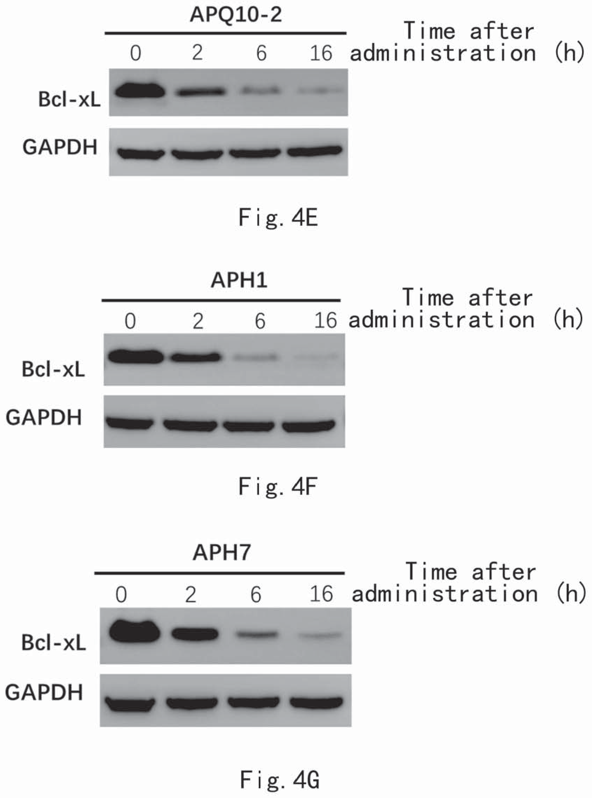

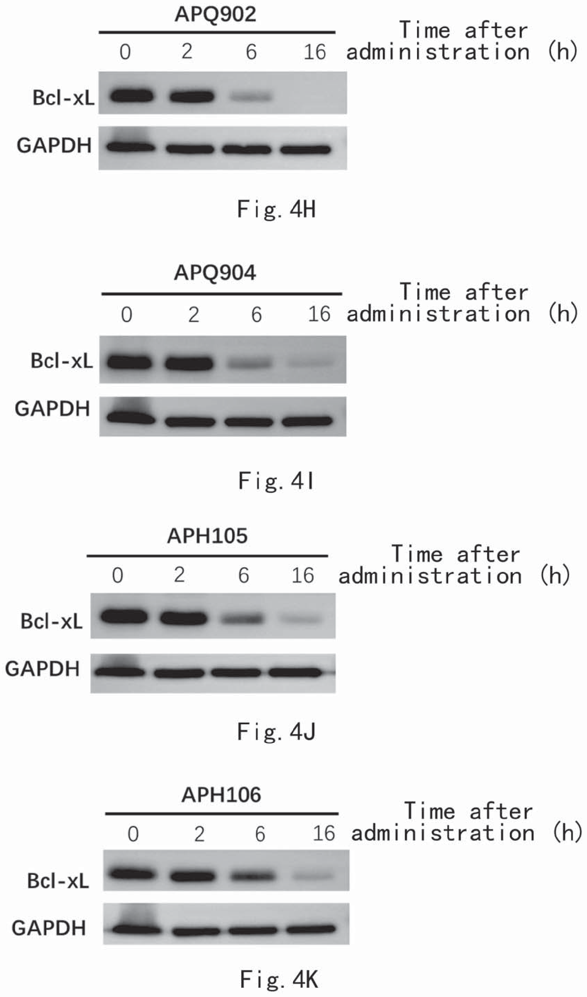

Figure 4 shows the results of the tested compounds on degrading the target protein Bcl-xL. Figures 4A, 4B, 4C, 4D, 4E, 4F, 4G, 4H, 4I, 4J, and 4K show the results of the PROTAC molecules APQ2, BF4, APQ9, APQ10, APQ10-2, APH1, APH7, APQ902, APQ904, APH105, and APH106 on degrading the Bcl-xL protein at various time points after administration respectively. The concentration for all PROTAC molecules administered in the test was 100 nM, and the detection time points were 0 h, 2 h, 6 h, and 16 h. Glyceraldehyde-3-phosphate dehydrogenase (GAPDH) was used as the internal control protein.

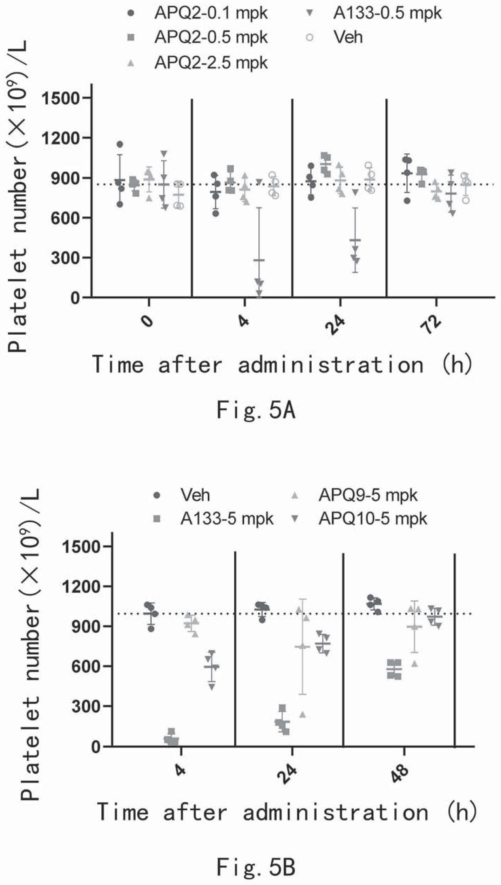

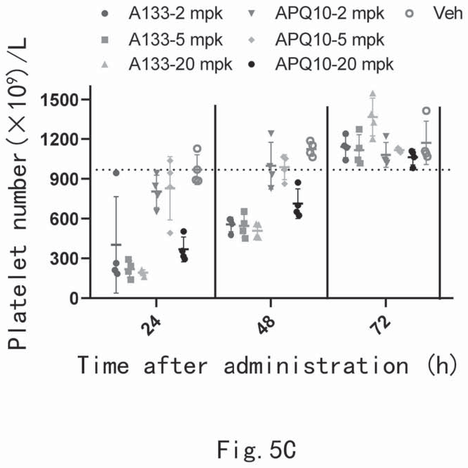

Figure 5 shows the results of platelet toxicity of the tested compounds administered in vivo. Figure 5A shows the results comparing the effects of the PROTAC molecule APQ2 administered at different doses with the control molecule A-1331852 on the number of platelets in the peripheral blood of mice. APQ2-0.1 mpk, APQ2-0.5 mpk, and APQ-2.5 mpk represent APQ2 groups administered at doses of 0.1, 0.5, and 2.5 mg/kg respectively; A133-0.5 mpk represents the A-1331852 group administered at a dose of 0.5 mg/kg; Veh represents the control group with the vehicle alone. The time points for blood routine detection were at 0, 4, 24, and 72 hours after administration. Figure 5B shows the results comparing the effects of the PROTAC molecules APQ9, APQ10 with the control molecule A-1331852 and the vehicle alone on the number of platelets in the peripheral blood of mice. Veh, APQ9-5 mpk, APQ10-5 mpk, and A133-5 mpk represent the control group with the vehicle alone, APQ9 group administered at a dose of 5 mg/kg, APQ10 group administered at a dose of 5 mg/kg, and A-1331852 group administered at a dose of 5 mg/kg, respectively. The time points for blood routine detection were at 4, 24, and 48 hours after administration. Figure 5C shows the results comparing the effects of APQ10 and A-1331852 administered at different doses on the number of platelets. APQ10-2 mpk, APQ10-5 mpk, and APQ10-20 mpk represent APQ10 groups administered at doses of 2, 5, and 20 mg/kg, respectively; A133-2 mpk, A133-5 mpk, and A133-20 mpk represent A-1331852 groups administered at doses of 2, 5, and 20 mg/kg, respectively; Veh represents the control group with the vehicle alone. The time points for blood routine detection were at 24, 48, and 72 hours after administration. The number of mice in each group in Figures 5A, 5B, and 5C is 4.

### Detailed Description of Invention

### Definitions

As used herein, "substituted" refers to all permissible substituents of a compound or functional group described herein. In the broadest sense, permissible substituents include acyclic or cyclic, branched or unbranched, carbocyclic or heterocyclic, and aromatic or non-aromatic substituents for organic compounds. Exemplary substituents include, but are not limited to, halogen, hydroxyl, or any other organic groups containing any number of carbon atoms, preferably 1-14 carbon atoms, which optionally comprise one or more heteroatoms such as oxygen, sulfur, or nitrogen in a linear, branched, or cyclic structural form. Representative substituents include alkyl, substituted alkyl, alkenyl, substituted alkenyl, alkynyl, substituted alkynyl, phenyl, substituted phenyl, aryl, substituted aryl, heteroaryl, substituted heteroaryl, halogen, hydroxyl, alkoxy, substituted alkoxy, phenoxy, substituted phenoxy, aryloxy, substituted aryloxy, alkylthio, substituted alkylthio, phenylthio, substituted phenylthio, arylthio, substituted arylthio, cyano, isocyano, substituted isocyano, carbonyl, substituted carbonyl, carboxyl, substituted carboxyl, amino, substituted amino, amido, substituted amido, sulfonyl, substituted sulfonyl, sulfonic acid group, phosphoryl, substituted phosphoryl, phosphonyl, substituted phosphonyl, polyaryl, substituted polyaryl, C₃-C₂₀ cyclic group, substituted C₃-C₂₀ cyclic group, heterocyclyl, substituted heterocyclyl, amino acid group, poly(lactic-co-glycolic acid) group, peptide group or polypeptide group. Such alkyl, substituted alkyl, alkenyl, substituted alkenyl, alkynyl, substituted alkynyl, phenyl, substituted phenyl, aryl, substituted aryl, heteroaryl, substituted heteroaryl, halogen, hydroxyl, alkoxy, substituted alkoxy, phenoxy, substituted phenoxy, aryloxy, substituted aryloxy, alkylthio, substituted alkylthio, phenylthio, substituted phenylthio, arylthio, substituted arylthio, cyano, isocyano, substituted isocyano, carbonyl, substituted carbonyl, carboxyl, substituted carboxyl, amino, substituted amino, amido, substituted amido, sulfonyl, substituted sulfonyl, sulfonic acid group, phosphoryl, substituted phosphoryl, phosphonyl, substituted phosphonyl, polyaryl, substituted polyaryl, C₃-C₂₀ cyclic group, substituted C₃-C₂₀ cyclic group, heterocyclyl, substituted heterocyclyl, amino acid group, poly(lactic-co-glycolic acid) group, peptide group, and polypeptide group can be further substituted.

Heteroatoms such as nitrogen may have hydrogen substituents and/or any permissible substituents of an organic compound described herein that satisfy the valency of the heteroatom. It should be appreciated that "substituted" or "substitution" includes the implicit condition that such substitution complies with the allowed valencies of the substituted atom and the substituent, and that the substitution yields a stable compound, i.e., a compound that does not spontaneously undergo transformation such as those via rearrangement, cyclization, elimination, or the like.

Unless otherwise specifically and explicitly stated, the term "substituted" refers to a structure, such as a compound or a moiety of a larger compound, regardless of how that structure is formed. The structure is not limited to a structure made by any particular method.

As used herein, "aryl" refers to C₅-C₂₆ aromatic, fused aromatic, fused heterocyclic, or bi-aromatic ring systems. As used herein, the broadly defined "aryl" includes 5-, 6-, 7-, 8-, 9-, 10-, 14-, 18- ,and 24-membered monocyclic aromatic groups, which may comprise zero to four heteroatoms, such as benzene, naphthalene, anthracene, phenanthrene, chrysene, pyrene, corannulene, coronene, or the like.

"Aryl" also includes polycyclic systems having two or more rings, wherein two or more carbons are shared by two adjacent rings (i.e., "fused rings"), of which at least one ring is aromatic, and the other cyclic ring(s) may be cycloalkyl, cycloalkenyl, cycloalkynyl, aryl, and/or heterocyclyl.

The term "substituted aryl" refers to an aryl group wherein one or more hydrogen atoms on one or more aromatic rings are replaced by one or more substituents, including but not limited to halogen, azide group, alkyl, aralkyl, alkenyl, alkynyl, cycloalkyl, hydroxyl, alkoxy, carbonyl (e.g., ketone group, aldehyde group, carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, imino, alkylthio, sulfate, sulfonate, sulfamido, sulfoxide group, sulfoamido, sulfonyl, heterocyclyl, alkylaryl, haloalkyl (e.g., CF₃, - CH₂-CF₃, -CCl₃), -CN, aryl, heteroaryl, and any combination thereof.

The terms "heterocycle", "heterocyclic" and "heterocyclyl" are used interchangeably, and refer to a cyclic group linked via a ring carbon or nitrogen atom, which is a monocyclic or bicyclic ring containing 3-10 or 3-8 ring atoms, preferably 5-6 ring atoms, consisting of carbon and one to four heteroatoms, each heteroatom selected from non-peroxide oxygen, sulfur, and N(Y), where Y is absent or is H, C₁-C₁₀ alkyl, phenyl or benzyl, and optionally containing 1-3 double bonds and optionally being substituted with one or more substituents. By definition, heterocyclyl is different from heteroaryl. Examples of heterocyclyl include, but are not limited to, piperazinyl, piperidinyl, piperidinonyl, 4-piperidinonyl, dihydrofurano[2,3-b]tetrahydrofuran, morpholinyl, piperazinyl, piperidinyl, piperidinonyl, 4-piperidinonyl, piperonyl, pyranyl, 2H-pyrrolyl, 4H-quinolyl, quinuclidinyl, tetrahydrofuranyl, and 6H-1,2,5-thiadiazinyl. Heterocyclyl may be optionally substituted with one or more substituents as defined above for alkyl and aryl.

The term "heteroaryl" refers to C₅-C₂₆ aromatic, fused aromatic, or bi-aromatic ring systems or combinations thereof, in which one or more carbon atoms on one or more aromatic ring structures have been replaced by heteroatoms. Suitable heteroatoms include, but are not limited to, oxygen, sulfur, and nitrogen. As used herein, the broadly defined "heteroaryl" includes 5-, 6-, 7-, 8-, 9-, 10-, 14-, 18- and 24-membered monocyclic aromatic groups, which may comprise one to four heteroatoms, for example pyrrole, furan, thiophene, imidazole, oxazole, thiazole, triazole, tetrazole, pyrazole, pyridine, pyrazine, pyridazine, and pyrimidine groups, and the like. Heteroaryl may also be referred to as "aryl heterocycles" or "heteroaromatic compounds". "Heteroaryl" also includes polycyclic systems having two or more rings, wherein two or more carbons are shared by two adjacent rings (i.e., "fused rings"), of which at least one ring is heteroaromatic, and the other ring(s) may be cycloalkyl, cycloalkenyl, cycloalkynyl, aryl, heterocyclyl, or combinations thereof. Examples of heteroaryl ring include, but are not limited to, benzimidazolyl, benzofuranyl, benzothiofuranyl, benzothienyl, benzoxazolyl, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, benzodihydropyranyl, benzopyranyl, cinnolinyl, decahydroquinolinyl, 2H,6H-1,5,2-dithiazinyl, furanyl, furazanyl, imidazolidinyl, imidazolinyl, imidazolyl, 1H-indazolyl, indolenyl, dihydroindolyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl, naphthyridinyl, octahydroisoquinolinyl, 1,2,3-oxadiazolyl, 1,2,4-oxadiazolyl, 1,2,5-oxadiazolyl, 1,3,4-oxadiazolyl, oxazolidinyl, oxazolyl, hydroxyindolyl, pyrimidinyl, phenanthridinyl, phenanthrolinyl, phenazinyl, phenothiazinyl, phenoxathinyl, phenoxazinyl, phthalazinyl, pteridinyl, purinyl, pyrazinyl, pyrazolidinyl, pyrazolinyl, pyrazolyl, pyridazinyl, pyridoxazolyl, pyridoimidazolyl, pyridothiazolyl, pyridinyl, pyridyl, pyrimidinyl, pyrrolidinyl, pyrrolinyl, pyrrolyl, quinazolinyl, quinolinyl, quinoxalinyl, tetrahydroisoquinolinyl, tetrahydroquinolinyl, tetrazolyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienoxazolyl, thienoimidazolyl, phenylthio and xanthenyl. One or more rings may be substituted, as defined below for "substituted heteroaryl".

The term "substituted heteroaryl" refers to a heteroaryl group in which one or more hydrogen atoms on one or more heteroaryl rings are replaced by one or more substituents, including but not limited to halogen, azide group, alkyl, aralkyl, alkenyl, alkynyl, cycloalkyl, hydroxyl, alkoxy, carbonyl (e.g., ketone group, aldehyde group, carboxyl, alkoxycarbonyl, formyl, or acyl), silyl, ether group, ester group, thiocarbonyl (e.g.,

thioester group, thioacetate, or thioformate), alkoxy, phosphoryl, phosphate, phosphonate, phosphinate, amino (or quaternized amino), amido, amidine group, imine group, cyano, nitro, azido, thiol, imino, alkylthio, sulfate, sulfonate, sulfamoyl, sulfoxide group, sulfonamido, sulfonyl, heterocyclyl, alkaryl, haloalkyl (e.g., CF₃, -CH₂-CF₃, -CCl₃), -CN, aryl, heteroaryl, and any combination thereof.

As used herein, "alkyl" refers to a saturated aliphatic group, including linear alkyl, branched alkyl, cycloalkyl (alicyclic), alkyl-substituted cycloalkyl, and cycloalkyl-substituted alkyl. In preferred embodiments, the linear or branched alkyl has 30 or less (e.g., C₁-C₃₀ for linear alkyl, C₃-C₃₀ for branched alkyl), preferably 20 or less, more preferably 15 or less, and most preferably 10 or less carbon atoms in its main chain. Similarly, a preferred cycloalkyl has 3-10 carbon atoms in the ring structure, and more preferably 5, 6, or 7 carbon atoms in the ring structure. The term "alkyl" (or "lower alkyl") as used throughout the specification, examples, and claims is intended to include both "unsubstituted alkyl" and "substituted alkyl", the latter means that one or more hydrogens on carbons of the main hydrocarbon chain are replaced by a moiety having one or more substituents. Such substituents include, but are not limited to, halogen, hydroxyl, carbonyl (e.g., carboxyl, alkoxycarbonyl, formyl, or acyl), thiocarbonyl (e.g., thioester group, thioacetate, or thioformate), alkoxy, phosphoryl, phosphate, phosphonate, phosphinate, amino, amido, amidine group, imine group, cyano, nitro, azido, thiol, alkylthio, sulfate, sulfonate, sulfamoyl , sulfoxide group, sulfonamido, sulfonyl, heterocyclyl, aralkyl, or aromatic or heteroaromatic moiety.

Unless otherwise specified for carbon atom number, the term "lower alkyl" as used herein refers to an alkyl group as defined above, but having 1 to 10 carbon atoms, more preferably 1 to 6 carbon atoms in its main chain structure. Similarly, "lower alkenyl" and "lower alkynyl" have similar chain lengths. Throughout this application, preferred alkyl is lower alkyl. In preferred embodiments, a substituent designated as alkyl herein is lower alkyl.

"Alkyl" includes one or more substitutions at one or more carbon atoms in a hydrocarbon or heteroalkyl group. Suitable substituents include, but are not limited to, halogen such as fluorine, chlorine, bromine, or iodine; hydroxyl; -NRR', wherein R and R' are independently hydrogen, alkyl, or aryl, and wherein the nitrogen atom is optionally quaternized; -SR, wherein R is hydrogen, alkyl, or aryl; -CN; -NO₂; -COOH; carboxylate; -COR, -COOR, or -CON(R)₂, wherein R is hydrogen, alkyl, or aryl; azide group; aralkyl; alkoxy; imino; phosphonate; phosphinate; silyl; ether group; sulfonyl; sulfonamido; heterocyclyl; aromatic or heteroaromatic moiety; haloalkyl (e.g., -CF₃, -CH₂-CF₃, -CCl₃); CN; -NCOCOCH₂CH₂; NCOCOCH; -NCS; and any combination thereof.

Those skilled in the art will appreciate that, if appropriate, the moiety that is substituted on the hydrocarbon chain may be substituted per se. For example, the substituent (s) on a substituted alkyl group may include halogen, hydroxyl, nitro, thiol, amino, azido, imino, amido, phosphoryl (including phosphonate and phosphinate), sulfonyl (including sulfate, sulfonamido, sulfamoyl, sulfoxide group, and sulfonate), and silyl, as well as ether group, alkylthio, carbonyl (including ketone group, aldehyde group, carboxylate, and ester group), haloalkyl, -CN, or the like. Cycloalkyl may be substituted in a similar way.

The terms "alkenyl" and "alkynyl" refer to unsaturated aliphatic groups that are similar to the aforementioned alkyl groups in terms of length and possible substitutions, but contain at least one double bond or triple bond, respectively.

The term "substituted alkenyl" refers to an alkenyl moiety having one or more substituents that replace one or more hydrogen atoms on one or more carbon atoms of the main hydrocarbon chain. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonamido, sulfonyl, heterocyclyl, alkaryl, haloalkyl, -CN, aryl, heteroaryl, and any combination thereof.

The term "substituted alkynyl" refers to an alkynyl moiety having one or more substituents that replace one or more hydrogen atoms on one or more carbon atoms of the main hydrocarbon chain. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonamido, sulfonyl, heterocyclyl, alkaryl, haloalkyl, -CN, aryl, heteroaryl, and any combination thereof.

As used herein, "amino" and "amine" are terms recognized in the art and refer to substituted and unsubstituted amines, for example, moieties that can be represented by the following general formula: 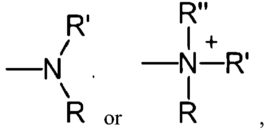 wherein R, R', and R" each independently represent hydrogen, substituted or unsubstituted alkyl, substituted or unsubstituted alkenyl, substituted or unsubstituted alkynyl, substituted or unsubstituted carbonyl, -(CH₂)ₘ-R''', or R and R' together with the nitrogen atom to which they are attached form a heterocyclic ring having 3 to 14 atoms in the ring structure; R"' represents hydroxyl, substituted or unsubstituted carbonyl, aryl, cycloalkyl, cycloalkenyl, heterocyclyl, or polycyclyl; and m is 0 or an integer from 1 to 8. In preferred embodiments, only one of R and R' may be carbonyl, for example, R and R' together with the nitrogen atom do not form diimide. In preferred embodiments, R and R' (and optional R") each independently represent hydrogen atom, substituted or unsubstituted alkyl, substituted or unsubstituted alkenyl, or -(CH₂)ₘ-R"'. Thus, the term "alkylamine" as used herein refers to an amine group as defined above that has substituted or unsubstituted alkyl attached thereto (i.e., at least one of R, R' or R" is alkyl).

As used herein, "carbonyl" is a term recognized in the art, and includes moieties that can be represented by the following general formula: 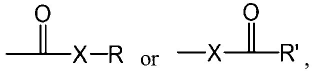 wherein X is a bond, or represents oxygen or sulfur; R represents hydrogen, substituted or unsubstituted alkyl, substituted or unsubstituted alkenyl, substituted or unsubstituted alkynyl, substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl, -(CH₂)ₘ-R", or a pharmaceutically acceptable salt thereof; R' represents hydrogen, substituted or unsubstituted alkyl, substituted or unsubstituted alkenyl, substituted or unsubstituted alkynyl, substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl, or (CH₂)ₘ-R"; R" represents hydroxyl, substituted or unsubstituted carbonyl, aryl, cycloalkyl, cycloalkenyl, heterocyclyl or polycyclyl; and m is 0 or an integer from 1 to 8. When X is oxygen and R is as defined above, this moiety is also called a carboxylic group. When X is oxygen and R is hydrogen, this formula represents a "carboxylic acid" group. When X is oxygen and R' is hydrogen, this formula represents a "formic acid" group. When X is oxygen and R or R' is not hydrogen, this formula represents an "ester" group. Generally, when the oxygen atom in the above formulae is replaced by a sulfur atom, this formula represents a "thiocarbonyl" group. When X is sulfur and R or R' is not hydrogen, this formula represents a "thioester" group. When X is sulfur and R is hydrogen, this formula represents a "thiocarboxylic acid" group. When X is sulfur and R' is hydrogen, this formula represents a "thioformate" group. When X is a bond and R is not hydrogen, the above formula represents a "ketone" group. When X is a bond and R is hydrogen, the above formula represents an "aldehyde" group.

The term "substituted carbonyl " refers to a carbonyl group as defined above, i.e., 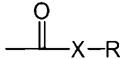 or 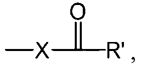 wherein one or more hydrogen atoms of R, R' or the groups to which they are attached are independently substituted. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonamido, sulfonyl, heterocyclyl, alkaryl, haloalkyl, -CN, aryl, heteroaryl, and any combination thereof.

The term "carboxyl" is defined for the following formulae: 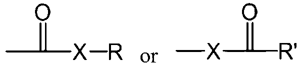 as above, and more specifically is defined by the formula -R^{iv}COOH, wherein R^{iv} is alkyl, alkenyl, alkynyl, cycloalkyl, heterocyclyl, alkaryl, aralkyl, aryl, or heteroaryl. In preferred embodiments, the linear or branched alkyl, alkenyl, and alkynyl have 30 or less (e.g., C₁-C₃₀ for linear alkyl, C₃-C₃₀ for branched alkyl, C₂-C₃₀ for linear alkenyl and alkynyl, and C₃-C₃₀ for branched alkenyl and alkynyl), preferably 20 or less, more preferably 15 or less, and most preferably 10 or less carbon atoms in their main chain. Similarly, preferred cycloalkyl, heterocyclyl, aryl, and heteroaryl have 3-10 carbon atoms in their ring structure, and more preferably 5, 6, or 7 carbon atoms in the ring structure.

The term "substituted carboxyl" refers to a carboxyl group as defined above, wherein one or more hydrogen atoms of R^{iv} are substituted. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonamido, sulfonyl, heterocyclyl, alkaryl, haloalkyl, -CN, aryl, heteroaryl, and any combination thereof.

As used herein, "heteroalkyl" refers to a linear, branched, or cyclic carbon-containing group, or a combination thereof, that contains at least one heteroatom. Suitable heteroatoms include, but are not limited to, O, N, Si, P, and S, wherein the nitrogen, phosphorus, and sulfur atoms are optionally oxidized, and the nitrogen heteroatom is optionally quaternized.

Examples of saturated hydrocarbon group include, but are not limited to, methyl, ethyl, n-propyl, isopropyl, n-butyl, tert-butyl, isobutyl, sec-butyl, cyclohexyl, (cyclohexyl)methyl, cyclopropylmethyl, and homologs and isomers thereof, such as n-pentyl, n-hexyl, n-heptyl, and n-octyl. Examples of unsaturated alkyl include, but are not limited to, vinyl, 2-propenyl, crotyl, 2-isopentenyl, 2-(butadienyl), 2,4-pentadienyl, 3-(1,4-pentadienyl), ethynyl, 1-propynyl, 3-propynyl, and 3-butynyl.

The terms "alkoxyl" or "alkoxy", "aroxy" or "aryloxy" generally describe compounds represented by the formula -OR^{v}, wherein R^{v} includes, but is not limited to, substituted or unsubstituted alkyl, alkenyl, alkynyl, cycloalkyl, heterocyclyl, cycloalkenyl, heterocycloalkenyl, aryl, heteroaryl, aralkyl, heteroalkyl, alkaryl, and alkylheteroaryl.

As used herein, the term "alkoxy" or "alkoxyl" refers to an alkyl group as defined above, to which an oxy group is attached. Representative alkoxy includes methoxy, ethoxy, propoxy, tert-butoxy, and the like. An "ether" is formed of two hydrocarbons covalently linked by oxygen. Thus, the substituent for alkyl that makes the alkyl become an ether is or is similar to an alkoxy group, for example, it may be represented by one of -O-alkyl, -O-alkenyl, and -O-alkynyl. If the valency permits, the term alkoxy also includes cycloalkyl, heterocyclyl, cycloalkenyl, heterocycloalkenyl, and aralkyl having an oxy group attached to at least one carbon atom.

The term "substituted alkoxy" refers to an alkoxy group having one or more substituents that replace one or more hydrogen atoms on one or more carbons of the main chain of the alkoxy. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonamido, sulfonyl, heterocyclyl, alkaryl, haloalkyl, -CN, aryl, heteroaryl, and any combination thereof.

The term "alkylthio" refers to an alkyl group as defined above that is linked to a sulfur group. An "alkylthio" moiety is represented by -S-alkyl. Representative alkylthio groups include methylthio, ethylthio, and the like. The term "alkylthio" also includes cycloalkyl to which a sulfur group is attached.

The term "substituted alkylthio" refers to an alkylthio group having one or more substituents that replace one or more hydrogen atoms on one or more carbon atoms of the main chain of the alkylthio. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonylamino, sulfonyl, heterocyclyl, alkaryl, haloalkyl, -CN, aryl, heteroaryl, and any combination thereof.

The term "arylthio" refers to -S-aryl or -S-heteroaryl, wherein the aryl and heteroaryl are as defined herein.

The term "substituted arylthio" represents -S-aryl or -S-heteroaryl, which has one or more substituents that replace one or more hydrogen atoms on one or more ring atims of the aryl or heteroaryl ring as defined herein. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonamido, sulfonyl, heterocyclyl, alkaryl, haloalkyl, -CN, aryl, heteroaryl, and any combination thereof.

As used herein, "aralkyl" refers to an alkyl group substituted with substituted or unsubstituted aryl or heteroaryl.

As used herein, "alkylaryl" refers to an aryl group (e.g., an aromatic or heteroaromatic group) substituted with substituted or unsubstituted alkyl.

The term "amide" or "amido" may be used interchangeably, and refers to both "unsubstituted amido" and "substituted amido", and is represented by the general formula below: 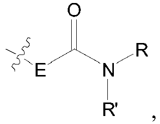 wherein E is absent, or E is substituted or unsubstituted alkyl, substituted or unsubstituted alkenyl, substituted or unsubstituted alkynyl, substituted or unsubstituted aralkyl, substituted or unsubstituted cycloalkyl, substituted or unsubstituted aryl, substituted or unsubstituted heteroaryl, or substituted or unsubstituted heterocyclyl; wherein, independently of E, R and R' each independently represent hydrogen, substituted or unsubstituted alkyl, substituted or unsubstituted alkenyl, substituted or unsubstituted alkynyl, substituted or unsubstituted carbonyl, substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl, -(CH₂)ₘ-R''', or R and R' together with the N atom to which they are attached form a heterocyclic ring having 3 to **14** atoms in the ring structure; R"' represents hydroxyl, substituted or unsubstituted carbonyl, aryl, cycloalkyl, cycloalkenyl, heterocyclyl or polycyclyl; and m is 0 or an integer from 1 to 8. In preferred embodiments, only one of R and R' may be carbonyl, for example, R and R' together with the nitrogen atom do not form a diimide. In preferred embodiments, R and R' each independently represent a hydrogen atom, substituted or unsubstituted alkyl, substituted or unsubstituted alkenyl, or -(CH₂)ₘ-R"'. When E is oxygen, a carbamate is formed. As understood by those skilled in the art, carbamate cannot be linked to another chemical substance, for example, to form an oxygen-oxygen bond or other unstable bonds.

The term "sulfonyl" is represented by the following formula: 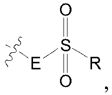 wherein E is absent, or E is alkyl, alkenyl, alkynyl, aralkyl, alkaryl, cycloalkyl, aryl, heteroaryl, or heterocyclyl; wherein, independently of E, R represents hydrogen, substituted or unsubstituted alkyl, substituted or unsubstituted alkenyl, substituted or unsubstituted alkynyl, substituted or unsubstituted amine group, substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl, -(CH₂)ₘ-R"', or E and R together with the S atom to which they are attached form a heterocyclic ring having 3 to **14** atoms in the ring structure; R"' represents hydroxyl, substituted or unsubstituted carbonyl, aryl, cycloalkyl, cycloalkenyl, heterocyclyl, or polycyclyl; and m is 0 or an integer from 1 to 8. In preferred embodiments, only one of E and R can be a substituted or unsubstituted amine group to form "sulfonamide" or "sulfonamido". Substituted or unsubstituted amine groups are as defined above.

The term "substituted sulfonyl" represents a sulfonyl group as defined above, wherein E and R are independently substituted. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonylamino, sulfonyl, heterocyclyl, alkaryl, haloalkyl, -CN, aryl, heteroaryl, and any combination thereof.

The term "sulfonic acid group" refers to a sulfonyl group as defined above, wherein R is hydroxyl and E is absent, or E is substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl.

The term "sulfate" refers to a sulfonyl group as defined above, wherein E is absent, oxygen, alkoxy, aryloxy, substituted alkoxy, or substituted aryloxy as defined above, and R is independently hydroxyl, alkoxy, aryloxy, substituted alkoxy, or substituted aryloxy as defined above. As understood by those skilled in the art, when E is oxygen, a sulfate group cannot be linked to another chemical species, for example, to form an oxygen-oxygen bond or other unstable bonds.

The term "sulfonate" refers to a sulfonyl group as defined above, wherein E is oxygen, alkoxy, aryloxy, substituted alkoxy, or substituted aryloxy as defined above, and R is independently hydrogen, substituted or unsubstituted alkyl, substituted or unsubstituted alkenyl, substituted or unsubstituted alkynyl, substituted or unsubstituted amine, substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl, -(CH₂)ₘ-R"', where R"' represents hydroxyl, substituted or unsubstituted carbonyl, aryl, cycloalkyl, cycloalkenyl, heterocyclyl, or polycyclyl; and m is 0 or an integer from 1 to 8. As understood by those skilled in the art, when E is oxygen, a sulfonate group cannot be linked to another chemical species, for example, to form an oxygen-oxygen bond or other unstable bonds.

The term "sulfamoyl" refers to a sulfonamide or a sulfonamide group represented by the following formula: 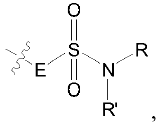 wherein E is absent, or E is substituted or unsubstituted alkyl, substituted or unsubstituted alkenyl, substituted or unsubstituted alkynyl, substituted or unsubstituted aralkyl, substituted or unsubstituted alkaryl, substituted or unsubstituted cycloalkyl, substituted or unsubstituted aryl, substituted or unsubstituted heteroaryl, or substituted or unsubstituted heterocyclyl; wherein E, R and R' each independently represent hydrogen, substituted or unsubstituted alkyl, substituted or unsubstituted alkenyl, substituted or unsubstituted alkynyl, substituted or unsubstituted carbonyl, substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl, -(CH₂)ₘ-R"', or R and R' together with the N atom to which they are attached form a heterocyclic ring having 3 to 14 atoms in the ring structure; R"' represents hydroxyl, substituted or unsubstituted carbonyl, aryl, cycloalkyl, cycloalkenyl, heterocyclyl, or polycyclyl; and m is 0 or an integer from 1 to 8. In preferred embodiments, only one of R and R' can be carbonyl, for example, R and R' together with the nitrogen atom do not form a diimide.

The term "sulfoxide group" is represented by the following formula: 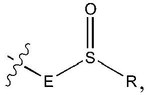 wherein E is absent, or E is alkyl, alkenyl, alkynyl, aralkyl, alkaryl, cycloalkyl, aryl, heteroaryl, or heterocyclyl; wherein, independently of E, R represents hydrogen, substituted or unsubstituted alkyl, substituted or unsubstituted alkenyl, substituted or unsubstituted alkynyl, substituted or unsubstituted amine, substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl, or -(CH₂)ₘ-R"', or E and R together with the S atom to which they are attached form a heterocyclic ring having 3 to **14** atoms in the ring structure; R"' represents hydroxyl, substituted or unsubstituted carbonyl, aryl, cycloalkyl, cycloalkenyl, heterocyclyl, or polycyclyl; and m is 0 or an integer from 1 to 8.

The term "phosphonyl" is represented by the following formula: 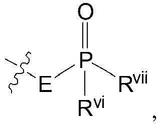 wherein E is absent, or E is substituted or unsubstituted alkyl, substituted or unsubstituted alkenyl, substituted or unsubstituted alkynyl, substituted or unsubstituted aralkyl, substituted or unsubstituted alkaryl, substituted or unsubstituted cycloalkyl, substituted or unsubstituted aryl, substituted or unsubstituted heteroaryl, or substituted or unsubstituted heterocyclyl; wherein, independently of E, R^{vi} and R^{vii} are independently hydrogen, substituted or unsubstituted alkyl, substituted or unsubstituted alkenyl, substituted or unsubstituted alkynyl, substituted or unsubstituted carbonyl, substituted or unsubstituted cycloalkyl, substituted or unsubstituted heterocyclyl, substituted or unsubstituted alkaryl, substituted or unsubstituted aralkyl, substituted or unsubstituted aryl, or substituted or unsubstituted heteroaryl, -(CH₂)ₘ-R''', or R and R' together with the P atom to which they are attached form a heterocyclic ring having 3 to 14 atoms in the ring structure; R"' represents hydroxyl, substituted or unsubstituted carbonyl, aryl, cycloalkyl, cycloalkenyl, heterocyclyl, or polycyclyl; and m is 0 or an integer from 1 to 8.

The term "substituted phosphonyl" represents a phosphonyl group in which E, R^{vi}, and R^{vii} are independently substituted. Such substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonamido, sulfonyl, heterocyclyl, alkaryl, haloalkyl, -CN, aryl, heteroaryl, and any combination thereof.

The term "phosphoryl" defines a phosphoryl group as defined above, wherein E is absent, or E is oxygen, alkoxy, aryloxy, substituted alkoxy, or substituted aryloxy as defined above, and independently of E, R^{vi} and R^{vii} are independently hydroxyl, alkoxy, aryloxy, substituted alkoxy, or substituted aryloxy as defined above. When E is oxygen, a phosphoryl group cannot be linked to another chemical species, for example, to form an oxygen-oxygen bond or other unstable bonds, as understood by those skilled in the art. When E, R^{vi}, and R^{vii} are substituted, the substituents include, but are not limited to, halogen, azide group, alkyl, aralkyl, alkenyl, alkynyl, cycloalkyl, hydroxyl, carbonyl (e.g., carboxyl, alkoxycarbonyl, formyl, or acyl), silyl, ether group, ester group, thiocarbonyl (e.g., thioester group, thioacetate, or thioformate), alkoxy, phosphoryl, phosphate, phosphonate, phosphinate, amino (or quaternized amino), amido, amidine group, imine group, cyano, nitro, azido, thiol, alkylthio, sulfate, sulfonate, sulfamoyl, sulfoxide group, sulfonamido, sulfonyl, heterocyclyl, alkaryl, haloalkyl, -CN, aryl, heteroaryl, and any combination thereof.

The term "C₃-C₂₀ cyclic group" refers to substituted or unsubstituted cycloalkyl, substituted or unsubstituted cycloalkenyl, substituted or unsubstituted cycloalkynyl, or substituted or unsubstituted heterocyclyl having 3 to 20 carbon atoms, where the geometric constraints allow. The cyclic structure is formed by a monocyclic or fused ring system. Substituted cycloalkyl, cycloalkenyl, cycloalkynyl, and heterocyclyl are substituted as defined above for alkyl, alkenyl, alkynyl, and heterocyclyl, respectively.

### Example 1. Synthesis of compounds C1 and C4 as PROTAC targeting ligands.

### a) Synthesis of Compound C1 as a PROTAC targeting ligand

### Synthesis scheme:

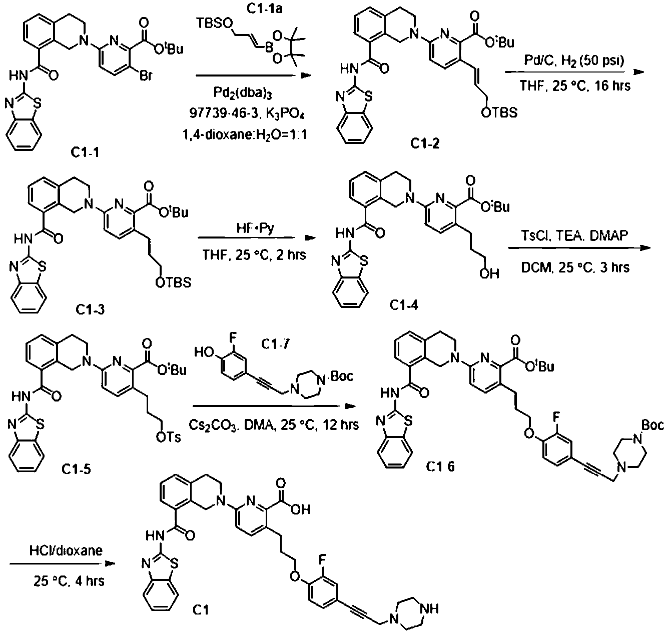

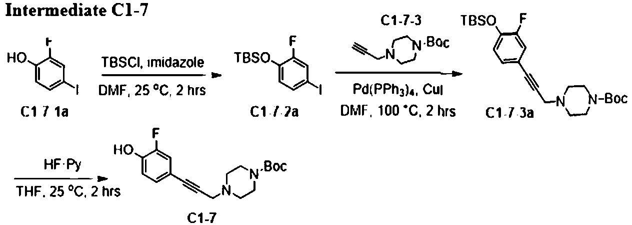

### 1. Synthesis of Compound C1-7-2a

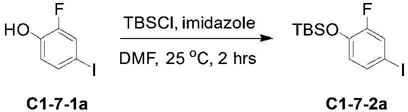

Compound C1-7-1a (5.00 g, 21.0 mmol, 1.00 eq.), TBSCl (4.12 g, 27.3 mmol, 3.35 mL, 1.30 eq.) and imidazole (2.86 g, 42.0 mmol, 2.00 eq.) were mixed in DMF (30.0 mL). The air was removed and replaced with N₂, and the mixture was stirred and reacted at 25°C under N₂ atmosphere for 2 hours. TLC (petroleum ether: ethyl acetate = 1:0, P1: R_{f}= 0.86, R1: R_{f}= 0.05) showed that C1-7-1a was reacted completely, and a new, low polar main peak was generated. To the reaction mixture, 300 mL of water was added to quench the reaction, and then extracted with a total of 300 mL of DCM (100 mL × 3). The resulting organic layer was washed with 300 mL of saturated brine, dried over Na₂SO₄, filtered under reduced pressure, and concentrated to obtain a crude product, which was purified by column chromatography (SiO₂, petroleum ether: ethyl acetate = 1:0) to provide Compound C1-7-2a (7.00 g, 19.9 mmol, 94.6% yield) as colorless liquid.

**HNMR:** EC10607-21-P1A, CDCl₃, 400MHz ¹H NMR (400 MHz, CDCl₃) δ ppm 7.16 (dd, *J =* 10.0, 2.0 Hz, 1H), 7.07 (d, *J*=8.4 Hz, 1H), 6.44 (t, *J*=8.4 Hz, 1H), 0.78 (s, 10H), 0.03 (s, 6H).

### 2. Synthesis of Compound C1-7-3a

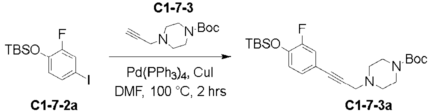

Compound C1-7-2a (2.00 g, 5.68 mmol, 1.00 eq.), C1-7-3 (1.91 g, 8.52 mmol, 1.50 eq.), CuI (54.1 mg, 284 µmol, 0.05 eq.) and Pd(PPh₃)₄ (399 mg, 568 µmol, 0.10 eq.) were mixed in DMF (30.0 mL), and the reaction was carried out under N₂ atmosphere at 75°C with stirring for 2 hours. LCMS (EC10607-30-p1a1, P1: RT = 0.460 minutes) showed that approximately 58.1% of the target product was generated. TLC (petroleum ether: ethyl acetate = 3:1, P1: R_{f}= 0.22, R1: R_{f}= 0.98) showed Compound C1-7-2a was reacted completely, and a new, high polar main peak was generated. To the reaction mixture, 200 mL of water was added to quench the reaction, and then extracted with 210 mL of DCM (70.0 mL × 3). The organic layer was washed with 200 mL of saturated saline, dried over Na₂SO₄, filtered under reduced pressure, and concentrated to obtain a crude product, which was purified by column chromatography (SiO₂, petroleum ether: ethyl acetate = 3:1, R_{f}= 0.22) to provide Compound C1-7-3a (1.98 g, 4.41 mmol, 77.7% yield) as a yellow oil.

LCMS: EC EC10607-30-p1a1, RT = 0.460 min, M/Z (M+H+) = 449.9.

### 3. Synthesis of Compound C1-7

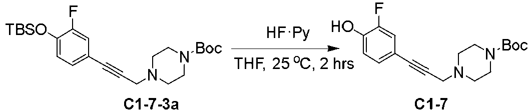

To a mixture of Compound C1-7-3a (1.98 g, 4.41 mmol, 1.00 eq.) dissolved in THF (20.0 mL), pyridine-hydrofluoride (875 mg, 8.83 mmol, 795 µL, 2.00 eq.) was added, and the mixture was stirred at 25°C under N₂ atmosphere for 2 hours. LCMS (EC10607-35-p1a1, P1: RT = 0.241 minutes) showed that approximately 89.2% of the target product was generated. TLC (petroleum ether: ethyl acetate = 2:1, R1: R_{f} = 0.22, P1: R_{f}= 0.98) showed Compound C1-7-3a was reacted completely, and a new, high polar main peak was generated. To the reaction mixture, 300 mL of water was added to quench the reaction, and then extracted with 300 mL DCM (100 mL × 3). The organic layer was washed with 300 mL of saline, dried over Na₂SO₄, filtered under reduced pressure, and concentrated to obtain a crude product, which was purified by column chromatography (SiO₂, petroleum ether: ethyl acetate = 2:1, R_{f} = 0.15) to provide Compound C1-7 (1.16 g, 3.47 mmol, 78.6% yield) as a red solid.
**LCMS:** EC EC10607-35-p1a1, RT = 0.241 min, M/Z (M+H⁺) = 335.4
**HNMR:** EC10607-21-P1A, CDCl₃, 400MHz
¹H NMR (400 MHz, CDCl₃) δ ppm 7.01 - 7.08 (m, 2H), 6.90 (t, J=8.8 Hz, 1H), 3.49 - 3.57 (m, 6H), 2.62 (br t, *J=*4.5 Hz, 4H), 1.47 (s, 9H).

### 4. Synthesis of Compound C1-2

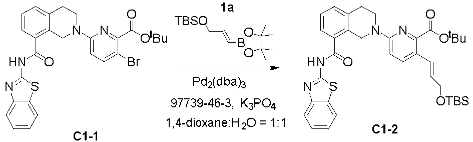

Compound C1-1 (1.50 g, 2.65 mmol, 1.00 eq.), Compound 1a (2.37 g, 7.96 mmol, 3.00 eq.), Pd₂(dba)₃ (243 mg, 265 µmol, 0.10 eq.), K₃PO₄ (1.97 g, 9.28 mmol, 3.50 eq.) and (1S,3R,5R,7S)-1,3,5,7-tetramethyl-8-phenyl-2,4,6-trioxa-8-phosphorus-tricyclo[3.3.1.13,7]decane (310 mg, 1.06 mmol, 0.40 eq.) were mixed in 1,4-dioxane (10.0 mL) and water (10.0 mL). The air was removed and replaced with N₂, and the mixture was stirred and reacted at 90°C under N₂ atmosphere for 2 hours. LCMS (EC10607-26-p1a2, P1: RT = 0.649 minutes) showed that approximately 67.5% of the target product was generated. TLC (petroleum ether: ethyl acetate = 3:1, P1: R_{f}= 0.29, R1: R_{f}= 0.25) showed Compound C1-1 was reacted completely, and multiple new peaks were generated. To the reaction mixture, 300 mL of water was added to quench the reaction, and then extracted with 300 mL DCM (100 mL × 3). The organic layer was washed with 300 mL of saline, dried over Na₂SO₄, filtered under reduced pressure, and concentrated to obtain a crude product, which was purified by column chromatography (SiO₂, petroleum ether: ethyl acetate = 3:1, R_{f}= 0.29) to provide Compound C1-2 (1.25 g, 1.90 mmol, 71.7% yield) as a yellow oil.

**LCMS:** EC10607-26-pla2, RT = 0.650 min, M/Z (M+H⁺) = 657.8.

### 5. Synthesis of Compound C1-3

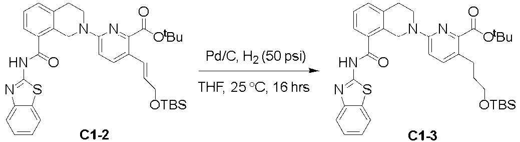

To a solution of Compound C1-2 (1.20 g, 1.83 mmol, 1.00 eq.) dissolved in THF (20.0 mL) and methanol (20.0 mL), Pd/C (1.00 g, 1.83 mmol, 10% purity, 1.00 eq.) was added, and the reaction mixture was stirred and reacted at 25°C under 50 psi H₂ (1.23 mg, 609 µmol) for 24 hours. TLC (petroleum ether: ethyl acetate = 3: 1, P1: R_{f}= 0.36, R1: R_{f}= 0.31) showed that Compound C1-2 was reacted completely, and a new peak was generated. Pd/C was removed by filtration with diatomaceous earth, and a crude product was obtained by concentrating under reduced pressure. The crude product was purified by column chromatography (SiO₂, petroleum ether: ethyl acetate = 3:1, R_{f}= 0.30) to provide Compound C1-3 (1.01 g, 1.53 mmol, 73.7% yield) as a yellow solid.

### 6. Synthesis of Compound C1-4

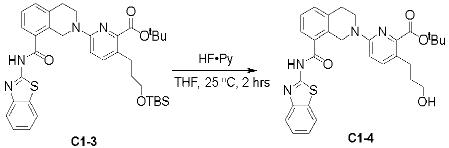

To a mixture of Compound C1-3 (1.00 g, 1.52 mmol, 1.00 eq.) dissolved in THF (15.0 mL), pyridine-hydrofluoride (694 mg, 4.55 mmol, 631 µL, 65% purity, 3.00 eq.) was added, and the mixture was stirred and reacted at 25°C under N₂ atmosphere for 2 hours. LCMS (EC10607-37-p1a1, P1: RT = 0.410 minutes) showed that approximately 46.4% of the target product was generated. TLC (petroleum ether: ethyl acetate = 1: 1, R1: R_{f}= 0.60, P1: R_{f}= 0.16) showed that C1-3 was reacted completely, and a new, high polar main peak was generated. To the reaction mixture, 300 mL of water was added to quench the reaction, and then extracted with 300 mL DCM (100 mL × 3). The organic layer was washed with 300 mL of saline, dried over Na₂SO₄, filtered and concentrated under reduced pressure to obtain a crude product, which was purified by Prep-TLC (SiO₂, petroleum ether: ethyl acetate = 1:1, R_{f} = 0.16) to provide Compound C1-4 (700 mg, 1.29 mmol, 84.7% yield) as a white solid.

**LCMS:** EC EC10607-37-p1a1, RT = 0.410 min, M/Z (M+H⁺) = 545.4.

### 7. Synthesis of Compound C1-5

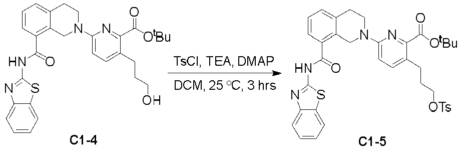

Compound C1-4 (680 mg, 1.25 mmol, 1.00 eq.), TEA (133 mg, 1.31 mmol, 182 µL, 1.05 eq.), DMAP (1.53 mg, 12.5 µmol, 0.01 eq.) and 4-methylbenzenesulfonyl chloride (250 mg, 1.31 mmol, 1.05 eq.) were mixed in DCM (5.00 mL). The air was removed and replaced with N₂, and the mixture was stirred and reacted at 25°C under N₂ atmosphere for 3 hours. LC-MS (EC10607-47-p1a1, P1: RT = 0.548 minutes) showed that approximately 46.7% of the target product was generated. TLC (petroleum ether: ethyl acetate = 2:1, P1: R_{f} = 0.36, R1: R_{f}= 0.20) showed that some C1-4 was reacted, and multiple new peaks were generated. To the reaction mixture, 300 mL of water was added to quench the reaction, and then extracted with 300 mL DCM (100 mL × 3). The organic layer was washed with 300 mL of saline, dried over Na₂SO₄, filtered under reduced pressure, and concentrated to obtain a crude product, which was purified by column chromatography (SiO₂, petroleum ether: ethyl acetate = 2:1, R_{f}= 0.36) to provide Compound C1-5 (400 mg, 572 µmol, 45.8% yield) as a yellow solid.

**LCMS:** EC (EC10607-47-p1a1, RT = 0.548 min, M/Z (M+H⁺) = 699.1

### 8. Synthesis of Compound C1-6

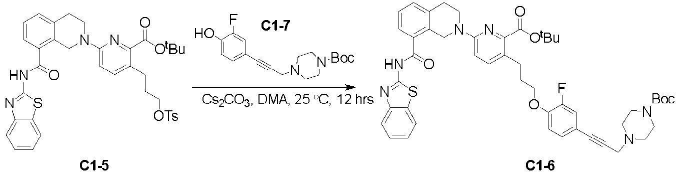

Compound C1-5 (100 mg, 143 µmol, 1.00 eq.), C1-7 (71.8 mg, 214 µmol, 1.50 eq.), Cs₂CO₃ (69.9 mg, 215 µmol, 1.50 eq.) were mixed in DMA (5.00 mL), the air was removed and replaced with N₂, and the mixture was stirred and reacted at 25°C under N₂ atmosphere for 12 hours. LC-MS (EC10607-49-p1a1, P1: RT = 0.493 minutes) showed that approximately 36.9% of the target product was generated. TLC (petroleum ether: ethyl acetate = 1:1, P1: R_{f}= 0.24, R1: R_{f}= 0.42) showed that C1-5 was completely consumed, and multiple new peaks were generated. To the reaction mixture, 30 mL of water was added to quench the reaction, and the mixture was extracted with 30.0 mL DCM (10.0 mL×3). The organic layer was washed with 30.0 mL saline, dried over Na₂SO₄, filtered and concentrated under reduced pressure to obtain a crude product, which was purified by Prep-TLC (SiO₂, petroleum ether: ethyl acetate = 1:1, R_{f}= 0.24) to provide Compound C1-6 (106 mg, 123 µmol, 86.0% yield) as a yellow solid.

**LCMS:** EC10607-49-p1a1, RT = 0.493 min, M/Z (M+H⁺) = 861.2

### 9. Synthesis of Compound C1

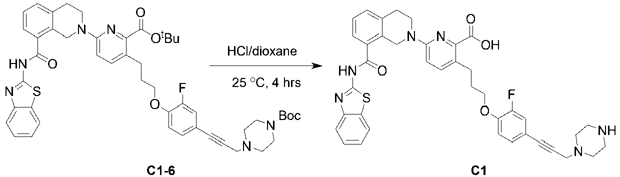

Compound C1-6 (100 mg, 116 µmol, 1.00 eq.) was dissolved in HCl/dioxacyclohexane (5.00 mL), The air was removed and replaced with N₂, and the mixture was stirred and reacted at 25°C under N₂ atmosphere for 12 hours. LC-MS (EC10607-51-p1a5, P1: RT = 0.412 minutes) showed that approximately 86.5% of the target product was generated. The reaction mixture was concentrated under reduced pressure to obtain a crude product, which was purified by Prep-HPLC (under HCl condition, chromatographic column: Welch Xtimate C18 150*25 mm*5 µm; mobile phase: [water (HCl)-ACN]; B%: 26%-46%, 10 minutes) to provide Compound C1 (25.0 mg, 35.5 µmol, 89.3% yield) as a yellow solid. LCMS (EC10607-57-p1a1, P1: RT = 0.42 minutes) showed that the product purity was approximately 100%. HPLC (EC10607-57-p1a2, P1: RT = 2.027 minutes) showed that the C1 product purity was approximately 6.1%.

LCMS: EC EC10607-51-pla5, RT = 0.412, M/Z (M+H⁺) = 705.2
¹H NMR (400 MHz, DMSO+D₂O) δ ppm 7.99 (d, *J=* 7.6 Hz, 1H), 7.77 (d, *J=* 8.0 Hz, 1H), 7.57 (d, *J* = 8.8 Hz, 1H), 7.54 (d, *J= 7.2* Hz, 1H), 7.47 (t, *J=* 7.2 Hz, 1H), 7.39 - 7.42 (m, 1H), 7.32 - 7.37 (m, 3H), 7.27 (d, *J* = 8.9 Hz, 1H), 7.06 - 7.12 (m, 1H), 7.01 (d, *J =* 8.8 Hz, 1H), 4.91 (s, 2H), 4.22 (s, 2H), 4.08 (s, 2H), 3.75 - 3.81 (m, 2H), 3.33 - 3.43 (m, 8H), 2.94 - 2.99 (m, 2H), 2.77 - 2.83 (m, 2H), 1.88 - 1.96 (m, 2H).

### b) Synthesis of Compound C4 as a PROTAC targeting ligand

### Synthesis scheme:

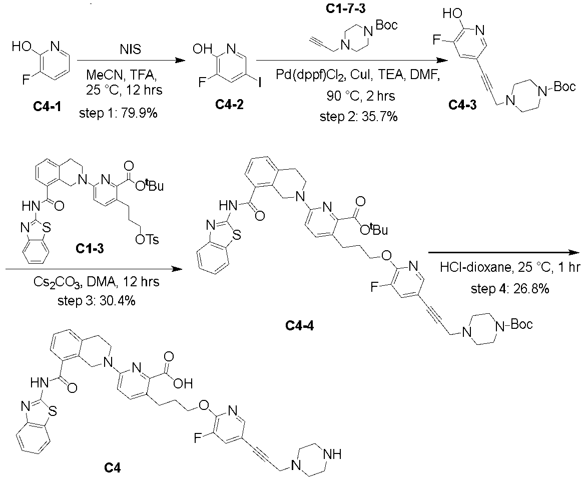

### 1. Synthesis of Compound C4-2

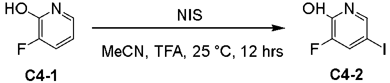

To a solution of Compound C4-1 (2.00 g, 17.7 mmol, 1.0 eq.) dissolved in MeCN (20.0 mL) and TFA (3.34 mL), NIS (4.38 g, 19.5 mmol, 1.1 eq.) was added, and the reaction mixture was stirred and reacted at 25 °C for 12 hours. TLC (petroleum ether: ethyl acetate = 0: 1, R1: R_{f}= 0.4; P1: R_{f}= 0.8) showed that C4-1 was completely consumed, and a new compound peak was generated. The reaction mixture was filtered to provide a crude product C4-2 (3.00 g, crude) as a white solid.

### 2. Synthesis of Compound C4-3

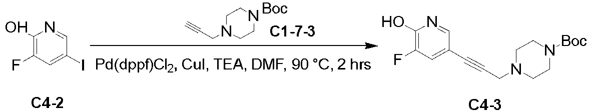

To a solution of Compound C4-2 (1.00 g, 4.18 mmol, 1.0 eq.) and C1-7-3 (1.41 g, 6.28 mmol, 1.5 eq.) dissolved in DMF (20.0 mL), CuI (39.9 mg, 209 µmol, 0.05 eq.), TEA (1.27 g, 12.6 mmol, 1.75 mL, 3.0 eq.) and Pd(dppf)Cl₂ (306 mg, 418 µmol, 0.1 eq.) were added, and the reaction mixture was stirred and reacted at 90°C for 2 hours. LCMS (EC13784-5-P1A, P1: RT = 0.242 minutes) showed that C4-2 was reacted completely, and a target product peak was generated. The reaction mixture was extracted by adding 20.0 mL of water and 60.0 mL of ethyl acetate. The resulting organic layer was washed with saturated saline (20.0 mL × 2), dried over Na₂SO₄, filtered and concentrated under reduced pressure to obtain a crude product C4-3 (500.0 mg) as a brown solid.

**LC-MS:** EC13784-5-P1A, product: RT = 0.242 min, m/z = 336.3 [M+H]⁺.

### 3. Synthesis of Compound C4-4

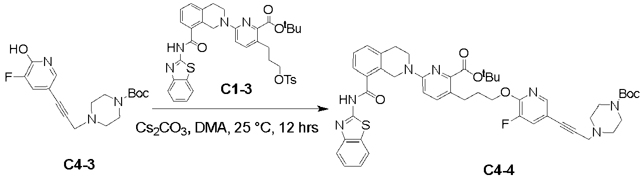

To a solution of Compound C4-3 (90.0 mg, 268 µmol, 1.0 eq.) dissolved in DMA (3.00 mL), Cs₂CO₃ (175 mg, 537 µmol, 2.0 eq.) and C1-3 (188 mg, 268 µmol, 1.0 eq.) were added, and the reaction mixture was stirred and reacted at 25 °C for 12 hours. LCMS (EC13784-6-P1A, P1: RT = 0.495 minutes) showed that Compound C4-3 was reacted completely, and a target product main peak with the expected m/z value was generated. TLC (petroleum ether: ethyl acetate = 1:2, R1: R_{f}= 0.1; R2: R_{f}= 0.85; P1: R_{f}= 0.55) showed that C4-3 was completely consumed, and two new compound peaks were generated. The reaction mixture was extracted by adding 10.0 mL of water and 10.0 mL of ethyl acetate. The resulting organic layer was washed with saturated saline (10.0 mL × 2), dried over Na₂SO₄, filtered and concentrated under reduced pressure to obtain a crude product. The crude product was subjected to TLC (petroleum ether: ethyl acetate = 1:2, P1: R_{f} = 0.55; P2: R_{f} = 0.30), and purified by column chromatography (SiO₂, petroleum ether: ethyl acetate = 1:2) to provide Compound C4-4 (55.0 mg) as a colorless oil.

**LC-MS:** EC13784-6-P1A, product: RT = 0.495 min, m/z = 862.6 [M+H]⁺.

### 4. Synthesis of Compound C4

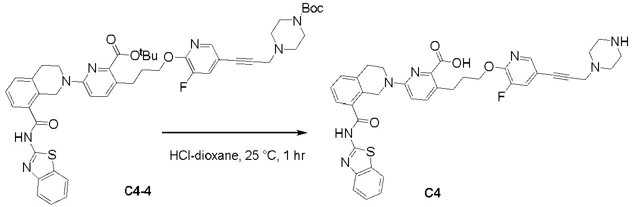

The mixture of Compound C4-4 (50.0 mg, 58.0 µmol, 1.0 eq.) dissolved in HCl-dioxacyclohexane (2.00 mL) was stirred and reacted at 25 °C for 1 hour. LCMS (EC13784-11-P1A2, P1: RT= 0.418 minutes) showed that Compound C4-4 was reacted completely, and a target product main peak with the expected m/z value was generated. The reaction mixture was filtered under reduced pressure to obtain a crude product, which was purified by Prep-HPLC column chromatography (chromatographic column: Welch Xtimate C18 150 * 25mm * 5µm; mobile phase: [water (HCl) -ACN]; gradient elution: 30%-60% B, 10 minutes) to provide Compound C4 (11.0 mg, 24.46% yield, 91.03% purity) as a colorless oil.
**LC-MS:** EC13784-11-P1A2, product: RT = 0.418 min, m/z = 706.3 [M+H]⁺
**HPLC:** EC13784-11-P1C; product: RT = 1.945 min
**¹H NMR:** EC13784-11-P1C1 (400 MHz DMSO) *δ* ppm 8.17 (d, *J =* 1.6 Hz, 1H), 8.02 (d, *J =* 7.6 Hz, 1H), 7.90 (dd, *J =* 10.8, 1.8 Hz, 1H), 7.78 (d, *J =* 8.0 Hz, 1H), 7.59 (t, *J =* 8.8 Hz, 2H), 7.44 - 7.50 (m, 1H), 7.40 - 7.44 (m, 1H), 7.32 - 7.38 (m, 2H), 7.02 (d, *J =* 8.8 Hz, 1H), 4.92 (s, 2H), 4.37 - 4.42 (m, 2H), 4.29 - 4.37 (m, 2H), 3.83 (t, *J =* 6.0 Hz, 3H), 3.57 (d, *J =* 4.0 Hz, 5H), 3.42 - 3.49 (m, 4H), 2.98 (t, *J =* 5.6 Hz, 2H), 2.81 (*t, J =* 7.6 Hz, 2H), 1.92 - 2.01 (m, 2H).

**Example 2. Synthesis of PROTAC molecules (AF3, AF4, BF3, BF4, APQ1, APQ2) with Compound C1 as the targeting ligand and recruiting the CRBN-E3 enzyme**

**a) Synthesis of PROTAC molecules AF3** 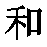 **AF4**

### Synthesis scheme:

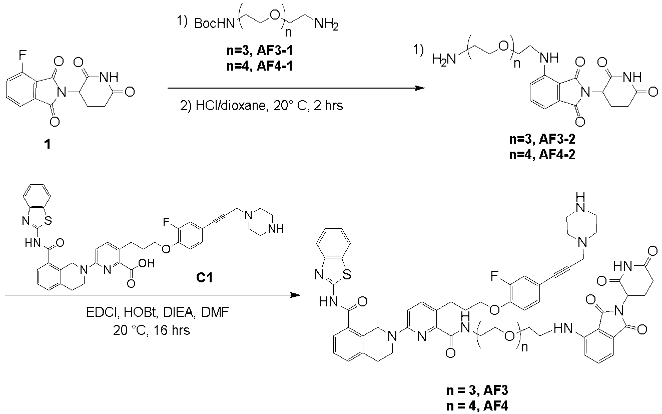

### Synthesis of AF3:

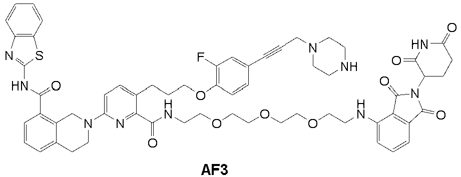

### 1. Synthesis of Compound AF3-2

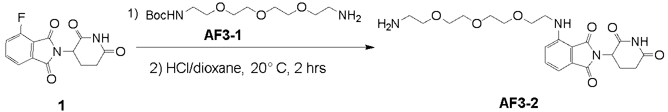

Compound AF3-1 (4.02 g, 13.7 mmol, 1.00 eq.), Compound 1 (3.80 g, 13.7 mmol, 1.00 eq.), and DIEA (3.56 g, 27.5 mmol, 4.79 mL, 2.00 eq.) were mixed in DMF (50.0 mL), and the air was removed and replaced with N₂, and the mixture was stirred and reacted at 90°C under N₂ atmosphere for 12 hours. LC-MS (EC11272-85-P1A, P1: RT = 0.415 minutes) showed that Compound 1 was completely reacted. To the reaction mixture, 50.0 mL of water was added at 0°C to quench the reaction, diluted with 50.0 mL of EA, and then extracted with 150.0 mL of EA (50.0 mL×3). The organic layer was washed with 50.0 mL of saturated saline, dried over Na₂SO₄, filtered and concentrated under reduced pressure to obtain a crude product, which was purified by column chromatography (SiO₂, petroleum ether: ethyl acetate = 3:1 to 0:1; when petroleum ether: ethyl acetate = 0:1, R_{f}= 0.24), then HCl/ dioxacyclohexane (4 M, 10.0 mL, 21.9 eq.) were added, and the air was removed and replaced with N₂. The mixture was stirred and reacted at 25°C under N₂ atmosphere for 2 hours, and then concentrated under reduced pressure to provide the crude product AF3-2 (500 mg, 1.11 mmol, 61.1% yield) as a yellow solid.

**LCMS:** EC11272-85-P1A, P1, RT = 0.415 min, M/Z (M+H⁺) = 449.2.

### 2. Synthesis of compound AF3

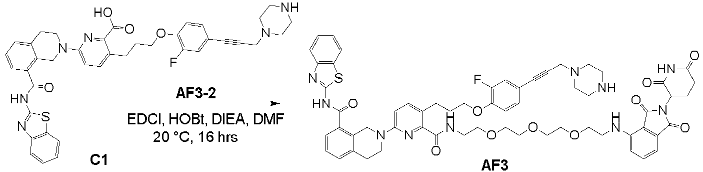

Compound C1 (50.0 mg, 67.4 µmol, 1.00 eq., HCl), AF3-2 (60.5 mg, 135 µmol, 2.00 eq.), EDCI (19.4 mg, 101 µmol, 1.50 eq.), HOBt (13.6 mg, 101 µmol, 1.50 eq.), and DIEA (43.6 mg, 337 µmol, 58.7 µL, 5.00 eq.) were mixed in DMF (1.0 mL), and the air was removed and replaced with N₂. The mixture was stirred and reacted at 20°C under N₂ atmosphere for 16 hours. LC-MS (EC13958-9-P1B1, P1: RT = 0.456 minutes) showed that C1 was completely reacted. The reaction mixture was concentrated under reduced pressure to obtain a crude product, which was purified by Prep-HPLC (neutral conditions) to provide compound AF3 (10 mg, 8.81 µmol, 13.0% yield) as a yellow solid.

**LCMS:** EC13958-9-P1B1, P1, RT = 0.456 min, M/Z (M+H⁺) = 1135.8
**HNMR:** EC13958-9-P1R *δ* ppm 8.42 (t, *J =* 5.6 Hz, 1H), 7.98 (d, *J=* 7.6 Hz, 1H), 7.74 (d, *J =* 8.0 Hz, 1H), 7.60 (d, *J =* 6.8 Hz, 1H), 7.46 - 7.56 (m, 2H), 7.40 - 7.46 (m, 1H), 7.21 - 7.37 (m, 4H), 7.15 (s, 1H), 7.05 - 7.12 (m, 2H), 6.95 - 7.03 (m, 2H), 6.56 (s, 1H), 5.03 (dd, *J =* 5.2, 12.8 Hz, 1H), 4.98 (s, 2H), 4.01 (t, *J* = 6.8 Hz, 2H), 3.86 (t, *J =* 5.6 Hz, 2H), 3.54 - 3.57 (m, 2H), 3.45 - 3.50 (m, 6H), 3.43 (d, *J =* 4.4 Hz, 8H), 3.39 (dd, *J= 8.4,* 5.75 Hz, 8H), 2.93 - 2.99 (m, 4H), 2.78 (br t, *J =* 4.8 Hz, 4H), 2.47 (s, 2H), 1.88 - 2.09 (m, 4H).

### Synthesis of AF4:

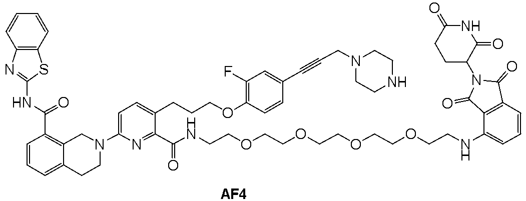

The synthesis procedure for the PROTAC molecule AF4 was similar to that for AF3, but the starting materials were replaced with Compound 1 and AF4-1, and the target product AF4 was obtained by purification after synthesis, as a yellow solid, with a yield of approximately 13%.

**LCMS:** EC13958-10-P1B1, P1, RT = 0.460 min, M/Z (M+H⁺) = 1180.3
**HNMR:** EC13958-10-P1R, 400 MHz, DMSO-*d*₆ *δ* ppm 8.39 - 8.46 (m, 1H), 7.97 (d, *J=* 8.0 Hz, 1H), 7.74 (d, *J =* 8.0 Hz, 1H), 7.58 - 7.62 (m, 1H), 7.52 - 7.58 (m, 1H), 7.49 (d, *J =* 8.8 Hz, 1H), 7.43 (t, *J =* 7.2 Hz, 1H), 7.23 - 7.37 (m, 4H), 7.14 - 7.19 (m, 1H), 7.06 - 7.13 (m, 2H), 6.94 - 7.03 (m, 2H), 6.57 (t, *J=* 5.6 Hz, 1H), 5.04 (dd, *J =* 12.8, 5.6 Hz, 1H), 4.99 (s, 2H), 4.01 (t, *J =* 6.4 Hz, 2H), 3.86 (t, *J=* 6.0 Hz, 2H), 3.55 - 3.59 (m, 2H), 3.49 - 3.51 (m, 2H), 3.45 - 3.48 (m, 4H), 3.43 (s, 8H), 3.42 (s, 4H), 3.37 - 3.41 (m, 6H), 2.93 - 2.99 (m, 4H), 2.76 (t, *J =* 4.8 Hz, 4H), 2.46 (d, *J =* 4.4 Hz, 4H), 1.90 - 2.06 (m, 4H).

**b) Synthesis of PROTAC molecules BF3** 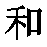 **BF4**

### Synthesis scheme:

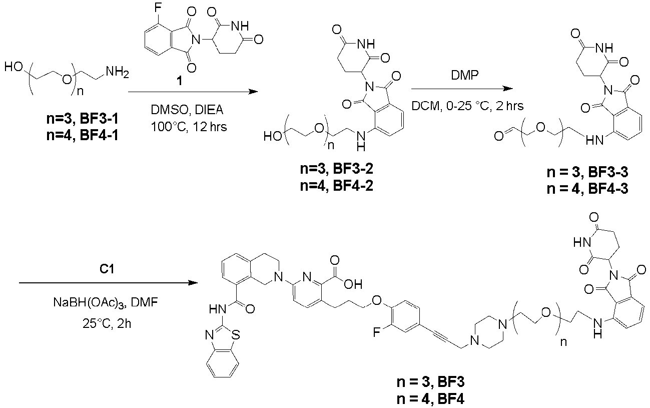

### Synthesis of BF3:

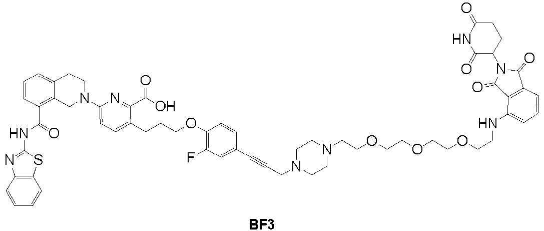

### 1. Synthesis of Compound BF3-2

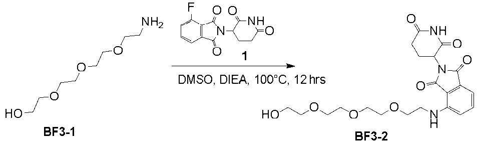

Compound BF3-1 (4.09 g, 21.1 mmol, 1.50 eq.), Compound 1 (3.90 g, 14.1 mmol, 1.00 eq.), and DIEA (10.9 g, 84.7 mmol, 14.7 mL, 6.00 eq.) were mixed in DMSO (40.0 mL), and the air was removed and replaced with N₂. The mixture was stirred and reacted at 100°C under N₂ atmosphere for 12 hours. LC-MS (EC13798-1-p1a1, P1 RT = 0.312 minutes) showed that a product with the target molecular weight was yielded. To the reaction mixture, 300.0 mL of water was added at 25°C to quench the reaction, diluted with 100.0 mL of DCM, and then extracted with DCM (100.0 mL×5). The organic layer was washed with 50.0 mL of saturated saline, dried over Na₂SO₄, filtered and concentrated under reduced pressure to obtain a crude product, which was purified by column chromatography (SiO₂, petroleum ether: ethyl acetate = 5:1 to 0:1; when petroleum ether: ethyl acetate = 0:1, R_{f}= 0.20) to provide the crude product BF3-2 (5.00 g, 11.1 mmol, 78.7% yield) as a green oil.

**LCMS:** EC13798-1-p1a1, P1 RT = 0.312 min, MS (ESI) *m*/*z* = 450.4 [M+H]⁺.

### 2. Synthesis of Compound BF3-3

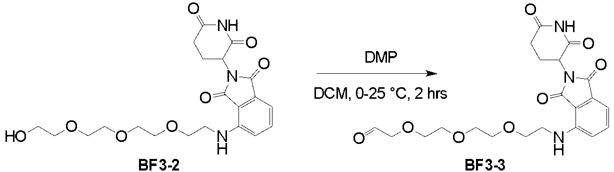

To a solution of Compound BF3-2 (200 mg, 444 µmol, 1.00 eq.) dissolved in DCM (2.00 mL), DMP (377.47 mg, 889.97 µmol, 275.73 µL, 2.00 eq.) was added at 0 °C, and the mixture was stirred and reacted at 25°C for 2 hours. LC-MS (EC13798-4-p1a1, P1 RT = 0.318 minutes) showed that a product with the target molecular weight was yielded. To the reaction mixture, saturated NaHCO₃ (10.0 mL) and saturated Na₂S₂O₃ (10.0 mL) were added at 25°C to quench the reaction, and stirred for another 30.0 minutes, and then extracted with DCM (20.0 mL × 3). The organic layer was washed with saturated saline, dried over Na₂SO₄, filtered and concentrated under reduced pressure to obtain a crude product BF3-3 (200 mg) as a yellow oil.

**LCMS:** EC13798-4-p1a1, P1 RT = 0.318 min, MS (ESI) *m*/*z* = 448.3 [M+H]⁺.

### 3. Synthesis of Compound BF3

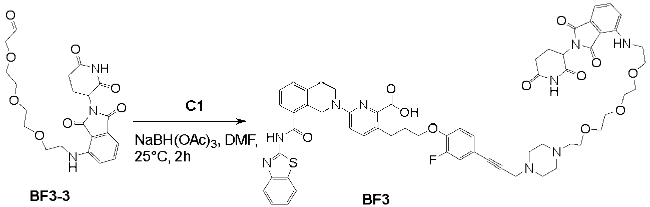

To a solution of Compound BF3-3 (19.0 mg, 42.5 µmol, 1.00 eq.) dissolved in DMF (2.00 mL), Compound C1 (30.0 mg, 42.5 µmol, 1.00 eq.) and NaBH(OAc)₃ (27.0 mg, 127 µmol, 3.00 eq.) were added, and the mixture was stirred and reacted at 25°C for 2 hours. LC-MS (EC13798-17-p1c8, P1 RT = 0.453minutes) showed that a product with the target molecular weight was yielded. The reaction mixture was concentrated under reduced pressure to obtain a crude product, which was purified by Prep-HPLC (under TFA conditions; chromatographic column: Phenomenex luna C18 150*25.0 mm* 10.0 µm; mobile phase: [water (TFA)-ACN]; gradient: 36.0% - 66.0% B, within 15.0 minutes) to provide Compound BF3 (10.0 mg, 8.27 µmol, 19.4% yield, 94.0% purity) as a yellow solid.

**LCMS:** EC13798-17-p1c8, RT = 0.453 min, MS (ESI) *m*/*z* = 1136.4 [M+H]⁺
**¹H NMR:** EC13798-17-pla2, 400 MHz, DMSO+D2O *δ* 8.02 (d, *J =* 7.6 Hz, 1H), 7.77 (d, *J =* 8.0 Hz, 1H), 7.52 - 7.61 (m, 3H), 7.47 (t, *J =* 8.0 Hz, 1H), 7.38 - 7.43 (m, 1H), 7.35 (m, 2 H), 7.26 (dd, *J =* 12.0, 1.6 Hz, 1H), 7.18 (d, *J =* 8.8 Hz, 1H), 7.05 - 7.12 (m, 2H), 7.03 (d, *J =* 7.2 Hz, 1H), 6.96 (d, *J =* 8.4 Hz, 1H), 5.03 (dd, *J =* 13.2, 5.6 Hz, 1H), 4.87 - 4.95 (m, 2H), 4.01 (t, *J =* 6.0 Hz, 2H), 3.84 (t, *J =* 5.6 Hz, 2H), 3.69 - 3.73 (m, 2H), 3.64 (s, 2H), 3.60 (s, 4H), 3.43 (t, *J =* 5.2 Hz, 3H), 3.26 (s, 2H), 2.97 (t, *J =* 5.6 Hz, 2H), 2.84 - 2.91 (m, 2H), 2.77 - 2.83 (m, 3H), 2.55 - 2.63 (m, 2H), 1.88 - 2.06 (m, 4H).

### Synthesis of BF4:

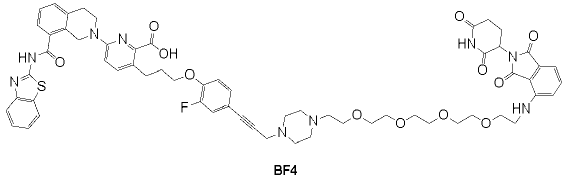

The synthesis procedure for the PROTAC molecule BF4 was similar to that for BF3, but the starting materials were replaced with Compound 1 and BF4-1, and the target product BF4 was obtained by purification after synthesis, as a yellow solid, with a purity of 94.1 % and a yield of approximately 16%.
**LCMS:** EC13798-11-pla2, RT = 0.456 min, MS (ESI) *m*/*z* = 1181.8 [M+H]⁺
**¹H NMR:** EC13798-11-pla3, 400 MHz, DMSO+D₂O *δ* 8.02 (d, *J =* 8.0 Hz, 1H), 7.78 (d, *J =* 7.6 Hz, 1H), 7.52 - 7.61 (m, 3H), 7.45 - 7.50 (m, 1H), 7.39 - 7.42 (m, 1H), 7.32 - 7.38 (m, 2H), 7.28 (dd, *J =* 11.6, 2.0 Hz, 1H), 7.19 (d, *J* = 9.2 Hz, 1H), 7.10 (d, *J =* 8.4 Hz, 2H), 7.03 (d, *J =* 6.8 Hz, 1H), 6.96 (d, *J =* 8.8 Hz, 1H), 5.03 (dd, *J =* 13.2, 6.0 Hz, 1H), 4.92 (s, 2H), 4.01 (t, *J =* 6.40 Hz, 2H), 3.83 (t, *J =* 6.00 Hz, 2H), 3.72 (s, 4H), 3.45 - 3.56 (m, 16H), 3.41 - 3.45 (m, 3H), 3.25 - 3.30 (m, 3H), 2.76 - 3.01 (m, 10H), 2.52 - 2.63 (m, 2H), 1.95 (m, 4H).

### c) Synthesis of PROTAC molecules APQ1

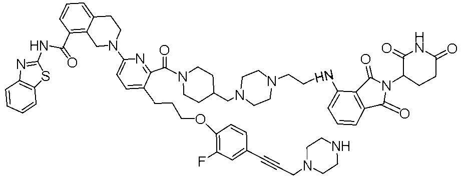

### Synthesis scheme:

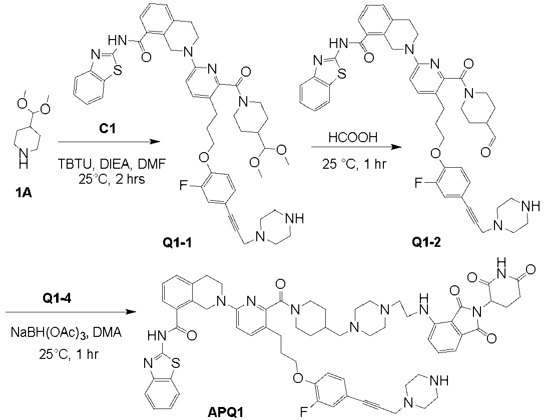

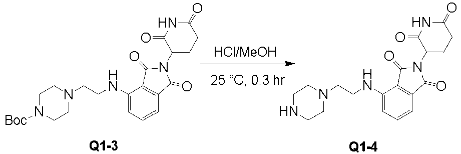

### 1. Synthesis of Q1-1

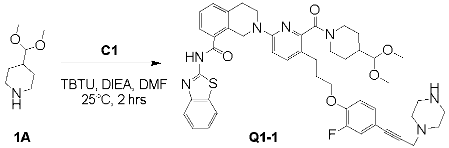

To a solution of Compound 1A (451 mg, 2.84 mmol, 4.00 eq.) and C1 (0.500 g, 709 µmol, 1.00 eq.) in DMF (5.00 mL), TBTU (455 mg, 1.42 mmol, 2.00 eq.) and DIEA (458 mg, 3.55 mmol, 617 mL, 5.00 eq.) were added, and the mixture was stirred and reacted at 25°C for 2 hours. To the reaction mixture, water (10.0 mL) was added to quench the reaction, diluted with DCM (10.0 mL), and then extracted with DCM (30.0 mL, 10.0 mL×3). The resulting organic layer was washed with saturated saline (20.0 mL, 10.0 mL×2), dried over Na₂SO₄, filtered and concentrated under reduced pressure to obtain a crude product, which was further purified by Prep-HPLC (chromatographic column: Phenomenex Luna C 18 150 * 25 mm * 10 µm; mobile phase: [water (FA)-ACN]; gradient elution: 30% - 60% B, within 10 minutes) to provide the product Q1-1 (90.0 mg, 106 µmol, 15.0% yield) as a white solid.

**LCMS:** EC13798-52-pla2, P1 RT = 0.426 min, MS (ESI) *m*/*z* = 846.4 [M+H]⁺.

### 2. Synthesis of Q1-2

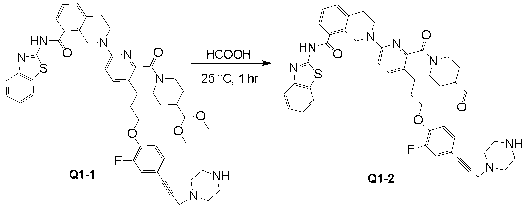

To Compound Q1-1 (30.0 mg, 35.4 µmol, 1.00 eq.), formic acid (2.00 mL) was added, and the mixture was stirred and reacted at 25°C for 1 hour. The reaction mixture was concentrated under reduced pressure to obtain a crude product Q1-2 (25.0 mg, 31.2 µmol, 88.1% yield) as a yellow solid, which was directly used in the next reaction without purification.

**LCMS:** EC13798-58-p1a1, P1 RT = 0.394 min, MS (ESI) *m*/*z* = 800.5 [M+H]⁺.

### 3. Synthesis of Q1-4

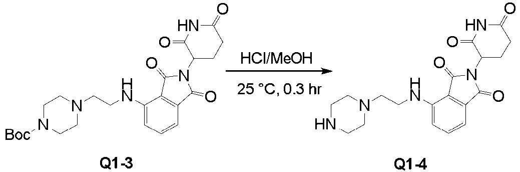

To Compound Q1-3 (0.500 g, 1.03 mmol, 1.00 eq.), HCl/methanol (4.00 M, 12.5 mL, 48.5 eq.) was added, and the mixture was stirred and reacted at 25°C for 0.3 hours. LCMS (P1 RT = 0.162 minutes) showed that a product with the target molecular weight was yielded. The reaction mixture was concentrated under reduced pressure to obtain the product Q1-4 (400 mg, crude product) as a yellow solid, which was directly used in the next reaction without purification.

**LCMS:** EC13798-46-p1a1, P1 RT = 0.162 min, MS (ESI) *m*/*z* = 386.3 [M+H]⁺.

### 4. Synthesis of APQ1

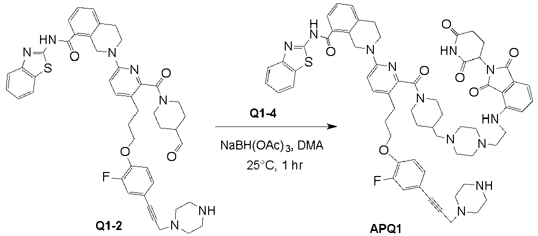

To a solution of Compound Q1-2 (25.0 mg, 31.2 µmol, 1.00 eq.) and Q1-4 (12.0 mg, 31.2 µmol, 1.00 eq.) in DMA (0.50 mL), NaBH(OAc)₃ (19.8 mg, 93.7 µmol, 3.00 eq.) was added, and the mixture was stirred and reacted at 25°C for 1 hour. LCMS (P1 RT = 0.375 minutes) showed that a product with the target molecular weight was yielded. To the reaction mixture, water (10.0 mL) was added at 25°C to quench the reaction, diluted with DCM (10.0 mL), and then extracted with DCM (30.0 mL, 10.0 mL×3). The resulting organic layer was washed with saturated saline (30.0 mL, 10.0 mL×3), dried over Na₂SO₄, filtered and concentrated under reduced pressure to obtain a crude product, which was further purified by Prep-HPLC (chromatographic column: Phenomenex luna C 18 150 * 25 mm * 10 µm; mobile phase: [water (HCl)-ACN]; gradient elution: 27% - 47% B, within 10.0 minutes) to provide the product APQ1 (1.00 mg, crude) as a yellow solid.

**LCMS:** EC13798-59-plc5, P1 RT = 0.375 min, MS (ESI) *m*/*z* = 585.7 [M/2+H]⁺
**HPLC:** EC13798-59-plc7, P1 RT = 2.385 min, 61.0% purity.

### d) Synthesis of PROTAC molecule APQ2

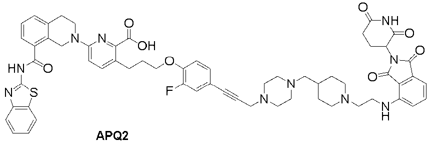

### Synthesis scheme:

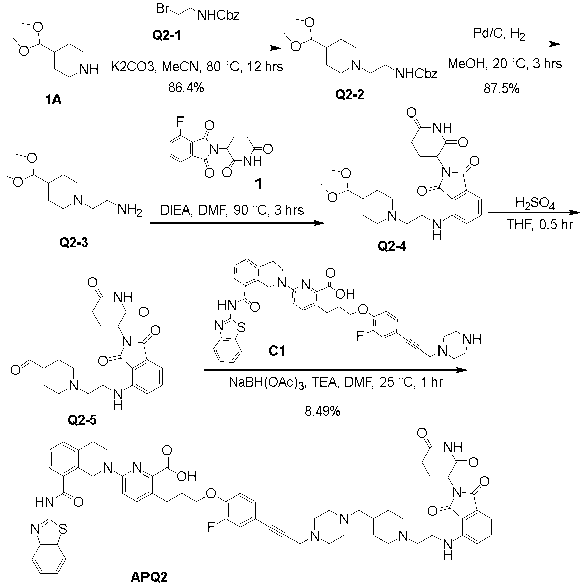

### 1. Synthesis of Compound Q2-2

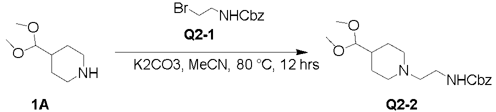

To a solution of Compound 1A (2.00 g, 12.5 mmol, 1.00 eq.) dissolved in CH₃CN (40.0 mL), K₂CO₃ (5.21 g, 37.7 mmol, 3.00 eq.) and Compound Q2-1 (3.24 g, 12.5 mmol, 1.00 eq.) were added, and the mixture was stirred and reacted at 80°C for 12 hours. LC-MS (EC13784-1-p1a, P1 RT = 0.263 minutes) showed that Q2-1 was reacted completely, and a product with the target molecular weight was yielded. The mixture was extracted by adding 40.0 mL of water and 40.0 mL of ethyl acetate. The resulting organic layer was washed with saturated saline (30.0 mL×2), dried over Na₂SO₄, filtered and concentrated under reduced pressure to obtain a crude product, which was purified by reverse-phase HPLC (0.1% FA condition) to provide Q2-2 (2.30 g, 5.91 mmol, 47.0% yield, 86.4% purity) as a colorless oil.

**LC-MS:** EC13784-1-pla, product: RT = 0.263 min, m/z = 337.5 [M+H]⁺
**¹H NMR:** EC13784-1-p1d (400 MHz DMSO) *δ* 8.19 (s, 1H), 7.26 - 7.42 (m, 5H), 7.05 - 7.24 (m, 1H), 5.01 (s, 3H), 4.02 (d, *J =* 6.8 Hz, 1H), 3.24 (s, 6H), 3.05 - 3.18 (m, 2H), 2.90 (d, *J=* 10.8 Hz, 2H), 2.33 - 2.45 (m, 2H), 1.96 (t, *J =* 11.6 Hz, 2H), 1.44 - 1.66 (m, 3H), 1.12 - 1.32 (m, 2H).

### 2. Synthesis of Compound Q2-3

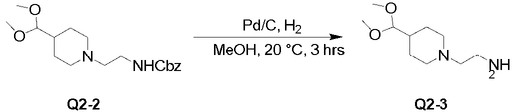

To a solution of Compound Q2-2 (1.90 g, 5.65 mmol, 1.0 eq.) dissolved in MeOH (60.0 mL), Pd-C (190 mg, 10% purity) was added, and the mixture was hydrogenated at 20°C under 50 Psi H₂ for 12 hours. LC-MS (EC13784-4-p1a, 0.446 minutes) showed that Q2-2 was reacted completely, and a product with the target molecular weight was yielded. The reaction mixture was concentrated under reduced pressure to obtain a crude product, which did not require purification. The crude product Q2-3 (1.00 g, 4.94 mmol, 87.53% yield) was a colorless oil.

**LC-MS:** EC13784-4-pla, product: RT = 0.446 min, m/z = 203.6 [M+H]⁺.

### 3. Synthesis of Compound Q2-4

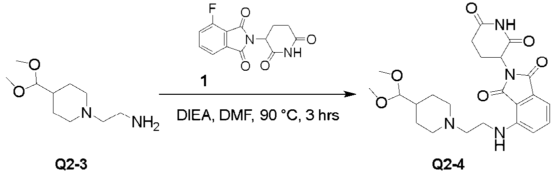

Compound Q2-3 (1.23 g, 4.45 mmol, 1.0 eq.), Compound 1 (1.23 g, 4.45 mmol, 1.0 eq.), and DIEA (1.15 g, 8.90 mmol, 1.55 mL, 2.0 eq.) were mixed in DMF (20.0 mL), and the air was removed and replaced with N₂. The mixture was stirred and reacted at 90°C under N₂ atmosphere for 3 hours. LC-MS (EC13784-14-p1a1, P1: RT = 0.252 minutes) showed that Q2-3 was reacted completely, and a product peak with the target molecular weight was generated. The reaction mixture was extracted by adding 30.0 mL of water and 90.0 mL of ethyl acetate. The resulting organic layer was washed with saturated saline (40.0 mL×2), dried over Na₂SO₄, filtered and concentrated under reduced pressure to obtain a crude product. Upon TLC detection (dichloromethane: methanol =10:1, P1: RT = 0.46), the crude product was purified by column chromatography (SiO2, DCM: MeOH=10:1) to provide Q2-4 (300 mg, crude) as a green oil.

**LC-MS:** EC13784-14-p1a1, product: RT = 0.252 min, m/z = 459.2 [M+H]⁺.

### 4. Synthesis of Compound Q2-5

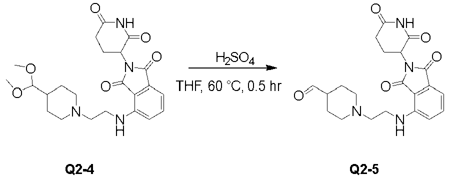

To a solution of Compound Q2-4 (80.0 mg, 174 µmol, 1.00 eq.) dissolved in THF (5.00 mL), H₂SO₄ (34.2 mg, 348 µmol, 18.6 µL, 2.00 eq.) was added, and the mixture was stirred and reacted at 60°C for 0.5 hours. LC-MS (EC13798-21-p1a1, P1 RT = 0.214 minutes) showed that a product with the target molecular weight was yielded. The reaction mixture was concentrated under reduced pressure to obtain a crude product, which did not require purification. The crude product Q2-5 (100 mg) was a yellow oil.

**LCMS:** EC13798-21-p1a1, P1 RT = 0.214 min, MS (ESI) *m*/*z* = 413.3 [M+H]⁺.

### 5. Synthesis of Compound APQ2

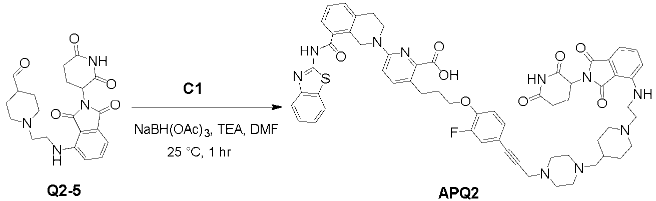

To a solution of Compound Q2-5 (100 mg, 121 µmol, 1.00 eq.) and C1 (85.4 mg, 121 µmol, 1.00 eq.) dissolved in DCM (5.00 mL), TEA (12.2 mg, 121 µmol, 16.8 µL, 1.00 eq.) was added dropwise over 5 minutes at 25°C, then NaBH(OAc)₃ (77.0 mg, 363 µmol, 3.00 eq.) was added at 25°C, and the mixture was stirred and reacted at 25°C for 1 hour. LC-MS (EC13798-22-p1c4, P1 RT = 1.825 minutes) showed that a product with the target molecular weight was yielded. The reaction mixture was concentrated under reduced pressure to obtain a crude product, which was further purified by Prep-HPLC (under TFA conditions; column: phenomenex luna C 18 150 * 25.0 mm* 10.0 µm; mobile phase: [water (TFA)-ACN]; gradient: 32%-62% B, whithin 10 minutes) to provide APQ2 (12.2 mg, 10.2 µmol, 8.49% yield, 92.9% purity) as a yellow solid.

**LCMS:** EC13798-22-plc4, P1 RT = 1.825 min, MS (ESI) *m*/*z* = 1102.8 [M+H]⁺
**¹H NMR:** EC13798-22-p1c1, 400 MHz, DMSO *δ* 12.83 (s, 1H), 11.10 (s, 1H), 9.40 (s, 1H), 8.04 (d, *J* = 8.0 Hz, 1H), 7.80 (d, *J =* 8.0 Hz, 1H), 7.55 - 7.65 (m, 3H), 7.42 - 7.49 (m, 1H), 7.37 - 7.42 (m, 1H), 7.29 - 7.37 (m, 3H), 7.16 - 7.24 (m, 2H), 7.09 - 7.16 (m, 2H), 6.97 (d, *J =* 8.8 Hz, 1H), 6.87 (t, *J =* 6.40 Hz, 1H), 5.07 (dd, *J =* 12.8, 5.2 Hz, 1H), 4.93 (s, 2H), 4.04 (t*, J =* 6.4 Hz, 2H), 3.86 (t*, J =* 6.4 Hz, 2H), 3.62 - 3.79 (m, 6H), 3.53 (s, 1H), 3.18 - 3.39 (m, 4H), 2.85 - 3.07 (m, 10H), 2.69 - 2.84 (m, 4H), 2.56 (m, 2H), 1.92 - 2.06 (m, 6H), 1.35 - 1.51 (m, 2H).

### e) Synthesis of PROTAC molecule APQ4

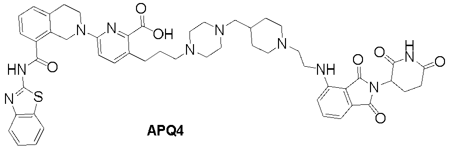

### Synthesis scheme:

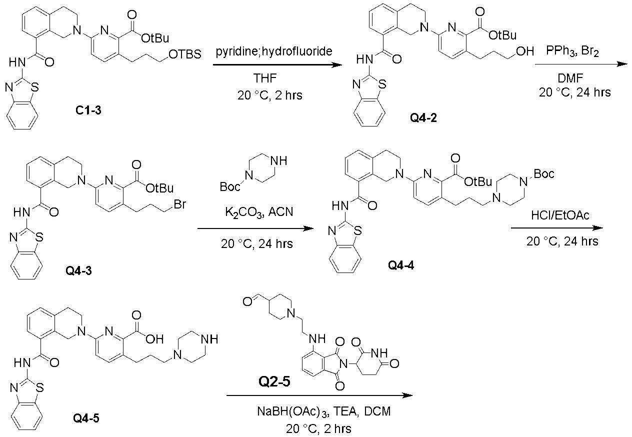

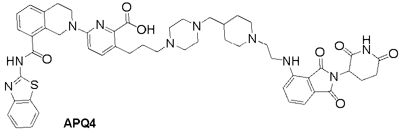

### 1. Synthesis of Compound Q4-2

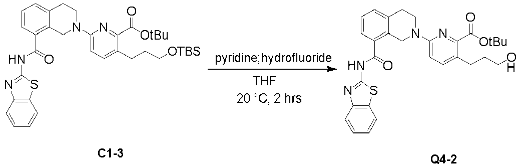

To a mixed solution of Compound C1-3 (1.00 g, 1.52 mmol, 1.00 eq.) in THF (15.0 mL), pyridine·HF (694 mg, 4.55 mmol, 631 µL, 65% purity, 3.00 eq.) was added, and the mixture was stirred and reacted at 20 °C for 2 hours. LC-MS (EC7111-187-pla, P1: RT = 0.464 minutes) showed that Compound C1-3 was completely reacted, and multiple new compound peaks were generated, where the product with the target m/z accounted for approximately 89.8%. The mixture was concentrated under reduced pressure to obtain a crude product, which was further purified by column chromatography (SiO₂, petroleum ether: ethyl acetate = 1:0 to 1:2) to provide Compound Q4-2 (760 mg, 1.31 mmol, 86.4% yield, 94.0% purity) as a white solid.

**LCMS:** EC7111-187-pla, P1: RT = 0.464 min, MS (M+H⁺) = 545.5.

### 2. Synthesis of Compound Q4-3

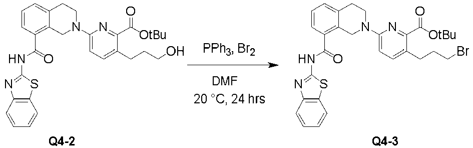

To a mixed solution of Compound Q4-2 (300 mg, 551 µmol, 1.00 eq.) in DMF (3.00 mL), PPh₃ (289 mg, 1.10 mmol, 2.00 eq.) and Br₂ (132 mg, 826 µmol, 42.6 µL, 1.50 eq.) were added, and the mixture was stirred and reacted at 20 °C for 24 hours. LC-MS (EC7111-195-pla, R1: RT = 0.425 min, P1: RT = 0.511 minutes) showed that approximately 4.73% of Compound Q4-2 was remained, and multiple new compound peaks were generated, where the product with the target m/z accounted for approximately 48.7%. To the reaction mixture, NaHCO₃ 50.0 mL was added at 20°C to quench the reaction, and extracted with ethyl acetate 60.0 mL (20.0 mL×3). The resulting organic layer was dried over Na₂SO₄, filtered and concentrated under reduced pressure to obtain a crude product, which was further purified by column chromatography (SiO₂, petroleum ether: ethyl acetate = 1:0 to 3:1) to provide Compound Q4-3 (220 mg, 347 µmol, 62.9% yield, 95.8% purity) as a white solid.

**LCMS:** EC7111-195-pla, P1: RT = 0.511 min, MS (M+H⁺) = 609.3.

### 3. Synthesis of Compound Q4-4

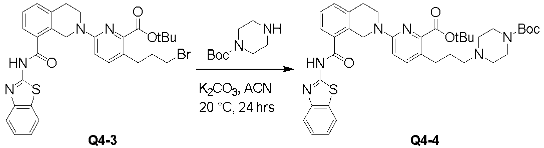

To a mixed solution of Compound Q4-3 (210 mg, 345 µmol, 1.00 eq.) in ACN (3.00 mL), tert-butyl piperazine-1-carboxylate hydrochloride (154 mg, 691 µmol, 2.00 eq.) and K₂CO₃ (239 mg, 1.73 mmol, 5.00 eq.) were added, and the mixture was stirred and reacted at 20 °C for 24 hours. LC-MS (EC7111-201-p1e, R1: RT = 0.481 min, P1: RT = 0.418 minutes) showed that approximately 4.11% of Compound Q4-3 was remained, and the product with the target m/z accounted for approximately 69.4%. The mixture was filtered and concentrated under reduced pressure to obtain a crude product (200 mg) as a yellow solid, which was directly used in the next reaction without purification.

**LCMS:** EC7111-201-ple, P1: RT = 0.418 min, MS (M+H⁺) = 713.3.

### 4. Synthesis of Compound Q4-5

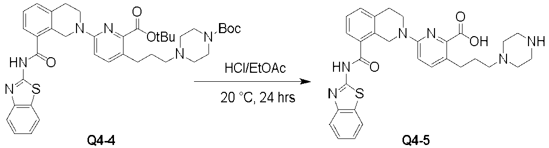

Compound Q4-4 (200 mg, 281 µmol, 1.00 eq.) was mixed in HCl/EtOAc (3.00 mL), and the mixture was stirred and reacted at 20 °C under N₂ atmosphere for 24 hours. LC-MS (EC15303-1-p1b, P1: RT = 0.311 minutes) showed that Compound Q4-4 was reacted completely, and multiple new compound peaks were generated, where the product with the target m/z accounted for approximately 58.7%. The mixture was filtered and concentrated under reduced pressure to provide Compound Q4-5 (crude, 250 mg) as a yellow solid, which was directly used in the next reaction without purification.

**LCMS:** EC15303-1-plb, P1: RT = 0.311 min, MS (M+H⁺) = 557.2.

### 5. Synthesis of Compound APQ4

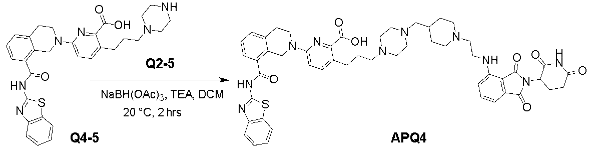

Compound Q4-5 (130 mg, 233 µmol, 1.00 eq.), Compound Q2-5 (96.3 mg, 233 µmol, 1.00 eq.), and TEA (189 mg, 1.87 mmol, 260 µL, 8.00 eq.) were mixed in DCM (2.00 mL), and the mixture was stirred and reacted at 20 °C for 15 minutes. Then, to the mixture, NaBH(OAc)₃ (148 mg, 700 µmol, 3.00 eq.) was added, and the mixture was stirred and reacted at 20 °C under N₂ atmosphere for 1.75 hours. LC-MS (EC15303-3-P1A, P1: RT = 0.313 minutes) showed that Compound Q4-5 was reacted completely, and multiple new compound peaks were generated, where the product with the target m/z accounted for approximately 53.7%. The reaction mixture was filtered and concentrated under reduced pressure to obtain a crude product, which was further purified by Prep-HPLC (FA conditions: chromatographic column: Welch Ultimate C18 150 * 25 mm * 5 µm; mobile phase: [water (FA) - ACN]; gradient elution: 10% - 40% B, within 11 minutes) to provide Compound APQ4 (11.0 mg, 11.3 µmol, 4.86% yield, 98.3% purity) as a white solid.

**LCMS:** EC15303-3-P1A, P1: RT = 0.313 min, MS (M+H⁺) = 477.7
**¹H NMR:** EC15303-3-pla (400 MHz, DMSO-*d*₆) *δ* 11.09 (s, 1H), 8.17 (s, 1H), 8.03 (d, *J =* 7.6 Hz, 1H), 7.79 (d, *J* = 8.2 Hz, 1H), 7.55 - 7.63 (m, 2H), 7.44 - 7.51 (m, 2H), 7.38 - 7.42 (m, 1H), 7.31 - 7.38 (m, 2H), 7.08 (d, *J =* 8.8 Hz, 1H), 7.02 (d, *J =* 7.2 Hz, 1H), 6.84 (d, *J =* 8.8 Hz, 1H), 6.75 (d, *J =* 3.2 Hz, 1H), 6.40 - 6.64 (m, 1H), 5.06 (dd, *J =* 12.8, 5.2 Hz, 1H), 4.89 (s, 2H), 3.84 (t, *J =* 6.0 Hz, 3H), 2.97 (t, *J =* 5.6 Hz, 3H), 2.86 (d, *J=* 11.6 Hz, 3H), 2.54 - 2.64 (m, 8H), 2.31 - 2.43 (m, 7H), 2.10 (d, *J =* 6.8 Hz, 2H), 1.88 - 2.06 (m, 4H), 1.72 - 1.79 (m, 2H), 1.61 - 1.68 (m, 2H), 1.40 - 1.50 (m, 1H), 1.03 - 1.15 (m, 2H).

### Example 3. Synthesis of PROTAC molecules (APQ3 and APL-1 to APL-6) with Compound C1 as a targeting ligand and recruiting VHL-E3 enzyme or IAP-E3 enzyme

### a) Synthesis of PROTAC molecule APQ3 containing a ligand structure for recruiting VHL-E3 enzyme

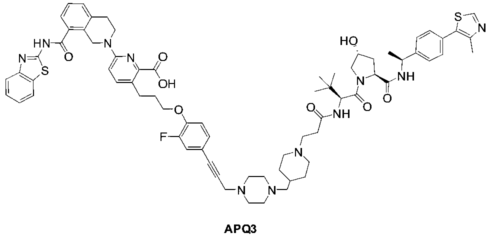

### Synthesis scheme:

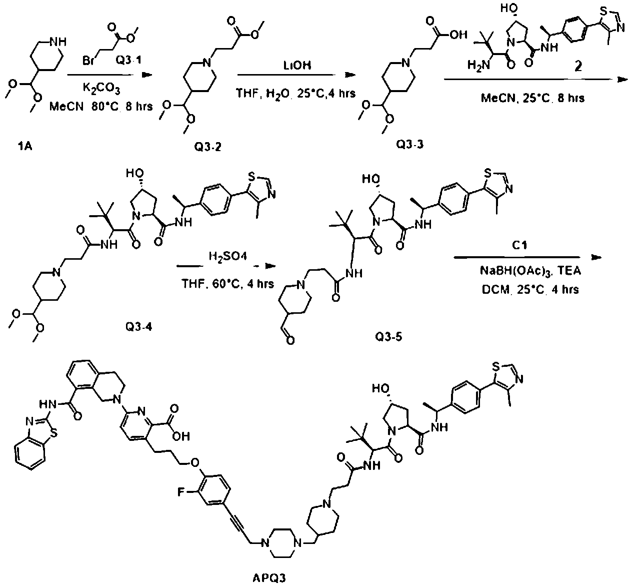

### 1. Synthesis of Compound Q3-2

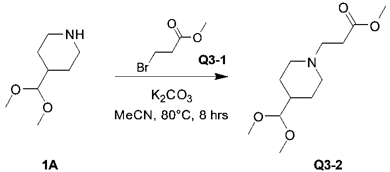

To a mixed solution of Compound 1A (1.00 g, 6.28 mmol, 1.00 eq.) and methyl 3-bromopropanoate (1.05 g, 6.28 mmol, 687 µL, 1.00 eq.) in MeCN (15.0 mL), K₂CO₃ (2.60 g, 18.8 mmol, 3.00 eq.) was added, and the mixture was stirred and reacted at 80°C for 8 hours. TLC (DCM/MeOH=10:1, R1: R_{f}= 0.10, P1: Rf = 0.75) showed that Compound 1A was reacted completely, and multiple new products were generated. The reaction mixture was filtered and concentrated under reduced pressure to obtain a crude product, which was purified by column chromatography (SiO₂, DCM/MeOH=100/1 to 0/1, P1: R_{f}= 0.75) to provide Compound Q3-2 (1.15 g, 4.69 mmol, 74.6% yield) as a white solid.

**¹H NMR:** EC12156-18-p1a1, DMSO, 400 MHZ δ 4.01 - 4.04 (m, 1H), 3.58 (s, 3H), 3.23 (s, 6H), 2.80 (d, *J = 11.2* Hz, 2H), 2.50 - 2.54 (m, 2H), 2.44 (d, *J =* 6.0 Hz, 2H), 1.99 (s, 2H), 1.83 (td, *J =* 11.6, 2.0 Hz, 2H), 1.56 (d, *J* = 13.2 Hz, 2H), 1.50 - 1.46 (m, 1H), 1.13 - 1.18 (m, 3H).

### 2. Synthesis of Compound Q3-3

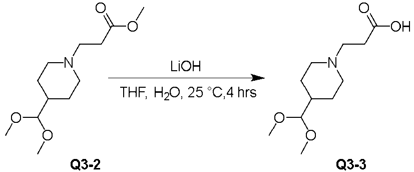

To a mixed solution of Compound Q3-2 (1.10 g, 4.48 mmol, 1.00 eq.) in THF (4.00 mL), LiOH·H₂O (564 mg, 13.4 mmol, 3.00 eq.) and H₂O (4.00 mL) were added, and the mixture was stirred and reacted at 25°C for 4 hours. TLC (DCM: MeOH=10:1, R1: R_{f} = 0.63, P1: R_{f}= 0.17) showed that Compound Q3-2 was reacted completely, and a compound with a single main peak was generated. The reaction mixture was concentrated under reduced pressure to remove the solvent, without purification, to obtain crude Compound Q3-3 (2.20 g) as a white solid.

**¹H NMR:** EC12156-19-p1a1, D₂O, 400 MHZ δ 4.16 - 4.26 (m, 1H), 3.36 (s, 6H), 2.89 (d, *J =* 11.6 Hz, 2H), 2.53 - 2.65 (m, 2H), 2.33 (dd, *J =* 8.8, 6.8 Hz, 2H), 2.01 (t, *J =* 11.2 Hz, 2H), 1.66 - 1.71 (m, 2H), 1.58 - 1.64 (m, 1H), 1.15 - 1.34 (m, 2H).

### 3. Synthesis of Compound Q3-4

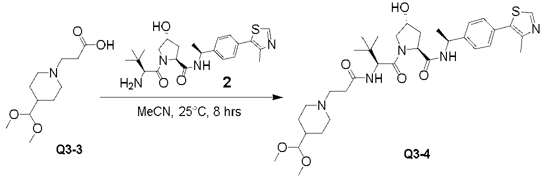

To a mixed solution of Compound 2 (384 mg, 864 µmol, 1.00 eq.) and Compound Q3-3 (200 mg, 864.72 µmol, 1.00 eq.) in CH₃CN (3.00 mL), HOBt (116 mg, 864 µmol, 1.00 eq.), Et₃N (350 mg, 3.46 mmol, 481 µL, 4.00 eq.), and BTU (327 mg, 864 µmol, 1.00 eq.) were added, and the mixture was stirred and reacted at 25 °C for 8 hours. LCMS (EC12156-24-pla2, P1: RT = 0.299 minutes) showed that Compound Q3-3 was reacted completely, and a main peak with the target m/z (m/z = 658.6[M+H]⁺) was generated, which accounted for approximately 61.5%. The reaction mixture was concentrated under reduced pressure to remove the solvent, and was further purified by Prep-TLC (SiO₂, DCM: MeOH=7:1, P1: R_{f}= 0.23) to provide Compound Q3-4 (179 mg, 272 µmol, 31.4% yield) as a white solid.

**LCMS:** EC12156-24-pla2, P1: RT = 0.299 min, MS (ESI) *m*/*z* = 658.6 [M+H]⁺.

### 4. Synthesis of Compound Q3-5

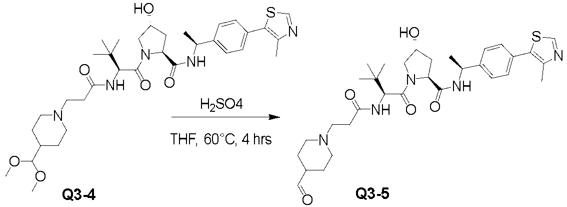

To Compound Q3-4 (50.0 mg, 76.0 µmol, 1.00 eq.), H₂SO₄ (14.9 mg, 152 µmol, 8.10 µL, 2.00 eq.) and THF (2.00 mL) were added, and the mixture was stirred and reacted at 60°C for 4 hours. LCMS (EC12156-30-p1a1, P1: RT = 0.298 minutes) showed that Compound Q3-4 was reacted completely, and a new main peak was generated, which accounted for approximately 89.4%, and the m/z was consistent with the target product (m/ z = 612.5 [M+H]⁺). The reaction mixture was diluted by adding water 15.0 mL, and extracted with DCM 300 mL (100 mL × 3). The resulting organic layer was dried over Na₂SO₄, and concentrated under reduced pressure to provide the crude product Compound Q3-5 (61.0 mg) as a yellow solid.

**LCMS:** EC12156-30-plal, P1: RT = 0.298 min, MS (ESI) *m*/*z* = 612.5 [M+H]⁺
**¹H NMR:** EC12156-30-plal, CDCl₃, 400 MHZ δ 9.68 (s, 1H), 9.17 (d, *J* = 7.4 Hz, 1H), 8.65 - 8.69 (m, 1H), 7.64 (d, *J* = 7.8 Hz, 1H), 7.39 (q, *J* = 8.2 Hz, 4H), 5.30 (s, 1H), 5.07 (m, *J* = 7.2 Hz, 1H), 4.77 (t, *J* = 7.6 Hz, 1H), 4.48 (s, 1H), 4.41 (d, *J =* 7.6 Hz, 1H), 4.16 (d, *J* = 10.4 Hz, 1H), 3.56 (dd, *J* = 11.2, 3.6 Hz, 1H), 2.87 - 3.00 (m, 2H), 2.59 - 2.63 (m, 2H), 2.53 (s, 3H), 2.37 - 2.42 (m, 2H), 2.27 - 2.37 (m, 2H), 2.14 - 2.24 (m, 2H), 1.94 - 2.10 (m, 4H), 1.87 - 1.92 (m, 1H), 1.66 - 1.86 (m, 5H), 1.46 (d, *J =* 6.8 Hz, 3H), 1.06 (s, 7H), 0.87 (d, *J* = 10.4 Hz, 5H).

### 5. Synthesis of Compound APQ3

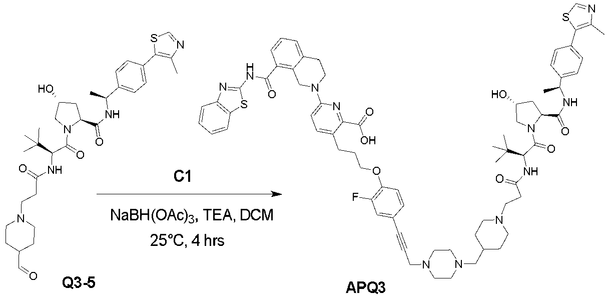

To a mixed solution of Compound C1 (50.6 mg, 71.9 µmol, 1.00 eq.) in DCM (3.00 mL), NaBH(OAc)₃ (45.7 mg, 215 µmol, 3.00 eq.), Compound Q3-**5** (44.0 mg, 71.9 µmol, 1.00 eq.), and TEA (21.8 mg, 215 µmol, 30.0 µL, 3.00 eq.) were added, and the mixture was stirred and reacted at 25 °C for 4 hours. LCMS (EC12156-36-pla3, P1: RT = 0.418 minutes) showed that Compound Q3-5 was reacted completely, and a new main peak was generated, which accounted for approximately 81.2%, and the m/z was consistent with the target product (m/ z = 1300.7 [M+H]⁺). To the reaction mixture, water (5.0 mL) was added at 25°C to quench the reaction, filtered and concentrated under reduced pressure to obtain a crude product, which was further purified by Prep-HPLC to provide Compound APQ3 (6.12 mg, 4.29 µmol, 5.96% yield, 91.1% purity) as a white solid.
**LCMS:** EC12156-36-pla3, P1: RT = 0.418 min, MS (ESI) *m*/*z* = 1300.7 [M+H]⁺
**HPLC:** EC12156-36-p1a10, RT = 1.899 min, 91.1% purity.
**¹H NMR:** EC12156-36-pla2, DMSO, 400 MHZ δ 8.97 (s, 1H), 8.57 - 8.67 (m, 1H), 8.38 (d, *J* = 8.0 Hz, 1H), 8.03 (d, *J* = 7.4 Hz, 1H), 7.78 (d, *J* = 8.4 Hz, 1H), 7.60 (d, *J =* 7.2 Hz, 1H), 7.31 - 7.51 (m, 10H), 7.26 (dd, *J =* 12.0, 2.0 Hz, 1H), 7.15 - 7.20 (m, 1H), 7.09 (t, *J* = 8.8 Hz, 1H), 6.89 (s, 1H), 4.86 - 4.96 (m, 3H), 4.47 - 4.56 (m, 1H), 4.36 - 4.47 (m, 1H), 4.27 (d, *J* = 1.6 Hz, 1H), 4.01 (t, *J =* 6.8 Hz, 2H), 3.8 (t, *J* = 5.6 Hz, 2H), 3.55 - 3.65 (m, 3H), 3.44 (s, 4H), 2.97 (t, *J=* 5.4 Hz, 3H), 2.72 - 2.81 (m, 2H), 2.64 - 2.70 (m, 2H), 2.45 (s, 6H), 2.29 - 2.36 (m, 6H), 2.09 (dd, *J* = 7.2, 2.8 Hz, 2H), 1.88 - 2.03 (m, 6H), 1.73 - 1.85 (m, 3H), 1.60 - 1.70 (m, 3H), 1.43 - 1.52 (m, 2H), 1.37 (d, *J* = 6.8 Hz, 3H), 1.23 (s, 1H), 1.04 - 1.19 (m, 3H), 0.94 (s, 9H).

### b) Synthesis of PROTAC molecules APL-1, APL-2, APL-3, APL-4, APL-5, and APL-6 containing a structure for recruiting the IAP-E3 enzyme

### Synthesis of APL-1:

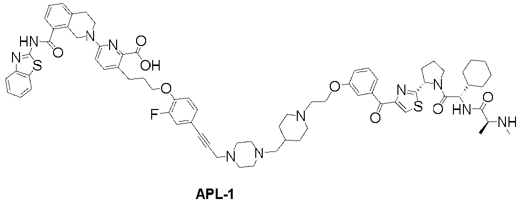

### Synthesis scheme:

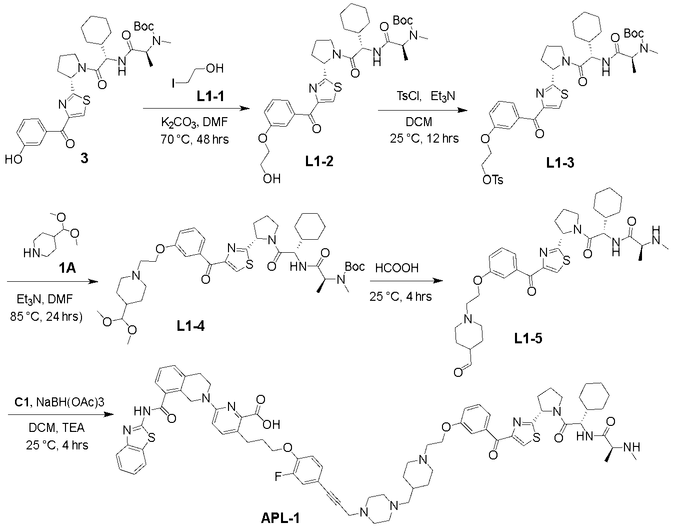

### 1. Synthesis of L1-2

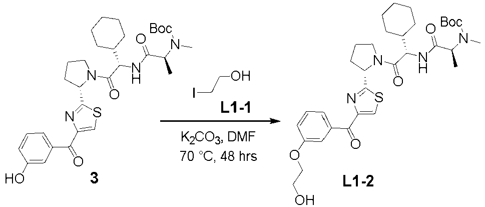

2-Iodoethanol (137 mg, 801 µmol, 62.6 µL, 2.4 eq.), Compound 3 (200 mg, 334 µmol, 1.0 eq.), and K₂CO₃ (46.1 mg, 334 µmol, 1.0 eq.) were mixed in DMF (2.0 mL), and the mixture was stirred and reacted at 70°C under N₂ atmosphere for 48 hours. LC-MS (EC16364-17-P1A, P1, RT= 0.432 minutes) showed that Compound 3 was completely consumed. To the reaction mixture, water (20.0 mL) was added at 0°C to quench the reaction. The mixture was diluted by adding ethyl acetate (20.0 mL), and extracted with ethyl acetate (60.0 mL, 20.0 mL×3). The resulting organic layer was washed with saturated saline (20.0 mL), dried over Na₂SO₄, filtered and concentrated under reduced pressure to provide the crude product L1-2 (140 mg, 217 µmol, 65.2% yield) as a white oil, which was directly used in the next reaction without purification.

LC-MS: EC16364-17-P1A, product: RT = 0.432 min, m/z = 643.3 [M+H]⁺.

### 2. Synthesis of L1-3

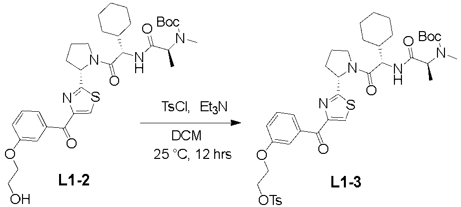

To a solution of Compound L1-2 (140 mg, 217 µmol, 1.0 eq.) in DCM (3.0 mL), TsCl (83.0 mg, 435µmol, 2.0 eq.) and Et₃N (66.1 mg, 653 µmol, 90.9 µL, 3.0 eq.) were added, and the mixture was stirred and reacted at 25°C for 12 hours. LC-MS (EC16364-18-P1A1, P1, RT = 0.495 minutes) showed that Compound L1-2 was completely reacted. To the reaction mixture, water (20.0 mL) was added at 0°C to quench the reaction. The mixture was diluted by adding DCM (20.0 mL), and extracted with DCM (60.0 mL, 20.0 mL×3). The resulting organic layer was washed with saturated saline (20.0 mL), dried over Na₂SO₄, filtered and concentrated under reduced pressure to provide the crude product L1-3 (170 mg, 213 µmol, 97.9% yield) as a white oil, which was directly used in the next reaction without purification.

**LC-MS:** EC16364-18-P1A1, product: RT = 0.495 min, m/z = 797.5 [M+H]⁺.

### 3. Synthesis of L1-4

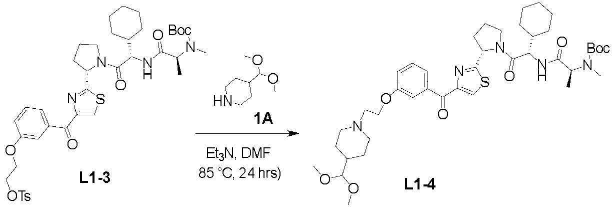

To a solution of Compound L1-3 (100 mg, 125 µmol, 1.0 eq.) in DCM (2.0 mL), Et₃N (63.5 mg, 627 µmol, 87.3 µL, 5.0 eq.) and Compound 1A (99.9 mg, 627 µmol, 5.0 eq.) were added, and the mixture was stirred and reacted at 85°C for 24 hours. LCMS (EC16364-42-pla, P1, RT = 0.303 minutes) showed that Compound L1-3 was completely reacted. To the reaction mixture, water (20.0 mL) was added at 0°C to quench the reaction. The mixture was diluted by adding ethyl acetate (20.0 mL), and extracted with ethyl acetate (60.0 mL, 20.0 mL×3). The resulting organic layer was washed with saturated saline (20.0 mL), dried over Na₂SO₄, filtered and concentrated under reduced pressure to provide the crude product L1-4, which was further purified by Prep-TLC (SiO₂, petroleum ether: ethyl acetate = 0:1, P1, R_{f} = 0.3) to provide the product L1-4 (20.0 mg, 25.5 µmol, 20.3% yield) as a white solid.

**LC-MS:** EC16364-42-pla, product: RT = 0.303 min, m/z = 684.4 [M+H]⁺.

### 4. Synthesis of L1-5

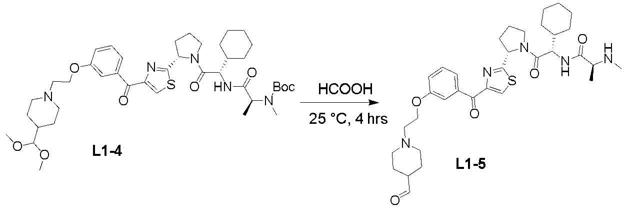

Compound L1-4 (20 mg, 25.51 µmol, 1.0 eq.) was added in formic acid (2 mL), and the mixture was stirred at 25°C under N₂ atmosphere for 2 hours. TLC (petroleum ether: ethyl acetate = 0:1) showed that L1-4 was reacted completely, and a more polar main peak was generated. The reaction mixture was concentrated under reduced pressure to provide the crude product L1-5 (15 mg, 20.3 µmol, 79.6% yield) as a yellow oil, which was directly used in the next reaction without purification.

### 5. Synthesis of APL-1

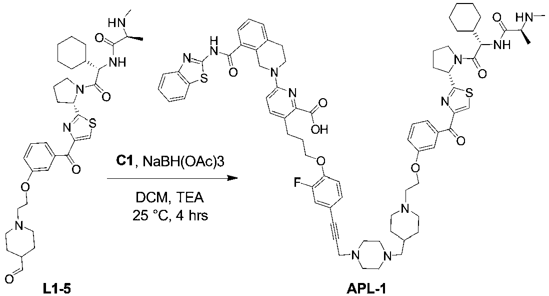

To a solution of Compound L1-5 (15.0 mg, 20.3 µmol, 1.0 eq.) in DCM (1 mL), TEA (20.6 mg, 203 µmol, 28.3 µL, 10.0 eq.) and Compound C1 (14.3 mg, 20.3 µmol, 1.0 eq.) were added, and the mixture was stirred and reacted at 25°C for 0.5 hours. Then, NaBH(OAc)₃ (12.9 mg, 61.0 µmol, 3.0 eq.) was added, and the mixture was stirred at 25°C for 3.5 hours. LC-MS (EC16364-45-P1A, P1, RT = 0.381 minutes) showed that L1-5 was completely reacted. The reaction mixture was concentrated under reduced pressure to obtain a crude product, which was further purified by Prep-HPLC column chromatography (FA conditions) to provide Compound APL-1 (10 mg, 7.34 µmol, 36.12% yield, 97.4 purity) as a white solid.

**LC-MS:** EC16364-45-P1A, product: RT = 0.381 min, m/z = 1326.8 [M+H]⁺
**¹H NMR:** EC16364-45-P1D (400 MHz DMSO) *δ* ppm 8.51 (s, 1H) 8.22 (s, 1H) 8.09 (d, *J* = 8.8 Hz, 1H) 8.00 - 8.06 (m, 1H) 7.79 (d, *J* = 8.0 Hz, 1H) 7.71 (s, 1H) 7.57 - 7.66 (m, 2H) 7.42 - 7.50 (m, 3H) 7.38 - 7.42 (m, 1H) 7.34 (td, *J* = 7.2, 3.38 Hz, 2H) 7.22 - 7.29 (m, 2H) 7.18 (d, *J* = 9.2 Hz, 1H) 7.05 - 7.14 (m, 1H) 6.84 (d, *J* = 9.2 Hz, 1H) 5.39 (dd, *J* = 7.6, 2.81 Hz, 1H) 4.90 (s, 2H) 4.47 (t, *J* = 7.6 Hz, 1H) 4.13 t, *J =* 5.2 Hz, 2H) 4.01 (t, *J =* 6.4 Hz, 2H) 3.78 - 3.85 (m, 4H) 3.46 (s, 2H) 3.17 (d, *J =* 6.8 Hz, 2H) 2.97 (t, *J* = 5.2 Hz, 2H) 2.91 (d, *J* = 10.0 Hz, 2H) 2.69 - 2.77 (m, 4H) 2.23 - 2.43 (m, 6H) 2.22 (s, 3H) 1.93 - 2.13 (m, 10H) 1.58 - 1.69 (m, 6H) 1.51 (d, *J* = 7.6 Hz, 2H) 1.46 (s, 1H) 1.15 (d, *J* = 6.8 Hz, 3H) 0.89 - 1.11 (m, 8H).

### Synthesis of APL-2:

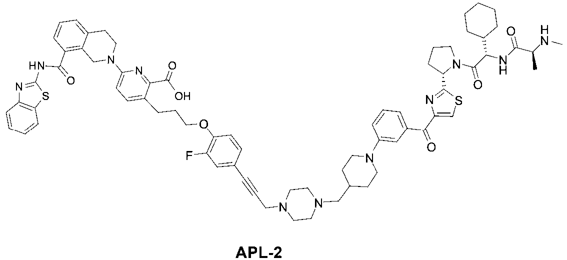

### Synthesis scheme:

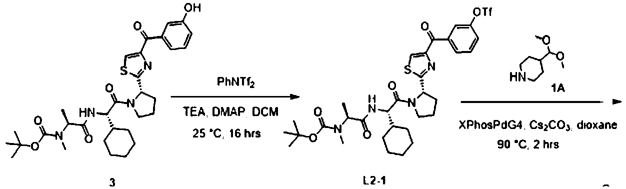

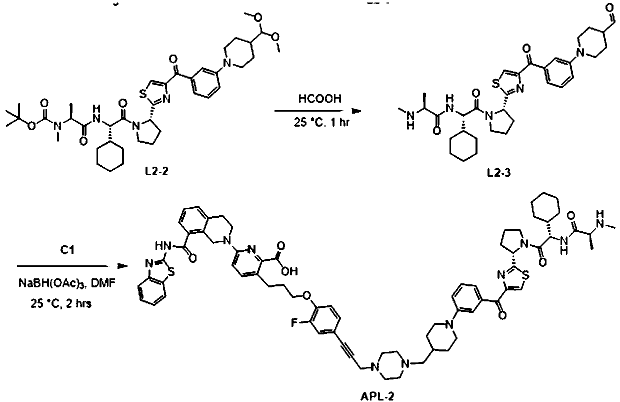

### 1. Synthesis of Compound L2-1

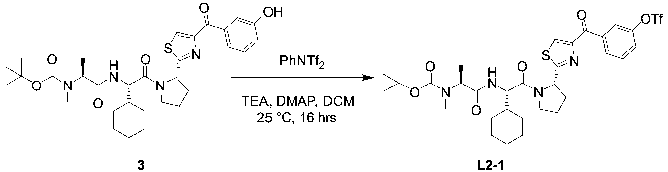

To a solution of Compound 3 (280 mg, 468 µmol, 1.0 eq.) in DCM (10.0 mL), PhNTf₂ (267 mg, 748 µmol, 1.6 eq.), TEA (94.6 mg, 935 µmol, 130 µL, 2.0 eq.), and DMAP (5.71 mg, 46.8 µmol, 0.1 eq.) were added, and the mixture was stirred and reacted at 25°C for 16 hours. The reaction mixture was concentrated under reduced pressure to remove the solvent, and the obtained crude product was further purified by reverse-phase HPLC (0.1% FA conditions) to provide Compound L2-1 (300 mg, approximately 98% purity) as a white solid.

LC-MS: (EC16331-27-P1A, product: RT = 0.568 min, m/z = 731.3 [M+H]⁺.

### 2. Synthesis of Compound L2-2

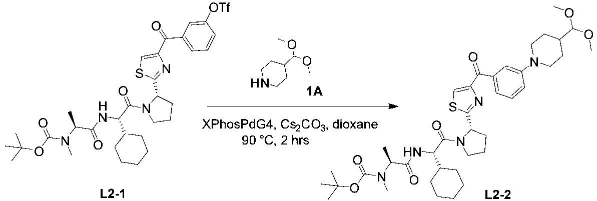

Compound L2-1 (160 mg, 219 µmol, 1.0 eq.), Compound 1A (174 mg, 1.09 mmol, 5.0 eq.), Cs₂CO₃ (214 mg, 657 µmol, 3.0 eq.), and XPhosPdG4 (18.8 mg, 21.9 µmol, 0.1 eq.) were mixed in dioxane (3.00 mL), and the mixture was stirred and reacted at 90°C under N₂ atmosphere for 2 hours. The reaction mixture was subjected to liquid-liquid separation by adding water (10.0 mL) and DCM (60.0 mL, 10.0 mL × 6). The resulting organic layer was washed with saturated saline (60.0 mL, 30.0 mL×2), dried over Na₂SO₄, filtered and concentrated under reduced pressure to obtain a crude product, which was purified by column chromatography (SiO₂, petroleum ether: ethyl acetate = 5:1) to provide Compound L2-2 (120 mg, 129.57 µmol, 59.18% yield, 79.9% purity) as a yellow oil.

**LC-MS:** EC16331-11-P1A, product: RT = 0.501 min, m/z = 740.3 [M+H]⁺
**¹H NMR:** EC16331-11-P1A (400 MHz CDCl₃) *δ* ppm 8.10 (s, 1H), 7.74 (d, *J =* 1.6 Hz, 1H), 7.56 - 7.66 (m, 1H), 7.34 (d, *J =* 1.2 Hz, 1H), 7.13 - 7.21 (m, 1H), 5.56 (dd, *J* = 7.6, 2.4 Hz, 1H), 4.64 (dd, *J* = 8.8, 6.4 Hz, 2H), 3.86 - 3.91 (m, 1H), 3.78 (d, *J* = 11.6 Hz, 2H), 3.39 (s, 6H), 2.81 (s, 3H), 2.75 (d, *J* = 8.0 Hz, 2H), 2.50 (dt, *J* = 6.4, 3.2 Hz, 1H), 2.27 - 2.32 (m, 1H), 2.18 (d, *J* = 7.2 Hz, 1H), 2.09 - 2.13 (m, 1H), 1.85 - 1.90 (m, 2H), 1.71 (s, 6H), 1.58 (s, 9H), 1.49 (s, 11H), 1.34 (d, *J* = 7.2 Hz, 3H).

### 3. Synthesis of Compound L2-3

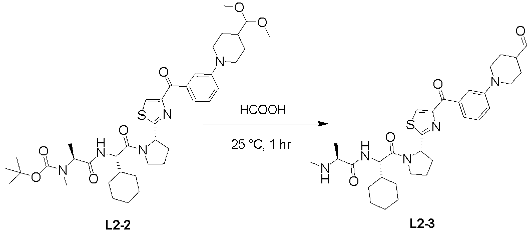

Compound L2-2 (50.0 mg, 67.6 µmol, 1.0 eq.) was mixed with formic acid (0.5 mL), and the mixture was stirred and reacted at 25°C for 1 hour. The reaction mixture was concentrated under reduced pressure to remove the solvent, and a crude product Compound L2-3 (30.0 mg) was obtained as a yellow oil, which was directly used in the next reaction without purification.

**LC-MS:** EC16331-15-P1A, product: RT = 0.322 min, m/z = 594.3 [M+H]⁺.

### 4. Synthesis of compound APL-2

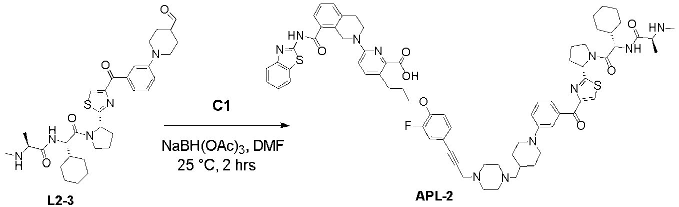

Compound L2-3 (30.0 mg, 50.5 µmol, 1.0 eq.), C1 (35.6 mg, 50.5 µmol, 1.0 eq.), and NaBH(OAc)₃ (32.1 mg, 152 µmol, 3.0 eq.) were mixed in DMF (0.5 mL), and the mixture was stirred and reacted at 25°C under N₂ atmosphere for 2 hours. The reaction mixture was filtered and concentrated under reduced pressure to obtain a crude product, which was further purified by reverse-phase HPLC (0.1% FA conditions) to provide compound APL-2 (10.0 mg, 7.61 µmol, 15.06% yield, 97.61% purity) as a yellow solid.

**LC-MS:** EC16331-16-P1A, product: RT = 0.420 min, m/z = 1283.5 [M+H]⁺
**¹H NMR:** EC16331-16-P1C (400 MHz DMSO) *δ* ppm 8.44 (s, 1H), 8.11 (d, *J* = 8.4 Hz, 1H), 8.02 (d, *J* = 7.6 Hz, 1H), 7.75 - 7.82 (m, 1H), 7.69 (s, 1H), 7.60 (d, *J =* 7.13 Hz, 1H), 7.44 - 7.50 (m, 2H), 7.39 (d, *J =* 5.2 Hz, 2H), 7.35 (dt, *J* = 7.2, 3.6 Hz, 3H), 7.14 - 7.30 (m, 4H), 7.04 - 7.14 (m, 1H), 6.77 - 6.85 (m, 1H), 5.38 (dd, *J* = 6.6, 3.6 Hz, 1H), 4.90 (s, 2H), 4.43 - 4.50 (m, 2H), 3.98 - 4.05 (m, 4H), 3.83 (d, *J* = 5.2 Hz, 6H), 3.70 - 3.79 (m, 10H), 3.20 (s, 2H), 2.97 (d, *J* = 4.8 Hz, 2H), 2.71 - 2.76 (m, 4H), 2.37 - 2.42 (m, 2H), 2.23 (s, 4H), 2.15 (d, *J* = 6.13 Hz, 2H), 2.00 (dd, *J* = 16.8, 6.8 Hz, 5H), 1.78 (d, *J* = 10.4 Hz, 2H), 1.68 (d, *J* = 3.2 Hz, 2H), 1.61 (s, 2H), 1.54 (d, *J* = 9.6 Hz, 2H), 1.24 (s, 2H), 1.15 (d, *J* = 6.8 Hz, 2H), 1.06 (t, *J* = 6.8 Hz, 3H).

### Synthesis of APL-3 and APL-4:

### Synthesis scheme:

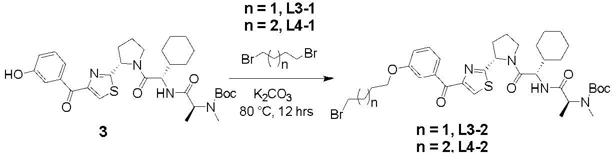

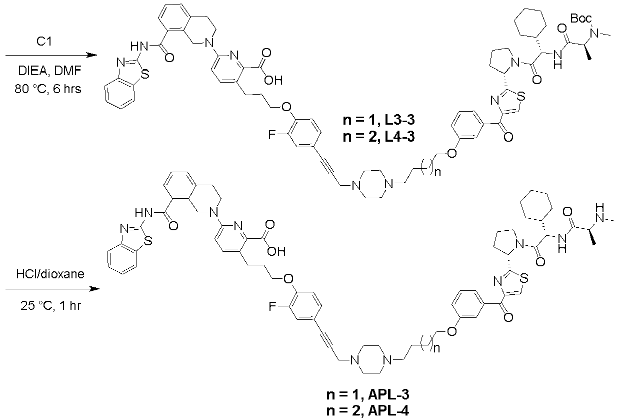

### Synthesis of APL-3:

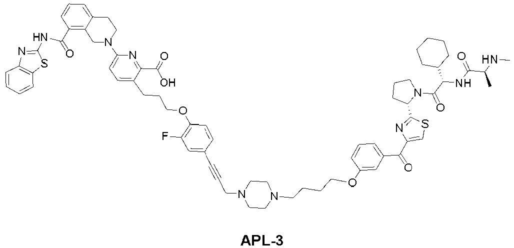

### 1. Synthesis of L3-2

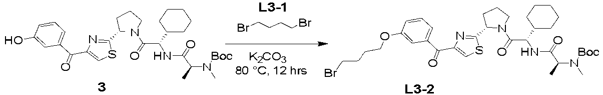

At 25°C, to a solution of Compound 3 (100 mg, 167 µmol, 1.0 eq.) in MeCN (1 mL), K₂CO₃ (69.2 mg, 501 µmol, 3.0 eq.) was added, and the mixture was stirred at 25°C for 30 minutes. Then, at 25°C, 1,4-dibromobutane (180 mg, 835 µmol, 100 µL, 5.0 eq.) was added dropwise, and the mixture was stirred and reacted at 80°C for 12 hours. To the reaction mixture, water (20.0 mL) was added at 0°C to quench the reaction. The mixture was diluted by adding ethyl acetate (20.0 mL), and extracted with ethyl acetate (60.0 mL, 20.0 mL×3). The resulting organic layer was washed with saturated saline (20.0 mL), dried over Na₂SO₄, filtered and concentrated under reduced pressure to provide the crude product L3-2, which was further purified by Prep-TLC (SiO₂, petroleum ether: ethyl acetate = 1:1, P1, R_{f} = 0.3) to provide the product L3-2 (100 mg, 136 µmol, 40.8% yield, 100% purity) as a white solid.

**LC-MS:** EC16364-66-P1A, product: RT = 0.591 min, m/z = 733.4 [M+H]⁺.

### 2. Synthesis of L3-3

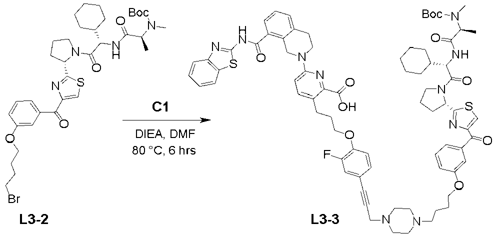

Compound C1 (76.8 mg, 109 µmol, 1.0 eq.), L3-2 (80.0 mg, 109 µmol, 1.0 eq.), and DIEA (42.3 mg, 327 µmol, 56.9 µL, 3.0 eq.) were mixed in DMF (1.0 mL), and the mixture was stirred and reacted at 80°C under N₂ atmosphere for 6 hours. LC-MS (EC16364-70-P1A1, P1, RT = 0.504 minutes) showed that L3-2 was completely reacted. The reaction mixture was concentrated under reduced pressure to obtain a crude product, which was further purified by Prep-HPLC column chromatography (FA conditions) to provide the product L3-3 (30.0 mg, 22.1 µmol, 20.2% yield) as a white solid.

**LC-MS:** EC16364-70-P1A1, product: RT = 0.504 min, m/z = 1357.6 [M+H]⁺.

### 3. Synthesis of APL-3

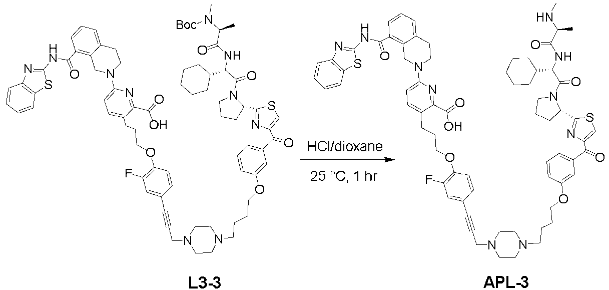

Compound L3-3 (30.0 mg, 22.1 µmol, 1.0 eq.) and HCl/dioxacyclohexane (4 M, 5.0 mL, 905 eq.) were mixed, and the mixture was stirred and reacted at 25°C under N₂ atmosphere for 1 hour. LC-MS (EC16364-80-P1B2, P1, RT = 0.428 minutes) showed that L3-3 was completely reacted. The reaction mixture was concentrated under reduced pressure to obtain a crude product, which was further purified by Prep-HPLC column chromatography (HCl conditions) to provide the product APL-3 (5 mg, 3.81 µmol, 17.2% yield, 95.7% purity) as a brown solid.

**LC-MS:** EC16364-80-P1B2, product: RT = 0.428 min, m/z = 1279.6 [M+H]⁺
**¹H NMR:** EC16364-80-P1D (400 MHz DMSO) *δ* ppm 9.30 (s, 1H) 9.07 (s, 1H) 8.87 (s, 1H) 8.75 (d, *J* = 7.6 Hz, 1H) 8.49 (s, 1H) 8.04 (d, *J* = 8.0 Hz, 1H) 7.79 (d, *J* = 8.0 Hz, 1H) 7.68 (d, *J* = 7.6 Hz, 1H) 7.53 - 7.63 (m, 4H) 7.47 (br t, *J =* 7.6 Hz, 3H) 7.39 - 7.43 (m, 1H) 7.30 - 7.39 (m, 4H) 7.22 - 7.27 (m, 2H) 7.09 - 7.15 (m, 1H) 6.95 - 7.00 (m, 1H) 5.35 - 5.41 (m, 1H) 4.92 (s, 2H) 4.47 *(t, J=* 7.6 Hz, 1H) 4.18 (s, 1H) 4.01 - 4.10 (m, 6H) 3.86 (t, *J* = 5.6 Hz, 4H) 3.76 - 3.82 (m, 4H) 2.98 (t, *J* = 5.2 Hz, 2H) 2.81 (d, *J* = 7.2 Hz, 2H) 2.32 (t, *J =* 4.8 Hz, 1H) 2.13 - 2.29 (m, 3H) 1.94 - 2.06 (m, 5H) 1.87 - 1.92 (m, 2H) 1.82 (d, *J* = 6.4 Hz, 2H) 1.59 - 1.72 (m, 5H) 1.52 - 1.58 (m, 3H) 1.35 (d, *J* = 6.8 Hz, 3H) 0.99 - 1.15 (m, 6H).

### Synthesis of APL-4:

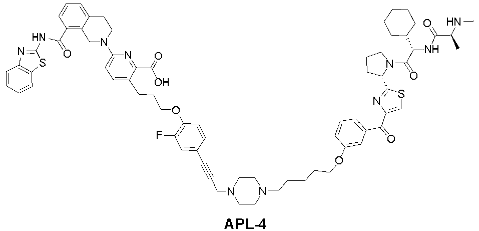

The synthesis procedure for the PROTAC molecule APL-4 was similar to that for APL-3, but the starting materials were replaced with Compound 3 and L4-1, and the target product APL-4 (5 mg, 3.59 µmol, 32.8% yield, 91.3% purity) was obtained by Prep-HPLC column chromatography (HCl conditions) after synthesis, as a white solid.

**LC-MS:** EC16364-88-P1B2, product: RT = 0.427 min, m/z = 1293.6 [M+Na]⁺
**¹H NMR:** EC16364-88-plc (400 MHz DMSO) *δ* ppm 9.18 (s, 1H) 8.84 (s, 1H) 8.73 - 8.73 (m, 1H) 8.74 (d, *J =* 8.0 Hz, 1H) 8.49 (s, 1H) 8.04 (d, *J =* 8.0 Hz, 1H) 7.79 (d, *J*=8.0 Hz, 1H) 7.68 (d, *J =* 7.6 Hz, 1H) 7.54 - 7.62 (m, 4H) 7.47 (td, *J* = 7.6, 4.13 Hz, 3H) 7.41 (d, *J =* 7.2 Hz, 2H) 7.36 (d, *J* = 6.0 Hz, 2H) 7.32 (d, *J =* 9.6 Hz, 1H) 7.23 - 7.29 (m, 2H) 7.17 - 7.22 (m, 1H) 7.13 *(t, J=* 8.8 Hz, 1H) 6.97 (d, *J =* 8.8 Hz, 1H) 5.39 (dd, *J =* 7.6, 2.69 Hz, 1H) 4.92 (s, 2H) 4.47 (t, *J =* 7.6 Hz, 1H) 4.05 (d, *J* = 9.2 Hz, 4H) 3.86 (d, *J =* 5.2 Hz, 4H) 3.81 (dd, *J* = 6.4, 2.25 Hz, 2H) 3.10 - 3.13 (m, 2H) 2.98 (t, *J* = 5.2 Hz, 2H) 2.78 - 2.83 (m, 2H) 2.54 (s, 2H) 2.17 - 2.25 (m, 2H) 2.02 - 2.08 (m, 2H) 1.95 - 2.01 (m, 2H) 1.75 - 1.80 (m, 4H) 1.61 - 1.70 (m, 4H) 1.53 - 1.58 (m, 2H) 1.45 - 1.51 (m, 2H) 1.34 (d, *J* = 6.8 Hz, 3H) 1.16 - 1.25 (m, 2H) 1.12 (d, *J* = 7.2 Hz, 2H) 1.07 (s, 1H) 1.05 (s, 2H) 1.03 - 1.04 (m, 1H).

### Synthesis of APL-5 and APL-6:

### Synthesis scheme:

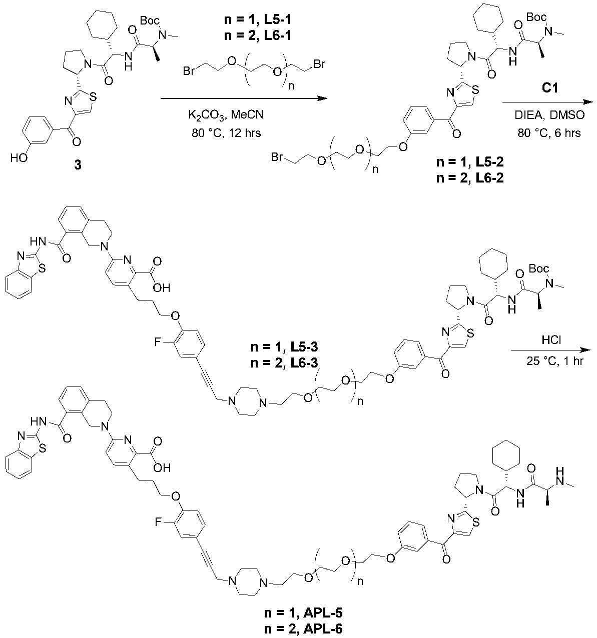

### Synthesis of APL-5:

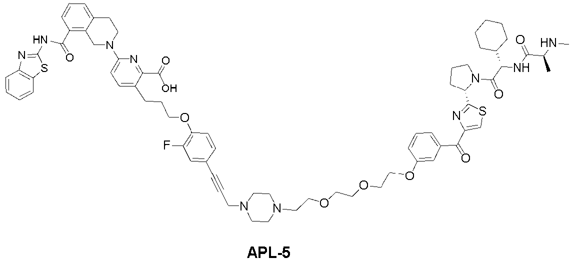

### 1. Synthesis of L5-2

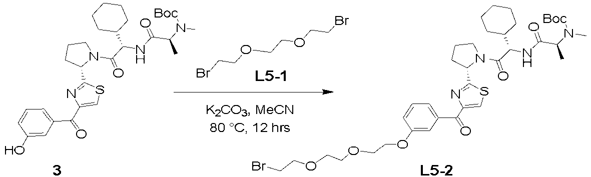

At 25°C, to a solution of Compound 3 (100 mg, 167 µmol, 1.0 eq.) in MeCN (3 mL), K₂CO₃ (69.2 mg, 501 µmol, 3.0 eq.) was added, and the mixture was stirred for 30 minutes. Then, at 25°C, L5-1 (230 mg, 835 µmol, 5.0 eq.) was added dropwise, and the mixture was stirred and reacted at 80°C for 12 hours. LC-MS (EC16364-78-P1A, P1, RT = 0.495 minutes) showed that Compound 3 was completely reacted. To the reaction mixture, water (20.0 mL) was added at 0°C to quench the reaction. The mixture was diluted by adding ethyl acetate (20.0 mL), and extracted with ethyl acetate (60.0 mL, 20.0 mL×3). The resulting organic layer was washed with saturated saline (20.0 mL), dried over Na₂SO₄, filtered and concentrated under reduced pressure to provide the crude product L5-2, which was further purified by Prep-TLC (SiO₂, petroleum ether: ethyl acetate = 1:2, P1, R_{f} = 0.3) to provide the product L5-2 (90.0 mg, 113 µmol, 67.9% yield, 100% purity) as a white solid.

**LC-MS:** EC16364-78-P1A, product: RT = 0.495 min, m/z = 793.3 [M+H]⁺.

### 2. Synthesis of L5-3

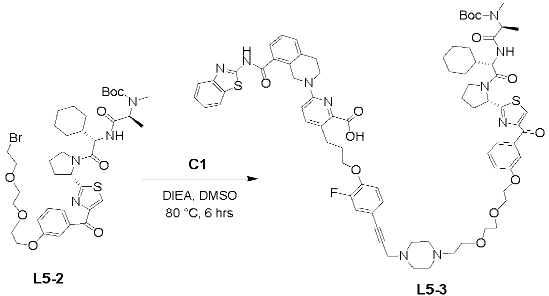

Compound L5-2 (50.0 mg, 63.0 µmol, 1.0 eq.), C1 (35.5 mg, 50.4 µmol, 0.8 eq.), and DIEA (24.4 mg, 188 µmol, 32.9 µL, 3.0 eq.) were mixed in DMF (1.0 mL), and the mixture was stirred and reacted at 80°C under N₂ atmosphere for 6 hours. LC-MS (EC16364-91-P1A1, P1, RT = 0.500 minutes) showed that L5-2 was completely reacted. The reaction mixture was concentrated under reduced pressure to obtain a crude product, which was further purified by Prep-HPLC column chromatography (FA conditions) to provide the product L5-3 (20.0 mg, 14.1 µmol, 22.4% yield) as a white solid.

**LC-MS:** EC16364-91-P1A1, product: RT = 0.500 min, m/z = 1418.6 [M+H]⁺.

### 3. Synthesis of APL-5

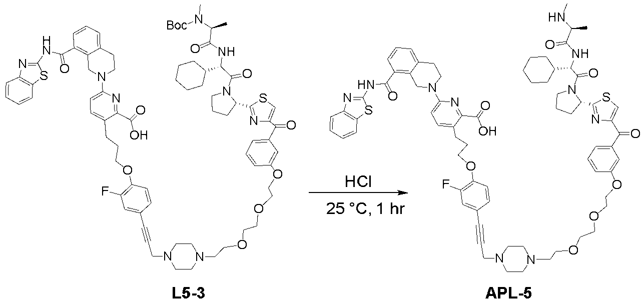

Compound L5-3 (20.0 mg, 14.1 µmol, 1.0 eq.) and HCl/ethyl acetate (4 M, 3.53 µL) were mixed, and the mixture was stirred and reacted at 25°C under N₂ atmosphere for 1 hour. LC-MS (EC16364-100-P1A1, P1, RT = 0.413 minutes) showed that L5-3 was completely reacted. The reaction mixture was concentrated under reduced pressure to obtain a crude product, which was further purified by Prep-HPLC column chromatography (HCl conditions) to provide the product APL-5 (13.1 mg, 9.07 µmol, 64.2% yield, 91.9% purity) as a brown solid.

**LC-MS:** EC16364-100-P1A1, product: RT = 0.413 min, m/z = 659.7 [M/2+H]⁺
**¹H NMR:** EC16364-100-P1C (400 MHz DMSO) *δ* ppm 9.26 (s, 1H) 8.80 - 8.93 (m, 1H) 8.74 (d, *J* = 8.0 Hz, 1H) 8.46 - 8.51 (m, 1H) 8.04 (d, *J* = 7.6 Hz, 1H) 7.79 (d, *J* = 7.6 Hz, 1H) 7.68 (d, *J* = 7.6 Hz, 1H) 7.54 - 7.62 (m, 3H) 7.39 - 7.50 (m, 4H) 7.31 - 7.39 (m, 3H) 7.26 (dd, *J* = 8.0, 2.63 Hz, 2H) 7.12 (t, *J* = 8.8 Hz, 1H) 6.97 (d, *J =* 9.2 Hz, 1H) 5.38 (dd, *J* = 7.6, 3.31 Hz, 1H) 5.32 (t, *J=* 4.4 Hz, 1H) 4.92 (s, 2H) 4.47 (t, *J =* 7.6 Hz, 1H) 4.15 - 4.19 (m, 2H) 4.03 (t, *J=* 6.4 Hz, 2H) 3.85 (dd, *J* = 11.6, 5.50 Hz, 6H) 3.77 - 3.80 (m, 4H) 3.33 (s, 4H) 2.98 (t, *J* = 5.6 Hz, 2H) 2.78 - 2.83 (m, 2H) 2.47 (s, 2H) 2.46 (s, 1H) 2.15 - 2.24 (m, 2H) 2.00 (dd, *J =* 15.2, 7.63 Hz, 8H) 1.60 - 1.70 (m, 4H) 1.56 (d, *J* = 8.4 Hz, 2H) 1.41 - 1.49 (m, 2H) 1.34 (d, *J* = 6.8 Hz, 3H) 1.03 - 1.16 (m, 6H) 0.83 - 0.88 (m, 2H).

### Synthesis of APL-6:

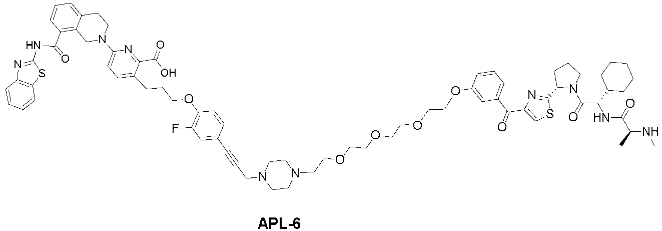

The synthesis procedure for the PROTAC molecule APL-6 was similar to that for APL-5, but the starting materials were replaced with Compound 3 and L6-1, and the target product APL-6 (11.0 mg, 7.32 µmol, 53.5% yield, 90.6% purity) was obtained by purification after synthesis, as a white solid.

**LC-MS:** EC16364-101-P1A1, product: RT = 0.418 min, m/z = 1384.5 [M+Na]⁺
**¹H NMR:** EC16364-101-P1C (400 MHz DMSO) *δ* ppm 9.24 - 9.36 (m, 1H) 8.80 - 8.94 (m, 1H) 8.48 (s, 1H) 8.04 (d, *J =* 7.6 Hz, 1H) 7.79 (d, *J* = 7.6 Hz, 1H) 7.68 (d, *J* = 7.6 Hz, 1H) 7.54 - 7.62 (m, 3H) 7.40 - 7.50 (m, 4H) 7.33 - 7.38 (m, 2H) 7.23 - 7.31 (m, 2H) 7.13 (t, *J =* 8.8 Hz, 1H) 6.97 (d, *J* = 8.8 Hz, 1H) 5.38 (dd, *J* = 7.6, 3.06 Hz, 1H) 5.32 (t, *J* = 4.8 Hz, 1H) 4.92 (s, 2H) 4.47 (t, *J* = 7.6 Hz, 1H) 4.14 - 4.17 (m, 2H) 4.01 - 4.04 (m, 2H) 3.87 (d, *J=* 5.6 Hz, 4H) 3.82 (d, *J* = 4.4 Hz, 4H) 3.76 (br s, 4H) 3.59 - 3.61 (m, 4H) 3.57 (s, 4H) 3.34 (d, *J* = 1.2 Hz, 2H) 2.98 (t, *J* = 5.6 Hz, 2H) 2.80 (t, *J =* 7.2 Hz, 2H) 2.46 (s, 1H) 2.33 (s, 3H) 2.19 - 2.23 (m, 1H) 2.02 (d, *J* = 7.2 Hz, 2H) 1.98 (d, *J* = 7.6 Hz, 2H) 1.64 (d, *J* = 8.0 Hz, 2H) 1.55 (dd, *J* = 8.0, 1.69 Hz, 2H) 1.43 - 1.48 (m, 1H) 1.35 (d, *J* = 6.8 Hz, 3H) 1.23 (s, 8H) 1.08 (d, *J* = 6.4 Hz, 2H) 0.83 - 0.86 (m, 1H).

### Example 4. Synthesis of PROTAC molecules (APF1, APF2, APF3, APF4, APF1A, APR1, APR2, APR3, and APR4) based on Compound A1331852 as the targeting ligand and the CRBN-E3 enzyme.

### a) Synthesis of PROTAC molecules APF1, APF2, APF3, APF4, and APF1A

### Synthesis scheme:

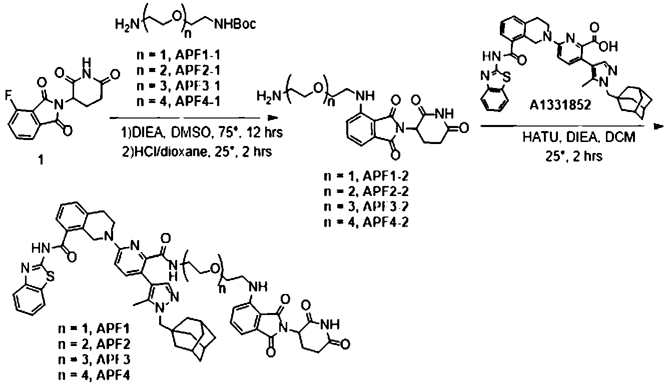

### Synthesis of APF1:

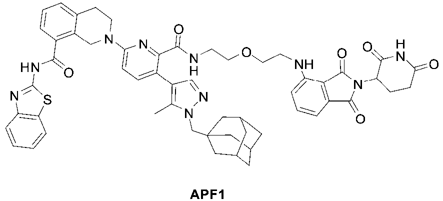

### 1. Synthesis of Compound APF1-2

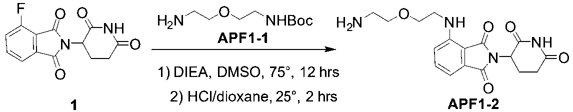

To a solution of Compound 1 (200 mg, 724 µmol, 1.00 eq.) and Compound APF1-1 (177 mg, 869 µmol, 1.20 eq.) dissolved in DMSO (2.00 mL), DIEA (281 mg, 2.17 mmol, 378 µL, 3.00 eq.) was added, and the mixture was stirred and reacted at 75°C for 12 hours. LC-MS (EC11255-27-P1A, P1 RT = 0.357 minutes) showed that Compound 1 was fully reacted, and multiple new compounds were generated, where approximately 56.0% of the target compound was detected. The mixture was extracted with ethyl acetate 45 mL (15.0 mL×3). The resulting organic layer was washed with saline, dried over Na₂SO₄, filtered and concentrated under reduced pressure to provide the crude product, which was purified by column chromatography (SiO2, PE: EA=1/0 to 1/5), and the resulting product was dissolved in 5 mL hydrochloric acid/dioxacyclohexane. The air was removed and replaced with N₂, and the mixture was stirred and reacted at 25°C for 2 hours. The reaction mixture was concentrated under vacuum to provide the crude product Compound APF1-2 (160 mg, 347 µmol, 48.0% yield) as a light yellow solid.

**LCMS:** EC11255-27-P1A, P1 RT = 0.357 min, MS (ESI) m/z = 361.2 [M+H]⁺.

### 2. Synthesis of Compound APF1

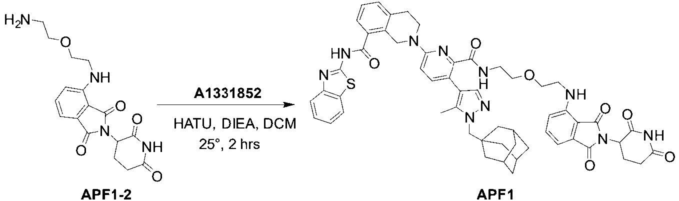

Compound A1331852 (60.0 mg, 91.0 µmol, 1.00 eq.), APF1-2 (49.2 mg, 136 µmol, 1.50 eq.), HATU (51.9 mg, 136 µmol, 1.50 eq.), and DIEA (58.8 mg, 455 µmol, 79.3 µL, 5.00 eq.) were mixed in DCM (1 mL). The air was removed and replaced with N₂, and the mixture was stirred and reacted at 25°C for 2 hours. LC-MS (EC11272-23-P1B) showed that Compound A1331852 was fully reacted, and the reaction mixture was concentrated under vacuum to obtain a crude product, which was further purified by Prep-HPLC (alkaline conditions) to provide Compound APF1 (21.6 mg, 20.0 µmol, 22.0% yield, 93.0% purity) as a yellow solid.
**LCMS:** EC11272-23-P1B, RT = 0.569 min, M/Z (M+H⁺) = 1001.3
**LCMS:** EC11272-23-P1C, RT = 0.563 min, M/Z (M+H⁺) = 1001.3
**HNMR:** EC11272-23-P1C, DMSO+D₂O, 400MHz *δ* 11.08 (s, 1H), 8.14 (t, *J =* 5.69 Hz, 1H), 7.99 (d, *J* = 7.6 Hz, 1H), 7.77 (d, *J* = 8.0 Hz, 1H), 7.58 (d, *J* = 7.6 Hz, 1H), 7.51 (t, *J* = 8.0 Hz, 1H), 7.42 - 7.48 (m, 2H), 7.29 - 7.41 (m, 3H), 7.25 (s, 1H), 7.06 (d, *J =* 8.4 Hz, 1H), 6.99 (d, *J* = 7.2 Hz, 1H), 6.94 (d, *J =* 8.8 Hz, 1H), 6.50 - 6.57 (m, 1H), 4.92 - 5.05 (m, 3H), 3.86 (t, *J =* 5.2 Hz, 2H), 3.64 (s, 2H), 3.38 (d, *J =* 6.4 Hz, 4H), 3.24 (d, *J =* 5.2 Hz, 2H), 2.96 - 3.01 (m, 2H), 2.70 - 2.92 (m, 2H), 2.02 (s, 3H), 1.91 - 2.00 (m, 2H), 1.88 (s, 3H), 1.57 - 1.64 (m, 3H), 1.46 - 1.54 (m, 9H).

### Synthesis of APF2:

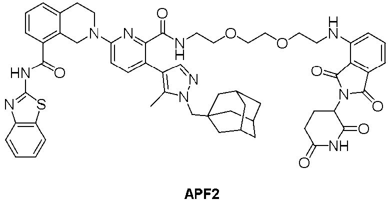

The synthesis procedure for the PROTAC molecule APF2 was similar to that for APF1, but the starting materials were replaced with Compound 1 and APF2-1, and the target product APF2 was obtained by purification after synthesis, as a yellow solid, with a purity of 93.7% and a yield of approximately 23.9%.
**LCMS:** EC11272-24-P1B, RT = 0.572 min, M/Z (M+H⁺) = 1045.3
**LCMS:** EC11272-24-P1C, RT = 0.567 min, M/Z (M+H⁺) = 1045.3
**HNMR:** EC11272-24-P1A, DMSO, 400MHz *δ* 11.07 - 11.11 (m, 1H), 8.18 (t, *J* = 5.6 Hz, 1H), 7.97 - 8.02 (m, 1H), 7.73 - 7.79 (m, 1H), 7.58 - 7.64 (m, 1H), 7.54 (t, *J =* 7.2 Hz, 1H), 7.41 - 7.48 (m, 2H), 7.29 - 7.41 (m, 3H), 7.25 (s, 1H), 7.08 (d, *J* = 8.4 Hz, 1H), 7.01 (d, *J =* 7.2 Hz, 1H), 6.94 (d, *J* = 8.8 Hz, 1H), 6.57 (t, *J* = 6.0 Hz, 1H), 4.94 - 5.08 (m, 3H), 3.89 (t, *J* = 6.0 Hz, 2H), 3.66 (s, 2H), 3.55 - 3.58 (m, 2H), 3.46 - 3.50 (m, 2H), 3.39 - 3.41 (m, 4H), 3.23 - 3.26 (m, 4H), 2.97 - 3.01 (m, 2H), 2.79 - 2.92 (m, 2H), 2.04 (s, 3H), 1.94 - 2.02 (m, 2H), 1.90 (s, 3H), 1.59 - 1.65 (m, 3H), 1.48 - 1.55 (m, 9H).

### Synthesis of APF3:

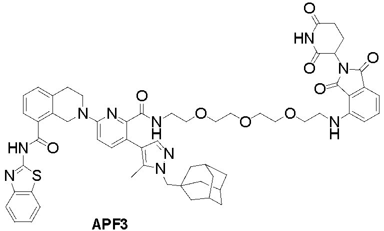

The synthesis procedure for the PROTAC molecule APF3 was similar to that for APF1, but the starting materials were replaced with Compound 1 and APF3-1, and the target product APF3 was obtained by purification after synthesis, as a yellow solid, with a purity of 97.4% and a yield of approximately 11.4%.
**LCMS:** EC12165-1-P1A, P1, RT = 0.513 min, M/Z (M+H⁺) = 1089.7
**LCMS:** EC12165-1-P1C, RT = 0.570 min, M/Z (M+H⁺) = 1089.2
**HNMR:** EC12165-1-P1C, DMSO, 400MHz *δ* 12.85 (s, 1H), 11.09 (s, 1H), 8.16 (t, *J* = 6.0 Hz, 1H), 8.01 (d, *J =* 7.6 Hz, 1H), 7.78 (d, *J* = 8.0 Hz, 1H), 7.52 - 7.63 (m, 2H), 7.43 - 7.49 (m, 2H), 7.39 - 7.43 (m, 1H), 7.31 - 7.37 (m, 2H), 7.25 (s, 1H), 7.10 *(d, J =* 8.8 Hz, 1H), 7.02 (d, J=7.2 Hz, 1H), 6.94 *(d, J =* 8.8 Hz, 1H), 6.58 (t, *J =* 5.6 Hz, 1H), 5.04 (dd, *J* = 12.8, 5.4 Hz, 1H), 4.97 (s, 2H), 3.89 (t, *J* = 6.0 Hz, 2H), 3.67 (s, 2H), 3.55 - 3.60 (m, 2H), 3.47 - 3.53 (m, 4H), 3.41 - 3.46 (m, 4H), 3.38 (dd, *J* = 6.0, 3.6 Hz, 4H), 3.22 - 3.26 (m, 2H), 3.00 (t, *J* = 5.6 Hz, 2H), 2.81 - 2.92 (m, 1H), 2.05 (s, 3H), 1.96 - 2.03 (m, 1H), 1.90 (s, 3H), 1.60 - 1.66 (m, 3H), 1.49 - 1.57 (m, 9H).

### Synthesis of APF4:

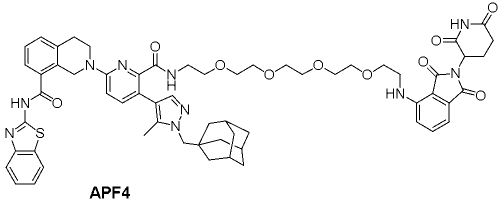

The synthesis procedure for the PROTAC molecule APF4 was similar to that for APF1, but the starting materials were replaced with Compound 1 and APF4-1, and the target product APF4 was obtained by purification after synthesis, as a yellow solid, with a purity of 93.4% and a yield of approximately 20.7%.

**LCMS:** EC12165-2-P1C, RT = 0.572 min, M/Z (M+Na⁺) = 1155.2
**HNMR:** EC12165-2-P1C, DMSO, 400MHz *δ* 12.84 (s, 1H), 11.09 (s, 1H), 8.17 (t, *J* = 5.6 Hz, 1H), 8.01 (d, *J* = 8.0 Hz, 1H), 7.78 (d, *J* = 8.0 Hz, 1H), 7.53 - 7.64 (m, 2H), 7.43 - 7.50 (m, 2H), 7.39 - 7.43 (m, 1H), 7.31 - 7.38 (m, 2H), 7.25 (s, 1H), 7.11 (d, *J* = 8.4 Hz, 1H), 7.02 (d, *J* = 7.2 Hz, 1H), 6.95 (d, *J* = 8.8 Hz, 1H), 6.58 (t, *J =* 5.6 Hz, 1H), 5.04 (dd, *J* = 12.8, 5.2 Hz, 1H), 4.98 (s, 2H), 3.89 (t, *J* = 6.0 Hz, 2H), 3.67 (s, 2H), 3.56 - 3.61 (m, 2H), 3.50 - 3.54 (m, 2H), 3.47 - 3.50 (m, 2H), 3.45 (s, 4H), 3.39 - 3.44 (m, 4H), 3.35 - 3.39 (m, 4H), 3.22 - 3.26 (m, 2H), 3.00 (t, *J* = 5.6 Hz, 2H), 2.87 (ddd, *J* = 17.2, 14.0, 5.2 Hz, 1H), 2.05 (s, 3H), 1.97 - 2.03 (m, 1H), 1.91 (s, 3H), 1.60 - 1.66 (m, 3H), 1.49 - 1.58 (m, 9H).

### Synthesis of APF1A:

### Synthesis scheme:

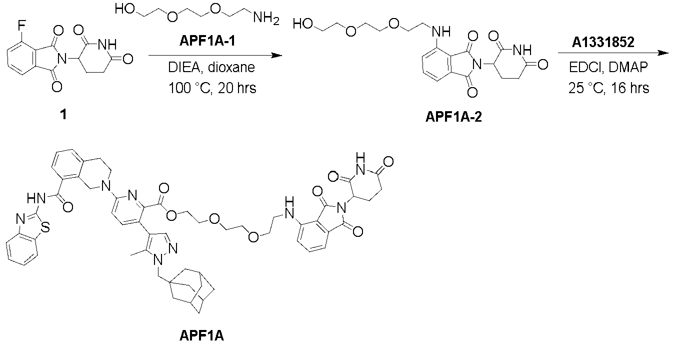

### 1. Synthesis of Compound APF1A-2

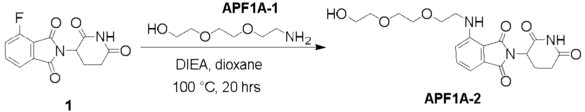

To a solution of Compound 1 (500 mg, 1.81 mmol, 1.00 eq.) and Compound APF1A-1 (405 mg, 2.72 mmol, 1.50 eq.) dissolved in dioxacyclohexane (10.0 mL), DIEA (1.40 g, 10.9 mmol, 1.89 mL, 6.00 eq.) was added, and the mixture was stirred and reacted at 100°C for 20 hours. LCMS (EC11255-19-P1a, P1 RT = 0.283 minutes) showed that Compound 1 was fully reacted, occurring multiple new compound peaks, where the target compound accounted for approximately 55.5%. Dioxacyclohexane was removed from the mixture under reduced pressure. The mixture was diluted by adding water 10.0 mL, and extracted with ethyl acetate 30 mL (10.0 mL×3). The resulting organic layer was washed with 10 mL saline, dried over Na₂SO₄, filtered and concentrated under reduced pressure to provide the crude product, which was purified by column chromatography (SiO2, PE: EA=1/0 to 9/1) to provide Compound APF1A-2 (140 mg, 345 µmol, 19.1% yield) as a light yellow oil. LC-MS (EC11255-19-P1z, P1 RT = 0.272 minutes) showed that the purity was approximately 97.2%.

**LCMS:** EC11255-19-P1a, P1 RT = 0.283 min, MS (ESI) *m*/*z* = 406.3 [M+H]⁺
**LCMS:** EC11255-19-P1z, P1 RT = 0.272 min, MS (ESI) *m*/*z* = 406.3 [M+H]⁺.

### 2. Synthesis of Compound APF1A

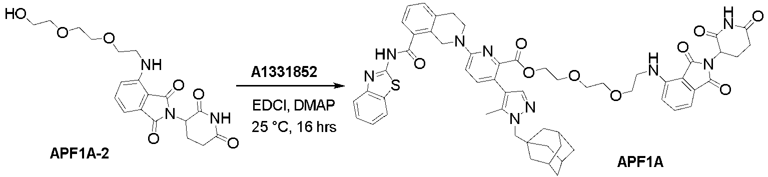

Compound A1331852 (100 mg, 151 µmol, 1.00 eq.), APF1A-2 (73.8 mg, 182 µmol, 1.20 eq.), DMAP (37.1 mg, 303 µmol, 2.00 eq.), and EDCl (116 mg, 607 µmol, 4.00 eq.) were mixed in DCM (1 mL). The air was removed and replaced with N₂, and the mixture was stirred and reacted at 25°C for 16 hours. LC-MS (EC11272-31-P1A, P1, RT = 0.594 minutes) showed that Compound A1331852 was fully reacted, and the reaction mixture was concentrated under vacuum to obtain a crude product, which was further purified by Prep-HPLC (alkaline conditions) to provide Compound APF1A (20.2 mg, 18.8 µmol, 12.4% yield, 97.3% purity) as a yellow solid.
**LCMS:** EC11272-31-P1A, RT = 0.569 min, M/Z (M/2+H⁺) = 524.2
**LCMS:** EC11272-31-P1C, RT = 0.593 min, M/Z (M+H⁺) = 1446.3
**HNMR:** EC11272-31-P1C, DMSO, 400MHz *δ* 12.85 (s, 1H), 11.08 (s, 1H), 8.02 (d, *J* = 7.6 Hz, 1H), 7.78 (d, *J* = 8.0 Hz, 1H), 7.60 (d, *J* = 8.0 Hz, 1H), 7.50 - 7.56 (m, 2H), 7.40 - 7.48 (m, 2H), 7.35 (q, *J* = 7.6 Hz, 2H), 7.24 (s, 1H), 7.08 (d, *J* = 8.2 Hz, 1H), 6.94 - 7.03 (m, 2H), 6.56 (t, *J* = 5.6 Hz, 1H), 5.00 - 5.07 (m, 1H), 4.95 (s, 2H), 4.02 - 4.09 (m, 2H), 3.84 (t, *J* = 5.6 Hz, 2H), 3.68 (s, 2H), 3.56 (t, *J* = 5.2 Hz, 2H), 3.45 - 3.49 (m, 2H), 3.40 (d, *J* = 5.2 Hz, 4H), 3.29 (s, 2H), 3.00 (t, *J* = 5.6 Hz, 2H), 2.77 - 2.92 (m, 2H), 2.07 (s, 3H), 1.95 - 2.04 (m, 2H), 1.89 (s, 3H), 1.58 - 1.64 (m, 3H), 1.47 - 1.55 (m, 9H).

### b) Synthesis of PROTAC molecules APR1, APR2, APR3, and APR4

### Synthesis scheme:

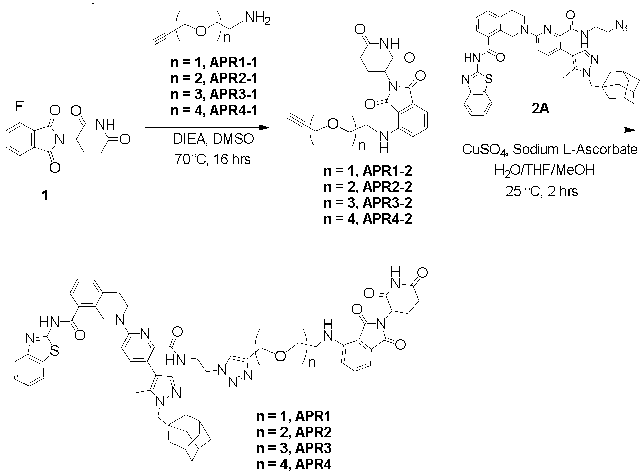

### Synthesis of APR1:

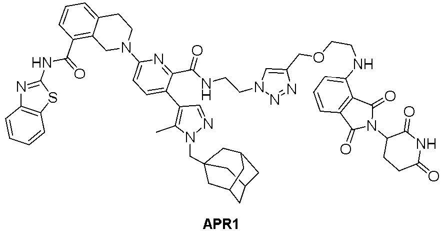

### 1. Synthesis of Compound APR1-2

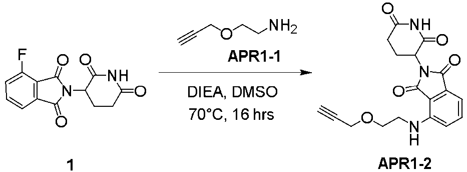

To a solution of Compound 1 (1.50 g, 5.43 mmol, 1.00 eq.) and Compound APR1-1 (538 mg, 5.43 mmol, 1.00 eq.) dissolved in DMSO (15.0 mL), DIPEA (1.40 g, 10.8 mmol, 1.89 mL, 2.00 eq.) was added, and the mixture was stirred and reacted at 70°C for 16 hours. LC-MS (EC7111-89-p1a1, rt [P1]= 0. 362 minutes) showed that Compound 1 was completely reacted, occurring multiple new compound peaks, where the target compound accounted for approximately 35.3%. The reaction mixture was diluted by adding water 30.0 mL, and extracted with ethyl acetate 60 mL (20.0 mL×3). The resulting organic layer was washed with 30 mL of saline, dried over Na₂SO₄, filtered and concentrated under reduced pressure to provide the crude product, which was purified by column chromatography (SiO2, PE: EA=5/1 to 0/1), and further purified by Prep-HPLC (neutral conditions, column: Phenomenex C18 250*50mm*10 µm; mobile phase: [water (NH₄HCO₃)-ACN]; B%: 22%-52%, 9minutes) to provide Compound APR1-2 (0.522 g, crude) as a yellow solid.

**LCMS:** EC7111-89-plal, RT = 0.362 min. M/Z (M+H⁺) = 356.3.

### 2. Synthesis of Compound APR1

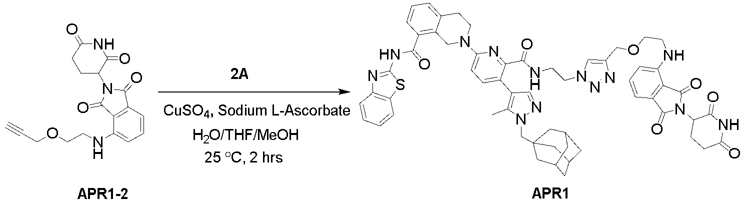

Intermediate Compound 2A (100 mg, 137µmol, 1.00 eq.), APR1-2 (53.8 mg, 151 µmol, 1.10 eq.), CuSO4 (548 µg, 3.44 µmol, 0.025 eq.), and sodium (2R)-2-[(1S)-1,2-dihydroxyethyl]-4-hydroxy-5-oxo-2H-furan-3-oate (1.36 mg, 6.88 µmol, 0.05 eq.) were mixed in THF (4.0 mL), water (1.0 mL) and methanol (6.0 mL). The air was removed and replaced with N₂, and the mixture was stirred and reacted at 25°C for 3 hours. LC-MS (EC12165-6-P1A, P1: RT = 0.508 minutes) showed that Compound 2A was fully reacted. To the reaction mixture, water 20.0 mL was added 0°C to quench the reaction. The mixture was diluted by adding DCM 20 mL, and extracted with ethyl acetate 60 mL (20.0 mL×3). The resulting organic layer was washed with 20 mL saturated saline, dried over Na₂SO₄, filtered and concentrated under reduced pressure to provide the crude product, which was purified by Prep-HPLC (neutral conditions) to provide Compound APR1 (60.0 mg, 55.4 µmol, 40.3% yield) as a yellow solid. LC-MS (EC12165-6-P1C, P1 RT = 0.515 minutes) showed that the purity was 96.0%.
**LCMS:** EC12165-6-P1a, RT = 0.508 min, M/Z (M+H⁺) = 1082.7
**LCMS:** EC12165-6-P1C, RT = 0.515 min, M/Z (M+H⁺) = 1083.6
**HNMR:** EC12165-6-P1C, DMSO, 400MHz *δ* 12.85 (s, 1H), 11.09 (s, 1H), 8.48 (t, *J* = 6.0 Hz, 1H), 7.99 (d, *J =* 7.8 Hz, 1H), 7.93 (s, 1H), 7.76 (d, *J =* 8.0 Hz, 1H), 7.52 - 7.64 (m, 2H), 7.40 - 7.50 (m, 3H), 7.30 - 7.38 (m, 2H), 7.23 (s, 1H), 7.08 (d, J=8.4 Hz, 1H), 7.02 (d, *J =* 7.2 Hz, 1H), 6.97 (d, *J* = 8.8 Hz, 1H), 6.57 (t, *J* = 6.0 Hz, 1H), 4.96 - 5.09 (m, 3H), 4.55 (s, 2H), 4.41 (t, *J =* 6.4 Hz, 2H), 3.90 (t, *J* = 6.0 Hz, 2H), 3.67 (s, 2H), 3.59 - 3.63 (m, 2H), 3.53 - 3.57 (m, 2H), 3.43 (q, *J* = 5.6 Hz, 2H), 3.01 (t, *J* = 5.6 Hz, 2H), 2.82 - 2.92 (m, 1H), 2.02 (s, 4H), 1.90 (s, 3H), 1.58 - 1.65 (m, 3H), 1.49 - 1.56 (m, 9H).

### Synthesis of APR2:

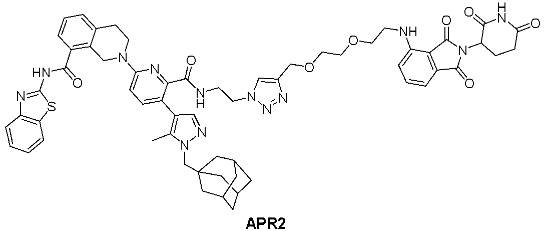

The synthesis procedure for the PROTAC molecule APR2 was similar to that for APR1, but the starting materials were replaced with Compound 1 and APR2-1, and the target product APR2 was obtained by purification after synthesis, as a yellow solid, with a purity of 96.4% and a yield of approximately 24.4%.

**LCMS:** EC12165-5-P1C, RT = 0.503 min, M/Z (M+H⁺) = 1126.7
**HNMR:** EC12165-5-P1C, DMSO, 400MHz *δ* 12.85 (s, 1H), 11.09 (s, 1H), 8.48 (t, *J* = 6.0 Hz, 1H), 7.99 (d, *J* = 8.0 Hz, 1H), 7.90 (s, 1H), 7.75 (d, *J =* 8.4 Hz, 1H), 7.52 - 7.62 (m, 2H), 7.40 - 7.49 (m, 3H), 7.29 - 7.38 (m, 2H), 7.22 (s, 1H), 7.10 *(d, J =* 8.8 Hz, 1H), 7.02 (d, *J =* 7.2 Hz, 1H), 6.96 (d, *J =* 8.8 Hz, 1H), 6.58 (t, *J =* 5.6 Hz, 1H), 5.04 (dd, *J =* 12.8, 5.2 Hz, 1H), 4.99 (s, 2H), 4.49 (s, 2H), 4.39 (t, *J* = 6.0 Hz, 2H), 3.90 (t, *J* = 6.0 Hz, 2H), 3.67 (s, 2H), 3.54 - 3.59 (m, 4H), 3.52 (s, 4H), 3.42 (q, *J* = 5.6 Hz, 2H), 3.00 (t, *J* = 5.6 Hz, 2H), 2.80 - 2.92 (m, 1H), 2.01 (s, 3H), 1.99 (s, 1H), 1.90 (s, 3H), 1.59 - 1.65 (m, 3H), 1.49 - 1.55 (m, 9H).

### Synthesis of APR3:

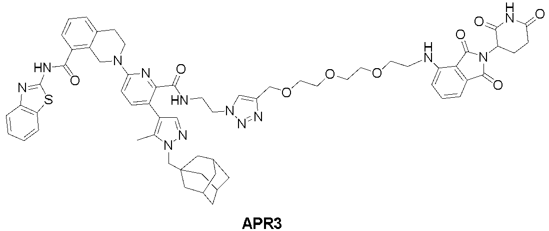

The synthesis procedure for the PROTAC molecule APR3 was similar to that for APR1, but the starting materials were replaced with Compound 1 and APR3-1, and the target product APR3 was obtained by purification after synthesis, as a yellow solid, with a purity of 97.7% and a yield of approximately 18.2%.

**LCMS:** EC12286-2-p1A, RT = 0.511 min, MS (M+H⁺) = 1171.7
**¹H NMR:** EC12886-2-P1C (400 MHz,DMSO) *δ* 12.01 (s, 1 H), 10.25 (s, 1 H), 7.58 - 7.67 (m, 1 H), 7.14 (d, *J =* 7.6 Hz, 1 H), 7.05 (s, 1 H), 6.91 (d, *J =* 8.0 Hz, 1 H), 6.67 - 6.78 (m, 2 H), 6.59 (dd, *J* = 18.0, 8.4 Hz, 3 H), 6.45 - 6.54 (m, 2 H), 6.37 (s, 1 H), 6.26 (d, *J =* 8.4 Hz, 1 H), 6.17 (d, *J =* 7.2 Hz, 1 H), 6.12 (d, *J* = 8.8 Hz, 1 H), 5.73 (t, *J* = 5.6 Hz, 1 H), 4.08 - 4.25 (m, 3 H), 3.62 (s, 2 H), 3.50 - 3.58 (m, 2 H), 3.05 (t, *J* = 5.2 Hz, 2 H), 2.82 (s, 2 H), 2.54 - 2.77 (m, 16 H), 2.13 - 2.20 (m, 2 H), 1.96 - 2.08 (m, 1 H), 1.16 (s, 4 H), 1.05 (s, 3 H), 0.74 - 0.81 (m, 3 H), 0.60 - 0.69 (m, 9 H).

### Synthesis of APR4:

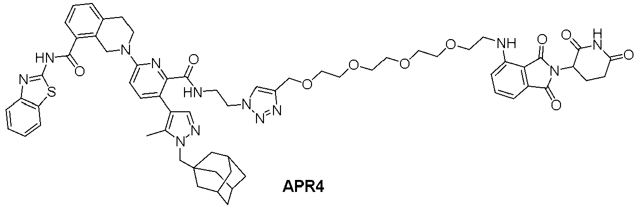

The synthesis procedure for the PROTAC molecule APR4 was similar to that for APR1, but the starting materials were replaced with Compound 1 and APR4-1, and the target product APR4 was obtained by purification after synthesis, as a light yellow solid, with a purity of 92.8% and a yield of approximately 27.4%.

**LCMS:** EC12886-1-p1a1, P1: RT = 0.500 min, MS (M+H⁺) = 1215.7
**¹H NMR:** ¹HNMR (EC12886-1-P1B4) (400 MHz, DMSO) *δ* 12.00 (s, 1H), 10.26 (s, 1H), 7.65 (t, *J*= 5.6 Hz, 1H), 7.15 (d, *J* = 5.2 Hz, 1H), 7.07 (s, 1H), 6.86 - 6.98 (m, 1H), 6.77 (d, *J* = 7.2 Hz, 1H), 6.71 - 6.75 (m, 1H), 6.56 - 6.65 (m, 3H), 6.47 - 6.54 (m, 2H), 6.39 (s, 1H), 6.29 (d, *J=* 8.8 Hz, 1H), 6.19 (d, *J=* 7.00 Hz, 1H), 6.05 - 6.17 (m, 1H), 5.76 (t, *J* = 5.6 Hz, 1 H), 4.22 (dd, *J* = 12.8, 5.2 Hz, 1H), 4.16 (s, 2H), 3.64 (s, 2H), 3.57 (d, *J* = 6.6 Hz, 2H), 3.01 - 3.14 (m, 2H), 2.83 (s, 2H), 2.74 - 2.78 (m, 3H), 2.69 - 2.71 (m, 2H), 2.65 - 2.67 (m, 4H), 2.59 - 2.64 (m, 10H), 2.18 (d, *J* = 5.6 Hz, 2H), 1.99 - 2.09 (m, 1H), 1.18 (s, 4 H), 1.07 (s, 3H), 0.76 - 0.81 (m, 3H), 0.67 - 0.72 (m, 9H).

### Synthesis of Intermediate Compound 2A:

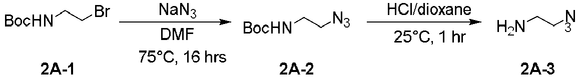

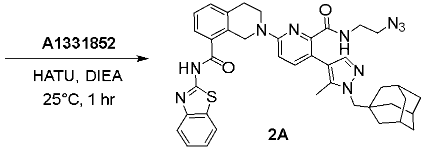

### 1. Synthesis of Compound 2A-2

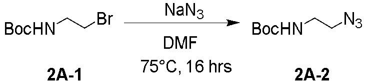

To a solution of Compound 2A-1 (10.0 g, 44.6 mmol, 1.00 eq.) dissolved in DMF (100 mL), NaN₃ (4.35 g, 66.9 mmol, 1.50 eq.) was added, and the mixture was stirred and reacted at 75°C for 16 hours. TLC (PE:EA=5:1, P1, R_{f} = 0.4) showed that Compound 2A-1 was completely reacted, and a high polar main peak was generated. The reaction was allowed to return to room temperature after completion, and 16.5 g of KOH was added gradually in small portions. The mixture was extracted with ethyl acetate 200 mL (40 mL×5) after returning to room temperature. The resulting organic layer was dried over Na₂SO₄, filtered and concentrated under reduced pressure to provide the crude product, which was purified by column chromatography (SiO₂, PE/EA=10/1 to 5/1, PE: EA = 5:1, P1, R_{f} = 0.4) to provide Compound 2A-2 (7.50 g, 40.3 mmol, 90.2% yield) as a white oil.

### 2. Synthesis of Compound 2A-3

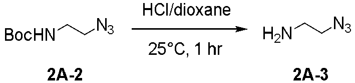

Compound 2A-2 (2.00 g, 10.7 mmol, 1.00 eq.) was added into hydrochloric acid/dioxacyclohexane (4 M, 26.8 mL, 10.0 eq.). The air was removed and replaced with N₂, and the mixture was stirred and reacted at 25°C under N₂ atmosphere for 1 hour. TLC (PE:EA=5:1, R1, R_{f} = 0.4) showed that Compound 2A-2 was fully reacted. The reaction mixture was concentrated under vacuum to obtain a crude product which was used directly in next reaction without further purification. Compound 2A-3 (1.20 g, 9.79 mmol, 91.16% yield, HCl) was obtained, as a white solid.

### 3. Synthesis of Compound 2A

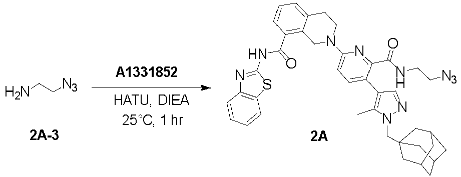

Compound A1331852 (200 mg, 303.58 µmol, 1 eq.), 2-azidoethane (186 mg, 1.52 mmol, 5.00 eq., HCl), HATU (173 mg, 455 µmol, 1.5.0 eq.), and DIEA (196 mg, 1.52 mmol, 264 µL, 5.00 eq.) were mixed in DCM (5 mL), and the air was removed and replaced with N₂. The mixture was stirred and reacted at 25°C under N₂ atmosphere for 1 hour. LC-MS (EC11272-32-P1A, P1, RT = 0.590 minutes) showed that Compound A1331852 was fully reacted. The reaction mixture was concentrated under vacuum to obtain a crude product., which was purified by column chromatography (SiO₂, PE: EA=3:1 to 2:1, PE: EA=2:1, P1: R_{f} = 0.3) to provide Compound 2A (90 mg, 112 µmol, 36.9% yield, 90.6% purity) as a creamy white solid.

**LCMS:** EC11272-32-P1A, RT = 0.590 min. M/Z (M+H⁺) = 727.4.

### Example 5. Synthesis of A1331852-deuterated compounds as PROTAC targeting ligands and corresponding deuterated PROTAC molecules (ligands A13D-1, A13D-2, A13D-5, A13D-6, as well as the deuterated PROTAC molecule A13D6-PF3)

### Synthesis scheme:

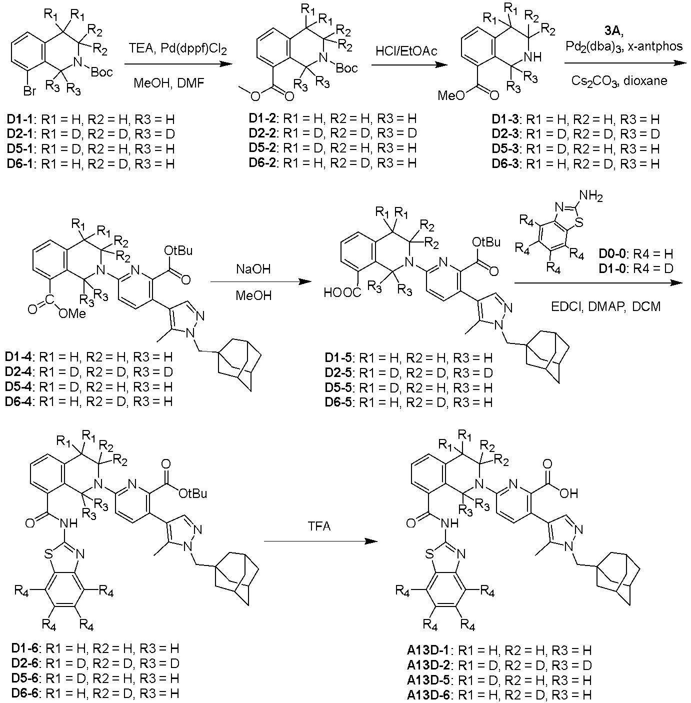

### Synthesis of A13D-1:

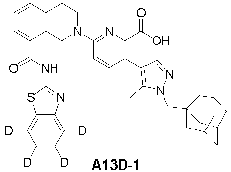

### Synthesis scheme:

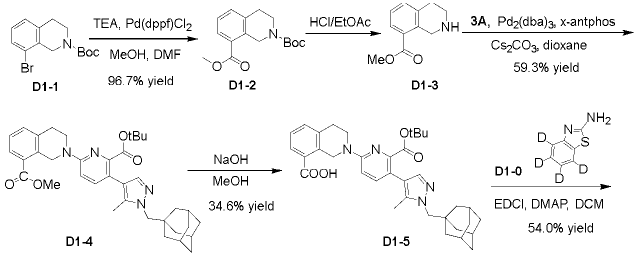

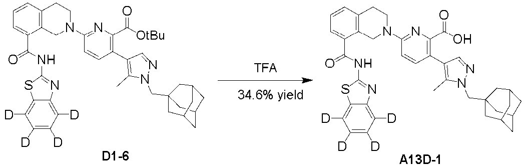

### 1. Synthesis of Compound D1-2

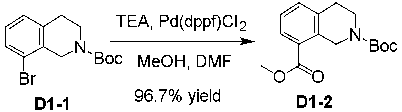

To Compound D1-1 (4.00 g, 12.8 mmol, 1.00 eq.), methanol (32.0 mL), DMF (8.00 mL), TEA (3.89 g, 38.4 mmol, 5.35 mL, 3.00 eq.), and Pd(dppf)Cl₂ (937 mg, 1.28 mmol, 0.10 eq.) were added at 25°C, and the mixture was stirred and reacted at 110°C under CO (2.00 Mpa) atmosphere for 24 hours. TLC (petroleum ether: ethyl acetate = 2:1) showed that Compound D1-1 was reacted completely, and a new compound main peak (R_{f} = 0.51) was generated. The reaction mixture was added water (50.0 mL) and ethyl acetate (100 mL), and extracted with ethyl acetate (50.0 mL×2). The resulting organic layer was washed with saturated saline (50.0 mL×5), dried over Na₂SO₄, filtered, and concentrated to provide the crude product. The crude product (TLC: petroleum ether: ethyl acetate = 2:1, R_{f} = 0.51) was purified by column chromatography (ethyl acetate: petroleum ether =0-5%) to provide Compound D1-2 (3.61 g, 12.4 mmol, 96.7% yield) as a yellow oil.

**¹H NMR:** EB8953-5-P1N2 (400 MHz, DMSO-*d*₆) δ ppm 1.41 (s, 9H), 2.85 (t, *J* = 6.40 Hz, 2H), 3.55 (br t, *J* = 5.60 Hz, 2H), 3.83 (s, 3H), 4.73 - 4.90 (m, 2 H), 7.26 - 7.36 (m, 1 H), 7.41 (d, *J* = 7.20 Hz, 1 H), 7.76 (d, *J* = 7.60 Hz, 1 H).

### 2. Synthesis of Compound D1-3

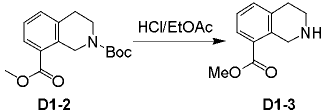

Under N₂ atmosphere, ethyl acetate (2.00 mL) and HCl/ethyl acetate (7.00 mL) were added at 25°C to Compound D1-**2** (1.02 g, 3.50 mmol, 1.00 eq.), and the mixture was stirred and reacted at 25°C for 2 hours. TLC (petroleum ether: ethyl acetate = 3:1) showed that Compound D1-2 was reacted completely, and a new compound main peak (R_{f} = 0.00) was generated. The reaction mixture was concentrated to provide the crude product Compound D1-3 (0.80 g).

### 3. Synthesis of Compound D1-4

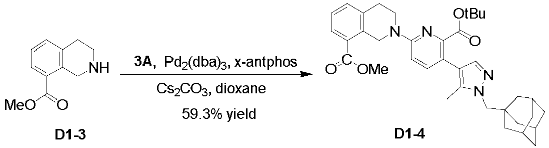

To Compound 3A (500 mg, 1.13 mmol, 1.00 eq.), dioxacyclohexane (5.00 mL), Compound D1-3 (216 mg, 1.13 mmol, 1.00 eq.), Cs₂CO₃ (1.47 g, 4.52 mmol, 4.00 eq.), x-antphos (131 mg, 226 µmol, 0.20 eq.), and Pd₂(dba)₃ (104 mg, 113 µmol, 0.10 eq.) were added in sequence at 25°C, and the mixture was stirred and reacted at 100°C for 12 hours. LCMS (EB9483-5-IPCL1) showed that Compound D1-3 was reacted completely, and a product having the target molecular weight (Rₜ = 1.96 minutes) was generated. To the reaction mixture, water (100 mL) was added, and extracted with ethyl acetate (100 mL×2). The resulting organic layer was washed with saturated saline (20.0 mL), dried over Na₂SO₄, filtered, and concentrated to provide the crude product, which was purified by column chromatography (petroleum ether: ethyl acetate = 100:1 to 10:1) to provide Compound D1-4 (400 mg, 670 µmol, 59.25% yield) as a yellow solid.

**LCMS:** EB9483-5-IPCL1, product: Rₜ = 1.964 min, MS (ESI) *m*/*z* = 597.4 [M+H]⁺
**¹H NMR:** EB9483-5-P1N1 (400 MHz, DMSO-*d*₆) δ ppm 1.32 (s, 10 H) 1.51 - 1.70 (m, 13 H) 1.92 (br s, 3H) 2.11 (s, 3 H) 2.98 (br t, *J =* 5.60 Hz, 2 H) 3.72 (s, 2 H) 3.80 - 3.95 (m, 5 H) 5.03 (s, 2 H) 6.94 (d, *J*= 8.80 Hz, 1 H) 7.23 (s, 1 H) 7.29 - 7.37 (m, 1 H) 7.41 - 7.52 (m, 2 H) 7.76 (dd, *J* = 7.60, 1.20 Hz, 1 H).

### 4. Synthesis of Compound D1-5

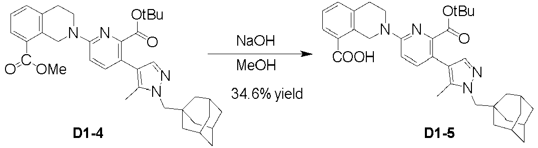

To Compound D1-4 (320 mg, 536 µmol, 1.00 eq.), methanol (2.50 mL), NaOH (42.9 mg, 1.07 mmol, 2.00 eq.), and H₂O (0.50 mL) were added in sequence at 25°C, and the mixture was stirred and reacted at 25°C for 12 hours. LCMS (EB9483-12-IPCL2) showed that Compound D1-4 was reacted completely, and a product having the target molecular weight (Rₜ = 1.038 minutes) was generated. To the reaction mixture, water (3.00 mL) was added, adjusted to about pH 5 with acetic acid, and extracted with ethyl acetate (20.0 mL×3). The resulting organic layer was washed with saturated saline (50.0 mL), dried over Na₂SO₄, filtered, and then concentrated to provide the crude product, which was purified by Prep-HPLC (column: Xtimate C18 150*40mm*10 µm; mobile phase: [water (NH₄HCO₃) - ACN]; B%: 20%-60%, 25minutes) to provide Compound D1-5 (108 mg, 185 µmol, 34.56% yield) as a yellow solid.

**LCMS:** EB9483-12-IPCL2, product: Rₜ = 1.038 min, MS (ESI) *m*/*z* = 583.3 [M+H]⁺
**¹H NMR:** EB9483-12-P1N1 (400 MHz, DMSO-*d*₆) δ ppm 1.29 (s, 9 H) 1.50 - 1.73 (m, 12 H) 1.88 - 1.96 (m, 3 H) 2.07 (br s, 2 H) 2.88 (br s, 2 H) 3.72 (s, 2 H) 3.82 (br t, *J* = 5.6 Hz, 2 H) 4.99 (s, 2 H) 6.88 (br d, *J =* 8.8 Hz, 1 H) 7.01 - 7.65 (m, 5 H).

### 5. Synthesis of Compound D1-6

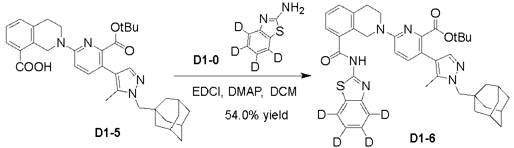

To Compound D1-5 (150 mg, 257 µmol, 1.00 eq.), CH₂Cl₂ (2.00 mL), EDCI (148 mg, 772 µmol, 3.00 eq.), DMAP (94.4 mg, 772 µmol, 3.00 eq.), and Compound D1-0 (59.6 mg, 386 µmol, 1.50 eq.) were added at 0°C in sequence, and the mixture was stirred and reacted at 25°C for 12 hours. LCMS (EB9483-13-IPCL2) showed that Compound D1-5 was reacted completely, and a product having the target molecular weight (Rₜ = 2.057 minutes) was generated. The reaction mixture was adjusted to about pH 6 with acetic acid, and extracted with CH₂Cl₂ (20.0 mL×3). The resulting organic layer was washed with saturated saline (30.0 mL), dried over Na₂SO₄, filtered, and then concentrated to provide the crude product, which was purified by Prep-HPLC (column: Xtimate C18 150*40mm*10 µm; mobile phase: [water (NH₄HCO₃)-ACN]; B%: 58%-98%, 25 minutes) to provide Compound D1-6 (110 mg, 139 µmol, 54.04% yield) as a yellow oil.

**LCMS:** EB9483-13-IPCL2, product: Rₜ = 2.057 min, MS (ESI) *m*/*z* = 719.3 [M+H]⁺
**¹H NMR:** EB9483-13-P1N1, (400 MHz, DMSO-*d*₆) δ ppm 1.21 (s, 9 H) 1.48 - 1.70 (m, 13 H) 1.92 (br s, 3 H) 2.08 (s, 3 H) 3.01 (br t, *J =* 5.60 Hz, 2 H) 3.71 (s, 2 H) 3.81 (br t, *J* = 6.40 Hz, 2 H) 4.89 (s, 2 H) 6.90 (d, *J =* 8.80 Hz, 1 H) 7.21 (s, 1 H) 7.36 (quin, *J* = 7.60 Hz, 2 H) 7.42 - 7.48 (m, 2 H) 10.68 (s, 1 H).

### 6. Synthesis of Compound A13D-1

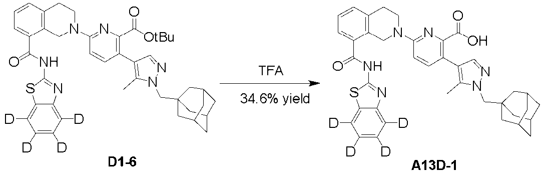

To Compound D1-6 (100 mg, 139 µmol, 1.00 eq.), CH₂Cl₂ (1.00 mL) and TFA(5.12 g, 44.9 mmol, 3.33 mL, 323 eq.) were added at 25°C in sequence, and the mixture was stirred at 25°C for 12 hours. LCMS (EB9484-16-IPCL1) showed that Compound D1-6 was reacted completely, and a product having the target molecular weight (Rₜ = 1.833 minutes) was generated. The reaction mixture was concentrated to obtain a crude product, which was purified by Prep-HPLC (column: Welch Xtimate C18 150*30mm*5µm; mobile phase: [water (FA)-ACN]; B%: 56%-96%, 25 minutes) to provide Compound A13D-1 (30.0 mg, 43.4 µmol, 34.64% yield, 95.8% purity) as a white solid.
**LCMS:** EB9483-16-IPCL1, product: Rₜ = 1.833 min, MS (ESI) *m*/*z* = 663.3 [M+H]⁺
**HPLC:** EB9483-16-P1H5, product: Rₜ = 4.492 min, purity: 95.8%
**HRMS:** X00470-2-P1S2, D2: 0.35%; D3: 3.12%; D4: 94.30%;
**¹H NMR:** EB9483-16-P1N1 (400 MHz, DMSO-*d*₆) δ ppm 1.50 - 1.60 (m, 10 H) 1.62 - 1.70 (m, 3 H) 1.93 (br s, 3 H) 2.10 (s, 3 H) 2.99 (br t, *J =* 5.60 Hz, 2 H) 3.71 (s, 2 H) 3.86 (t, *J* = 6.00 Hz, 2 H) 4.88 (s, 2 H) 6.91 (d, *J =* 8.80 Hz, 1 H) 7.20 (s, 1 H) 7.32 - 7.41 (m, 2 H) 7.44 - 7.52 (m, 2 H) 10.69 (s, 1 H) 12.80 (br s, 1 H).

### Synthesis of A13D-2:

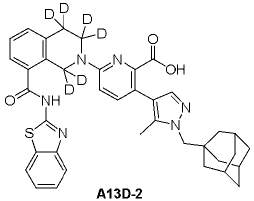

The synthesis procedure for the deuterated molecule A13D-2 was similar to that for A13D-1, but the starting materials were replaced with the Intermediate Compound D2-1 and D0-0, and the target product A13D-2 was obtained by purification after synthesis, as a yellow solid, with a purity of 99.1% and a yield of approximately 18.8%.
**LCMS:** EB9776-40-IPCL2, product: R_{T} =1.793 min, MS (ESI) *m*/*z* = 665.2 [M+H]⁺
**HPLC:** EB9776-40-P1H2, purity 99.1% (area %)
**¹HNMR:** EB9776-40-P1N5 (400 MHz, DMSO-*d*₆) *δ* 8.21 (br s, 1H), 8.03 (br d, *J* = 7.6 Hz, 1H), 7.79 (br d, *J* = 8.0 Hz, 1H), 7.62 (br d, *J =* 7.6 Hz, 1H), 7.51 - 7.32 (m, 5H), 7.28 (s, 1H), 6.89 (br d, *J =* 8.8 Hz, 1H), 3.69 (br s, 2H), 2.10 (s, 3H), 1.92 (br s, 3H), 1.72 - 1.35 (m, 13H).

### Synthesis of A13D-5:

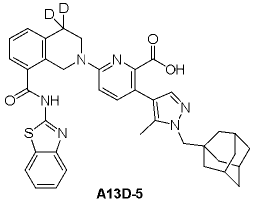

The synthesis procedure for the deuterated molecule A13D-5 was similar to that for A13D-1, but the starting materials were replaced with the Intermediate Compound D5-1 and D0-0, and the target product A13D-5 was obtained by purification after synthesis, as a yellow solid, with a purity of 98.9% and a yield of approximately 42.9%.
**LCMS:** EB9776-26-IPCL2, product: R_{T} = 0.82 min, MS (ESI) *m*/*z* = 661.4 [M+H]⁺
**HPLC:** EB9776-26-P1H1, purity 98.9% (area %)
**¹HNMR:** EB9776-26-P1N3 (400 MHz, DMSO-*d*₆) *δ* 13.27 - 12.28 (m, 1H), 8.04 (d, *J* = 7.6 Hz, 1H), 7.79 (d, *J* = 8.0 Hz, 1H), 7.62 (d, *J* = 7.6 Hz, 1H), 7.52 - 7.32 (m, 5H), 7.29 (s, 1H), 6.92 (br d, *J* = 8.8 Hz, 1H), 4.95 (s, 2H), 3.87 (s, 2H), 3.70 (s, 2H), 2.11 (s, 3H), 1.93 (br s, 3H), 1.69 - 1.49 (m, 13H).

### Synthesis of A13D-6:

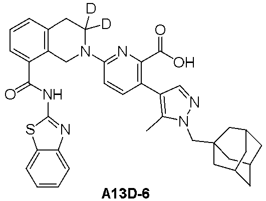

The synthesis procedure for the deuterated molecule A13D-6 was similar to that for A13D-1, but the starting materials were replaced with the Intermediate Compound D6-1 and D0-0, and the target product A13D-6 was obtained by purification after synthesis, as a white solid, with a purity of 98.4% and a yield of approximately 18.8%.
**LCMS:** EB9776-48-IPCL1, product: R_{T} =0.815 min, MS (ESI) *m*/*z* = 661.4 [M+H]⁺
**HPLC:** EB9776-48-P1H3, purity 98.4% (area %)
**¹HNMR:** EB9776-48-P1N1 (400 MHz, DMSO-*d*₆) 8.15 (s, 1H), 8.04 (d, *J =* 7.6 Hz, 1H), 7.79 (d, *J* = 8.0 Hz, 1H), 7.61 (d, *J* = 7.2 Hz, 1H), 7.52 - 7.32 (m, 5H), 7.27 (s, 1H), 6.93 (d, *J* = 8.8 Hz, 1H), 4.95 (s, 2H), 3.70 (s, 2H), 2.99 (s, 2H), 2.10 (s, 3H), 1.92 (br s, 3H), 1.69 - 1.49 (m, 13H).

### Synthesis of Intermediate Compound 3A:

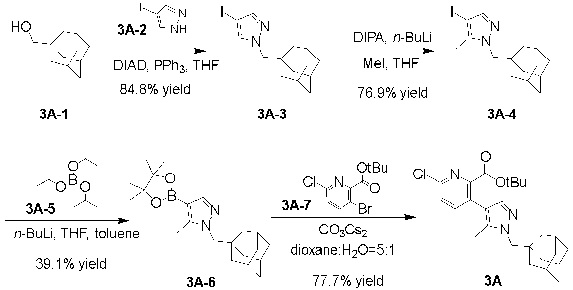

### 1. Synthesis of Compound 3A-3

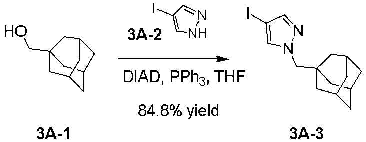

Under N₂ atmosphere, THF (300 mL), Compound 3A-2 (19.3 g, 99.2 mmol, 1.50 eq.), PPh₃ (26.0 g, 99.2 mmol, 1.50 eq.), and DIAD (20.1 g, 99.2 mmol, 19.3 mL, 1.50 eq.) were added to Compound 3A-1 (11.0 g, 66.2 mmol, 1.00 eq.) at 25°C, and the mixture was stirred and reacted at 25°C for 36 hours. LCMS (EB8953-61-IPCL4) showed that Compound 3A-1 was reacted completely, and a product having the target molecular weight (Rₜ = 1.344 minutes) was generated. The reaction mixture was concentrated to obtain a crude product, and the crude product (TLC: petroleum ether: ethyl acetate = 5:1, Rf = 0.71) was purified by column chromatography (ethyl acetate: petroleum ether = 0-5%) to provide Compound 3A-3 as a white solid.

**LCMS:** EB8953-61-IPCL4, product: Rₜ = 1.344 min, MS (ESI) m/z = 557.3 [M+H]⁺
**¹H NMR:** EB8953-61-P1N1 (400 MHz, DMSO-*d*₆) δ ppm 1.42 (d, *J* = 2.00 Hz, 6H), 1.48 - 1.56 (m, 3H), 1.59 - 1.67 (m, 3H), 1.91 (br s, 3H), 3.78 (s, 2H), 7.48 (s, 1H), 7.80 (s, 1H).

### 2. Synthesis of Compound 3A-4

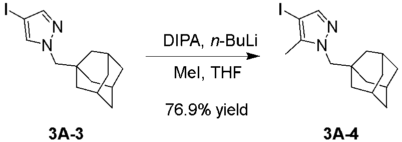

Under N₂ atmosphere, DIPA (6.24 g, 61.7 mmol, 8.72 mL, 1.10 eq.) was added to THF (160 mL) at 25°C. At -78°C, n-BuLi (2.50 M, 29.2 mL, 1.30 eq.) was added and stirred for 30 minutes. At -78°C, Compound 3A-3 (19.2 g, 56.1 mmol, 1.00 eq.) dissolved in THF (80.0 mL) was added and stirred for 1 hour, and at -78°C, MeI (9.08 g, 64.0 mmol, 3.98 mL, 1.14 eq.) was added and stirred for 4 hours. LCMS (EB8953-61-IPCL4) showed that Compound 3A-3 was reacted completely, and a product having the target molecular weight (Rₜ = 1.130 minutes) was generated. To the reaction mixture, a saturated NH₄Cl solution was added at 0°C to quench the reaction. Water (100 mL) and ethyl acetate (200 mL) were, and the mixture was extracted with ethyl acetate (200 mL×2). The resulting organic layer was washed with saturated saline (100 mL), dried over Na₂SO₄, filtered, and then concentrated to provide the crude product, which was ground in MeOH (100 mL) at 25°C to provide the crude product Compound 3A-4, as a white solid.

**LCMS:** EB8953-68-IPCL1, product: Rₜ = 1.130 min, MS (ESI) m/z = 357.0 [M+H]⁺
**¹H NMR:** EB8953-68-P1N1 (400 MHz, CDCl₃) δ ppm 1.55 - 1.76 (m, 12H), 1.99 (br s, 3H), 2.29 (s, 3H), 3.78 (s, 2H), 7.44 (s, 1H).

### 3. Synthesis of Compound 3A-6

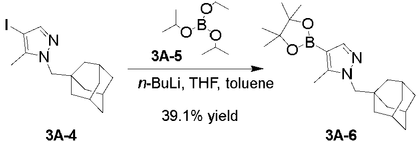

Under N₂ atmosphere, toluene (100 mL), Compound 3A-4 (15.6 g, 43.8 mmol, 1.00 eq.), and triisopropyl borate (14.8 g, 78.8 mmol, 18.1 mL, 1.80 eq.) were added to THF (100 mL) at 25°C. At -78°C, n-BuLi (2.50 M, 26.3 mL, 1.50 eq.) was added and stirred for 4 hours, and at -78°C, 2,3-dimethylbutane-2,3-dione (7.76 g, 65.7 mmol, 1.50 eq.) dissolved in toluene (80.0 mL) wad added. The mixture was slowly warmed to 20°C and maintained for 1 hour, and the mixture was stirred and reacted at 25°C for 12 hours. LCMS (EB8953-69-IPCL1) showed that Compound 3A-4 was reacted completely, and a product having the target molecular weight (Rₜ = 1.145 minutes) was generated. To the reaction mixture, a saturated NH₄Cl solution was added at 0°C to quench the reaction. Water (100 mL) and THF (200 mL) were added, and the mixture was extracted with THF (300 mL). The resulting organic layer was washed with saturated saline (200 mL), dried over Na₂SO₄, filtered, and then concentrated to provide the crude product, which was recrystallized at 25°C in DMSO (180 mL) to provide Compound 3A-6 as a white solid.

**LCMS:** EB8953-69-IPCL1, product: Rₜ = 1.145 min, MS (ESI) m/z = 357.2 [M+H]⁺
**¹H NMR:** EB8953-69-P1N2 (400 MHz, CDCl₃) δ ppm 1.31 (s, 12H), 1.56 - 1.72 (m, 12H), 1.97 (br s, 3H), 2.42 (s, 3H), 3.71 (s, 2H), 7.69 (s, 1 H).

### 4. Synthesis of Compound 3A

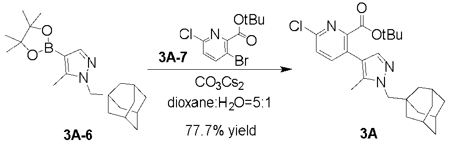

Under N₂ atmosphere, dioxacyclohexane (30.0 mL), water (6.00 mL), Compound 3A-6 (2.80 g, 7.86 mmol, 1.00 eq.), Cs₂CO₃ (7.68 g, 23.6 mmol, 3.00 eq.), and 4-di-tert-butylphosphoyl-N,N-dimethylaniline dichloropalladium (556 mg, 786 µmol, 556 µL, 0.10 eq.) were added to Compound 3A-7 (2.76 g, 9.43 mmol, 1.20 eq.) at 25°C, and the mixture was stirred and reacted at 80°C for 12 hours. TLC (petroleum ether: ethyl acetate = 3:1) showed that Compound 3A-6 was reacted completely, and a new compound main peak (R_{f} = 0.56) was generated. To the reaction mixture, water (200.0 mL) was added, and extracted with ethyl acetate (200.0 mL×2). The resulting organic layer was washed with saturated saline (200 mL), dried over Na₂SO₄, filtered, and then concentrated to provide a crude product. The crude product (TLC: petroleum ether: ethyl acetate = 3:1, R_{f} = 0.51) was purified by column chromatography (petroleum ether: ethyl acetate = 100:1 to 10:1) to provide Compound 3A (2.70 g, 6.11 mmol, 77.7% yield) as a white solid.

**¹H NMR:** EB9483-4-P1N1 (400 MHz, DMSO-*d*₆) δ ppm 1.35 (s, 9 H) 1.49 - 1.70 (m, 12 H) 1.88 - 2.02 (m, 3 H) 2.17 (s, 3 H) 3.77 (s, 2 H) 7.36 (s, 1 H) 7.66 (d, *J* = 8.40 Hz, 1 H) 7.85 (d, *J* = 8.40 Hz, 1 H).

### Synthesis of Intermediate D2-1:

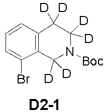

### Synthesis scheme:

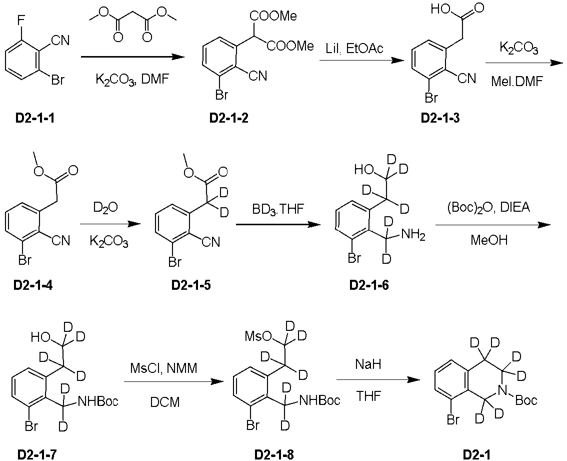

### 1. Synthesis of Compound D2-1-2

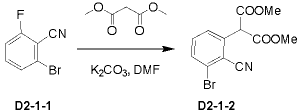

Compound D2-1-1 (19.4 g, 97.0 mmol, 1.00 eq.), dimethyl malonate (19.2 g, 145 mmol, 16.7 mL, 1.50 eq.), and K₂CO₃ (33.5 g, 242 mmol, 2.50 eq.) were mixed in DMF (100 mL) at 25°C, and the mixture was stirred and reacted at 60°C for 12 hours. TLC (petroleum ether: ethyl acetate = 3:1) showed that Compound D2-1-1 was reacted completely, and a new compound main peak (R_{f} = 0.39) was generated. To the reaction mixture, water (200 mL) and ethyl acetate (200 mL) were added. The organic layer was separated, and extracted with ethyl acetate (100 mL×2). The resulting organic layer was washed with saturated saline (200 mL), dried over Na₂SO₄, filtered, and then concentrated to provide a crude product, which was purified by column chromatography (petroleum ether: ethyl acetate = 100:1 to 10:1) to provide Compound D2-1-2 (19.1 g, 61.1 mmol, 63.0% yield) as a white solid.

**¹H NMR:** EB9915-3-P1N1 (400 MHz, DMSO-*d*₆) *δ* 7.92 (dd, *J* = 1.2, 8.1 Hz, 1H), 7.72 - 7.66 (m, 1H), 7.64 - 7.60 (m, 1H), 5.36 (s, 1H), 3.74 (s, 6H).

### 2. Synthesis of Compound D2-1-3

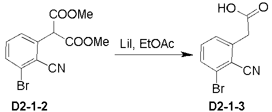

To Compound D2-1-2 (19.1 g, 61.1 mmol, 1.00 eq.), ethyl acetate (190 mL) and LiI (81.8 g, 611 mmol, 23.4 mL, 10.0 eq.) was added at 25°C, and the mixture was stirred and reacted at 80°C for 4 hours. TLC (petroleum ether: ethyl acetate = 2:1) showed that Compound D2-1-2 was reacted completely, and a new compound main peak (R_{f} = 0.19) was generated. To the reaction mixture, citric acid (100 mL) was added to quench the reaction, and the mixture was extracted with ethyl acetate (200 mL×2). The resulting organic layer was washed with saturated saline (200 mL), dried over Na₂SO₄, filtered, and then concentrated to provide a crude product. The crude product (TLC: petroleum ether: ethyl acetate = 2:1, R_{f} = 0.19) was purified by column chromatography (ethyl acetate /petroleum ether =0-10%)) to provide Compound D2-1-3 (5.60 g, 23.3 mmol, 38.2% yield) as a white solid.

**¹H NMR:** EB9915-4-P1N1 (400 MHz, DMSO-*d*₆) *δ* 12.82 (br s, 1H), 7.79 (dd, *J* = 1.2, 7.9 Hz, 1H), 7.64 - 7.50 (m, 2H), 3.90 (s, 2H).

### 3. Synthesis of Compound D2-1-4

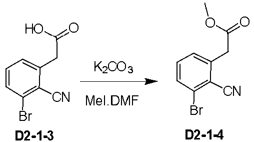

At 25°C, Compound D2-1-3 (1.00 g, 4.17 mmol, 1.00 eq.) and K₂CO₃ (691 mg, 5.00 mmol, 1.20 eq.) were added to DMF, and at 0°C, MeI (887 mg, 6.25 mmol, 389 µL, 1.50 eq.) was added, and the mixture was stirred and reacted at 25°C for 1 hour. TLC (petroleum ether: ethyl acetate = 1:1) showed that Compound D2-1-3 was reacted completely, and a new compound main peak (Rf = 0.40) was generated. To the reaction mixture, water (10.0 mL) was added to quench the reaction, and the mixture was extracted with ethyl acetate (20.0 mL×2). The resulting organic layer was washed with saturated saline (20.0 mL×3), dried over Na₂SO₄, filtered, and then concentrated to provide a crude product. The crude product (TLC: petroleum ether: ethyl acetate = 1:1, R_{f} = 0.40) was purified by column chromatography (ethyl acetate /petroleum ether =0-20%) to provide Compound D2-1-4 (640 mg, 2.52 mmol, 60.5% yield) as a white solid.

**¹H NMR:** EB9915-10-P1N1 (400 MHz, DMSO-*d*₆) *δ* 7.81 (dd, *J* = 1.2, 8.0 Hz, 1H), 7.64 - 7.54 (m, 2H), 4.05 - 3.96 (m, 2H), 3.66 (s, 3H).

### 4. Synthesis of Compound D2-1-5

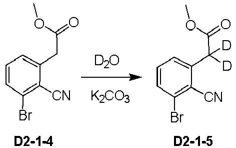

THF (1.00 mL) was added to Compound D2-1-4 (640 mg, 2.52 mmol, 1.00 eq.) at 25°C, and at 0°C, K₂CO₃ (17.4 mg, 126 µmol, 0.05 eq.) dissolved in D₂O (1.01 g, 50.4 mmol, 20.0 eq.) was added, and the mixture was stirred and reacted at 25°C for 12 hours. To the reaction mixture, D₂O (5.00 mL) was added, and extracted with ethyl acetate (20.0 mL×2). The resulting organic layer was dried over Na₂SO₄, filtered, and then concentrated to provide the crude product Compound D2-1-5 (590 mg, 2.30 mmol, 91.5% yield) as a white solid.

**¹H NMR:** EB9915-11-P1N1 (400 MHz, DMSO-*d₆*) *δ* 7.81 (dd, *J* = 1.2, 7.9 Hz, 1H), 7.66 - 7.50 (m, 2H), 4.02 - 3.97 (m, 1H), 3.66 (s, 3H).

### 5. Synthesis of Compound D2-1-6

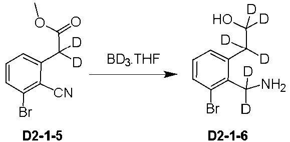

Under N₂ atmosphere, BD₃.THF (0.75 M, 7.65 mL, 3.00 eq.) was added to Compound D2-1-5 (490 mg, 1.91 mmol, 1.00 eq.) at 0°C, and the mixture was stirred and reacted at 25°C for 12 hours. LCMS (EB9776-22-IPCL1) showed that Compound D2-1-5 was fully reacted, and a product having the target molecular weight (Rₜ = 0.335 minutes) was generated. To the reaction mixture, CD₃OD (15.0 mL) was added, and the mixture was stirred and reacted at 80°C for 16 hours. Then, the mixture was concentrated to provide the crude product Compound D2-1-6 (2.30 g) as a yellow oil.

**LCMS:** EB9776-22-IPCL1, product: R_{T} = 0.335 min, MS (ESI) *m*/*z* = 237.5 [M+H]⁺.

### 6. Synthesis of Compound D2-1-7

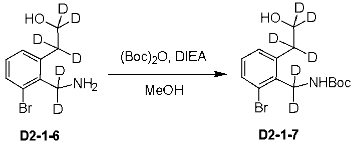

To Compound D2-1-6 (2.46 g, 10.4 mmol, 1.00 eq.), methanol (15.0 mL), DIEA (1.62 g, 12.5 mmol, 2.18 mL, 1.20 eq.), and (Boc)₂O (2.27 g, 10.4 mmol, 2.39 mL, 1.00 eq.) were added, and the mixture was stirred and reacted at 25°C for 16 hours. LCMS (EB9776-30-IPCL1) showed that Compound D2-1-6 was fully reacted, and a product having the target molecular weight (Rₜ = 0.657 minutes) was generated. The reaction mixture was concentrated to obtain a crude product. The crude product (petroleum ether: ethyl acetate = 1:1, R_{f} = 0.25) was purified by column chromatography (ethyl acetate: petroleum ether =0-20%) to provide Compound D2-1-7 (250 mg, 743 µmol, 7.14% yield).

**LCMS:** EB9776-30-IPCL1, product: R_{T} = 0.657 min, MS (ESI) *m*/*z* = 412.3 [M+H]⁺
**¹H NMR:** EB9776-30-P1N1 (400 MHz, DMSO-*d₆*) *δ* 7.46 (dd, *J* = 1.2, 7.9 Hz, 1H), 7.20 (dd, *J* = 1.2, 7.6 Hz, 1H), 7.14 - 7.09 (m, 1H), 5.20 (br s, 1H), 4.52 (br d, *J* = 5.6 Hz, 1H), 3.80 (s, 1H), 3.12 - 3.03 (m, 1H), 1.45 (s, 9H).

### 7. Synthesis of Compound D2-1-8

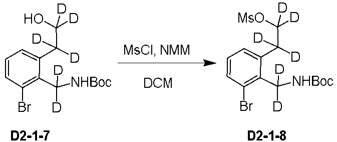

Under N₂ atmosphere, at 0°C, DCM (15.0 mL) and 4-methylmorpholine (749 mg, 7.41 mmol, 814 µL, 3.00 eq.) were added to Compound D2-1-7 (830 mg, 2.47 mmol, 1.00 eq.), and the mixture was stirred at 0°C for 0.5 hours. Then, methanesulfonyl chloride (650 mg, 5.67 mmol, 439 µL, 2.30 eq.) was added, and the mixture was stirred and reacted at 0°C for 2 hours. LCMS (EB9776-32-IPCL1) showed that Compound D2-1-7 was fully reacted, and a product having the target molecular weight (Rₜ = 0.613 minutes) was generated. To the reaction mixture, water (10.0 mL) was added, and extracted with CH₂Cl₂ (10.0 mL×3). The resulting organic layer was washed with saturated saline (10.0 mL), dried over Na₂SO₄, filtered, and then concentrated to provide a crude product. The crude product (petroleum ether: ethyl acetate = 1:1, R_{f} = 0.34) was purified by column chromatography (ethyl acetate: petroleum ether =0-20%) to provide Compound D2-1-8 (780 mg, 1.88 mmol, 76.3% yield) as a yellow oil.

**LCMS:** EB9776-32-IPCL1, product: R_{T} = 0.613 min, MS (ESI) *m*/*z* = 357.9 [M-*t*-Bu+H]⁺
**¹H NMR:** EB9776-32-P1N1 (400 MHz, DMSO-*d₆*) *δ* 7.51 (dd, *J* = 1.2, 8.0 Hz, 1H), 7.24 - 7.19 (m, 1H), 7.17 - 7.08 (m, 1H), 5.08 (br s, 1H), 2.94 (s, 3H), 1.45 (s, 9H).

### 8. Synthesis of Compound D2-1

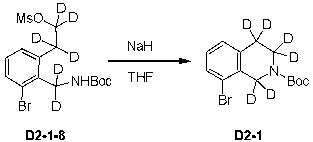

Under N₂ atmosphere, at 0°C, THF (10.0 mL) and NaH (72.2 mg, 1.81 mmol, 60.0% purity, 1.10 eq.) were added to Compound D2-1-8 (680 mg, 1.64 mmol, 1.00 eq.), and the mixture was stirred at 25°C for 3 hours. LCMS (EB9776-34-IPCL1) showed that a product having the target molecular weight (Rₜ = 0.813 minutes) was generated. To the reaction mixture, water (10.0 mL) was added, and extracted with ethyl acetate (10.0 mL×3). The resulting organic layer was washed with saturated saline (10.0 mL), dried over Na₂SO₄, filtered, and then concentrated to provide the product D2-1 (590 mg) as a yellow oil.

**LCMS:** EB9776-34-IPCL1, product: R_{T} = 0.813 min, MS (ESI) *m*/*z* = 217.9 [M-Boc+H]⁺
**¹H NMR:** EB9776-34-P1N1 (400 MHz, CHLOROFORM-d) *δ* 7.42 (dd, *J* = 1.2, 7.6 Hz, 1H), 7.13 - 6.98 (m, 2H), 4.51 (br s, 1H), 3.02 (s, 1H), 1.51 (s, 9H).

### Synthesis of Intermediate D5-1:

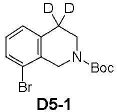

### Synthesis scheme:

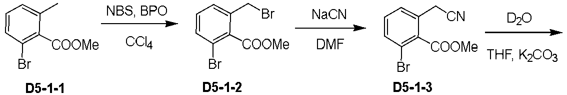

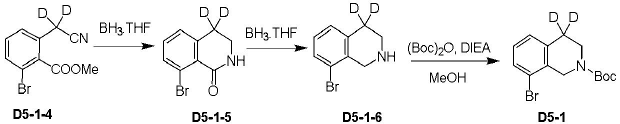

### 1. Synthesis of Compound D5-1-2

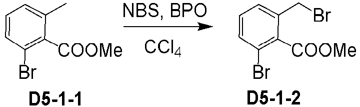

To a midxed solution of Compound D5-1-1 (45.0 g, 196 mmol, 1.00 eq.) dissolved in CCl₄ (500 mL), benzoate peroxide (4.76 g, 19.6 mmol, 0.10 eq.) and NBS (35.0 g, 196 mmol, 1.00 eq.) were added at 25°C under N₂ atmosphere, and the mixture was stirred and reacted at 80°C for 12 hours. TLC (petroleum ether: ethyl acetate = 5:1) showed that Compound D5-1-1 was reacted completely, and a new compound main peak (R_{f} = 0.48) was generated. The reaction mixture was filtered after returning to room temperature. The filtrate was washed with saturated saline (600 mL), dried over Na₂SO₄, filtered and concentrated under reduced pressure to provide a crude product, which was purified by column chromatography (petroleum ether: ethyl acetate = 100:1 to 10:1) to provide Compound D5-1-2 (39.0 g, 127 mmol, 64.5% yield) as a colorless oil.

**¹H NMR:** EB9495-2-P1N1 (400 MHz, DMSO-*d₆*) *δ* 7.69 (dd, *J* = 0.8, 8.1 Hz, 1H), 7.59 (d, *J* = 7.6 Hz, 1H), 7.47 - 7.39 (m, 1H), 4.64 (s, 2H), 3.91 (s, 3H).

### 2. Synthesis of Compound D5-1-3

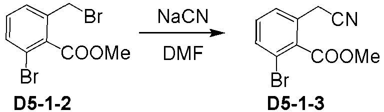

Compound D5-1-2 (34.0 g, 110 mmol, 1.00 eq.) and NaCN (6.43 g, 131 mmol, 1.19 eq.) were mixed in DMF (170 mL) at 25°C, and the mixture was stirred and reacted at 60°C for 6 hours. TLC (petroleum ether: ethyl acetate = 10:1) showed that Compound D5-1-2 was reacted completely, and a new compound main peak was generated. To the reaction mixture, water (300 mL) was added to quench the reaction, and the mixture extracted with ethyl acetate (200 mL×2). The resulting organic layer was washed with saturated saline (200 mL), dried over Na₂SO₄, filtered and concentrated under reduced pressure to provide a crude product, which was subjected to a TLC detection (petroleum ether: ethyl acetate = 10:1, R_{f} = 0.33), and was purified by column chromatography (petroleum ether: ethyl acetate = 10:1 to 10:0) to provide Compound D5-1-3 (20.2 g, 79.5 mmol, 72.0% yield) as a light yellow oil.

**¹H NMR:** EB9527-2-P1N1 (400 MHz, DMSO-*d₆*) *δ* 7.73 (d, *J* = 8.0 Hz, 1H), 7.57 - 7.52 (m, 1H), 7.51 - 7.45 (m, 1H), 4.05 (s, 2H), 3.91 (s, 3H).

### 3. Synthesis of Compound D5-1-4

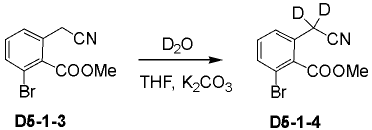

To a mixed solution of Compound K₂CO₃ (136 mg, 984 µmol, 0.05 eq.) dissolved in D₂O (7.88 g, 394 mmol, 20.0 eq.), Compound D5-1-3 (5.00 g, 19.7 mmol, 1.00 eq.) and THF (10.0 mL) were added at 25°C under N₂ atmosphere, and the mixture was stirred and reacted at 25°C for 12 hours. HNMR showed that Compound D5-1-3 was reacted completely, and the target product was generated. The reaction mixture was transferred to a separatory funnel to obtain the water phase, and extracted with MTBE (10.0 mL×2). The resulting organic layer was dried over Na2SO4, filtered and concentrated under reduced pressure to provide the crude product Compound D5-1-4 (4.80 g, 10.0 mmol, 50.9 % yield, 53.4% purity) as a colorless oil.

**¹H NMR:** EB9495-10-P1N1 (400 MHz, DMSO-*d₆*) *δ* 7.73 (dd, *J* = 1.2, 8.0 Hz, 1H), 7.56 - 7.52 (m, 1H), 7.50 - 7.45 (m, 1H), 3.91 (s, 3H)
EB9495-10-P1N2 (400 MHz, DMSO-*d₆*) *δ* 7.73 (dd, *J* = 1.2, 7.9 Hz, 1H), 7.56 - 7.52 (m, 1H), 7.50 - 7.45 (m, 1H), 3.91 (s, 3H).

### 4. Synthesis of Compound D5-1-5

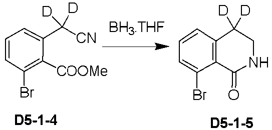

BH₃.THF (1.00 M, 77.3 mL, 6.00 eq.) was added to Compound D5-1-4 (3.30 g, 12.9 mmol, 1.00 eq.) at 0°C under N₂ atmosphere, and the mixture was stirred and reacted at 25°C for 16 hours. LCMS (E B9776-9-IPCL2) showed that Compound D5-1-4 was reacted completely, and a new compound having the target molecular weight (Rₜ =1.070 minutes) was generated. The reaction mixture was transferred to a new bottle, to which methanol (155 mL) was added, and the mixture was stirred and reacted at 80°C for 16 hours. The mixture was concentrated under reduced pressure to obtain a crude product (dichloromethane: methanol =10: 1, R_{f} = 0.20), which was purified by column chromatography (petroleum ether: ethyl acetate = 1:0 to 1:1) to provide Compound D5-1-5 (1.69 g, 7.41 mmol, 57.5% yield) as a yellow oil.

**¹H NMR:** EB9776-9-P1N1 (400 MHz CHLOROFORM-d) *δ* 7.61 (dd, *J =* 1.6, 7.8 Hz, 1H), 7.25 - 7.16 (m, 2H), 6.54 (br s, 1H), 3.48 (d, *J* = 3.6 Hz, 2H)
**LCMS:** EB9776-9-IPCL2, product: R_{T} = 1.070 min, MS (ESI) *m*/*z* = 227.9 [M+H]⁺.

### 5. Synthesis of Compound D5-1-6

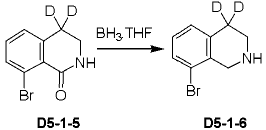

BH₃.THF (1.00 M, 51.9 mL, 7.00 eq.) was added to Compound D5-1-5 (1.69 g, 7.41 mmol, 1.00 eq.) at 0°C under N₂ atmosphere, and the mixture was stirred at 25°C for 16 hours. Then, at 0°C, BH₃.THF (1.00 M, 22.2 mL, 3.00 eq.) was further added, and the mixture was stirred at 25°C for 16 hours. Then, at 0°C, BH₃.THF (1.00 M, 14.8 mL, 2.00 eq.) was further added, and the mixture was stirred at 25°C for 4 hours. Then, at 0°C, BH₃.THF (1.00 M, 22.2 mL, 3.00 eq.) was further added, and the mixture was stirred at 25°C for 4 hours. Then, at 0°C, BH₃.THF (1.00 M, 14.8 mL, 2.00 eq.) was further added, and the mixture was stirred at 25°C for 4 hours. LCMS (EB9776-11-IPCL15) showed that a product with the target molecular weight was yielded (Rₜ = 0.441 minutes). The reaction mixture was transferred to a new bottle, to which methanol (220 mL) was added, and the mixture was stirred and reacted at 80°C for 16 hours. The mixture was concentrated under reduced pressure to obtain a crude product Compound D5-1-6 (2.62 g) as a yellow oil.

**LCMS:** EB9776-11-IPCL15, product: R_{T} = 0.441 min, MS (ESI) *m*/*z* = 214.05 [M+H]⁺.

### 6. Synthesis of Compound D5-1

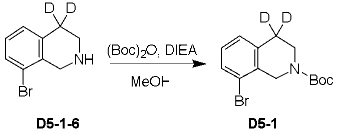

To a mixed solution of Compound D5-1-6 (2.62 g, 12.2 mmol, 1.00 eq.) in methanol (25.0 mL), DIEA (1.90 g, 14.7 mmol, 2.56 mL, 1.20 eq.) and (Boc)₂O (2.67 g, 12.2 mmol, 2.81 mL, 1.00 eq.) were added, and the mixture was stirred and reacted at 25°C for 16 hours. LCMS (EB9776-14-IPCL2) showed that Compound D5-1-6 was reacted completely, and a product with the target molecular weight was yielded (Rₜ = 1.803 minutes). The mixture was concentrated under reduced pressure to obtain a crude product, and the crude product (petroleum ether: ethyl acetate = 5:1, R_{f} = 0.62) was purified by column chromatography to provide Compound D5-1 (870 mg, 2.77 mmol, 22.6% yield) as a yellow oil.

**LCMS:** EB9776-14-IPCL2, product: R_{T} = 1.803 min, MS (ESI) *m*/*z* = 214.0 [M-Boc+H]⁺
**¹H NMR:** EB9776-14-P1N1 (400 MHz CHLOROFORM-d) *δ* 7.42 (dd, *J* = 0.8, 7.7 Hz, 1H), 7.13 - 6.98 (m, 2H), 4.54 (br s, 2H), 3.63 (br s, 2H), 1.51 (s, 9H).

### Synthesis of Intermediate D6-1:

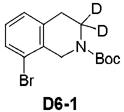

### Synthesis scheme:

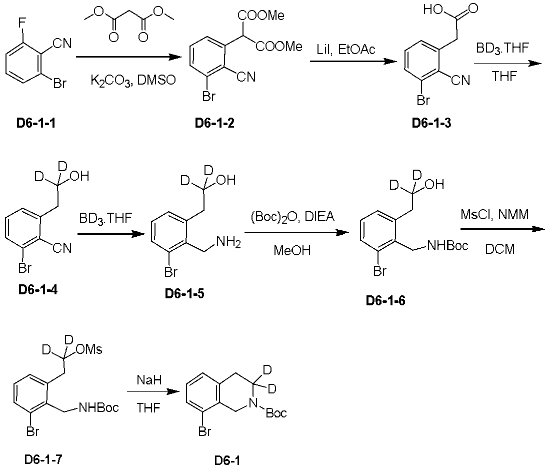

### 1. Synthesis of Compound D6-1-2

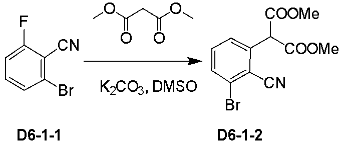

Compound D6-1-1 (10.0 g, 50.0 mmol, 1.00 eq.), dimethyl malonate (9.91 g, 75.0 mmol, 8.59 mL, 1.50 eq.) and K₂CO₃ (17.3 g, 125 mmol, 2.50 eq.) were mixed in DMF (50.0 mL) at 25°C, and the mixture was stirred and reacted at 60°C for 16 hours. TLC (petroleum ether: ethyl acetate = 3:1) showed that Compound D6-1-1 was reacted completely, and a new compound main peak (R_{f} = 0.53) was generated. To the reaction mixture, water (200 mL) and ethyl acetate (200 mL) were added. The organic layer was separated, and extracted with ethyl acetate (100 mL×2). The resulting organic layer was washed with saturated saline (200 mL), dried over Na₂SO₄, filtered, and then concentrated to provide a crude product, which was purified by column chromatography (petroleum ether: ethyl acetate = 100:1 to 10:1) to provide Compound D6-1-2 (7.70 g, 24.7 mmol, 49.3% yield) as a white solid.

**¹H NMR:** EB9495-16-P1N1 (400 MHz, DMSO-*d₆*) *δ* 7.91 *(d, J=* 7.6 Hz, 1H), 7.71 - 7.65 (m, 1H), 7.64 - 7.59 (m, 1H), 5.36 (s, 1H), 3.74 (s, 6H).

### 2. Synthesis of Compound D6-1-3

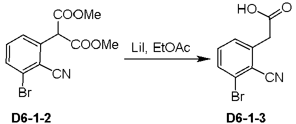

To Compound D6-1-2 (6.70 g, 21.5 mmol, 1.00 eq.), ethyl acetate (70 mL) and LiI (28.7 g, 215 mmol, 8.23 mL, 10.0 eq.) was added at 25°C, and the mixture was stirred and reacted at 80°C for 4 hours. TLC (petroleum ether: ethyl acetate = 3:1) showed that Compound D6-1-2 was reacted completely, and a new compound main peak (R_{f} = 0.34) was generated. To the reaction mixture, citric acid (3.0 mL) was added to quench the reaction, and the mixture was extracted with ethyl acetate (20 mL×2). The resulting organic layer was washed with saturated saline (10 mL), dried over Na₂SO₄, filtered, and then concentrated to provide a crude product. The crude product (TLC: petroleum ether: ethyl acetate = 3:1, R_{f} = 0.34) was purified by column chromatography (ethyl acetate /petroleum ether =0-10%) to provide Compound D6-1-3 (3.90 g, 16.3 mmol, 75.7% yield) as a white solid.

**¹H NMR:** EB9495-20-P1N1 (400 MHz, DMSO-*d₆*) δ 7.79 (dd, *J =* 0.8, 7.9 Hz, 1H), 7.62 - 7.57 (m, 1H), 7.56 - 7.52 (m, 1H), 3.89 (s, 2H).

### 3. Synthesis of Compound D6-1-4

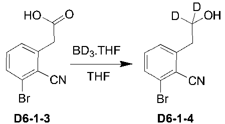

To Compound D6-1-3 (1.70 g, 7.08 mmol, 1.00 eq.), THF (10.0 mL) was added at 0°C, then trideuteroborane (0.75 M, 13.2 mL, 1.40 eq.) was added at 25°C, and the mixture was stirred and reacted at 0°C for 4 hours. TLC (petroleum ether: ethyl acetate = 1:1) showed that Compound D6-1-3 was reacted completely, and a new compound main peak (R_{f} = 0.41) was generated. The reaction mixture was concentrated to obtain a crude product, and the crude product (TLC: petroleum ether: ethyl acetate = 1:1, R_{f} = 0.41) was purified by column chromatography (petroleum ether: ethyl acetate = 100:1 to 10:1) to provide Compound D6-1-4 (1.40 g, 4.91 mmol, 69.3% yield, 80% purity) as a colorless oil.

**LCMS:** EB9495-21-P1L1, product: R_{T} = 1.108 min, MS (ESI) *m*/*z* = 227.8 [M+H]⁺
**¹H NMR:** EB9495-21-P1N1 (400 MHz, DMSO-*d₆*) *δ* 7.72 (dd, *J* = 0.8, 7.8 Hz, 1H), 7.62 - 7.47 (m, 2H), 4.81 (s, 1H), 2.95 (s, 2H).

### 4. Synthesis of Compound D6-1-5

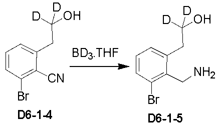

under N₂ atmosphere, to Compound D6-1-4 (400 mg, 1.75 mmol, 1.00 eq.) THF (3.00 mL) was added at 25°C, then BH₃.THF (1.00 M, 5.26 mL, 3.00 eq.) was added at 0°C, and the mixture was stirred and reacted at 25°C for 10 hours. At 0°C, BH₃.THF (1.00 M, 3.51 mL, 2.00 eq.) was added, and the mixture was stirred and reacted at 25°C for 10 hours, then at 0°C, BH₃-Me₂S (10.0 M, 5.00 mL, 28.5 eq.) was further added, and the mixture was stirred and reacted at 25°C for 10 hours. LCMS (EB9495-38-IPCL10) showed that Compound D6-1-4 was reacted completely, and a product with the target molecular weight was yielded (R_{T} = 0.965minutes). To the mixture, methanol (10.0 mL) was added 80°C, and after 12 hours, the reaction was quenched. The mixture was concentrated to provide Compound D6-1-5 (820 mg, crude) as a yellow oil.

**LCMS:** EB9495-38-IPCL10, product: R_{T} = 0.965 min, MS (ESI) *m*/*z* = 231.8 [M+H]⁺.

### 5. Synthesis of Compound D6-1-6

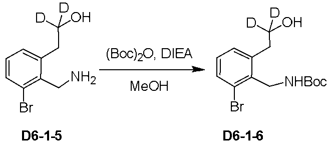

Under N₂ atmosphere, THF (5.00 mL), DIEA (548 mg, 4.24 mmol, 738 µL, 1.20 eq.), and (Boc)₂O (771 mg, 3.53 mmol, 812 µL, 1.00 eq.) were added to Compound D6-1-5 (820 mg, 3.53 mmol, 1.00 eq.) at 25°C, and the mixture was stirred and reacted at 25°C for 4 hours. TLC (petroleum ether: ethyl acetate = 1:1) showed that Compound D6-1-5 was reacted completely, and a new compound main peak (R_{f} = 0.30) was generated. The reaction mixture was concentrated to obtain a crude product, and the crude product (TLC: petroleum ether: ethyl acetate = 1:1, R_{f} = 0.30) was purified by column chromatography (ethyl acetate: petroleum ether =0-20%) to provide Compound D6-1-6 (150 mg, 452 µmol, 12.8% yield) as a yellow oil.

**¹H NMR:** EB9495-42-P1N4 (400 MHz, DMSO-*d₆*) *δ* 7.45 (d, *J =* 7.6 Hz, 1H), 7.23 (d, *J* = 7.6 Hz, 1H), 7.17 - 7.11 (m, 1H), 6.90 (br s, 1H), 4.74 - 4.67 (m, 1H), 4.33 (br d, *J* = 4.8 Hz, 2H), 2.85 (s, 2H), 1.44 - 1.36 (m, 11H).

### 6. Synthesis of Compound D6-1-7

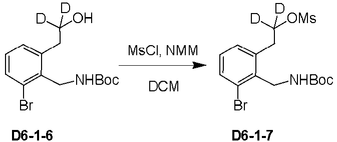

Under N₂ atmosphere, DCM (10.0 mL) and 4-methylmorpholine (365 mg, 3.61 mmol, 397 µL, 3.00 eq.) were added to Compound D6-1-6 (400 mg, 1.20 mmol, 1.00 eq.) at 0°C, and the mixture was stirred at 0°C for 0.5 hours. At 0°C, methanesulfonyl chloride (340 mg, 2.97 mmol, 230 µL, 2.47 eq.) was added, and the mixture was stirred at 0°C for 2 hours. LCMS (EB9776-41-IPCL1) showed that Compound D6-1-6 was reacted completely, and a product with the target molecular weight was yielded (R_{T} = 0.702 minutes). To the reaction mixture, ice-water (10.0 mL) was added, and the mixture was extracted with CH₂Cl₂ (10.0 mL×3). The resulting organic layer was washed with saturated saline (10 mL), dried over Na₂SO₄, filtered, and then concentrated to provide a crude product. The crude product (TLC: petroleum ether: ethyl acetate = 1:1, R_{f} = 0.34) was purified by column chromatography (ethyl acetate /petroleum ether =0-20%) to provide Compound D6-1-7 (450 mg, 1.10 mmol, 91.1% yield) as a yellow oil.

**LCMS:** EB9776-41-IPCL1, product: R_{T} = 0.702 min, MS (ESI) *m*/*z* = 354.0 [M-*t*-Bu+H]⁺
**¹H NMR:** EB9776-41-P1N1 (400 MHz, CHLOROFORM-d) *δ* 7.51 (dd, *J* = 0.8, 7.9 Hz, 1H), 7.23 (d, *J =* 7.2 Hz, 1H), 7.16 - 7.10 (m, 1H), 5.10 (br s, 1H), 4.51 (d, *J* = 6.0 Hz, 2H), 3.31 (s, 2H), 2.94 (s, 3H), 1.47 - 1.42 (m, 9H).

### 7. Synthesis of Compound D6-1

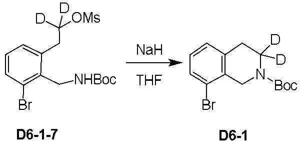

Under N₂ atmosphere, THF (8.00 mL) and NaH (48.3 mg, 1.21 mmol, 60.0% purity, 1.10 eq.) were added to Compound D6-1-7 (450 mg, 1.10 mmol, 1.00 eq.) at 0°C, and the mixture was stirred at 25°C for 3 hours. LCMS (EB9776-42-IPCL2) showed that Compound D6-1-7 was reacted completely, and a product with the target molecular weight was yielded (R_{T} = 0.804 minutes). To the reaction mixture, water (10.0 mL) was added, and the mixture was extracted with ethyl acetate (10.0 mL×3). The resulting organic layer was washed with saturated saline (10 mL), dried over Na₂SO₄, filtered, and then concentrated to provide the crude product Compound D6-1 (330 mg, 1.05 mmol, 95.8%) as a yellow oil.

**LCMS:** EB9776-42-IPCL2, product: R_{T} = 0.804 min, MS (ESI) *m*/*z* = 215.3 [M-Boc+H]⁺
**¹H NMR:** EB9776-42-P1N1 (400 MHz, CHLOROFORM-d) *δ* 7.42 (d, J = 7.6 Hz, 1H), 7.13 - 6.96 (m, 2H), 4.55 (br s, 2H), 2.83 (s, 2H), 1.51 (s, 9H).

### Synthesis of Intermediate D1-0:

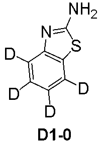

### Synthesis scheme:

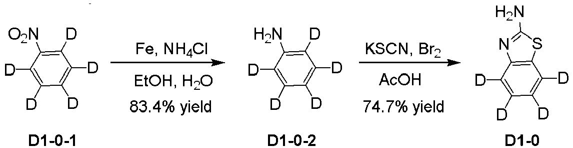

### 1. Synthesis of Compound D1-0-2

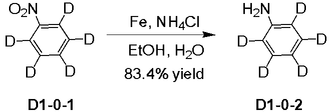

Under N₂ atmosphere, water (60.0 mL), Compound D1-0-1 (10.0 g, 78.0 mmol, 1.00 eq.), Fe (21.8 g, 390 mmol, 5.00 eq.), and NH₄Cl (20.9 g, 390 mmol, 5.00 eq.) were added to ethanol (180 mL) at 25°C, and the mixture was stirred and reacted at 60°C for 12 hours. TLC (petroleum ether: ethyl acetate = 10:1) showed that Compound D1-0-1 was reacted completely, and a new compound main peak (R_{f} = 0.15) was generated. The reaction mixture was filtered after returning to room temperature. The filter cake was washed with ethyl acetate (500 mL×2), and extracted with ethyl acetate (300 mL×2). The resulting organic layer was washed with saturated saline (500 mL), dried over Na₂SO₄, filtered, and then concentrated to provide a crude product. The crude product (TLC: petroleum ether: ethyl acetate = 10:1, R_{f} = 0.15) was purified by column chromatography (ethyl acetate /petroleum ether =0-10%) to provide Compound D1-0-2 (83.4% yield), as a red liquid.

**¹H NMR:** EB8953-2-P1N2 (400 MHz, DMSO-*d*₆) δ ppm 4.98 (br s, 2H).

### 2. Synthesis of Compound D1-0

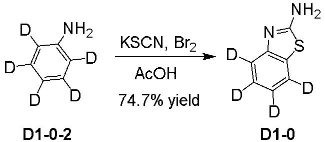

Under N₂ atmosphere, KSCN (4.98 g, 51.3 mmol, 4.98 mL, 3.00 eq.) and Compound D1-0-2 (2.00 g, 17.1 mmol, 1.96 mL, 1.00 eq.) were added to acetic acid (30.0 mL) at 10°C, and the mixture was stirred for 30 minutes. Then, at 10°C, Br₂ (2.73 g, 17.1 mmol, 881 µL, 1.00 eq.) dissolved in acetic acid (10.0 mL) was further added, and the mixture was stirred and reacted at 25°C for 6 hours. LCMS (EB8953-44-IPCL2) showed that Compound D1-0-2 was reacted completely, and a product with the target molecular weight was yielded (Rₜ = 1.068 minutes). The reaction mixture was adjusted to about pH 8 with ammonia (50%), water (100 mL) was added, and extracted with ethyl acetate (100 mL×2). The resulting organic layer was washed with saturated saline (100 mL), dried over Na₂SO₄, filtered, and then concentrated to provide a crude product. The crude product (TLC: petroleum ether: ethyl acetate = 2:1, R_{f} = 0.54) was purified by column chromatography (ethyl acetate /petroleum ether =0-10%) to provide Compound D1-0 (74.7% yield) as a yellow solid.

**LCMS:** EB8953-44-IPCL2, product: Rt = 1.068 min, MS (ESI) m/z = 331.8 [2M+Na]⁺
**¹H NMR:** EB8953-44-P1N1 (400 MHz, DMSO-*d*₆) δ ppm 5.78 (s, 2H).

### Synthesis of the deuterated PROTAC molecule A13D6-PF3:

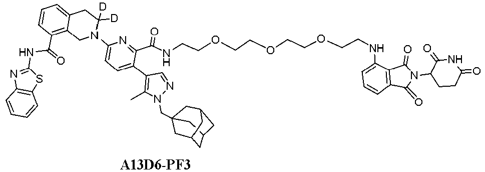

The synthesis procedure for the deuterated molecule A13D6-PF3 was similar to that for the undeuterated molecule APF3, but the starting material A-1331852 was replaced with the deuterated molecule A13D-6, and the target product A13D6-PF3 (12.6 mg, 11.2 µmol, 36.9% yield, 96.9% purity) was obtained by reverse-phase HPLC (neutral conditions), as a yellow solid.

**LCMS:** EC15866-4-P1C2, RT = 0.567 min, M/Z (M+H⁺) = 1091.4
**¹HNMR:** EC15866-4-P1A (400MHz, DMSO-*d₆*) *δ* ppm 12.84 (s, 1H) 11.08 (s, 1H) 8.17 (t, *J* = 5.6 Hz, 1H) 8.01 (d, *J =* 7.6 Hz, 1H) 7.77 (d, *J =* 8.4 Hz, 1H) 7.52-7.62 (m, 2H) 7.43-7.48 (m, 2H) 7.38-7.42 (m, 1H) 7.31-7.37 (m, 2H) 7.25 (s, 1H) 7.10 (d, *J* = 8.4 Hz, 1H) 6.99-7.05 (m, 1H) 6.91-6.98 (m, 1H) 6.58 (t, *J* = 5.6 Hz, 1H) 5.04 (dd, *J* = 12.8, 5.32 Hz, 1H) 4.93-5.01 (m, 2H) 3.67 (s, 2H) 3.55-3.62 (m, 2H) 3.47-3.53 (m, 4H) 3.41-3.46 (m, 4H) 3.37-3.40 (m, 2H) 3.33-3.36 (m, 2H) 3.22-3.26 (m, 2H) 2.95-3.01 (m, 3H) 2.81-2.92 (m, 1H) 2.58 (s, 2H) 2.05 (s, 3H) 1.98-2.02 (m, 1H) 1.90 (s, 3H) 1.60-1.65 (m, 3H) 1.49-1.56 (m, 9H).

### Example 6. Synthesis of PROTAC molecules based on novel ligand structures for recruiting the CRBN-E3 enzyme

### a) Synthesis of APQ5a and APQ5b

### Synthesis of APQ5a:

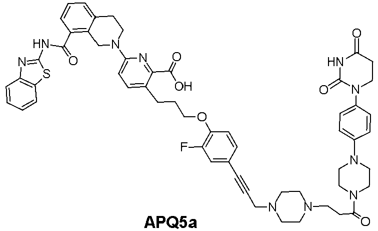

### Synthesis scheme:

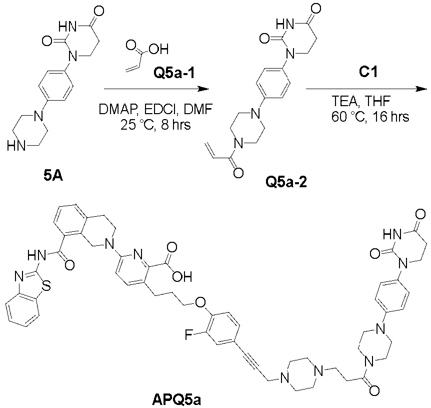

### 1. Synthesis of Compound Q5a-2

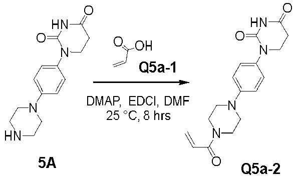

To a solution of Compound 5A (1.00 g, 3.65 mmol, 1.0 eq.) in DMF (20.0 mL), DMAP (22.3 mg, 182 µmol, 0.05 eq.), EDCI (769 mg, 4.01 mmol, 1.1 eq.), and acrylic acid (525 mg, 7.29 mmol, 500 µL, 2.0 eq.) were added, and the mixture was stirred and reacted at 25°C for 8 hours. LC-MS (EC16331-35-P1G, P1: RT = 0.390 min, R1: RT = 0.341 minutes) showed that Compound 5A was reacted completely, and a target product main peak with the expected m/z value was generated. The reaction mixture was concentrated under reduced pressure to remove the solvent and obtain a crude product, which was further purified by reverse-phase HPLC (0.1% NH3·H2O) column chromatography to provide Compound Q5a-2 (200 mg, 609 µmol, 16.7% yield) as a brown solid.

LC-MS: EC16331-35-P1G, product: RT = 0.390 min, m/z = 329.1 [M+H]+.

### 2. Synthesis of Compound APQ5a

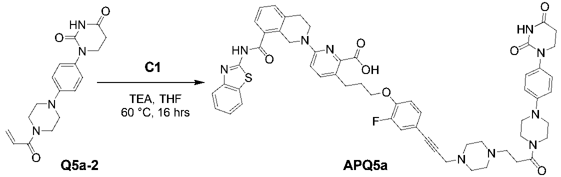

To a solution of Compound C1 (150 mg, 213 µmol, 1.0 eq.) in THF (2.00 mL), TEA (216 mg, 2.13 mmol, 296 µL, 10 eq.) and Compound Q5a-2 (70.0 mg, 213 µmol, 1.0 eq.) were added, and the mixture was stirred and reacted at 25°C for 16 hours. LC-MS (EC16331-41-P1E, P1: RT = 0.413 minutes) showed that Compound Q5a-2 was reacted completely, and a target product main peak with the expected m/z value was generated. The reaction mixture was concentrated under reduced pressure to remove the solvent and obtain a crude product, which was further purified by Prep-HPLC (chromatographic column: Phenomenex luna C18 150 * 25 mm * 10 µm; mobile phase: [water (FA)-ACN]; gradient elution: 31%-51% B, within 15 minutes) column chromatography to provide Compound APQ5a (20.0 mg, 17.9 µmol, 8.39% yield, 92.41% purity) as a white solid.

LC-MS: EC16331-41-P1E, product: RT = 0.413 min, m/z = 1033.5 [M+H]⁺
1H NMR: EC16331-41-P1K (400 MHz DMSO) δ ppm 10.27 (s, 1H), 8.20 (s, 1H), 8.03 (d, J = 7.8 Hz, 1H), 7.79 (d, J = 7.8 Hz, 1H), 7.59 (d, J = 6.6 Hz, 1H), 7.44 - 7.52 (m, 2H), 7.38 - 7.42 (m, 1H), 7.32 - 7.37 (m, 2H), 7.26 (dd, J = 12.0, 2.0 Hz, 1H), 7.14 - 7.19 (m, 3H), 7.05 - 7.11 (m, 1H), 6.94 (d, J = 9.2 Hz, 2H), 6.89 (d, J = 8.8 Hz, 1H), 4.91 (s, 2H), 4.00 (t, J = 6.4 Hz, 2H), 3.85 (t, J = 5.6 Hz, 2H), 3.69 (t, J = 6.6 Hz, 3H), 3.59 (d, J = 3.6 Hz, 5H), 3.45 (s, 3H), 3.13 (s, 3H), 3.07 (s, 3H), 2.97 (t, J = 5.6 Hz, 3H), 2.77 (t, J = 7.6 Hz, 3H), 2.67 (t, J = 6.6 Hz, 3H), 2.54 (s, 4H), 1.92 - 2.00 (m, 2H).

### Synthesis of APQ5b:

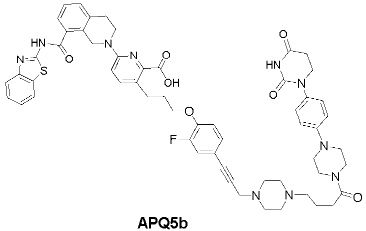

### Synthesis scheme:

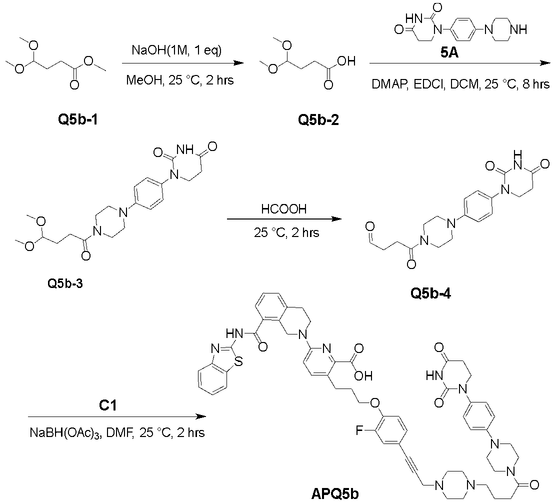

### 1. Synthesis of Compound Q5b-2

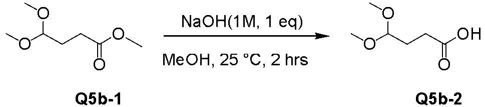

To a solution of Compound Q5b-1 (1.00 g, 6.17 mmol, 1.0 eq.) in methanol (10.0 mL), NaOH (1M, 6.17 mL, 1.0 eq.) was added, and the mixture was stirred and reacted at 25°C for 2 hours. The reaction mixture was adjusted to a pH of less than 6 by HCl (1 M), and extracted with H₂O (20.0 mL) and DCM (30.0 mL, × 3). The resulting organic layer was washed with saturated saline (30.0 mL, × 2), dried over Na₂SO₄, and then concentrated under reduced pressure to provide the crude product Compound Q5b-2 (500 mg, 3.37 mmol, 54.73% yield) as a colorless oil.

### 2. Synthesis of Compound Q5b-3

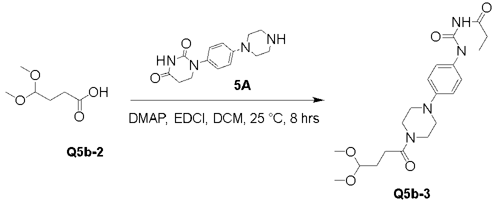

Compound Q5b-2 (500 mg, 3.37 mmol, 1.0 eq.), Compound 5A (925 mg, 3.37 mmol, 1.0 eq.), DMAP (20.6 mg, 168 µmol, 0.05 eq.), and EDCI (711 mg, 3.71 mmol, 1.1 eq.) were mixed in DCM (10.0 mL), and the mixture was stirred and reacted at 25°C under N₂ atmosphere for 8 hours. The reaction mixture was extracted with H₂O (10.0 mL) and DCM (10.0 mL, 3). The resulting organic layer was washed with saturated saline (20.0 mL, × 2), dried over Na₂SO₄, and then concentrated under reduced pressure to provide the crude product Compound Q5b-3 (400 mg) as a colorless oil.

### 3. Synthesis of Compound Q5b-4

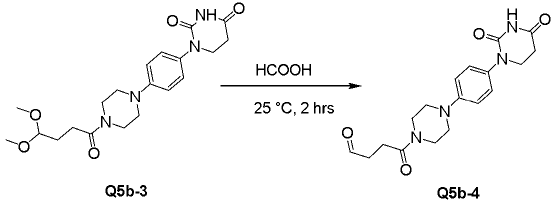

Compound Q5b-3 (500 mg, 1.24 mmol, 1.0 eq.) was dissolved in formic acid (5.00 mL), and the mixture was stirred and reacted at 25°C for 2 hours. The reaction mixture was concentrated under reduced pressure to obtain a crude product, which was further purified by reverse-phase HPLC (0.1% FA conditions) to provide Compound Q5b-4 (30.0 mg, approximately 75% purity) as a brown solid.

### 4. Synthesis of APQ5b

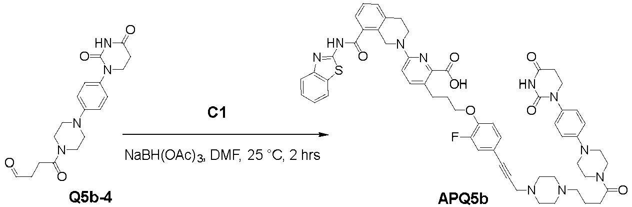

Compound Q5b-4 (20.0 mg, 55.8 µmol, 1.0 eq.), Compound C1 (39.3 mg, 55.8 µmol, 1.0 eq.), and NaBH(OAc)₃ (35.5 mg, 167 µmol, 3.0 eq.) were mixed in DMF (1.00 mL), and the mixture was stirred and reacted at 25°C under N₂ atmosphere for 2 hours. The reaction mixture was concentrated under reduced pressure to remove the solvent and obtain a curde target product. The crude product was purified by Prep-HPLC (chromatographic column: Phenomenex luna C18 150 * 25 mm * 10 µm; mobile phase: [water (FA)-ACN]; gradient: 32%-52% B, within 10 minutes) column chromatography to provide Compound APQ5b (6.3 mg, 9.47 µmol, 16.97% yield, 99.2% purity) as a white solid.

**LC-MS:** EC16331-37-P1C1, product: RT = 0.418 min, m/z = 1047.5 [M+H]⁺
**¹H NMR:** EC16331-37-P1A (400 MHz DMSO) *δ* ppm 10.26 (s, 1H), 8.18 (s, 1H), 8.03 (d, *J =* 7.6 Hz, 1H), 7.79 (d, *J* = 8.4 Hz, 1H) 7.60 (d, *J* = 7.2 Hz, 1H), 7.51 (d, J = 8.8 Hz, 1H), 7.45 - 7.48 (m, 1H), 7.39 - 7.42 (m, 1H), 7.32 - 7.38 (m, 2H), 7.26 (dd, *J* = 11.6, 1.6 Hz, 1H), 7.13 - 7.19 (m, 3H), 7.08 (d, *J* = 8.8 Hz, 1H), 6.89 - 6.94 (m, 3H), 4.91 (s, 2H), 4.00 (t, *J* = 6.4 Hz, 2H), 3.85 (t, *J =* 5.6 Hz, 2H), 3.69 (t, *J* = 6.8 Hz, 3H), 3.57 - 3.60 (m, 4H), 3.45 (br s, 3H), 3.12 (s, 3H), 3.06 (s, 2H), 2.97 (t, *J* = 5.6 Hz, 3H), 2.78 (t, *J* = 7.38 Hz, 2H) 2.67 (t, *J =* 6.8 Hz, 3H), 2.32 - 2.37 (m, 3H), 2.28 (t, *J =* 7.2 Hz, 2H), 1.91 - 2.00 (m, 3H), 1.67 (q, *J* = 7.2 Hz, 3H).

### b) Synthesis of APQ6a and APQ6b

### Synthesis of APQ6a:

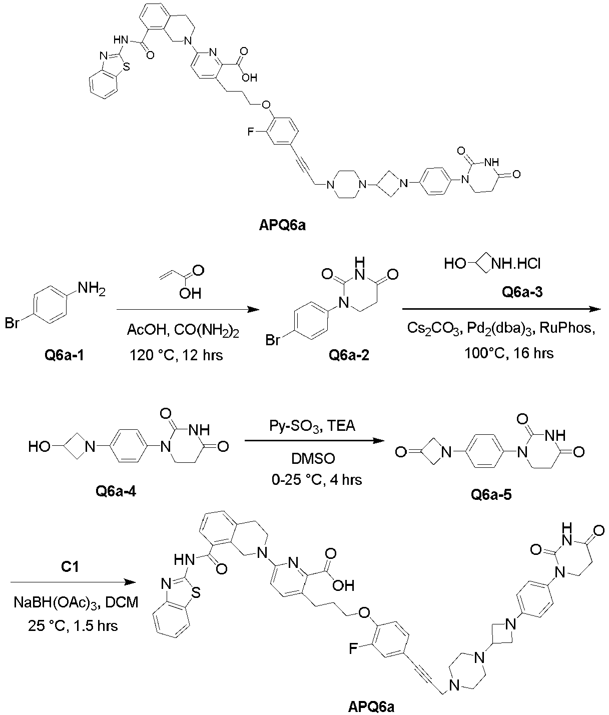

### 1. Synthesis of Q6a-2

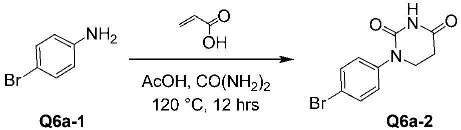

To Compound Q6a-1 (2.00 g, 11.6 mmol, 1.00 eq.), acrylic acid (3.35 g, 46.5 mmol, 3.19 mL, 4.00 eq.) was added, and the mixture was stirred at 110°C for 3 hours. Then, CO(NH₂)₂ (4.48 g, 74.6 mmol, 4.01 mL, 6.40 eq.) and AcOH (20 mL) were added, and the mixture was stirred and reacted at 120°C for 12 hours. The reaction mixture was added into water (50.0 mL) at 0°C, and filtered to obtain a filter cake. The filter cake was washed with water (20.0 mL), and then concentrated under reduced pressure to provide the crude product Compound Q6a-2 (2.20 g, 8.18 mmol, 70.3% yield) as a brown solid.

**LCMS:** EC16416-3-pla, P1 RT = 0.265 min, m/z = 269.0 [M+H]⁺.

### 2. Synthesis of Q6a-4

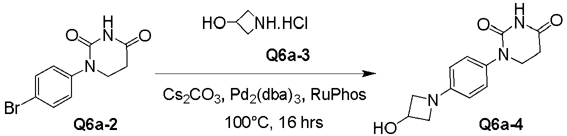

Compound Q6a-3 (2.44 g, 22.3 mmol, 3.00 eq., HCl), Q6a-2 (2.00 g, 7.43 mmol, 1.00 eq.), Cs₂CO₃ (7.26 g, 22.3 mmol, 3.00 eq.), RuPhos (693 mg, 1.49 mmol, 0.200 eq.), and Pd₂(dba)₃ (681 mg, 743 µmol, 0.100 eq.) were mixed in dioxacyclohexane (20.0 mL), and the mixture was stirred and reacted at 100°C under N₂ atmosphere for 16 hours. The reaction mixture was concentrated under reduced pressure to obtain a crude product, which was further purified by Prep-HPLC (neutral conditions) to provide the product Q6a-4 (300 mg, 1.15 mmol, 15.4% yield) as a white solid.

**LCMS:** EC16364-67-P1A1, P1 RT = 0.465 min, m/z = 262.1 [M+H]⁺.

### 3. Synthesis of Q6a-5

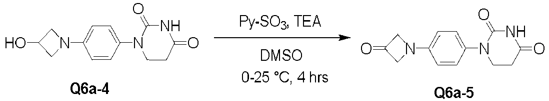

To a solution of Py-SO₃ (122 mg, 765 µmol, 4.00 eq.) in DMSO (1.00 mL), TEA (96.8 mg, 957 µmol, 133 µL, 5.00 eq.) was added dropwise at 0°C, and the mixture was stirred at 0°C for 15 minutes. Then, Compound Q6a-4 (50.0 mg, 191 µmol, 1.00 eq.) dissolved in DMSO (1.00 mL) was added dropwise at 0°C, and the mixture was stirred and reacted at 25°C for 3.75 hours. LCMS (P1, RT = 0.331 minutes) showed that Q6a-4 was completely reacted. The reaction mixture was concentrated under reduced pressure to obtain a crude product, which was purified by Prep-HPLC (FA conditions) to provide the product Q6a-5 (25.0 mg, 96.4 µmol, 50.4% yield) as a white solid.

**LCMS:** EEC16364-119-P1A1, P1 RT = 0.331 min, m/z = 260.0 [M+H]⁺.

### 4. Synthesis of Q6a

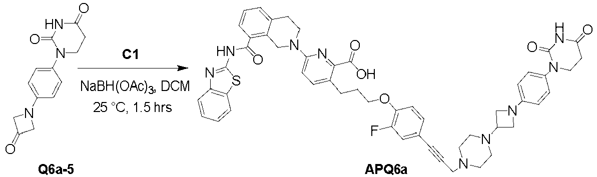

To a solution of Compound C1 (27.2 mg, 38.6 µmol, 1.00 eq.) in DCM (1 mL), TEA (39.0 mg, 386 µmol, 53.7 µL, 10.0 eq.) and Compound Q6a-5 (10.0 mg, 38.6 µmol, 1.00 eq.) were added, and the mixture was stirred at 25°C for 0.5 hours. Then, NaBH(OAc)₃ (24.5 mg, 116 µmol, 3.00 eq.) was added, and the mixture was stirred and reacted at 25°C for 1 hour. The reaction mixture was concentrated under reduced pressure to obtain a crude product, which was purified by Prep-HPLC (neutral conditions) to provide the product APQ6a (8.05 mg, 8.44 µmol, 21.9% yield) as a white solid.

**LCMS:** EC16364-125-P1X, P1 RT = 0.470 min, m/z = 948.1 [M+H]⁺
**¹H NMR:** EC16364-125-P1C (400 MHz DMSO-*d₆*) *δ* 10.22 (s, 1H) 8.03 (d, *J=* 7.6 Hz, 1H) 7.79 (d, *J* = 8.0 Hz, 1H) 7.59 (d, *J* = 7.2 Hz, 1H) 7.44-7.54 (m, 2H) 7.38-7.42 (m, 1H) 7.32-7.38 (m, 2H) 7.26 (dd, *J* = 12.0, 1.88 Hz, 1H) 7.14-7.20 (m, 1H) 7.09 (d, *J* = 8.8 Hz, 3H) 6.90 (d, *J* = 8.8 Hz, 1H) 6.41 (d, *J* = 8.8 Hz, 2H) 4.91 (s, 2H) 4.01 (t, *J* = 6.4 Hz, 2H) 3.85 (t, *J* = 6.0 Hz, 2H) 3.90 (t, *J* = 7.2 Hz, 2H) 3.66 (t, *J* = 6.8 Hz, 2H) 3.54-3.58 (m, 2H) 3.48 (s, 2H) 3.21-3.26 (m, 5H) 2.97 (t, *J* = 5.6 Hz, 2H) 2.77 (t, *J* = 7.2 Hz, 2H) 2.66 (t, J= 6.8 Hz, 2H) 2.52-2.57 (m, 2H) 2.38 (d, *J* = 2.4 Hz, 2H) 1.92-2.00 (m, 2H).

### Synthesis of APQ6b:

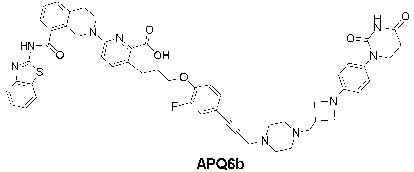

### Synthesis scheme:

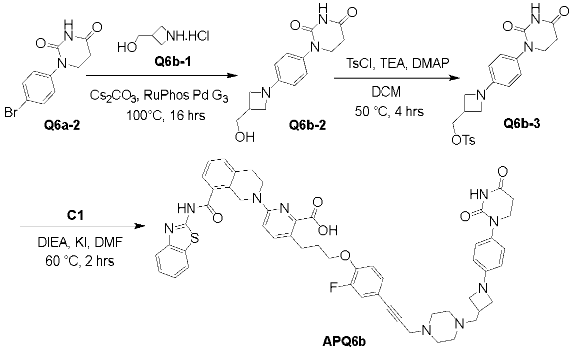

### 1. Synthesis of Q6b-2

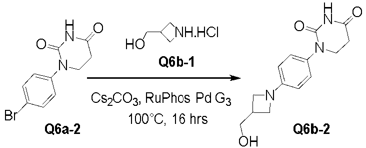

Compound Q6a-2 (2.00 g, 7.43 mmol, 1.00 eq.), Q6b-1 (1.01 g, 8.18 mmol, 1.01 eq., HCl), Cs₂CO₃ (7.26 g, 22.3 mmol, 3.00 eq.), and RuPhos Pd G₃ (1.24 g, 1.49 mmol, 0.200 eq.) were mixed in dioxacyclohexane (20.0 mL), and the mixture was stirred and reacted at 100°C under N₂ atmosphere for 16 hours. The reaction mixture was diluted by adding water (30.0 mL), and extracted with ethyl acetate (120.0 mL, 40.0 mL×3). The resulting organic layer was washed with saturated saline (120.0 mL, 60.0 mL×2), dried over Na₂SO₄, filtered and concentrated under reduced pressure to obtain a crude product, which was further purified by reverse-phase HPLC (FA conditions) to provide the product Q6b-2 (300 mg, crude product) as a brown solid.

**LCMS:** EC16331-20-P1B, P1 RT = 0.297 min, m/z = 275.9 [M+H]⁺.

### 2. Synthesis of Q6b-3

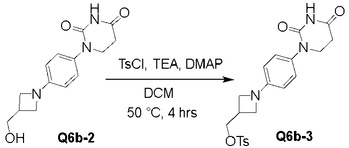

To a solution of Compound Q6b-2 (100 mg, 363 µmol, 1.00 eq.) in DCM (1.00 mL), TEA (110 mg, 1.09 mmol, 152 µL, 3.00 eq.), TsCl (208 mg, 1.09 mmol, 3.00 eq.), and DMAP (178 mg, 1.45 mmol, 4.00 eq.) were added, and the mixture was stirred and reacted at 50°C for 4 hours. To the reaction mixture, water (20.0 mL) was added at 0°C to quench the reaction. The reaction mixture was diluted by adding ethyl acetate (20.0 mL), and extracted with ethyl acetate (60.0 mL, 20.0 mL×3). The resulting organic layer was washed with saturated saline (20.0 mL), dried over Na₂SO₄, filtered and concentrated under reduced pressure to obtain a crude product, which was further purified by column chromatography (SiO₂, petroleum ether: ethyl acetate = 10:1 to 5:1) to provide the product Q6b-3 (70.0 mg, 163 µmol, 44.9% yield) as a white solid.

**LCMS:** EC16364-56-P1A, product: RT = 0.395 min, m/z = 430.1 [M+H]⁺.

### 3. Synthesis of APQ6b

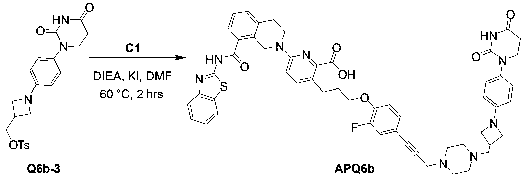

To a solution of Compound Q6b-3 (50.0 mg, 116 µmol, 1.00 eq.) and Cl (49.2 mg, 69.8 µmol, 0.600 eq.) in DMF (0.500 mL), DIEA (60.2 mg, 465 µmol, 81.1 µL, 4.00 eq.) and KI (48.3 mg, 291 µmol, 2.50 eq.) were added, and the mixture was stirred and reacted at 60°C for 2 hours. The reaction mixture was filtered and concentrated under reduced pressure to obtain a crude product, which was further purified by Prep-HPLC (FA conditions and neutral conditions) to provide the product APQ6b (2.40 mg, 2.49 µmol, 2.14% yield) as a white solid.

**LCMS:** EC16331-43-P1B, P1 RT = 0.427 min, m/z = 962.3 [M+H]⁺
**¹H NMR:** EC16364-64-P1D (400 MHz DMSO-*d₆*) *δ* 10.23 (s, 1H) 7.87 (d, *J* = 7.2 Hz, 1H) 7.68 (d, *J* = 8.8 Hz, 1H) 7.57-7.63 (m, 1H) 7.54 (d, *J* = 8.8 Hz, 1H) 7.23-7.50 (m, 3H) 7.22-7.28 (m, 2H) 7.18 (d, *J* = 8.8 Hz, 1H) 7.08-7.12 (m, 1H) 7.04 *(d, J=* 7.2 Hz, 2H) 6.94-6.98 (m, 1H) 6.31 *(d, J=* 7.6 Hz, 1H) 4.95 (s, 2H) 4.28 (d, *J* = 6.0 Hz, 2H) 3.98-4.03 (m, 2H) 3.75 *(d, J =* 6.0 Hz, 2H) 3.70-3.74 (m, 2H) 3.59-3.65 (m, 4H) 3.48-3.52 (m, 4H) 3.42 (s, 2H) 2.93 (d, *J* = 5.6 Hz, 2H) 2.73 (s, 4H) 2.66 (s, 2H) 2.44 (s, 2H) 2.33 (s, 1H) 1.89-2.01 (m, 4H).

### c) Synthesis of APQ7a and APQ7b

### Synthesis of APQ7a:

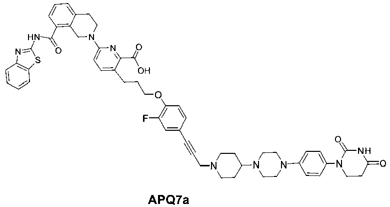

### Synthesis scheme:

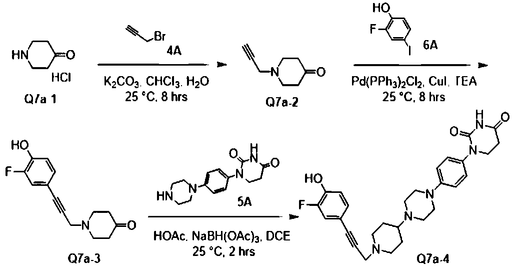

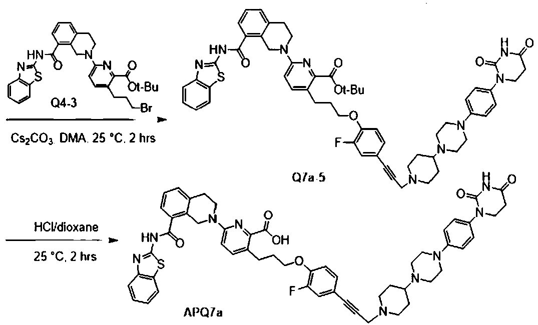

### 1. Synthesis of Compound Q7a-2

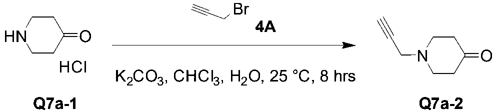

To a mixture of Compound 4A (6.58 g, 55.3 mmol, 4.77 mL, 1.5 eq.) dissolved in H₂O (50.0 mL) and CHCl₃ (50.0 mL), K₂CO₃ (15.3 g, 110 mmol, 3 eq.) and Compound Q7a-1 (5.00 g, 36.9 mmol, 1.0 eq.) were added, and the mixture was stirred and reacted at 20°C for 8 hours. The organic layer was separated from the reaction mixture, washed with saturated saline (30.0 mL, ×2), dried over Na₂SO₄, and concentrated under reduced pressure to obtain a crude product, which was further purified by column chromatography (SiO₂, dichloromethane: methanol =97:3) to provide Compound Q7a-2 as a yellow oil.

**¹H NMR:** EC9174-266-P1C (400 MHz CDCl₃) *δ* ppm 3.45 (d, *J =* 2.4 Hz, 2H), 2.87 *(t, J=* 6.0 Hz, 4H), 2.50 (t, *J =* 6.0 Hz, 4H), 2.29 (t, *J* = 2.4 Hz, 1H).

### 2. Synthesis of Compound Q7a-3

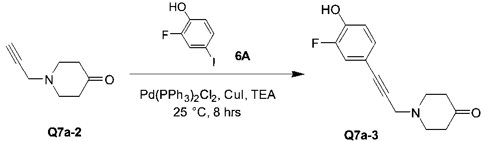

Compound 6A (867 mg, 3.64 mmol, 1.0 eq.), Pd(PPh₃)₂Cl₂ (51.2 mg, 72.9 µmol, 0.02 eq.), CuI (27.8 mg, 146 µmol, 0.04 eq.), and TEA (10.0 mL) were mixed, and Compound Q7a-2 (500 mg, 3.64 mmol, 1.0 eq.) was added under N₂ atmosphere. The mixture was stirred and reacted at 25°C for 8 hours. The reaction mixture was extracted with H₂O (30.0 mL) and ethyl acetate (40.0 mL), and concentrated under reduced presssure to provide the crude product Q7a-3 (1.00 g, crude) as a brown solid.

**LC-MS:** EC9174-317-P1E7, product: RT = 0.302 min, m/z = 246.3 [M+H]⁺
**¹H NMR:** EC9174-317-P1A (400 MHz CDCl₃) *δ* ppm 7.09 - 7.15 (m, 2H), 6.93 (t, *J* = 8.8 Hz, 1H), 3.64 (s, 2H), 2.95 (t, *J* = 6.0 Hz, 4H), 2.55 (t, *J* = 6.0 Hz, 4H).

### 3. Synthesis of Compound Q7a-4

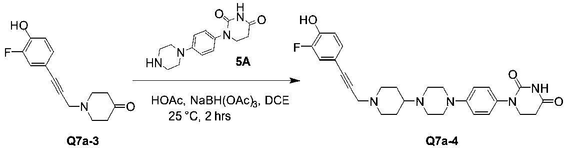

To a mixed solution of Compound Q7a-3 (400 mg, 1.62 mmol, 1.0 eq.), Compound 5A (466 mg, 1.70 mmol, 1.05 eq.), and NaBH(OAc)₃ (686 mg, 3.24 mmol, 2.0 eq.) in DCE (10.0 mL), HOAc (117 mg, 1.94 mmol, 111 µL, 1.2 eq.) was added dropwise at 25°C over 30 minutes, and the mixture was stirred and reacted at 25°C for 1.5 hours. The reaction mixture was concentrated under reduced pressure to obtain a crude target product, which was further purified by reverse-phase HPLC (0.1% NH₃·H₂O) column chromatography to provide Compound Q7a-4 (100 mg) as a brown solid.

**LC-MS:** EC16331-24-P1A, product: RT = 0.392 min, m/z = 506.2 [M+H]⁺.

### 4. Synthesis of Compound Q7a-5

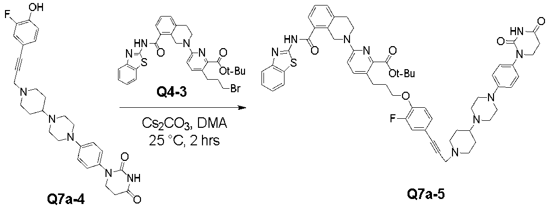

Compound Q7a-4 (80.0 mg, 158 µmol, 1.0 eq.), Compound Q4-3 (106 mg, 174 µmol, 1.1 eq.) and Cs₂CO₃ (103 mg, 316 µmol, 2.0 eq.) were mixed in DMA (2.00 mL), and the mixture was stirred and reacted at 25°C under N₂ atmosphere for 2 hours. The reaction mixture was concentrated under reduced pressure to obtain a crude product, which was further purified by reverse-phase HPLC column chromatography (chromatographic column: Welch Xtimate C18 150 * 25 mm * 5 µm; mobile phase: [water (FA)-ACN]; gradient elution: 27%-57% B, within 10 minutes) to provide Compound Q7a-5 (30.0 mg) as a white solid.

### 5. Synthesis of Compound APQ7a

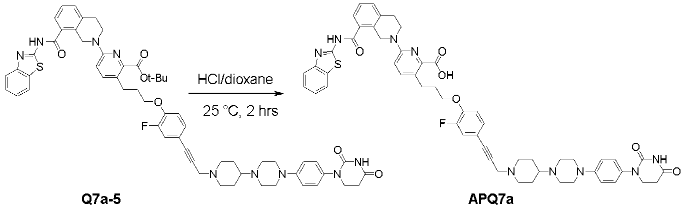

Compound Q7a-5 (30.0 mg, 29.1 µmol, 1.0 eq.) was dissolved in HCl-dioxane (2.00 mL), and the mixture was stirred and reacted at 25°C for 2 hours. The reaction mixture was concentrated under reduced pressure to obtain a crude product, which was further purified by reverse-phase HPLC column chromatography (chromatographic column: Daisogel SP ODS RPS 150 * 25 mm * 5 µm; mobile phase: [water (NH₄HCO₃)-ACN]; gradient elution: 28%-58% B, within 10 minutes) to provide Compound APQ7a (8.60 mg, 10.2 µmol, 35.25% yield, 95.76% purity) as a white solid.

**LC-MS:** EC16331-38-P1D2, product: RT = 1.578 min, m/z = 976.2 [M+H]⁺
**¹H NMR:** EC16331-38-P1A (400 MHz DMSO) *δ* ppm 10.25 (s, 1H), 8.03 (d, *J* = 7.50 Hz, 1H), 7.78 *(d, J=* 7.6 Hz, 1H), 7.60 *(d, J=* 7.2 Hz, 1H), 7.48 - 7.51 (m, 1H), 7.47 (s, 1H), 7.38 - 7.42 (m, 1H), 7.35 (dd, *J =* 7.2, 2.8 Hz, 2H), 7.27 (dd, *J =* 11.6, 2.0 Hz, 1H), 7.16 - 7.19 (m, 1H), 7.14 (d, *J* = 9.2 Hz, 2H), 7.10 (d, *J* = 8.8 Hz, 1H), 6.92 (d, *J =* 9.2 Hz, 2H), 6.89 (s, 1H), 4.91 (s, 2H), 4.01 (t, *J* = 6.4 Hz, 2H), 3.85 (t, *J* = 6.0 Hz, 2H), 3.69 (t, *J* = 6.4 Hz, 3H), 3.45 (s, 3H), 3.11 (s, 4H), 2.97 (t, *J* = 5.6 Hz, 3H), 2.89 (d, *J* = 10.8 Hz, 3H), 2.77 *(t, J=* 7.2 Hz, 3H), 2.67 (t, *J* = 6.8 Hz, 3H), 2.62 (s, 5H), 2.13 - 2.23 (m, 4H), 1.93 - 2.00 (m, 2H), 1.80 (d, *J* = 11.6 Hz, 2H), 1.38 - 1.52 (m, 3H).

### Synthesis of APQ7b:

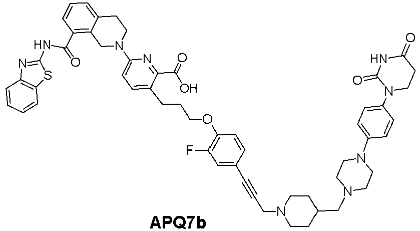

### Synthesis scheme:

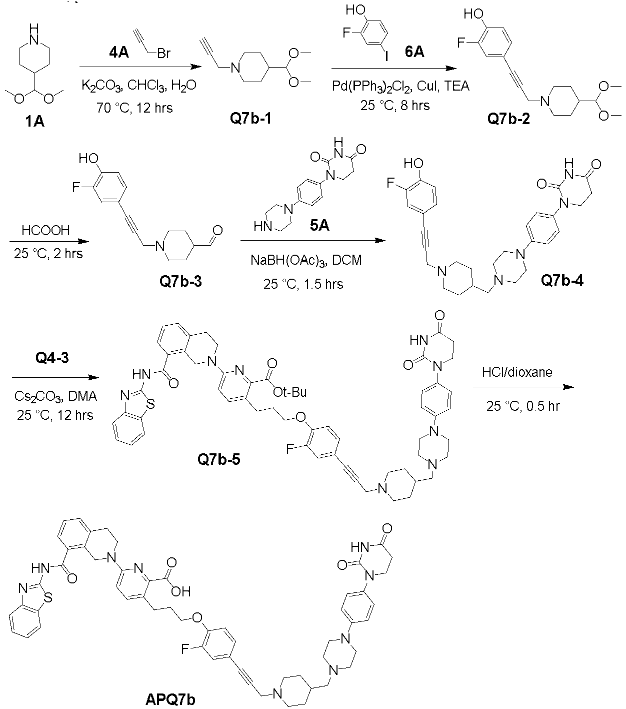

The synthesis procedure for the PROTAC molecule APQ7b was similar to that for APQ7a, but the starting material Compound Q7a-1 was replaced with Compound 1A, and after synthesis, Compound APQ7b (13.7 mg, 13.4 µmol, 82.4% yield, 96.8% purity) was obtained by purification via Prep-HPLC (chromatographic column: Welch Xtimate C18 150 * 25 mm * 5 µm; mobile phase: [water (HCl)-ACN]; gradient elution: 18%-48% B, within 8 minutes) column chromatography, as a brown solid.

LC-MS: EC11255-211-P1A1, product: RT = 0.372 min, m/z = 496.0 [M/2+H]⁺
**¹H** NMR: EC11255-211-P1B (400 MHz DMSO) δ ppm 8.04 (d, *J =* 7.8 Hz, 1H), 7.79 (d, *J =* 8.0 Hz, 1H), 7.54 - 7.65 (m, 2H), 7.29 - 7.50 (m, 6H), 7.13 - 7.24 (m, 3H), 6.97 - 7.05 (m, 3H), 4.93 (s, 2H), 4.30 (s, 2H), 4.06 (t, *J =* 6.4 Hz, 2H), 3.86 (t, *J =* 5.6 Hz, 2H), 3.79 (d, *J =* 12.0 Hz, 2H), 3.70 (t, *J =* 6.6 Hz, 2H), 3.64 (s, 4H), 3.22 - 3.30 (m, 2H), 3.04 - 3.18 (m, 6H), 2.98 (t, *J =* 5.8 Hz, 2H), 2.78 - 2.86 (m, 2H), 2.69 (t, *J* = 6.6 Hz, 2H), 2.13 (d, *J =* 13.8 Hz, 3H), 1.92 - 2.02 (m, 2H), 1.52 - 1.72 (m, 2H).

### d) Synthesis of APQ8, APQ9, APQ10 and APQ11

### Synthesis of APQ8:

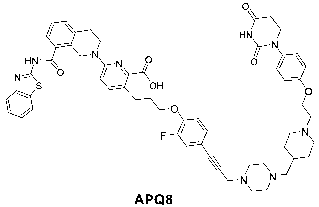

### Synthesis scheme:

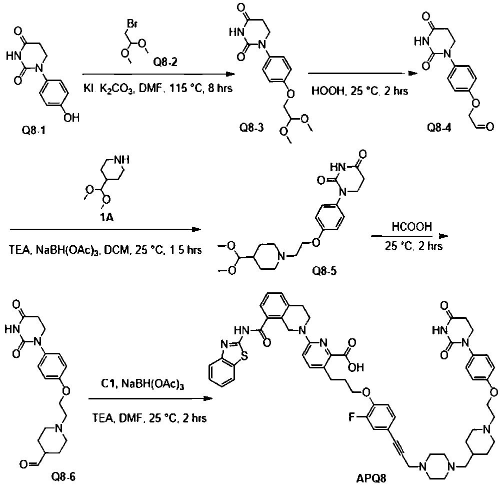

### 1. Synthesis of Compound Q8-3

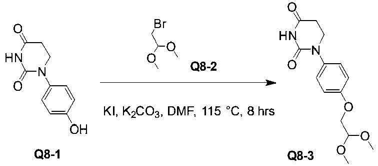

To a solution fo Compound Q8-2 (2.95 g, 17.4 mmol, 2.05 mL, 1.2 eq.) in DMF (35.0 mL), Compound Q8-1 (3.00 g, 14.5 mmol, 1.0 eq.), KI (2.66 g, 16.00 mmol, 1.1 eq.) and K₂CO₃ (4.02 g, 29.1 mmol, 2.0 eq.) were added, and the mixture was stirred and reacted at 115°C for 16 hours. Then, Q8-1 (1.50 g, 8.87 mmol, 1.04 mL, 0.6 eq.) was further added, and the mixture was further stirred and reacted at 115°C for 3 hours. The reaction mixture was filtered and concentrated under reduced pressure to obtain a crude product, which was purified by Prep-HPLC column chromatography (under TFA conditions) to provide Compound Q8-3 (1.60 g, 5.44 mmol, 37.37% yield) as a white solid.

**LC-MS:** EC16416-5-Plda, product: RT = 0.326 min, m/z = 231.1 [M+H]⁺.

### 2. Synthesis of Compound Q8-4

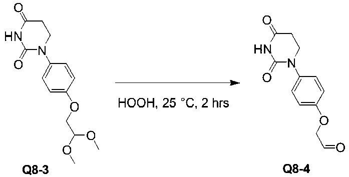

Compound Q8-3 (300 mg, 1.02 mmol, 1.0 eq.) was dissolved in formic acid (5.00 mL), and the mixture was stirred and reacted at 25°C for 2 hours. The reaction mixture was concentrated under reduced pressure to provide the crude product Compound Q8-4 (385 mg) as a yellow oil.

**LC-MS:** EC11532-166-p1al, product: RT = 0.183 min, m/z = 231.1 [M+H]⁺.

### 3. Synthesis of Compound Q8-5

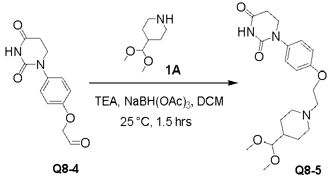

To a solution of Compound Q8-4 (288 mg, 1.16 mmol, 1.0 eq.) in DCM (10.0 mL), TEA (1.17 g, 11.6 mmol, 1.61 mL, 10 eq.) and Compound 1A (184 mg, 1.16 mmol, 1.0 eq.) was added, and the mixture was stirred and reacted at 25°C for 0.5 hours. Then, NaBH(OAc)₃ (737 mg, 3.48 mmol, 3.0 eq.) was added, and the mixture was stirred and reacted at 25°C for 1 hour. To the mixture, water (5.00 mL) was added at 25°C to quench the reaction, and then the mixture was extracted with DCM (20.0 mL, 5.00 mL×4). The resulting organic layer was washed with saturated saline (10.0 mL, 5.00 mL×2), dried over Na₂SO₄, filtered, and then concentrated under reduced pressure to provide the crude product Compound Q8-5 (388 mg, 991 µmol, 85.43% yield) as a yellow solid, which was directly used in the next reaction without purification.

**LC-MS:** EC11532-169-p1al, product: RT = 0.188 min, m/z = 392.1 [M+H]⁺.

### 4. Synthesis of Compound Q8-6

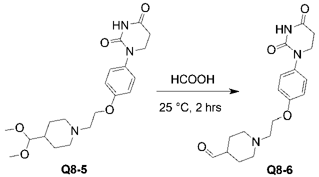

Compound Q8-5 (100 mg, 255 µmol, 1.0 eq.) was dissolved in formic acid (0.5 mL), and the mixture was stirred and reacted at 25°C for 2 hours. The reaction mixture was concentrated under reduced pressure to remove the solvent, and obtain the crude product Compound Q8-6 (80.0 mg) as a colorless oil.

**LC-MS:** EC16331-29-P1A, product: RT= 0.172 min, m/z = 346.1 [M+H]⁺.

### 5. Synthesis of Compound APQ8

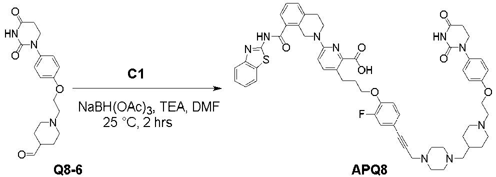

To a solution of Compound Q8-6 (80.0 mg, 231 µmol, 1.0 eq.) and C1 (163 mg, 231 µmol, 1 eq.) in DMF (2.00 mL), TEA (234 mg, 2.32 mmol, 322 µL, 10.0 eq.) and NaBH(OAc)₃ (147 mg, 695 µmol, 3.0 eq.) was added, and the mixture was stirred and reacted at 25°C for 2 hours. The reaction mixture was concentrated under reduced pressure to obtain a crude product, which was purified by Prep-HPLC column chromatography (chromatographic column: Phenomenex luna C18 150 * 25 mm * 10 µm; mobile phase: [water (FA)-ACN]; gradient elution: 20%-50% B, within 10 minutes) to provide Compound APQ8 (30.0 mg, 28.6 µmol, 12.33% yield, 98.45% purity) as a white solid.

**LC-MS:** EC16331-30-P1A1, product: RT = 0.379 min, m/z = 1034.5 [M+H]⁺
**¹H NMR:** EC16331-30-P1A (400 MHz DMSO) δ ppm 10.29 (s, 1H), 8.18 (s, 1H), 8.03 (d, *J* = 7.6 Hz, 1H), 7.79 (d, *J* = 8.0 Hz, 1H), 7.59 (d, *J* = 7.6 Hz, 1H), 7.51 (d, *J* = 8.8 Hz, 1H), 7.45 - 7.49 (m, 1H), 7.39 - 7.42 (m, 1H), 7.35 (s, 1H), 7.34 (s, 1H), 7.26 (dd, *J* = 11.6, 1.6 Hz, 1H), 7.21 (d, *J* = 9.2 Hz, 2H), 7.17 (d,*J* = 10.4 Hz, 1H), 7.06 - 7.12 (m, 1H), 6.94 (s, 1H), 6.92 (s, 1H), 6.89 - 6.92 (m, 1H), 4.91 (s, 2H), 4.03 (dt, *J* = 16.8, 6.4 Hz, 5H), 3.85 (t, *J* = 6.0 Hz, 2H), 3.70 (t, *J* = 6.8 Hz, 3H), 3.44 (s, 3H), 2.97 (t, *J =* 5.6 Hz, 2H), 2.90 (d, *J* = 11.6 Hz, 2H), 2.78 (t, *J =* 7.6 Hz, 3H), 2.65 - 2.71 (m, 5H), 2.29 - 2.40 (m, 4H), 2.09 (d, *J* = 7.2 Hz, 2H), 1.92 - 2.04 (m, 5H), 1.60 - 1.67 (m, 2H), 1.41 - 1.49 (m, 1H), 1.02 - 1.14 (m, 2H).

### Synthesis of APQ9:

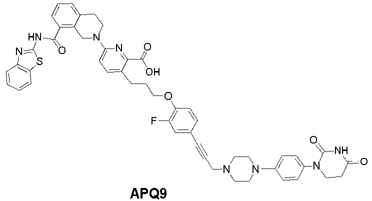

### Synthesis scheme:

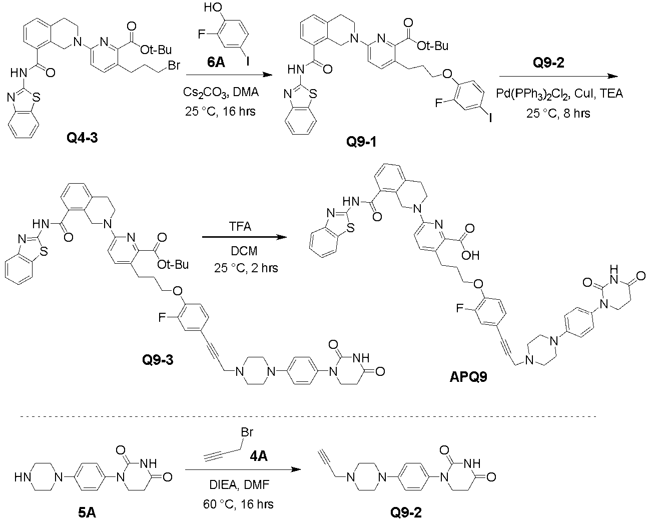

### 1. Synthesis of Compound Q9-1

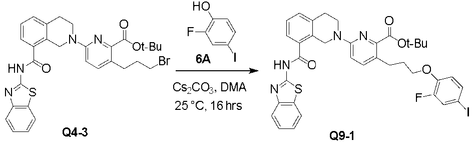

To a solution of Compound Q4-3 (100 mg, 164 µmol, 1.0 eq.) in DMA (2 mL), Cs₂CO₃ (134 mg, 411 µmol, 2.5 eq.) and compound 6A (58.7 mg, 246 µmol, 1.5 eq.) were added, and the mixture was stirred and reacted at 25 °C for 16 hours. To the mixture, water (10.0 mL) was added at 0°C to quench the reaction, and the mixture was extracted with ethyl acetate (30.0 mL, 10.0 mL×3). The resulting organic layer was washed with saturated saline (10.0 mL), dried over Na₂SO₄, filtered, and concentrated under reduced pressure to obtain a crude product, which was further purified by column chromatography (SiO₂, ethyl acetate: petroleum ether =3:1 to 1:1) to provide Compound Q9-1 (60.0 mg, 72.4 µmol, 43.9% yield, 92.2% purity) as a yellow solid.

**LC-MS:** EC16364-30-P1A, product: RT = 0.619 min, m/z = 765.1 [M+H]⁺.

### 2. Synthesis of Compound Q9-2

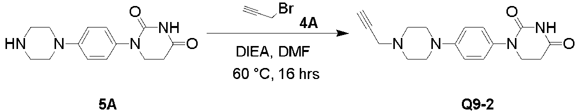

3-Bromo-1-propyne (104 mg, 874 µmol, 75.4 µL, 1.2 eq.), Compound 5A (200 mg, 729 µmol, 1.0 eq.) and DIEA (565 mg, 4.37 mmol, 762 µL, 6.0 eq.) were mixed in DMF (5 mL), and the mixture was stirred and reacted at 60°C under N₂ atmosphere for 16 hours. The reaction mixture was concentrated under reduced pressure to obtain a crude product, which was further purified by Prep-HPLC column chromatography (alkaline conditions) to provide Compound Q9-2 (140 mg, 448 µmol, 61.5% yield) as a yellow oil.

LC-MS: EC16364-15-P1A, product: RT = 0.411 min, m/z = 313.3 [M+H]⁺.

### 3. Synthesis of Compound Q9-5

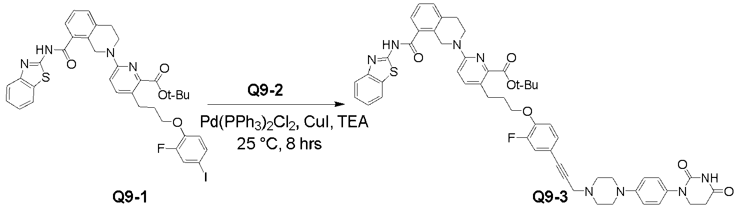

Compound Q9-1 (70.0 mg, 91.5 µmol, 1.0 eq.), Pd(PPh₃)₂Cl₂ (1.29 mg, 1.83 µmol, 0.02 eq.), CuI (697 µg, 3.66 µmol, 0.04 eq.) and TEA (1 mL) were mixed. With protection under N₂, Compound Q9-2 (42.9 mg, 137.3 µmol, 1.5 eq.) was added, and the mixture was stirred and reacted at 25°C for 8 hours. The mixture was concentrated under reduced pressure, and was ground with water to remove impurities and to provide the crude product Compound Q9-3 (80.0 mg, 84.3 µmol, 92.1%) as a yellow solid.

**LC-MS:** EC16364-32-P1A, product: RT = 0.484 min, m/z = 949.3 [M+H]⁺
**¹H NMR:** EC17015-67-P1B (400 MHz DMSO) δ ppm 12.54 - 13.15 (m, 1H), 10.26 (s, 1H), 8.01 (d, *J* = 7.8 Hz, 1H), 7.76 (d, *J =* 7.8 Hz, 1H), 7.56 (d, *J* = 7.4 Hz, 1H), 7.50 (d, *J =* 8.8 Hz, 1H), 7.42 - 7.48 (m, 1H), 7.37 - 7.42 (m, 1H), 7.31 - 7.36 (m, 2H), 7.29 (dd, *J* = 12.0, 1.8 Hz, 1H), 7.17 - 7.22 (m, 1H), 7.15 (d, *J* = 8.8 Hz, 2H), 7.07 - 7.13 (m, 1H), 6.94 (d, *J =* 9.0 Hz, 2H), 6.91 (d, *J =* 8.8 Hz, 1H), 4.93 (s, 2H), 4.02 (t, *J* = 6.0 Hz, 2H), 3.79 (t, *J =* 6.0 Hz, 2H), 3.69 (t, *J =* 6.6 Hz, 2H), 3.55 (s, 2H) 3.17 (d, *J =* 3.8 Hz, 4H), 2.98 (t, *J* = 6.0 Hz, 2H), 2.63 - 2.70 (m, 8H), 1.88 - 1.98 (m, 2H), 1.30 (s, 9H).

### 4. Synthesis of Compound APQ9

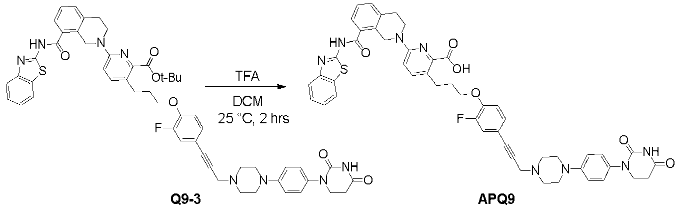

Compound Q9-3 (50.0 mg, 52.7 µmol, 1.0 eq.) and TFA (1.54 g, 13.5 mmol, 1 mL, 255 eq.) were mixed in DCM (1 mL), and the mixture was stirred and reacted at 25°C under N₂ atmosphere for 2 hours. The mixture was concentrated under reduced pressure to obtain a crude product, which was further purified by Prep-HPLC column chromatography (HCl conditions and neutral conditions) to provide Compound APQ9 (6 mg, 6.24 µmol, 11.85% yield, 92.9% purity) as a white solid.

**LC-MS:** EC16364-35-P1R; product: RT = 0.416 min
**¹H NMR:** EC16364-35-P1R (400 MHz DMSO) *δ* ppm 10.26 (s, 1H) 8.03 (d, *J =* 8.0 Hz, 1H) 7.78 (d, *J* = 8.0 Hz*,* 1H) 7.60 (d, *J* = 7.2 Hz, 1H) 7.43 - 7.55 (m, 2H) 7.38 - 7.42 (m, 1H) 7.32 - 7.37 (m, 2H) 7.28 (dd, *J =* 12.0, 1.75 Hz, 1H) 7.13 - 7.21 (m, 3H) 7.07 - 7.12 (m, 1H) 6.88 - 6.96 (m, 3H) 4.92 (s, 2H) 4.02 (t, *J* = 6.4 Hz, 2H) 3.85 (t, *J* = 6.0 Hz, 2H) 3.69 (t*, J =* 6.8 Hz, 2H) 3.55 (s, 2H) 3.16 (s, 4H) 2.97 (t*, J =* 5.6 Hz, 2H) 2.78 (t, *J* = 7.2 Hz, 2H) 2.65 - 2.70 (m, 6H) 1.91 - 2.02 (m, 2H).

### Synthesis of APQ10:

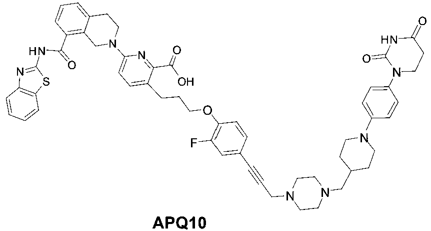

### Synthesis scheme:

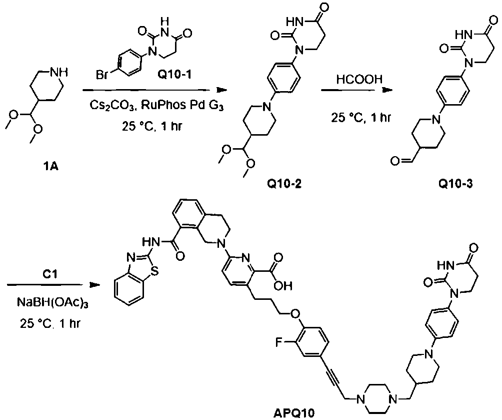

### 1. Synthesis of Compound Q10-2

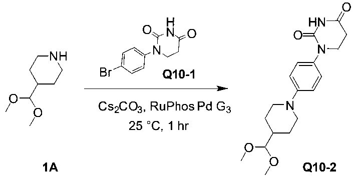

Compound 1A (218 mg, 1.11 mmol, 3.0 eq., HCl), 1-(4-bromophenyl)hexahydropyrimidine-2,4-dione (100 mg, 371.62 µmol, 1 eq.), Cs₂CO₃ (363 mg, 1.11 mmol, 3.0 eq.) and RuPhos Pd G3 (31.1 mg, 37.1 µmol, 0.1 eq.) were mixed in dioxane (5 mL). With protection under N₂, the mixture was stirred and reacted at 90°C for 2 hours. The mixture was concentrated under reduced pressure to obtain a crude produc, which was further purified by Prep-HPLC column chromatography (FA conditions) to provide Compound Q10-2 (60.0 mg, 172 µmol, 46.5% yield) as a white oil.

**LC-MS:** EC16364-40-P1A, product: RT = 0.206 min, m/z = 348.1 [M+H]⁺.

### 2. Synthesis of Compound Q10-3

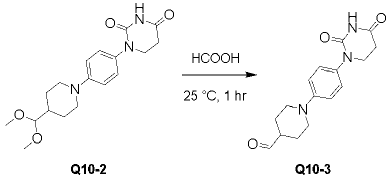

Compound Q10-2 (60 mg, 172 µmol, 1 eq.) was mixed with formic acid (1.0 mL), and the mixture was stirred and reacted at 25°C under N₂ atmosphere for 1 hour. The mixture was concentrated under reduced pressure to obtain a crude product, which was directly used in the next reaction without purification. The obtained crude product Compound Q10-3 (35.0 mg, 116.1 µmol, 67.2% yield) was a yellow oil.

### 3. Synthesis of Compound APQ10

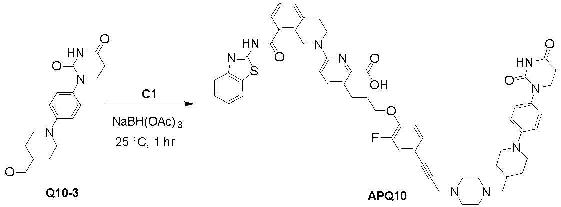

To a solution of Compound Q10-3 (35 mg, 116 µmol, 1.0 eq.) in DCM (5.0 mL), TEA (117mg, 1.16 mmol, 161 µL, 10.0 eq.) and Compound C1 (90 mg, 127.69 µmol, 1.1 eq.) were added, and the mixture was stirred and reacted at 25°C for 0.5 hours. Then, NaBH(OAc)₃ (73.81 mg, 348.26 µmol, 3.0 eq.) was added, and the mixture was stirred and reacted at 25°C for 1 hour. The mixture was concentrated under reduced pressure to obtain a crude product, which was further purified by Prep-HPLC column chromatography (FA conditions) to provide Compound APQ10 (13 mg, 12.8 µmol, 11.1% yield, 98.0% purity) as a yellow solid.

**LC-MS:** 16364-47-P1A, product: RT = 0.386 min, m/z = 990.5 [M+H]⁺
**¹H NMR:** EC16364-47-P1C (400 MHz DMSO) *δ* ppm 12.85 (s, 1H), 11.69 (s, 1H), 10.44 (s, 1H), 8.04 (d, *J* = 8.0 Hz, 1H), 7.79 (d, *J =* 8.0 Hz, 1H), 7.69 - 7.77 (m, 1H), 7.52 - 7.61 (m, 3H), 7.44 - 7.51 (m, 3H), 7.40 - 7.43 (m, 1H), 7.42 (d, *J =* 8.0 Hz, 1H), 7.29 - 7.39 (m, 3H), 7.10 - 7.19 (m, 1H), 6.98 (d, *J =* 8.8 Hz, 1H), 4.93 (s, 2H), 4.30 (s, 2H), 4.02 - 4.08 (m, 2H), 3.86 (t, *J =* 6.0 Hz, 2H), 3.80 (t, *J =* 6.4 Hz, 4H), 3.60 - 3.64 (m, 2H), 3.33 - 3.53 (m, 4H), 3.14 - 3.27 (m, 2H), 2.98 (t, *J =* 5.6 Hz, 2H), 2.78 - 2.86 (m, 2H), 2.71 (t, *J* = 6.4 Hz, 2H), 2.16 - 2.27 (m, 2H), 1.97 (dt, *J =* 14.4, 7.04 Hz, 2H), 1.88 (s, 1H).

### Synthesis of APQ11:

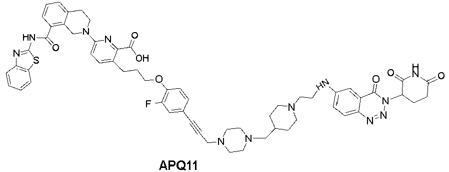

### Synthesis scheme:

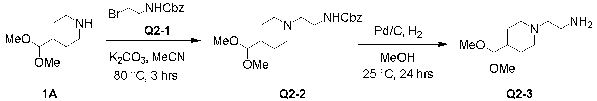

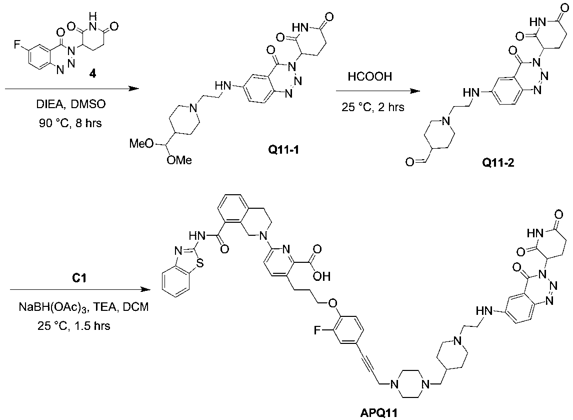

The sysnthesis procedure for the PROTAC molecule APQ11 was similar to that for APQ2, but the intermediate material Compound 1 was replaced with Compound 4, and after synthesis, Compound APQ11 (30.0 mg, 26.5 µmol, 18.6% yield, 97.1% purity) was obtained by purification via column chromatography (FA conditions), as a white solid.

**LC-MS:** EC16364-14-P1A1, product: RT = 1.33 min, m/z = 1101.7 [M+H]⁺
**¹H NMR:** EC16364-14-P1C (400 MHz DMSO) δ ppm 11.12 (s, 1H) 8.19 (s, 1H) 8.03 (d, *J* = 8.0 Hz, 1H) 7.89 (d, *J* = 9.2 Hz, 1H) 7.78 (d, *J* = 8.0 Hz, 1H) 7.59 (d, *J* = 7.2 Hz, 1H) 7.44 - 7.53 (m, 2H) 7.38 - 7.42 (m, 1H) 7.30 - 7.38 (m, 3H) 7.24 - 7.29 (m, 1H) 7.13 - 7.20 (m, 2H) 7.04 - 7.13 (m, 2H) 6.90 (d, *J* = 8.8 Hz, 1H) 5.84 (dd, *J =* 12.4, 5.25 Hz, 1H) 4.91 (s, 2H) 4.01 (t, *J =* 6.4 Hz, 2H) 3.85 (t, *J =* 6.0 Hz, 2H) 3.44 (s, 2H) 3.30 (dd, *J =* 12.0, 5.57 Hz, 4H) 2.86 - 3.00 (m, 6H) 2.77 (t, *J* = 7.6 Hz, 2H) 2.64 - 2.71 (m, 2H) 2.29 - 2.41 (m, 4H) 2.21 (d, *J =* 5.2 Hz, 2H) 2.10 (d, *J =* 6.8 Hz, 2H) 1.92 - 2.01 (m, 4H) 1.65 (d, *J =* 12.0 Hz, 2H) 1.40 - 1.54 (m, 2H) 1.04 - 1.18 (m, 2H).

### e) Synthesis of APQ9-1, APQ9-2, APQ10-1, APQ10-2, and APQ10-3

### Synthesis of APQ9-1:

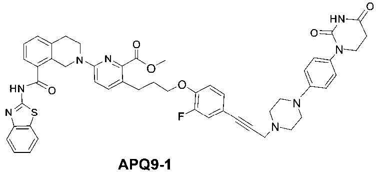

### Synthesis scheme:

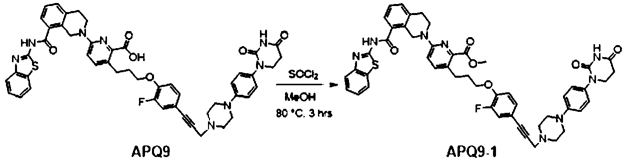

To a solution of Compound APQ9 (20.0 mg, 22.40 µmol, 1.00 eq.) in methanol (5.0 mL), SOCl₂ (13.3 mg, 112 µmol, 8.13 µL, 5.00 eq.) was added, and the mixture was stirred and reacted at 80°C for 3 hours. LCMS (EC17015-82-P1D, P1: RT = 0.449 minutes) showed that 47.6% of the target compound was generated. The reaction mixture was extracted with CH₂Cl₂ (10.0 mL, X 2). The resulting organic layer was washed with saturated saline (10.0 mL), dried over Na₂SO₄, filtered, and then concentrated under reduced pressure to obtain a crude product, which was further purified by Prep-HPLC (chromatographic column: Phenomenex luna C18 150 * 25 mm * 10 µm; mobile phase: [water (FA)-ACN]; gradient elution: 37%-57% B, within 13 minutes) to provide Compound APQ9-1 (6.20 mg, 12.2% yield, 96.3% purity) as a white solid.

**LC-MS:** EC17015-82-P1D, product: RT = 0.449 min, m/z = 454.4 [M/2+H]⁺
**¹H NMR:** EC19038-3-P1A1 (400 MHz DMSO) *δ* ppm 10.26 (s, 1H), 8.03 (d, *J =* 7.6 Hz, 1H), 7.78 (d, *J* = 8.0 Hz, 1H), 7.52 - 7.61 (m, 2H), 7.43 - 7.50 (m, 1H), 7.37 - 7.42 (m, 1H), 7.32 - 7.37 (m, 2H), 7.29 (dd, *J* = 12.0, 1.8 Hz, 1H), 7.20 (d, *J =* 9.0 Hz, 1H), 7.15 (d, *J =* 8.8 Hz, 2H), 7.05 - 7.12 (m, 1H), 6.95 (dd, *J =* 8.8, 5.0 Hz, 3H), 4.90 (s, 2H), 4.00 (t, *J =* 6.4 Hz, 2H), 3.77 - 3.86 (m, 2H), 3.69 (t, *J =* 6.8 Hz, 2H),3.62 (s, 3H), 3.55 (s, 2H), 3.17 (s, 4H), 2.98 (t*, J =* 5.8 Hz, 2H), 2.62 - 2.77 (m, 8H), 1.86 - 1.99 (m, 2H).

### Synthesis of APQ9-2:

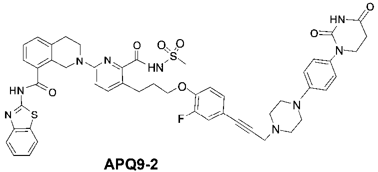

### Synthesis scheme:

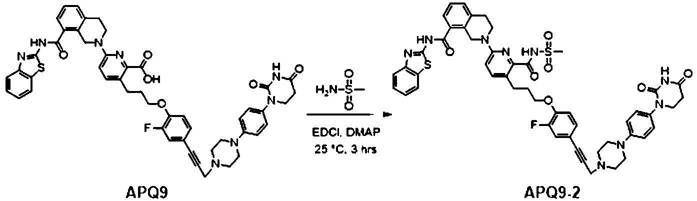

Compound APQ9 (50.0 mg, 56.0 µmol, 1.00 eq.), methylsulfonamide (7.99 mg, 84.0 µmol, 1.50 eq.), EDCI (17.2 mg, 89.6 µmol, 1.60 eq.) and DMAP (13.7 mg, 112 µmol, 2.0 eq.) were mixed in DCM (1.00 mL), and the mixture was stirred and reacted at 25 °C under N₂ atmosphere for 3 hours. LCMS (EC17015-45-P1A, P1: RT= 0.435 minutes) showed that 75.6% of the target compound was generated. The reaction mixture was concentrated under reduced pressure to obtain a crude product, which was further purified by Prep-HPLC (chromatographic column: Phenomenex luna C18 150 * 25 mm * 10 µm; mobile phase: [water (FA)-ACN]; gradient elution: 30%-60% B, within 10 minutes) to provide Compound APQ9-2 (19.9 mg, 18.5 µmol, 33.0% yield, 90.1% purity) as a white solid.

**LC-MS:** EC17015-45-P1A, product: RT = 0.435 min, m/z = 970.3 [M+H]⁺
**¹H NMR:** EC17015-45-P1A (400 MHz DMSO) *δ* ppm 10.25 (s, 1H), 8.14 (s, 1H), 8.02 (d, *J* = 7.2 Hz, 1H), 7.78 (d, *J =* 8.2 Hz, 1H), 7.59 (d, *J =* 7.6 Hz, 1H), 7.44 - 7.52 (m, 2H), 7.39 - 7.43 (m, 1H), 7.32 - 7.37 (m, 2H), 7.25 - 7.30 (m, 1H), 7.07 - 7.22 (m, 5H), 6.94 (d, *J* = 9.0 Hz, 3H), 4.86 (s, 2H), 4.02 (t, *J =* 6.0 Hz, 2H), 3.84 - 3.93 (m, 2H), 3.69 (t*, J* = 6.6 Hz, 2H), 3.54 (s, 2H), 3.16 (s, 4H), 2.99 (d, *J =* 5.6 Hz, 2H), 2.72 - 2.78 (m, 2H), 2.67 (dd, *J =* 3.8, 1.8 Hz, 9H), 1.92 - 2.01 (m, 2H).

### Synthesis of APQ10-1:

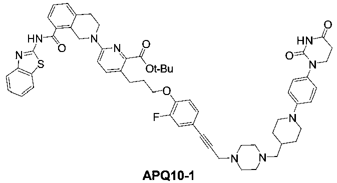

### Synthesis scheme:

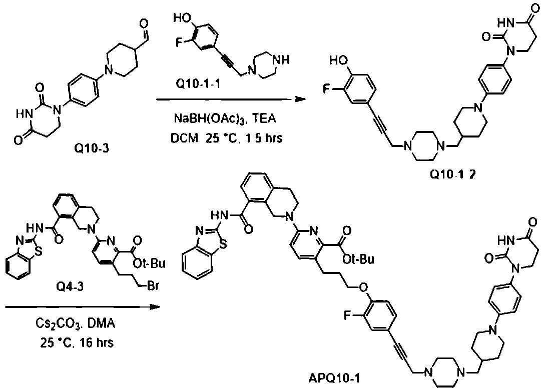

### 1. Synthesis of Q10-1-2

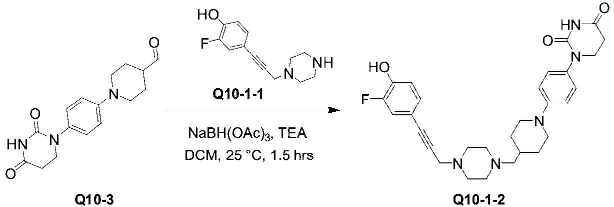

To a solution of Compound Q10-1-1 (334 mg, 1.43 mmol, 1.00 eq.) in DCM (10.0 mL), TEA (1.44 g, 14.3 mmol, 1.99 mL, 10.0 eq.) and Compound Q10-3 (430 mg, 1.43 mmol, 1.00 eq.) were added, and the mixture was stirred and reacted at 25°C for 0.5 hours. Then, NaBH(OAc)₃ (907 mg, 4.28 mmol, 3.00 eq.) was added, and the mixture was further stirred and reacted at 25 °C for 1 hour. The reaction mixture was diluted by adding water (5.0 mL), and extracted with DCM (5.0 mL * 4). The resulting organic layer was washed with saturated saline (10.0 mL), dried over Na₂SO₄, filtered and concentrated under reduced pressure to obtain a crude product, which was purified by reverse-phase HPLC (0.1% FA conditions) to provide Compound Q10-1-2 (160 mg, 308 µmol, 21.6% yield) as a yellow solid.

**LCMS:** EC19038-2-P1A1, RT = 0.238 min, MS (ESI) *m*/*z* = 520.3 [M+H]⁺.

### 2. Synthesis of APQ10-1

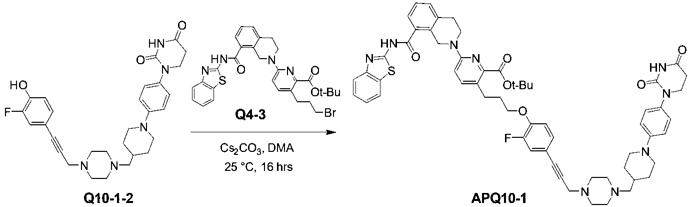

Compound Q4-3 (117 mg, 192 µmol, 1.00 eq.), Q10-1-2 (100 mg, 192 µmol, 1.00 eq.) and Cs₂CO₃ (157 mg, 481 µmol, 2.50 eq.) were mixed in DMA (2.00 mL), and the mixture was stirred and reacted at 25°C under N₂ atmosphere for 16 hours. LCMS (EC17015-91-P1A, P1: RT = 0.433 minutes) showed that 57.6% of the target compound was generated. The reaction mixture was concentrated under reduced pressure to obtain a crude product, which was further purified by Prep-HPLC (chromatographic column: Phenomenex luna C18 150 * 25 mm * 10 µm; mobile phase: [water (FA) - ACN]; gradient elution: 36%-66% B, within 10 minutes) to provide Compound APQ10-1 (63.8 mg, 56.0 µmol, 31.7% yield, 95.8% purity) as a white solid.

**LCMS:** EC17015-91-P1A, product: RT = 0.433 min, m/z = 1046.4 [M+H]⁺
**¹H NMR:** EC17015-93-P1A (400 MHz DMSO) *δ* ppm 10.25 (s, 1H), 8.20 (s, 1H), 8.02 (d, *J* = 7.8 Hz, 1H), 7.77 (d, *J* = 8.2 Hz, 1H), 7.56 (d, *J* = 7.4 Hz, 1H), 7.43 - 7.52 (m, 2H), 7.38 - 7.42 (m, 1H), 7.34 (td, *J* = 7.2, 2.2 Hz, 2H), 7.24 - 7.30 (m, 1H), 7.15 - 7.23 (m, 1H), 7.05 - 7.14 (m, 3H), 6.91 (dd, *J =* 8.8, 4.2 Hz, 3H), 4.93 (s, 2H), 4.02 (t, *J =* 6.2 Hz, 2H), 3.79 (t, *J*=5.94 Hz, 2H), 3.58 - 3.72 (m, 5H), 3.46 (s, 3H), 2.98 (t, *J* = 5.6 Hz, 2H), 2.57 - 2.73 (m, 7H), 2.27 - 2.45 (m, 4H), 2.15 (d, *J =* 6.8 Hz, 2H), 1.87 - 1.98 (m, 2H), 1.76 (d, *J* = 11.0 Hz, 2H), 1.56 - 1.70 (m, 1H), 1.30 (s, 9H) 1.10 - 1.25 (m, 3H).

### Synthesis of APQ10-2:

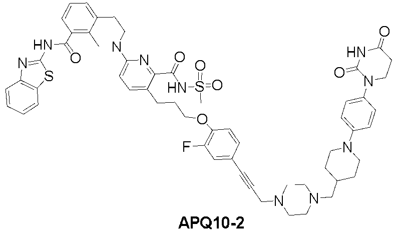

### Synthesis scheme:

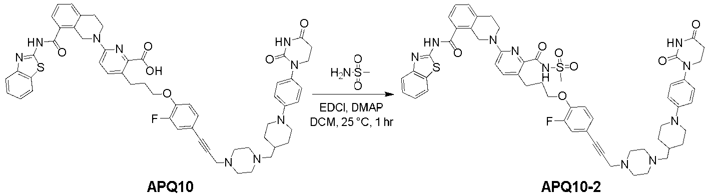

Compound APQ10 (50.0 mg, 50.5 µmol, 1.00 eq.), methylsulfonamide (7.21 mg, 75.8 µmol, 1.50 eq.), EDCI (15.5 mg, 80.8 µmol, 1.60 eq.) and DMAP (12.3 mg, 101 µmol, 2.00 eq.) were mixed in DCM (2.00 mL), and the mixture was stirred and reacted at 25 °C under N₂ atmosphere for 1 hour. LCMS (EC17015-89-P1B, P1: RT = 0.416 minutes) showed that approximately 76.1 % of the target compound was generated. The reaction mixture was diluted by adding water (20.0 mL), and extracted with DCM (20.0 mL * 3). The resulting organic layer was washed with saturated saline (30.0 mL), dried over Na₂SO₄, filtered and concentrated under reduced pressure to obtain a crude product, which was purified by Prep-HPLC (chromatographic column: Phenomenex luna C18 150 * 25 mm * 10 µm; mobile phase: [water (FA) - ACN]; gradient elution: 28%-58% B, within 10 minutes) to provide Compound APQ10-2 (17.0 mg, 15.1 µmol, 29.8% yield, 94.6% purity) as a white solid.

**LCMS:** EC17015-89-P1B, product: RT = 0.416 min, m/z = 1067.5 [M+H]⁺
**¹H NMR:** EC17015-89-P1A (400 MHz DMSO) *δ* ppm 10.24 (s, 1H), 8.16 (s, 1H), 8.03 (d, *J =* 8.4 Hz, 1H), 7.78 (d, *J* = 8.2 Hz, 1H), 7.58 (d, *J* = 7.8 Hz, 1H), 7.44 - 7.50 (m, 1H), 7.40 (d, *J* = 7.8 Hz, 2H), 7.31 - 7.37 (m, 2H), 7.26 (d, *J =* 12.6 Hz, 1H), 7.09 - 7.19 (m, 4H), 6.91 (d, *J =* 9.0 Hz, 2H), 6.67 - 6.75 (m, 1H), 4.83 (s, 2H), 4.01 (t, *J =* 6.8 Hz, 2H), 3.86 (t, *J =* 5.8 Hz, 2H), 3.63 - 3.73 (m, 4H), 3.46 (s, 2H), 2.97 (t, *J =* 5.2 Hz, 2H), 2.91 (s, 3H), 2.58 - 2.73 (m, 8H), 2.44 (s, 4H), 2.17 (d, *J =* 6.4 Hz, 2H), 1.91 - 2.03 (m, 2H), 1.76 (d, *J =* 12.6 Hz, 2H), 1.65 (s, 2H), 1.08 - 1.29 (m, 3H).

### Synthesis of APQ10-3:

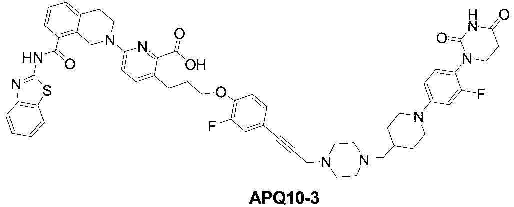

### Synthesis scheme:

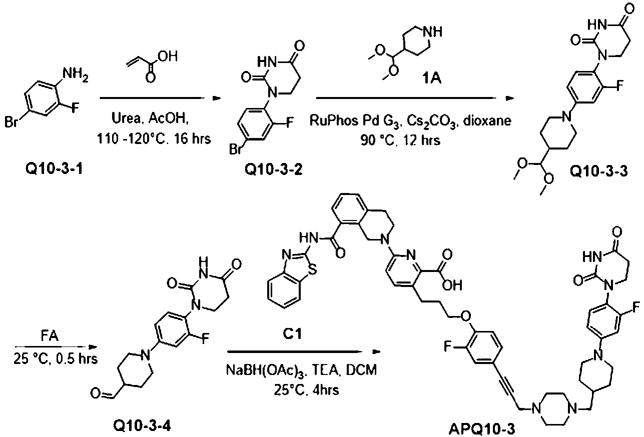

### 1. Synthesis of Q10-3-2

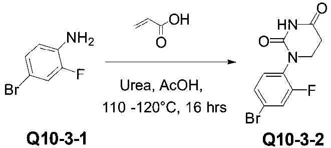

At 20°C, to Compound Q10-3-1 (5.00 g, 26.3 mmol, 1.00 eq.), acrylic acid (3.79 g, 52.6 mmol, 3.61 mL, 2.00 eq.) was added, and the mixture was stirred at 110 °C for 5 hours. Then, acetic acid (50.0 mL) and urea (7.11 g, 118 mmol, 6.37 mL, 4.50 eq.) were added, and the mixture was stirred and reacted at 120°C for 12 hours. LC-MS (EC21184-306-P1B, P1: RT = 0.598 minutes) showed that approximately 76.5% of the target product was generated. The reaction mixture was diluted by adding water (200 mL), filtered and concentrated under reduced pressure to provide the product Compound Q10-3-2 (2.50 g, 8.11 mmol, 30.8% yield, 93.1% purity) as a white solid.

**LC-MS:** EC21184-306-P1B, P1: RT = 0.598 min, m/z = 288.9 [M+H]⁺.

### 2. Synthesis of Q10-3-3

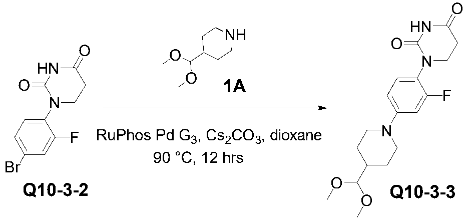

Compound Q10-3-2 (2.20 g, 7.66 mmol, 1.00 eq.), Compound 1A(6.10 g, 38.3 mmol, 5.00 eq.), RuPhos Pd G₃ (641 mg, 766 µmol, 0.10 eq.) and Cs₂CO₃ (4.99 g, 15.3 mmol, 2.00 eq.) were mixed in dioxane (25.0 mL), and the mixture was stirred and reacted at 90°C under N₂ atmosphere for 12 hours. LC-MS (EC21184-315-P1, P1: RT = 0.272 minutes) showed that approximately 12.0% of the target product was generated. The reaction mixture was filtered and concentrated under reduced pressure, and purified by Prep-HPLC (neutral conditions) column chromatography to provide the product Compound Q10-3-3 (200 mg, 321 µmol, 4.20% yield, 58.7% purity) as a brown solid.

**LC-MS:** EC21184-315-P1, P1: RT = 0.272 min, m/z = 366.2 [M+H]⁺.

### 3. Synthesis of Q10-3-4

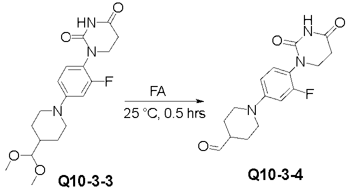

To Compound Q10-3-3 (50.0 mg, 137 µmol, 1.00 eq.), formic acid (0.50 mL) was added, and the mixture was stirred and reacted at 25°C under N₂ atmosphere for 0.5 hours. LC-MS (EC21184-321-P1A, P1: RT = 0.221 minutes) showed that approximately 60.9% of the target product was generated. The reaction mixture was filtered and concentrated under reduced pressure to provide the crude product Compound Q10-3-4 (50.0 mg, crude) as a white solid.

**LC-MS:** EC21184-321-P1A, P1: RT = 0.221 min, m/z = 320.2 [M+H]⁺.

### 4. Synthesis of APQ10-3

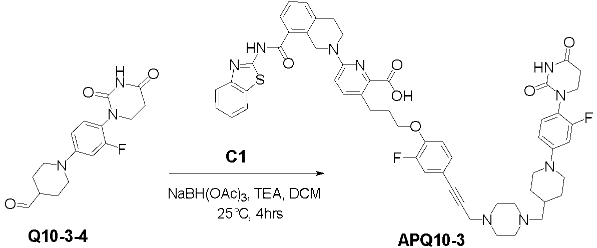

To a solution of Compound Q10-3-4 (50.0 mg, 157 µmol, 1.00 eq.) and Compound C1 (66.2 mg, 93.9 µmol, 0.60 eq.) in DCM (1.00 mL), TEA (158 mg, 1.57 mmol, 218 µL, 10.0 eq.) and NaBH(OAc)₃ (100 mg, 470 µmol, 3.00 eq.) were added, and the mixture was stirred and reacted at 25 °C for 4 hours. LC-MS (EC21184-322-P1B, P1: RT = 1.447 minutes) showed that approximately 44.1% of the target product was generated. The reaction mixture was diluted by adding water (2.0 mL), filtered and concentrated under reduced pressure to provide the crude product, which was further purified by Prep-HPLC (chromatographic column: CD02-Waters Xbidge BEH C18 150*25*10um; mobile phase: [water (NH₄HCO₃)-ACN]; gradient: 28%-58% B, within 10 minutes) to provide the product Compound APQ10-3 (8.00 mg, 7.94 µmol, 4.57% yield, 90.3% purity) as a white solid.

**LC-MS:** EC17015-651-P1B1, P1: RT = 0.424 min, m/z = 1008.5 [M+H]⁺
**¹H NMR:** EC17015-651-P1A (400 MHz DMSO) *δ* 10.36 (s, 1H), 8.03 (d*, J =* 7.6 Hz, 1H), 7.78 (d, *J*= 8.0 Hz, 1H), 7.60 (d, *J* = 6.8 Hz, 1H), 7.52 (d, *J =* 8.8 Hz, 1H), 7.47 (t, *J* = 7.2 Hz, 1H), 7.38 - 7.43 (m, 1H), 7.31 - 7.38 (m, 2H), 7.27 (dd, *J* = 11.6, 1.6 Hz, 1H), 7.13 - 7.22 (m, 2H), 7.05 - 7.12 (m, 1H), 6.92 (d, *J* = 8.4 Hz, 1H), 6.70 - 6.82 (m, 2H), 4.92 (s, 2H), 4.02 (t, *J =* 6.4 Hz, 2H), 3.86 (t, *J =* 6.0 Hz, 2H), 3.68 - 3.77 (m, 2H), 3.61 (t, *J =* 6.8 Hz, 2H), 3.43 - 3.48 (m, 4H), 2.94 - 3.01 (m, 2H), 2.79 (t, *J =* 7.2 Hz, 2H), 2.63 - 2.74 (m, 6H), 2.30 - 2.42 (m, 4H), 2.14 (d, *J* = 7.2 Hz, 2H), 1.91 - 2.02 (m, 2H), 1.63 - 1.81 (m, 3H), 1.05 - 1.21 (m, 2H).

### f) Synthesis of APQ901, APQ902, APQ902a, APQ903, amd APQ904

### Synthesis scheme:

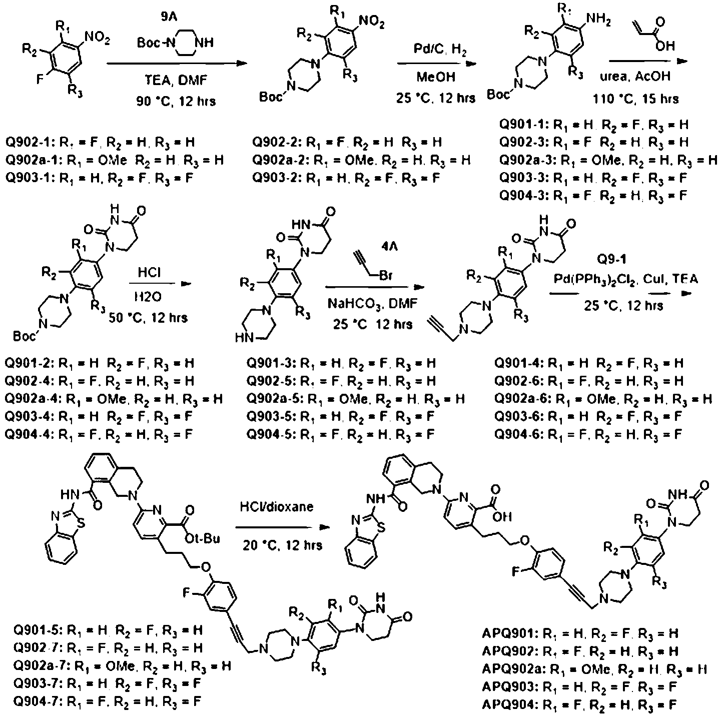

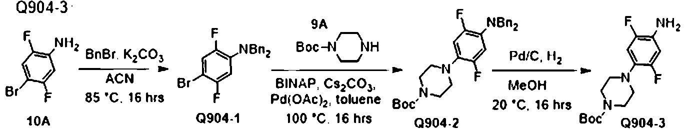

### Synthesis of APQ901:

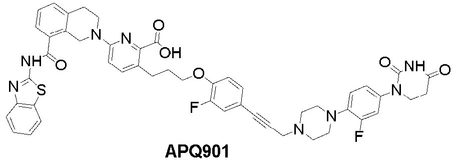

The synthesis procedure for the PROTAC molecule APQ901 was similar to that for APQ902, and the raw material Q901-1 was available by direct purchase, but the starting materials were replaced with Q901-1 and acrylic acid, and the target product APQ901 (17.0 mg, 16.0 µmol, 19.3% yield, 85.6% purity) was obtained by purification after synthesis, as a white solid.

**LC-MS:** EC21184-138-pla, P1: RT = 1.820 min, m/z = 911.6 [M+H]⁺
**¹H NMR:** EC21184-138-P1CC (400 MHz DMSO) *δ* 12.40 - 13.11 (m, 2H), 10.37 (s, 1H), 8.04 (d, *J=* 8.0 Hz, 1H), 7.79 (d, *J* = 8.0 Hz, 1H), 7.59 (d, *J* = 7.2 Hz, 1H), 7.54 (d, *J =* 8.8 Hz, 1H), 7.44 - 7.50 (m, 1H), 7.39 - 7.43 (m, 1H), 7.32 - 7.38 (m, 2H), 7.30 (dd*, J =* 12.0, 2.0 Hz, 1H), 7.15 - 7.22 (m, 2H), 7.11 (d, *J =* 8.8 Hz, 1H), 7.03 - 7.09 (m, 2H), 6.95 (d, *J =* 8.8 Hz, 1H), 4.92 (s, 2H), 4.02 (t, *J =* 6.4 Hz, 2H), 3.86 (t, *J* = 6.0 Hz, 2H), 3.74 (t, *J =* 6.8 Hz, 2H), 3.55 (s, 2H), 3.04 (s, 4H), 2.98 (t, *J =* 5.6 Hz, 2H), 2.80 (t, J = 7.6 Hz, 2H), 2.68 (t, *J* = 6.4 Hz, 6H), 1.92 - 2.02 (m, 2H).

### Synthesis of APQ902:

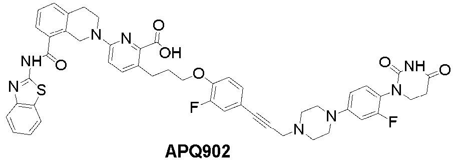

### Synthesis scheme:

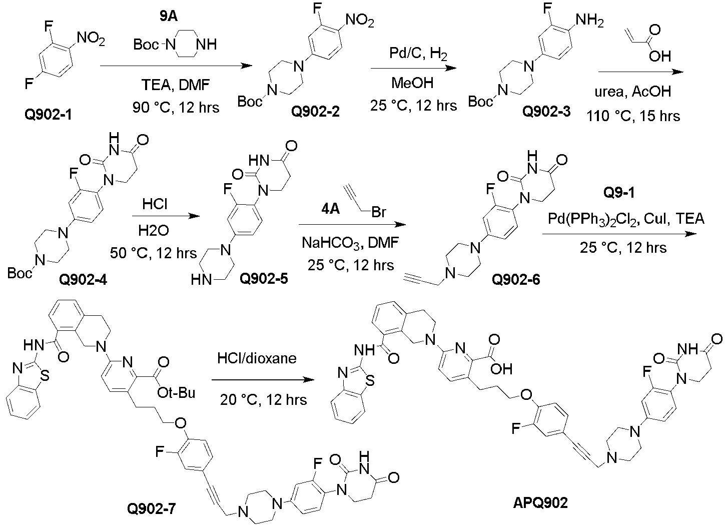

### 1. Synthesis of Compound Q902-2

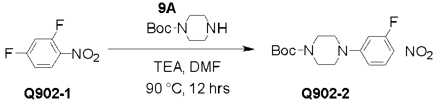

Compound Q902-1 (20.0 g, 126 mmol, 13.8 mL, 1.00 eq.), Compound 9A (23.4 g, 126 mmol, 1.00 eq.) and TEA (38.2 g, 377 mmol, 52.5 mL, 3.00 eq.) were mixed in DMF (200 mL), and the mixture was stirred and reacted at 90 °C under N₂ atmosphere for 12 hours. To the reaction mixture, water (600 mL) was added, and extracted with ethyl acetate (200 mL * 3). The resulting organic layer was washed with saturated saline (200 mL), dried over Na₂SO₄, filtered and concentrated under reduced pressure to obtain a crude product, which was further purified by column chromatography (SiO₂, petroleum ether /ethyl acetate =1/0 to 9/1) to provide the product Compound Q902-2 (13.1 g, crude) as a yellow solid.

**LC-MS:** EC17015-352-P1A, product: RT = 0.445 min
**¹H NMR:** EC17015-352-P1A (400 MHz CDCl₃) *δ* ppm 8.01 (t, *J =* 9.2 Hz, 1H), 6.38 - 6.64 (m, 2H), 3.53 - 3.71 (m, 4H), 3.36 - 3.47 (m, 4H), 1.48 (s, 9H).

### 2. Synthesis of Compound Q902-3

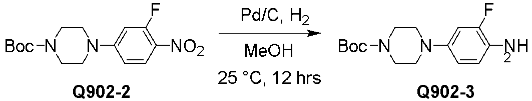

To a solution of Compound Q902-2 (2.00 g, 6.15 mmol, 1.00 eq.) in methanol (20.0 mL), Pd/C (1.00 g, 10% purity) was added, and the mixture was shook and reacted at 25 °C under H₂ (30 psi) atmosphere for 12 hours. The reaction mixture was filtered, and washed with methanol. The resulting organic layer was collected and concentrated, and then was dried to provide the crude product Compound Q902-3 (1.70 g, 5.76 mmol, 93.6% yield) as a brown solid.

**¹H** NMR: EC17015-356-P1A (400 MHz CDCl₃) *δ* ppm 6.69 - 6.77 (m, 1H), 6.65 (dd, *J =* 13.2, 2.4 Hz, 1H), 6.58 (dd, *J =* 8.4, 1.8 Hz, 1H), 3.52 - 3.63 (m, 4H), 3.48 (d, *J* = 10.4 Hz, 2H), 2.90 - 3.04 (m, 4H), 1.49 (s, 9H).

### 3. Synthesis of Compound Q902-4

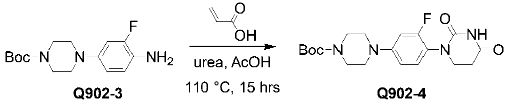

Compound Q902-3 (5.00 g, 16.9 mmol, 1.00 eq.) and acrylic acid (4.88 g, 67.7 mmol, 4.64 mL, 4.00 eq.) were mixed, and the mixture was stirred and reacted at 110°C for 3 hours. Then, urea (6.53 g, 109 mmol, 5.84 mL, 6.42 eq.) and AcOH (50.0 mL) were added, and the mixture was stirred and reacted at 120°C for 12 hours. The reaction mixture was concentrated under reduced pressure to provide the product Compound Q902-4 (6.60 g, crude) as a dark brown oil, which was directly used in the next reaction without purification.

### 4. Synthesis of Compound Q902-5

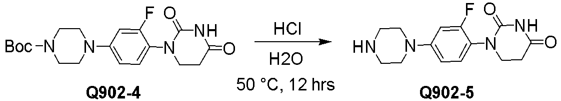

Compound Q902-4 (6.60 g, 16.8 mmol, 1.00 eq.) was mixed in HCl (33.0 mL) and H₂O (33.0 mL), and the mixture was stirred and reacted at 50°C for 12 hours. LCMS (EC17015-374-P1A, P1: RT= 0.509 minutes) showed that approximately 49.1 % of the target product was generated. The reaction mixture was concentrated under reduced pressure to obtain a crude product, which was purified by Prep-HPLC (chromatographic column: CD19-Daisogel SP-100-8-ODS-PK 200*50*10 µm; mobile phase: [water (NH₃H₂O) -ACN]; gradient elution: 8%-36% B, within 10 minutes) to provide the product Compound Q902-5 (160 mg, 547 µmol, 8.00% yield) as a brown solid.

**LC-MS:** EC17015-374-P1A, product: RT = 0.509 min, m/z = 293.1 [M+H]⁺.

### 5. Synthesis of Compound Q902-6

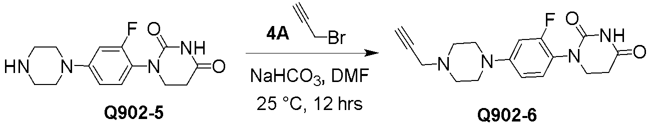

Compound Q902-5 (140 mg, 479 µmol, 1.00 eq.), Compound 4A (57.0 mg, 479 µmol, 41.3 µL, 1.00 eq.) and NaHCO₃ (120.7 mg, 1.44 mmol, 55.9 µL, 3.00 eq.) were mixed in DMF (2.00 mL), and the mixture was stirred and reacted at 25 °C for 12 hours. LCMS (EC17015-400-P1W, P1: RT = 0.566 minutes) showed that approximately 81.3% of the target product was generated. The reaction mixture was filtered and concentrated under reduced pressure to obtain a crude product, which was purified by Prep-HPLC (chromatographic column: CD02-Waters Xbidge BEH C18 150*25*10 µm; mobile phase: [water (NH₃H₂O)-ACN]; gradient elution: 12%-42% B, within 10 minutes) to provide the product Compound Q902-6 (90.0 mg, 272 µmol, 56.9% yield) as a brown solid.

**LC-MS:** EC17015-400-P1W, product: RT = 0.566 min, m/z = 331.2 [M+H]⁺
**¹H NMR:** EC17015-400-P1A (400 MHz DMSO) *δ* ppm 10.24 - 10.44 (m, 1H), 7.20 (t, *J* = 9.0 Hz, 1H), 6.72 - 6.90 (m, 2H), 3.62 (t, *J =* 6.6 Hz, 2H), 3.33 - 3.34 (m, 2H), 3.15 - 3.24 (m, 5H), 2.68 (t, *J =* 6.6 Hz, 2H), 2.54 - 2.60 (m, 4H).

### 6. Synthesis of Compound Q902-7

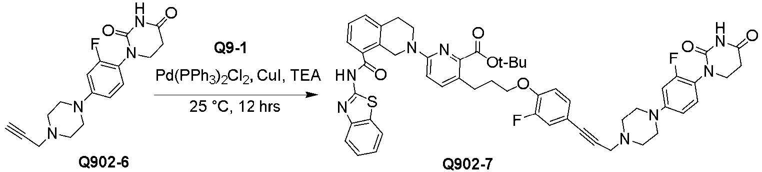

Q9-1 (108 mg, 141 µmol, 1.00 eq.), Compound Q902-6 (70.0 mg, 212 µmol, 1.50 eq.), CuI (1.35 mg, 7.06 µmol, 0.05 eq.) and Pd(PPh₃)₂Cl₂ (9.92 mg, 14.1 µmol, 0.10 eq.) were mixed in TEA (1.50 mL) and DMF (0.50 mL), and the mixture was stirred and reacted at 25 °C under N₂ atmosphere for 12 hours. LCMS (EC17015-412-P1A, P1: RT = 0.449 minutes) showed that approximately 31.6% of the target product was generated. To the reaction mixture, ice-water (20.0 mL) was added, filtered and concentrated under reduced pressure to obtain a crude product, which was further purified by column chromatography (SiO₂, dichloromethane/ethyl acetate =1/4 to 0/1) to provide the product Compound Q902-7 (60.0 mg, 62.0 µmol, 43.9% yield) as a yellow solid.

**LC-MS:** EC17015-412-P1A, product: RT = 0.449 min, m/z = 967.4 [M+H]⁺.

### 7. Synthesis of Compound APQ902

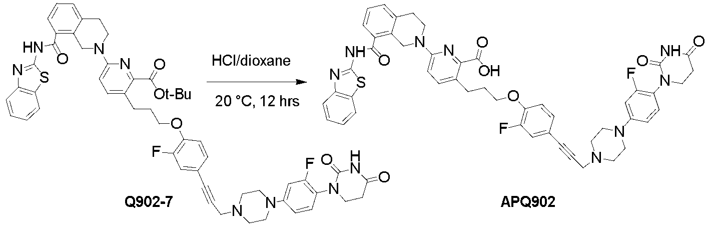

Compound Q902-7 (60.0 mg, 62.0 µmol, 1.00 eq.) was mixed in HCl/dioxane (5.00 mL), and the mixture was stirred and reacted at 20°C for 12 hours. LCMS (EC17015-419-P1B1, P1: RT = 0.422 minutes) showed that approximately 71.4% of the target product was generated. The reaction mixture was concentrated under reduced pressure to obtain a crude product, which was further purified by Prep-HPLC (chromatographic column: CD03-Welch Xtimate C18 150*25*5 µm; mobile phase: [water (FA)-ACN]; gradient elution: 28%-58% B, within 10 minutes) to provide the product Compound APQ902(15.0 mg, 14.6 µmol, 23.6% yield, 88.8% purity) as a white solid.

**LC-MS:** EC17015-419-P1B1, product: RT = 0.422 min, m/z = 911.3 [M+H]⁺
**¹H NMR:** EC17015-419-P1A (400 MHz DMSO) *δ* ppm 10.38 (s, 1H), 8.17 (s, 1H), 8.03 (d, *J* = 7.8 Hz, 1H), 7.79 (d, *J =* 8.0 Hz, 1H), 7.59 (d, *J* = 7.2 Hz, 1H), 7.52 (d, *J* = 8.8 Hz, 1H), 7.47 (t, *J* = 7.6 Hz, 1H), 7.39 - 7.42 (m, 1H), 7.32 - 7.38 (m, 2H), 7.28 (dd, *J* = 12.0, 1.6 Hz, 1H), 7.16 - 7.24 (m, 2H), 7.07 - 7.14 (m, 1H), 6.92 (d, *J* = 8.6 Hz, 1H), 6.81 - 6.87 (m, 1H), 6.74 - 6.80 (m, 1H), 4.91 (s, 2H), 4.02 (t, *J =* 6.4 Hz, 2H), 3.85 (t, *J* = 5.6 Hz, 2H), 3.60 - 3.64 (m, 2H), 3.55 (s, 2H), 3.21 (s, 4H), 2.97 (t, *J =* 5.4 Hz, 2H), 2.75 - 2.82 (m, 2H), 2.63 - 2.71 (m, 6H), 1.89 - 2.07 (m, 2H).

### Synthesis of APQ902a:

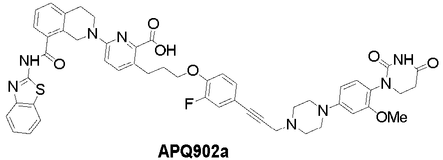

The synthesis procedure for the PROTAC molecule APQ902a was similar to that for APQ902, but the starting materials were replaced with Compound Q902a-1 and 9A, and the target product APQ902a (8.00 mg, 8.46 µmol, 6.90% yield, 97.6% purity) was obtained by purification after synthesis, as a white solid.

**LC-MS:** EC17015-420-P1B1, product: RT = 0.413 min, m/z = 923.3 [M+H]⁺
**¹H NMR:** EC17015-420-P1A (400 MHz DMSO) *δ* ppm 10.20 (s, 1H), 8.03 (d, *J =* 8.0 Hz, 1H), 7.78 (d, *J* = 7.6 Hz, 1H), 7.60 (d, *J =* 7.6 Hz, 1H), 7.48 - 7.53 (m, 1H), 7.42 - 7.48 (m, 1H), 7.38 - 7.42 (m, 1H), 7.31 - 7.37 (m, 2H), 7.25 - 7.31 (m, 1H), 7.19 (d, *J =* 8.8 Hz, 1H), 7.10 (t, *J* = 8.8 Hz, 1H), 7.03 (d, *J =* 8.4 Hz, 1H), 6.90 (d, *J* = 9.2 Hz, 1H), 6.62 (d, *J =* 1.6 Hz, 1H), 6.49 (dd, *J =* 8.8, 2.4 Hz, 1H), 4.91 (s, 2H), 4.02 (t, *J* = 6.4 Hz, 2H), 3.85 (t, *J =* 6.0 Hz, 2H), 3.77 (s, 3H), 3.55 (s, 2H), 3.49 (d, *J =* 6.4 Hz, 2H), 3.21 (s, 4H), 2.94 - 3.01 (m, 2H), 2.74 - 2.80 (m, 2H), 2.61 - 2.68 (m, 6H), 1.93 - 2.02 (m, 2H).

### Synthesis of APQ903:

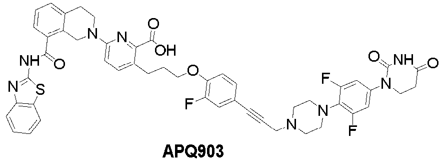

The synthesis procedure for the PROTAC molecule APQ903 was similar to that for APQ902, but the starting materials were replaced with Compound Q903-1 and 9A, and the target product APQ903(12.0 mg, 12.0 µmol, 29.5% yield, 92.6% purity) was obtained by purification after synthesis, as a white solid.

**LC-MS:** EC17015-421-P1B1, product: RT = 0.418 min, m/z = 929.3 [M+H]⁺
**¹H NMR:** EC17015-421-P1A (400 MHz DMSO) *δ* ppm 10.47 (s, 1H), 8.03 (d, *J =* 8.4 Hz, 1H), 7.78 (d, *J* = 8.0 Hz, 1H), 7.59 (d, *J* = 7.2 Hz, 1H), 7.52 (d, *J =* 8.8 Hz, 1H), 7.47 (t, *J =* 7.2 Hz, 1H), 7.38 - 7.43 (m, 1H), 7.27 - 7.37 (m, 3H), 7.20 (d, *J* = 8.8 Hz, 1H), 7.06 - 7.13 (m, 3H), 6.92 (d, *J* = 8.0 Hz, 1H), 4.85 - 4.99 (m, 2H), 4.02 (t, *J =* 6.4 Hz, 2H), 3.86 (t, *J =* 5.6 Hz, 2H), 3.76 (t, *J =* 6.8 Hz, 2H), 3.54 (s, 2H), 3.13 (s, 4H), 2.95 - 3.00 (m, 2H), 2.76 - 2.81 (m, 2H), 2.62 - 2.68 (m, 6H), 1.92 - 2.02 (m, 2H).

### Synthesis of APQ904:

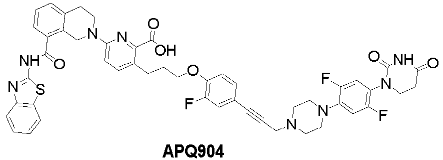

The synthesis procedure for the PROTAC molecule APQ904 was similar to that for APQ902, but the Intermediate APQ904-3 was synthesized using a different synthesis method, where the synthesis method from APQ904-3 to APQ904 was identical to that for APQ902. The target product APQ904 (18.7 mg, 19.5 µmol, 32.0% yield, 96.9% purity) was obtained by purification after synthesis, as a white solid.

**LC-MS:** EC21184-159-pla, P1: RT = 0.409 min, m/z = 929.2 [M+H]⁺
**¹H NMR:** EC21184-159-p1dg1 (400 MHz DMSO) *δ* 12.41 - 13.04 (m, 2H), 10.47 (s, 1H), 8.04 (d, *J* = 8.0 Hz, 1H), 7.79 (d, *J =* 8.0 Hz, 1H), 7.60 (d, *J=* 7.2 Hz, 1H), 7.55 (d, *J* = 8.8 Hz, 1H), 7.47 (t, *J =* 7.2 Hz, 1H), 7.39 - 7.43 (m, 1H), 7.27 - 7.38 (m, 4H), 7.17 - 7.22 (m, 1H), 7.07 - 7.13 (m, 1H), 6.92 - 7.01 (m, 2H), 4.92 (s, 2H), 4.02 (t, *J =* 6.4 Hz, 2H), 3.86 (t, *J =* 6.0 Hz, 2H), 3.66 (t, *J =* 6.8 Hz, 2H), 3.55 (s, 2H), 3.08 (s, 4H), 2.98 (t, *J* = 5.6 Hz, 2H), 2.80 (t, *J* = 7.6 Hz, 2H), 2.63 - 2.71 (m, 6H), 1.93 - 2.00 (m, 2).

### Synthesis of Intermediate APQ904-3:

### 1. Synthesis of Compound Q904-1

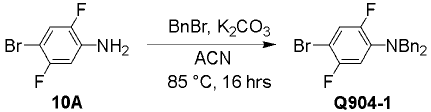

To a solution of Compound 10A (24.0 g, 115 mmol, 1.00 eq.) in ACN (240 mL), BnBr (59.2 g, 346 mmol, 41.1 mL, 3.0 eq.) and K₂CO₃ (47.8 g, 346 mmol, 3.00 eq.) were added, and the mixture was stirred and reacted at 85°C for 16 hours. LC-MS (EC21184-81-pld, P1: RT = 0.577 minutes) showed that approximately 65.7% of the target product was generated. The reaction mixture was concentrated under reduced pressure to obtain a crude product, which was further purified by column chromatography (SiO₂, petroleum ether /ethyl acetate =100/0) to provide the product Compound Q904-1 (40.0 g, crude) as a white solid.

**LC-MS:** EC21184-81-pld, P1: RT = 0.577 min, m/z = 387.9 [M+H]⁺
**¹H NMR:** EC21184-81-p1gz (400 MHz DMSO) *δ* 7.54 (dd, *J =* 12.4, 6.8 Hz, 1H), 7.16 - 7.40 (m, 10H), 6.91 (dd, *J* = 11.2, 7.6 Hz, 1H), 4.40 (s, 4H).

### 2. Synthesis of Compound Q904-2

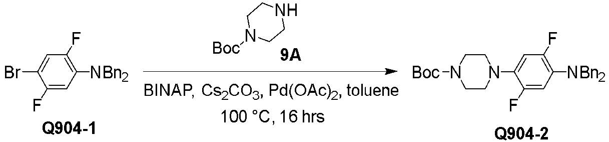

Compound Q904-1 (34.0 g, 87.6 mmol, 1.00 eq.), Compound 9A(17.9 g, 96.3 mmol, 1.10 eq.), Cs₂CO₃ (57.1 g, 175.2 mmol, 2.00 eq.), BINAP (10.9 g, 17.5 mmol, 0.20 eq.) and Pd(OAc)₂ (1.97 g, 8.76 mmol, 0.10 eq.) were mixed in toluene (340 mL), and the mixture was stirred and reacted at 100°C under N₂ atmosphere for16 hours. LC-MS (EC21184-91-pla, P1: RT = 0.602 minutes) showed that approximately 52.7% of the target product was generated. The reaction mixture was concentrated under reduced pressure to obtain a crude product, which was further purified by column chromatography (SiO2, petroleum ether /ethyl acetate =100/0 to 85/15) to provide the product Compound Q904-2 (36.0 g, 67.8 mmol, 77.5% yield, 93.0% purity) as a white solid.

**LC-MS:** EC21184-91-pla, P1: RT = 0.602 min, m/z = 494.2 [M+H]⁺

### 3. Synthesis of Compound Q904-3

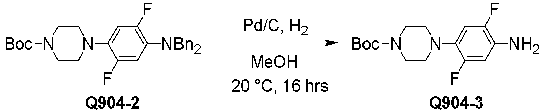

To a solution of Compound Q904-2 (34.0 g, 68.9 mmol, 1.00 eq.) in methanol (700 mL), Pd/C (3.67 g, 3.44 mmol, 10% purity, 0.05 eq.) was added under N₂ atmosphere, then H₂ was filled, and the mixture was stirred and reacted at 20°C under H₂ (40 Psi) atmosphere for 16 hours. LC-MS (EC21184-96-p1b2, R1: RT = 0.391 min, P1: RT= 0.391 minutes) showed that approximately 81.3% of the target product was generated. The reaction mixture was concentrated under reduced pressure to obtain a crude product, which was further purified by column chromatography (SiO₂, petroleum ether /ethyl acetate =100/0 to 88/12) to provide the product Compound Q904-3 (18.0 g, 57.5 mmol, 83.4% yield, 100% purity) as a white solid.

**LC-MS:** EC21184-96-p1b2, P1: RT = 0.391 min, m/z = 258.0 [M-55+H]⁺
**¹H NMR:** EC21184-96-p1gz (400 MHz CHLOROFORM) *δ* 6.62 - 6.88 (m, 1H), 6.52 (dd, *J* = 12.8, 8.0 Hz, 1H), 3.56 - 3.62 (m, 4H), 2.91 (s, 4H), 1.48 (s, 9H).

### Example 7. Synthesis of PROTAC molecules (APH1-APH20 and APH101-APH106) for recruiting the VHL-E3 enzyme

### a) Synthesis of APH1, APH2, APH3, APH4, and APH5

### Synthesis of APH1:

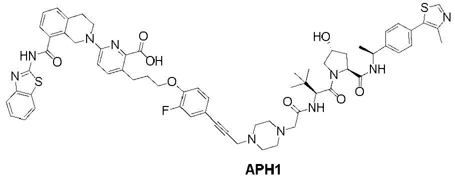

### Synthesis scheme:

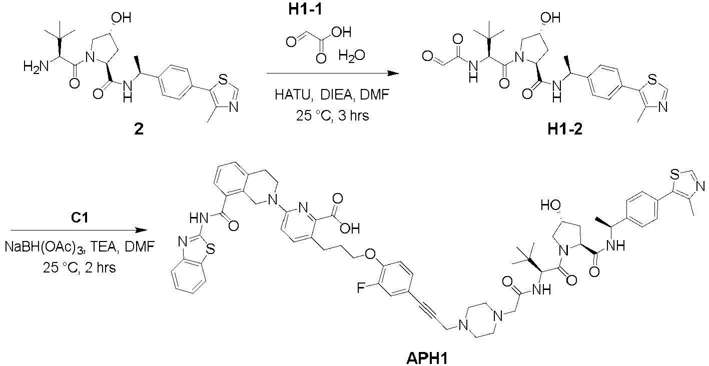

### 1. Synthesis of Compound H1-2

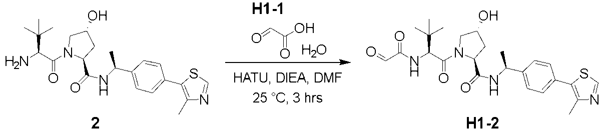

To a solution of Compound 2 (200 mg, 450 µmol, 1.0 eq.) in DMF (2.00 mL), HATU (257 mg, 674 µmol, 1.5 eq.), DIEA (174 mg, 1.35 mmol, 235 µL, 3.0 eq.) and H1-1 (200 mg, 1.35 mmol, 3.0 eq.) were added, and the mixture was stirred and reacted at 25°C for 3 hours. LCMS (EC16331-51-P1A6, P1: RT = 0.359 minutes) showed that Compound 2 was reacted completely, and a target product main peak with the expected m/z value was generated. The reaction mixture was and separated and extracted by adding water (5.00 mL) and DCM (5.00 mL * 3). The resulting organic layer was washed with saturated saline (5.00 mL * 2), dried over Na₂SO₄, filtered and concentrated under reduced pressure to provide the crude product Compound H1-2 (120 mg, 186.97 µmol, 13.85% yield, 78% purity) as a white solid.

**LC-MS:** EC16331-51-P1C; product: RT = 0.361 min
**¹H NMR:** EC16331-51-P1C (400 MHz DMSO) *δ* ppm 8.99 (s, 1H), 8.46 (br d, *J* = 7.6 Hz, 1H), 7.32 - 7.47 (m, 5H), 4.97 - 5.00 (m, 1H), 4.86 - 4.96 (m, 1H), 4.40 - 4.51 (m, 2H), 4.28 (br s, 1H), 3.56 (br s, 5H), 2.45 (s, 3H), 2.01 - 2.10 (m, 1H), 1.77 (ddd, *J =* 12.8, 8.8, 4.4 Hz, 1H), 1.37 (br d, *J =* 6.8 Hz, 3H), 0.93 (s, 9H).

### 2. Synthesis of Compound APH1

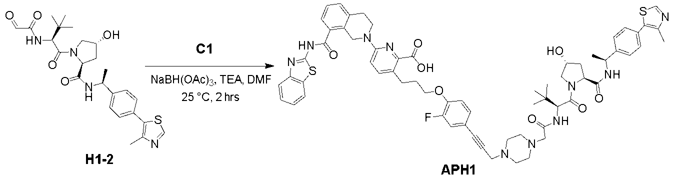

To a solution of Compound H1-2 (100 mg, 200 µmol, 1.0 eq.) in DMF (2.00 mL), TEA (202 mg, 2.00 mmol, 278 µL, 10.0 eq.), NaBH(OAc)₃ (127 mg, 600 µmol, 3.0 eq.) and Compound C1 (70.4 mg, 99.9 µmol, 0.5 eq.) were added, and the mixture was stirred and reacted at 25°C for 2 hours. LCMS (EC16331-55-P1A, P1: RT = 0.462 min; R2: RT = 0.409 minutes) showed that Compound C1 was reacted completely, and a target product main peak with the expected m/z value was generated. The reaction mixture was filtered and concentrated under reduced pressure to provide the crude product for Compound APH1, which was purified by preparative -HPLC (FA conditions; chromatographic column: Phenomenex luna C18 150 * 25mm * 10µm; mobile phase: [water (FA)-ACN]; gradient: 23%-53% B, within 10 minutes) column chromatography to provide the product APH1 (7.00 mg, 5.31 µmol, 2.66% yield, 90.23% purity) as a white solid.

**LC-MS:** EC16331-55-P1E; product: RT = 0.456 min
**¹H NMR:** EC16331-55-P1T (400 MHz DMSO) *δ* ppm 8.94 - 8.99 (m, 1H), 8.39 - 8.46 (m, 1H), 8.21 (s, 1H), 8.03 (br d, *J* = 7.6 Hz, 1H), 7.73 - 7.81 (m, 2H), 7.59 (br d, *J =* 6.8 Hz, 1H), 7.47 (br dd, *J =* 14.0, 8.2 Hz, 2H), 7.40 (br t, *J* = 7.2 Hz, 3H), 7.32 - 7.36 (m, 3H), 7.26 (br d, *J* = 12.0 Hz, 1H), 7.19 (br d, *J* = 8.2 Hz, 1H), 7.06 - 7.12 (m, 1H), 6.88 (br d, *J =* 8.4 Hz, 1H), 4.83 - 4.93 (m, 3H), 4.37 - 4.53 (m, 3H), 4.28 (br s, 1H), 4.01 (br *t, J* = 6.0 Hz, 3H), 3.84 (br s, 3H), 3.58 (br s, 3H), 3.48 (br s, 4H), 2.90 - 3.08 (m, 6H), 2.72 - 2.82 (m, 3H), 2.42 - 2.46 (m, 3H), 2.00 - 2.08 (m, 1H), 1.97 (br d, *J =* 6.0 Hz, 2H), 1.72 - 1.80 (m, 1H), 1.38 (br s, 1H), 1.28 - 1.37 (m, 3H), 1.23 (br s, 1H), 0.93 (br s, 9H).

### Synthesis of APH2:

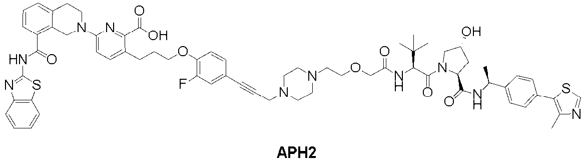

### Synthesis scheme:

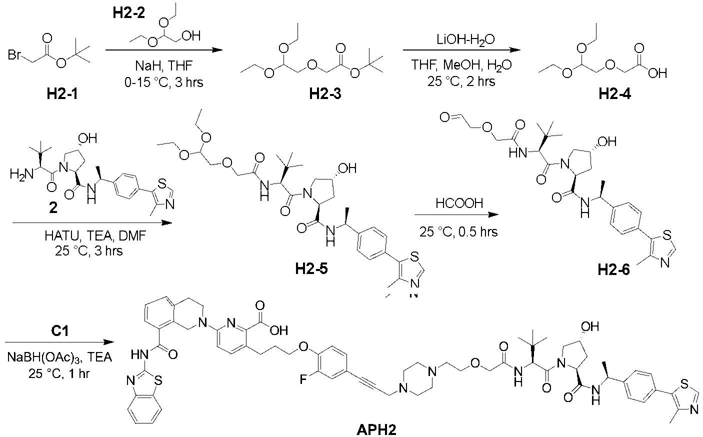

### 1. Synthesis of Compound H2-3

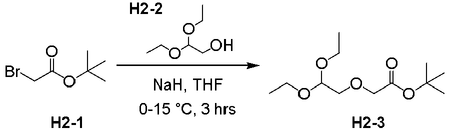

To a solution of Compound H2-2 (10.3 g, 76.9 mmol, 1.5 eq.) in THF (120 mL), NaH (3.08 g, 76.9 mmol, 60% purity, 1.5 eq.) was added at 0 °C under N₂ atmosphere, and the mixture was stirred at 0 °C for 0.5 hours. Then, at 0 °C, a solution of Compound H2-1 (10.0 g, 51.3 mmol, 7.57 mL, 1.0 eq.) dissolved in THF (20.0 mL) was added dropwise, and the mixture was stirred and reacted at 15°C under N₂ atmosphere for 2 hours. TLC (PE: EA = 15:1, R1: R_{f}= 0.92; R2: R_{f} = 0.1; P1: R_{f}= 0.44) showed that Compound H2-1 was reacted completely, and a new product peak was generated. To the reaction mixture, NH₄Cl (200 mL) was added at 0 °C to quench the reaction. The mixture was diluted by adding water (200 mL), and extracted with ethyl acetate (160 mL * 3). The resulting organic layer was washed with saturated saline (200 mL * 2), dried over Na₂SO₄, filtered and concentrated under reduced pressure to obtain a crude product, which was purified by column chromatography (SiO₂, petroleum ether: ethyl acetate = 98:1 to 95:1) to provide the product Compound H2-3 (1.30 g, 5.24 mmol, 10.21% yield) as a colorless liquid.

**¹H NMR:** EC9174-340-P1A (400 MHz DMSO) *δ* ppm 4.58 (t, *J* = 5.2 Hz, 1H), 4.00 (s, 2H), 3.56 - 3.65 (m, 2H), 3.45 - 3.52 (m, 2H), 3.43 (d, *J =* 5.2 Hz, 2H), 1.42 (s, 9H), 1.11 (t*, J =* 7.2 Hz, 6H).

### 2. Synthesis of Compound H2-4

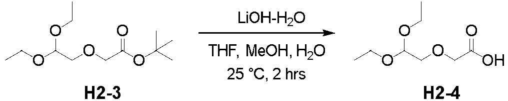

To a solution of Compound H2-3 (210 mg, 846 µmol, 1.0 eq.) in THF (1.00 mL), methanol (1.00 mL) and H₂O (2.00 mL), LiOH-H₂O (355 mg, 8.46 mmol, 10.0 eq.) was added, and the mixture was stirred at 25 °C for 2 hours. TLC (PE: EA = 15:1, R1: R_{f}= 0.38) showed that Compound H2-3 was reacted completely, and a new compound peak was generated. The reaction mixture was adjusted to pH 6 with hydrochloric acid (1 M), and extracted with ethyl acetate (4.00 mL * 3). The resulting organic layer was washed with saturated saline (10.0 mL * 2), dried over Na₂SO₄, filtered and concentrated under reduced pressure to provide the crude product Compound H2-4 (110 mg, 572 µmol, 67.67% yield) as a yellow oil, which was directly used in the next reaction without purification.

**¹H NMR:** EC16331-59-P1A1 (400 MHz DMSO) *δ* ppm 4.58 (t, *J =* 5.2 Hz, 1H), 4.00 (s, 2H), 3.56 - 3.65 (m, 2H), 3.45 - 3.52 (m, 2H), 3.43 (d, *J =* 5.2 Hz, 2H), 1.42 (s, 9H), 1.11 (t*, J =* 7.2 Hz, 6H).

### 3. Synthesis of Compound 4

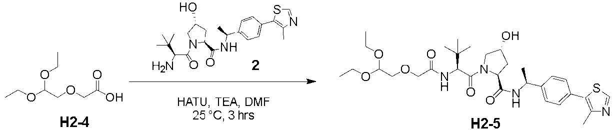

Compound H2-4 (80.0 mg, 416 µmol, 1.0 eq.), Compound 2 (185 mg, 416 µmol, 1.0 eq.), HATU (237 mg, 624 µmol, 1.5 eq.) and TEA (126 mg, 1.25 mmol, 174 µL, 3.0 eq.) were mixed in DMF (2.00 mL), and the mixture was stirred and reacted at 25°C under N₂ atmosphere for 3 hours. LC-MS (EC16331-60-P1A1, P1: RT = 0.440 min; R2: RT = 0.313 minutes) showed that Compound H2-4 was reacted completely, and a target product main peak with the expected m/z value was generated. The reaction mixture was filtered and concentrated under reduced pressure to obtain a crude product, which was purified by Prep-HPLC (FA conditions; chromatographic column: Phenomenex luna C₁₈ 150 * 25mm * 10µm; mobile phase: [water (FA)-ACN]; gradient: 30%-60% B, within 10 minutes) to provide the product Compound H2-5 (80.0 mg, 114 µmol, 27.33% yield, 88% purity).

**LC-MS:** EC16331-60-P1C; product: RT = 0.405 min
**¹H NMR:** EC16331-60-P1S (400 MHz DMSO)δ ppm 8.99 (s, 1H), 8.45 (br d, *J =* 7.6 Hz, 1H), 7.41 - 7.45 (m, 2H), 7.35 - 7.40 (m, 3H), 4.91 (quin, *J =* 7.2 Hz, 1H), 4.63 (t, *J* = 5.2 Hz, 1H), 4.55 (br d, *J =* 9.6 Hz, 1H), 4.41 - 4.47 (m, 1H), 4.28 (br s, 1H), 3.98 (s, 2H), 3.44 - 3.67 (m, 9H), 2.45 (s, 3H), 1.99 - 2.10 (m, 1H), 1.77 (ddd, *J =* 12.8, 8.8, 4.4 Hz, 1H), 1.37 (br d, *J =* 7.2 Hz, 3H), 1.09 - 1.16 (m, 6H), 0.94 (s, 9H).

### 4. Synthesis of Compound H2-6

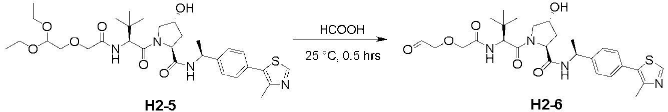

To Compound H2-5 (70.0 mg, 113 µmol, 1.0 eq.), formic acid (1.00 mL) was added, and the mixture was stirred and reacted at 25 °C for 0.5 hours. LC-MS (EC16331-68-P1A2, P1: RT = 0.325 minutes) showed that Compound H2-5 was reacted completely, and a target product main peak with the expected m/z value was generated. The reaction mixture was filtered and concentrated under reduced pressure to provide the crude product Compound H2-6 (80.0 mg) as a yellow oil, which was directly used in the next reaction without purification.

**LC-MS:** EC16331-68-P1A2, product: RT = 0.440 min, m/z = 545.2 [M+H]⁺.

### 5. Synthesis of Compound APH2

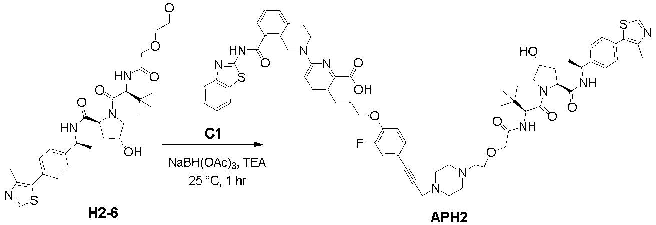

To a solution of Compound H2-6 (80.0 mg, 147 µmol, 1.0 eq.) in DMF (1.00 mL), TEA (149 mg, 1.47 mmol, 204.44 µL, 10.0 eq.), NaBH(OAc)₃ (93.4 mg, 441 µmol, 3.0 eq.) and Compound C1 (72.5 mg, 103 µmol, 0.7 eq.) were added, and the mixture was stirred and reacted at 25 °C for 1 hour. LC-MS (EC16331-69-P1A, P1: RT = 0.448 minutes) showed that Compound H2-6 was reacted completely, and a target product main peak with the expected m/z value was generated. The reaction mixture was filtered and concentrated under reduced pressure to obtain a crude product, which was purified by Prep-HPLC (chromatographic column: Waters Xbridge 150 * 25mm * 5µm; mobile phase: [water (NH₃H₂O)-ACN]; gradient: 15%-45% B, within 10 minutes) to provide the product Compound APH2 (20.0 mg, 14.6 µmol, 9.96% yield, 90.18% purity) as a white solid.

**LC-MS:** EC16331-69-P1E; product: RT = 0.497 min
**¹H NMR:** EC16331-69-P1W (400 MHz DMSO) *δ*ppm 8.95 - 9.00 (m, 1H), 8.45 (br d, *J* = 7.6 Hz, 1H), 8.26 (s, 1H), 8.02 (d, *J* = 7.6 Hz, 1H), 7.78 (d, *J =* 8.0 Hz, 1H), 7.59 (br d, *J =* 7.6 Hz, 1H), 7.40 - 7.51 (m, 6H), 7.31 - 7.39 (m, 6H), 7.26 (br d, *J* = 12.0 Hz, 1H), 7.15 - 7.19 (m, 1H), 7.09 - 7.09 (m, 1H), 7.05 - 7.12 (m, 1H), 6.83 (br d, *J* = 8.8 Hz, 1H), 4.90 (s, 3H), 4.49 - 4.57 (m, 2H), 4.44 (br t, *J* = 8.0 Hz, 2H), 4.28 (br s, 2H), 3.97 - 4.02 (m, 3H), 3.92 - 3.96 (m, 2H), 3.83 (br t, *J* = 5.6 Hz, 3 H) 3.56 - 3.61 (m, 5H), 3.45 (s, 3H), 2.95 - 2.95 (m, 1H), 2.96 (br t, *J =* 5.2 Hz, 1H), 2.74 (br t, *J =* 6.8 Hz, 2H), 2.43 - 2.46 (m, 4H), 2.01 - 2.08 (m, 1H), 1.91 - 2.01 (m, 2H), 1.76 (ddd, *J* = 12.8, 8.8, 4.4 Hz, 1H), 1.34 - 1.38 (m, 3H), 0.90 - 0.97 (m, 12H).

### Synthesis of APH3:

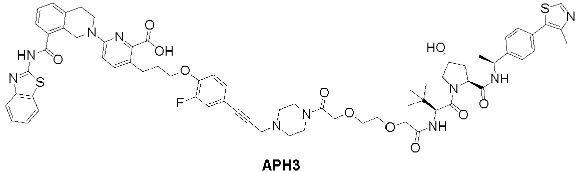

### Synthesis scheme:

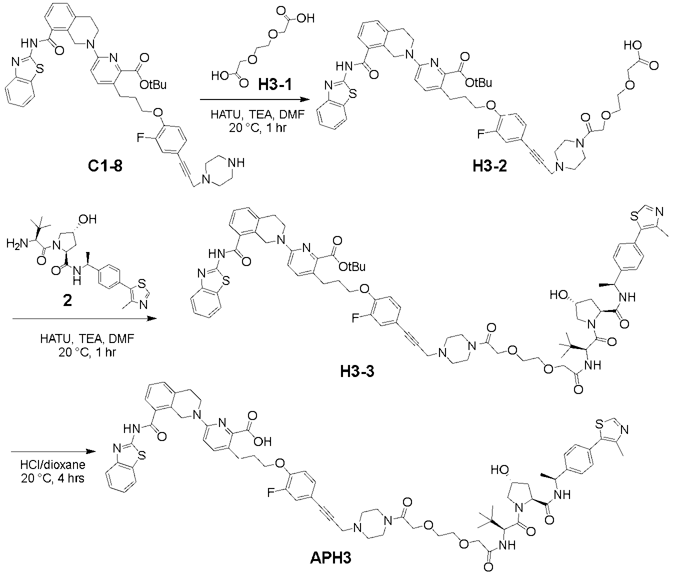

### 1. Synthesis of Compound H3-2

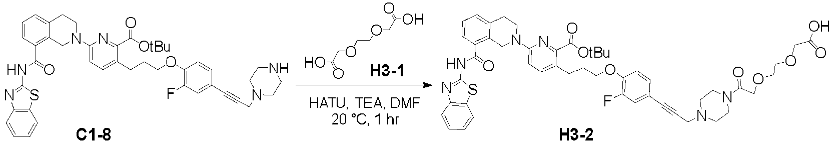

To a solution of Compound C1-8 (150 mg, 197 µmol, 1.00 eq.) and Compound H3-1 (141 mg, 789 µmol, 4.00 eq.) in DMF (2.00 mL), HATU (113 mg, 296 µmol, 1.50 eq.) and TEA (59.8 mg, 591 µmol, 82.3 µL, 3 .00 eq.) were added, and the mixture was stirred and reacted at 20°C for 1 hour. LC-MS (EC21184-30-p1a, P1: RT = 0.439 minutes) showed that Compound C1-8 was completely reacted, and the target product was generated, accounting for approximately 84.7%. To the reaction mixture, water (50.0 mL) was added, and extracted with ethyl acetate (30.0 mL * 2). The resulting organic layer was dried over Na₂SO₄, filtered and concentrated under reduced pressure to provide the crude product Compound H3-2 (180 mg, crude product) as a white solid.

**LC-MS:** EC21184-30-pla, P1: RT = 0.439 min, m/z = 921.4 [M+H]⁺.

### 2. Synthesis of Compound APH3-3

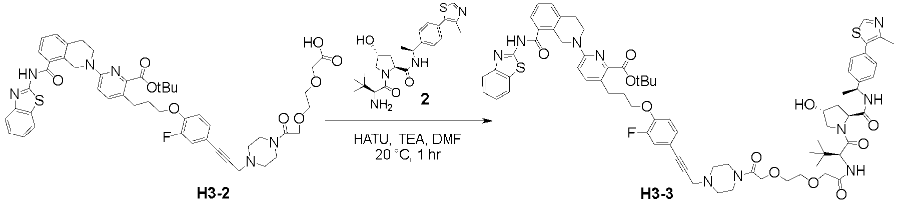

To a solution of Compound H3-2 (180 mg, 195 µmol, 1.00 eq.) and Compound 2 (86.9 mg, 195 µmol, 1.00 eq.) in DMF (2.00 mL), HATU (111 mg, 293 µmol, 1.50 eq.) and TEA (59.3 mg, 586 µmol, 81.6 µL, 3.00 eq.) were added, and the mixture was stirred and reacted at 20°C for 1 hour. LC-MS (EC21184-31-p1a1, P1: RT = 0.810 minutes) showed that Compound H3-2 was reacted completely, and the target product was generated, accounting for approximately 8.41%. To the reaction mixture, water (100 mL) was added, and extracted with ethyl acetate (25.0 mL * 3). The resulting organic layer was dried over Na₂SO₄, filtered and concentrated under reduced pressure to obtain a crude product, which was further purified by Prep-HPLC (neutral conditions; chromatographic column: CD07-Daisogel SP-100-8-ODS-PK 150*25*10µm; mobile phase: [water (NH₄HCO₃)-ACN]; gradient: 57%-87% B, within 10 minutes) to provide Compound H3-3 (50.0 g, 36.1 µmol, 18.5% yield, 97.3% purity) as a white solid.

**LC-MS:** EC21184-31-pla1, P1: RT = 0.810 min, m/z = 1347.5 [M+H]⁺
**¹H NMR:** EC21184-31-p1qc (400 MHz DMSO) *δ* ppm 12.76 - 12.93 (m, 1H), 8.97 (s, 1H), 8.43 (d, *J* = 7.6 Hz, 1H), 8.02 (d, *J =* 8.0 Hz, 1H), 7.77 (d, *J* = 8.4 Hz, 1H), 7.56 (d, *J =* 8.0 Hz, 1H), 7.30 - 7.53 (m, 10H), 7.27 (dd, *J =* 12.0, 2.0 Hz, 1H), 7.17 (d, *J =* 8.8 Hz, 1H), 7.05 - 7.12 (m, 1H), 6.91 (d, *J =* 8.8 Hz, 1H), 5.13 (d, *J* = 3.2 Hz, 1H), 4.86 - 4.96 (m, 3H), 4.54 (d, *J* = 9.2 Hz, 1H), 4.44 (t, *J =* 8.0 Hz, 1H), 4.27 (s, 1H), 4.20 (d, *J* = 2.4 Hz, 2H), 4.01 (t, *J =* 6.0 Hz, 2H), 3.96 (s, 2H), 3.78 (br t, *J =* 6.0 Hz, 2H), 3.54 - 3.65 (m, 6H), 3.39 - 3.53 (m, 6H), 2.98 (t, J = 5.6 Hz, 2H), 2.62 - 2.71 (m, 2H), 2.44 (s, 5H), 2.00 - 2.09 (m, 1H), 1.87 - 1.97 (m, 2H), 1.71 - 1.82 (m, 1H), 1.36 (d, *J=* 7.2 Hz, 3H), 1.30 (s, 9H), 0.93 (s, 9H).

### 3. Synthesis of Compound APH3

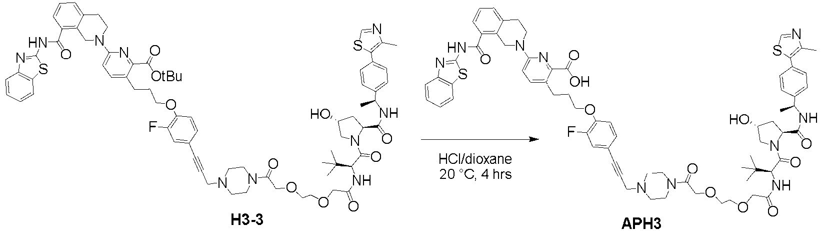

Compound H3-3 (45.0 g, 33 µmol, 1.00 eq.) was mixed in HCl/dioxane (25.0 mL), and the mixture was stirred and reacted at 20 °C under N₂ atmosphere for 4 hours. LC-MS (EC21184-35-p1a1, P1: RT = 0.450 minutes) showed that the target product was generated, accounting for approximately 88.5%. The reaction mixture was filtered and concentrated under reduced pressure to obtain a crude product, which was further purified by Prep-HPLC (neutral conditions; chromatographic column: CD02-Waters Xbidge BEH C18 150*25*10µm; mobile phase: [water (NH₄HCO₃)-ACN]; gradient: 25%-55% B, within 10 minutes) to provide Compound APH3 (13.0 mg, 9.70 µmol, 29.0% yield, 96.4% purity) as a white solid.

**LC-MS:** EC21184-35-p1a1, P1: RT = 0.450 min, m/z = 1313.3 [M+Na]⁺
**¹H NMR:** EC21184-35-plqc (400 MHz DMSO) *δ* 8.97 (s, 1H), 8.42 (d, *J =* 7.6 Hz, 1H), 8.03 (d, *J* = 8.0 Hz, 1H), 7.78 (d, *J* = 8.0 Hz, 1H), 7.60 (d, *J* = 8.0 Hz, 1H), 7.30 - 7.54 (m, 10H), 7.26 (dd, *J* = 12.0, 2.0 Hz, 1H), 7.14 - 7.19 (m, 1H), 7.05 - 7.12 (m, 1H), 6.89 (d, *J* = 8.4 Hz, 1H), 4.85 - 4.95 (m, 3H), 4.54 (d*, J* = 9.6 Hz, 1H), 4.44 (t, *J =* 8.0 Hz, 1H), 4.28 (s, 1H), 4.20 (d, *J =* 1.6 Hz, 2H), 4.01 (t, *J =* 6.4 Hz, 2H), 3.96 (s, 2H), 3.85 (t, *J =* 5.6 Hz, 2H), 3.55 - 3.67 (m, 7H), 3.42 - 3.55 (m, 10H), 2.97 (t*, J =* 5.6 Hz, 2H), 2.77 (t, *J =* 7.2 Hz, 2H), 2.44 (s, 5H), 2.01 - 2.10 (m, 1H), 1.91 - 2.00 (m, 2H), 1.77 (ddd, *J =* 13.2, 8.8, 4.4 Hz, 1H), 1.37 (d, *J* = 6.8 Hz, 3H), 0.93 (s, 9H).

### Synthesis of APH4:

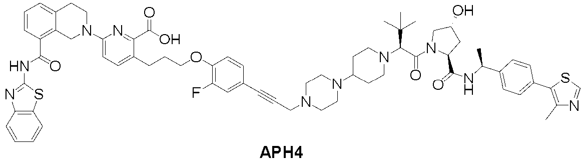

### Synthesis scheme:

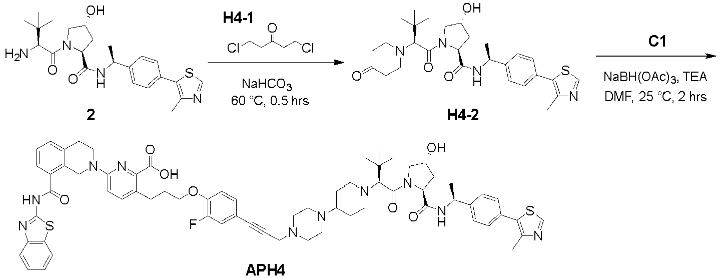

### 1. Synthesis of Compound H4-2

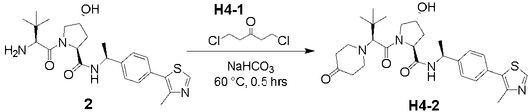

To a solution of Compound 2 (200 mg, 450 µmol, 1.0 eq.) in methanol (3.00 mL), NaHCO₃ (189 mg, 2.25 mmol, 87.52 µL, 5.0 eq.) and Compound H4-1 (105 mg, 675 µmol, 1.5 eq.) were added, and the mixture was stirred and reacted at 60°C for 0.5 hours. LC-MS (EC16331-49-P1A, P1: RT = 0.497 minutes) showed that Compound 2 was reacted completely, and a target product main peak with the expected m/z value was generated. The reaction mixture was filtered and concentrated under reduced pressure to obtain a crude product, which was purified by Prep-HPLC (neutral conditions; chromatographic column: Waters Xbridge BEH C18 150 * 25 mm * 5 µm; mobile phase: [water (NH₄HCO₃)-ACN]; gradient: 25%-55% B, within 10 minutes) to provide the product Compound H4-2 (160 mg, 292 µmol, 64.83% yield, 96% purity) as a white solid.

**LC-MS:** EC16331-49-P1C; product: RT = 0.488 min
**¹H NMR:** EC16331-49-P1C (400 MHz DMSO) δ ppm 8.98 (s, 1H), 8.34 (d, J = 7.6 Hz, 1H), 7.34 - 7.45 (m, 4H), 5.07 (d, J = 3.6 Hz, 1H), 4.85 - 4.94 (m, 1H), 4.44 (t, J = 7.6 Hz, 1H), 4.27 (brd, J = 2.0 Hz, 1H), 3.51 - 3.62 (m, 3H), 3.29 (s, 1H), 3.17 (dt, J = 11.6, 5.6 Hz, 2H), 2.83 (dt, J = 11.6, 6.0 Hz, 2H), 2.45 (s, 3H), 2.26 - 2.35 (m, 4H), 1.99 - 2.07 (m, 1H), 1.79 (ddd, J = 12.8, 7.6, 4.8 Hz, 1H), 1.37 (d, J = 7.2 Hz, 3H), 0.99 (s, 9H).

### 2. Synthesis of Compound APH4

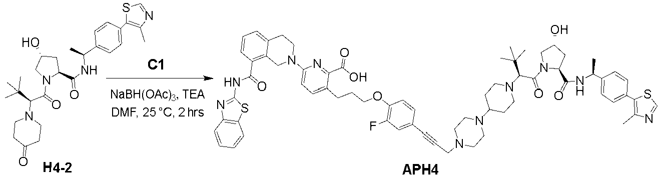

To a solution of Compound H4-2 (100 mg, 190 µmol, 1.0 eq.) in DMF (2.00 mL), TEA (192 mg, 1.90 mmol, 264 µL, 10.0 eq.), NaBH(OAc)₃ (121 mg, 570 µmol, 3.0 eq.) and Compound C1 (66.9 mg, 94.9 µmol, 0.5 eq.) were added, and the mixture was stirred and reacted at 25 °C for 2 hours. LC-MS (EC16331-54-P1A1, P1: RT = 0.769 min; R1: RT = 0.700 minutes) showed that the target product having the expected m/z was generated. The reaction mixture was filtered and concentrated under reduced pressure to obtain a crude product, which was purified by Prep-HPLC (neutral conditions; chromatographic column: Daisogel SP ODS RPS 150 * 25mm * 5µm; mobile phase: [water (NH₄HCO₃)-ACN]; gradient: 37%-67% B, within 10 minutes) to provide the product Compound APH4 (20.0 mg, 16.5 µmol, 8.67% yield, 100% purity) as a white solid.

**LC-MS:** EC16331-54-P1T; product: RT = 0.417 min
**¹H NMR:** EC16331-54-P1M (400 MHz DMSO)δ ppm 8.98 (s, 1H), 8.31 (br d, *J =* 7.6 Hz, 1H), 8.02 (d, *J* = 7.6 Hz, 1H), 7.78 (d, *J* = 8.0 Hz, 1H), 7.59 (br d, *J =* 7.2 Hz, 1H), 7.31 - 7.50 (m, 9H), 7.25 (dd, *J =* 12.0, 1.2 Hz, 1H), 7.16 (br d, *J =* 8.6 Hz, 1H), 7.04 - 7.11 (m, 1H), 6.85 (br d, *J =* 8.8 Hz, 1H), 4.86 - 4.93 (m, 3H), 4.43 (brt, *J =* 7.6 Hz, 1H), 4.26 (br s, 2H), 4.00 (br t, *J =* 6.4 Hz, 2H), 3.84 (br t, *J =* 5.6 Hz, 3H), 3.57 (br s, 4H), 3.16 - 3.23 (m, 4H), 3.07 (br s, 2H), 2.97 (br d, *J =* 5.2 Hz, 3H), 2.85 - 2.92 (m, 2H), 2.70 - 2.79 (m, 3H), 2.45 (s, 3H), 1.94 (br s, 6H), 1.76 - 1.85 (m, 1H), 1.62 - 1.72 (m, 2H), 1.37 (br d, *J =* 6.8 Hz, 2H), 1.28 (br d, *J*= 9.2 Hz, 1H), 1.23 (br s, 1H), 0.92 (s, 9H).

### Synthesis of APH5:

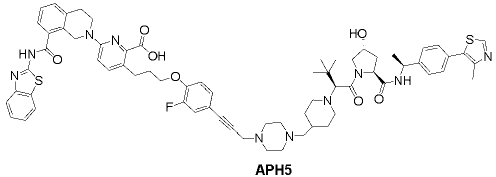

### Synthesis scheme:

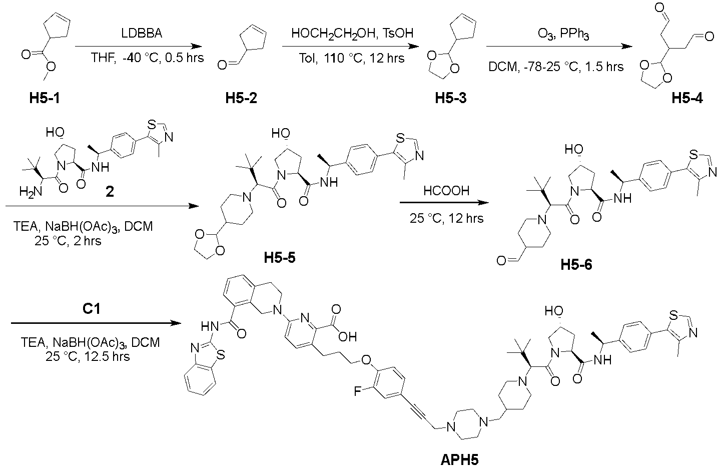

### 1. Synthesis of Compound H5-2

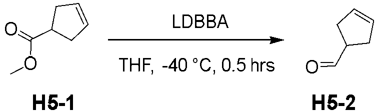

To a solution of Compound H5-1 (5.00 g, 39.63 mmol, 1.00 eq.) in THF (238 mL), LDBBA (0.5 M, 118 mL, 1.50 eq.) was added dropwise at -40 °C, and the mixture was stirred and reacted at -40°C for 0.5 hours. TLC (PE: EA=7:1, R1: R_{f}= 0.80; P1: R_{f}= 0.75) showed that Compound H5-1 was reacted completely, and two new compound peaks were generated. To the reaction mixture, HCl (1 M, 30.0 mL) was added at -60°C to quench the reaction. The mixture was dried over Na₂SO₄, filtered and concentrated under reduced pressure to provide the crude product H5-2 (5.20 g) as a brown liquid, which was directly used in the next reaction without purification.

**¹H NMR:** EC16331-78-P1A (400 MHz DMSO) *δ* ppm 9.59 (d, *J* = 1.2 Hz, 1H), 5.63 (s, 2H), 3.11 - 3.15 (m, 1H), 2.55 - 2.61 (m, 4H).

### 2. Synthesis of Compound H5-3

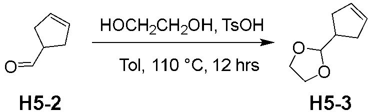

To a solution of Compound H5-2 (5.00 g, 52.0 mmol, 1.00 eq.) in toluene (60.0 mL), HOCH₂CH₂OH (32.3 g, 520 mmol, 29.01 mL, 10.0 eq.) and TsOH (89.6 mg, 520 µmol, 0.01 eq.) were added under N₂ atmosphere, and the mixture was stirred and reacted at 110°C for 12 hours. TLC (PE: EA=7:1, R1: R_{f}= 0.80; P1: Rᵣ = 0.75) showed that Compound H5-2 was reacted completely, and a new compound peak was generated. The reaction mixture was extracted by adding water (100.0 mL) and ethyl acetate (80.0 mL * 3). The resulting organic layer was washed with saturated saline (100 mL * 2), dried over Na₂SO₄, filtered and concentrated under reduced pressure to obtain a crude product, which was further purified by column chromatography (SiO₂, petroleum ether: ethyl acetate = 99:1) to provide the product Compound H5-3 (700 mg, 4.99 mmol, 9.60% yield) as a yellow oil.

**¹H NMR:** EC16331-82-P1A (400 MHz DMSO) *δ* ppm 5.60 - 5.70 (m, 2H), 4.65 - 4.73 (m, 1H), 3.73 - 3.95 (m, 4H), 2.15 - 2.46 (m, 6H).

### 3. Synthesis of Compound H5-4

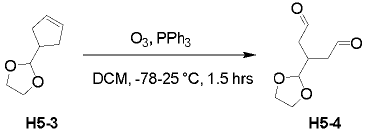

To a solution of Compound H5-3 (400 mg, 2.85 mmol, 1.00 eq.) in DCM (20.0 mL), O₃ (1.37 g, 2.85 mmol, 1.00 eq.) was introduced at -78 °C for 1 minute. Then, PPh₃ (898.11 mg, 3.42 mmol, 1.20 eq.) was added, and the mixture was stirred at -78 °C for 0.5 hours, and then was stirred at 25 °C for 1 hour. To the reaction mixture, HCl (1 M, 30.0 mL) was added at -60 °C to quench the reaction. The mixture was dried over Na₂SO₄, filtered and concentrated under reduced pressure to obtain a crude product, which was further purified by Prep-TLC (SiO₂, PE: EA=0:1; P1: R_{f}= 0.30) to provide the product Compound H5-4 (100 mg, crude product) as a colorless oil.

### 4. Synthesis of Compound H5-5

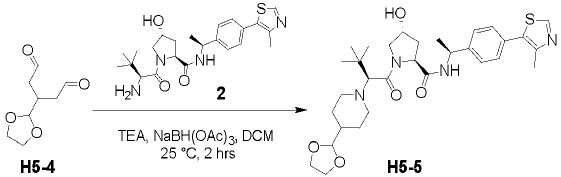

To a solution of Compound H5-4 (50.0 mg, 290 µmol, 1.00 eq.) in DCM (1.00 mL), TEA (2.93 g, 2.90 mmol, 404 µL, 10.0 eq.), NaBH(OAc)₃ (185 mg, 871 µmol, 3.0 eq.) and Compound 2 (103 mg, 232 µmol, 0.8 eq.) were added, and the mixture was stirred and reacted at 25 °C for 2 hours. The reaction mixture was extracted by adding water (5.00 mL) and ethyl acetate (5.00 mL * 3). The resulting organic layer was washed with saturated saline (30.0 mL), dried over Na₂SO₄, filtered and concentrated under reduced pressure to obtain a crude product, which was further purified by Prep-HPLC (FA conditions; chromatographic column: Phenomenex luna C18 150 * 25mm * 10µm; mobile phase: [water (FA) - ACN]; gradient: 9%-39% B, within 10 minutes) to provide the product Compound H5-5 (60.0 mg, 99.19 µmol, 34.16% yield, 96.67% purity) as a yellow oil.

**LC-MS:** EC16331-100-P1C; product: RT = 0.307 min
**¹H NMR:** EC16331-100-P1A (400 MHz DMSO)δ ppm 8.70 (s, 1H), 8.36 (s, 1H), 7.66 (br d, *J* = 7.6 Hz, 1H), 7.40 (q, *J =* 8.2 Hz, 4H), 7.28 (s, 1H), 5.06 (quin, *J =* 7.2 Hz, 1H), 4.78 - 4.95 (m, 2H), 4.65 (d*, J =* 4.0 Hz, 1H), 4.60 (br s, 1H), 3.84 - 3.97 (m, 4H), 3.77 - 3.83 (m, 1H), 3.66 (br dd, *J* = 10.8, 4.8 Hz, 1H), 3.52 (s, 1H), 3.45 (br d, *J* = 12.4 Hz, 1H), 3.18 (br d, *J =* 9.6 Hz, 1H), 2.96 (br s, 9H), 2.75 - 2.84 (m, 1H), 2.64 - 2.72 (m, 1H), 2.57 (br s, 1H), 2.51 - 2.53 (m, 1H), 2.03 (ddd, *J =* 12.8, 8.4, 4.0 Hz, 1H), 1.70 - 1.82 (m, 2H), 1.60 (br d, *J* = 7.2 Hz, 2H), 1.49 (br d, *J =* 6.8 Hz, 3 H), 1.10 (s, 9H).

### 5. Synthesis of Compound H5-6

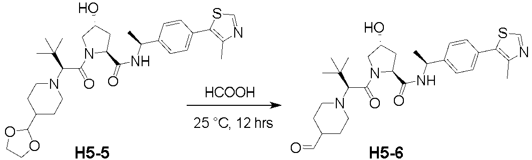

Compound H5-5 (30.0 mg, 51.3 µmol, 1.00 eq.) was dissolved in formic acid (1.00 mL), and the mixture was stirred and reacted at 25°C for 12 hours. LCMS (EC17015-215-P1A, P1: RT = 1.353 minutes) showed that the target product was generated. The reaction mixture was filtered and concentrated under reduced pressure to provide the product Compound H5-6 (28.0 mg, crude), which was directly used in the next reaction without purification.

**LC-MS:** EC17015-215-P1A, product: RT = 1.353 min, m/z = 541.3 [M+H]⁺.

### 6. Synthesis of Compound APH5

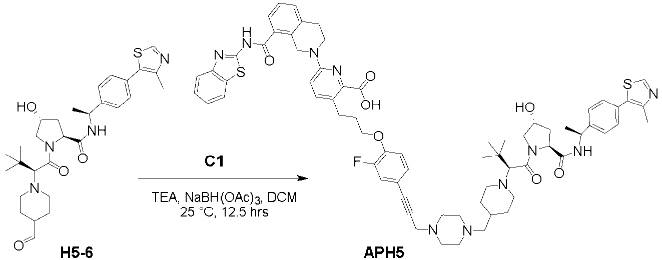

To a solution of Compound H5-6 (28.0 mg, 51.8 µmol, 1.00 eq.) in DCM (1.00 mL), TEA (52.4 mg, 518 µmol, 72.1 µL, 10.0 eq.) and Compound C1 (18.2 mg, 25.9 µmol, 0.50 eq.) were added, and the mixture was stirred and reacted at 25 °C for 0.5 hours. Then, NaBH(OAc)₃ (32.9 mg, 155 µmol, 3.00 eq.) was added, and the mixture was stirred and reacted at 25 °C for 12 hours. LCMS (EC17015-217-P1B, P1: RT = 1.388 minutes) showed that the target product was generated. The reaction mixture was filtered and concentrated under reduced pressure to obtain a crude product, which was further purified by Prep-HPLC (chromatographic column: CD02-Waters Xbidge BEH C18 150*25*10 µm; mobile phase: [water (NH₃H₂O)-ACN]; gradient: 24%-54% B, within 10 minutes) to provide the product Compound APH5 (5.00 mg, 3.69 µmol, 7.13% yield, 90.8% purity) as a white solid.

**LC-MS:** EC17015-217-P1B, product: RT = 1.388 min, m/z = 1230.5 [M+H]⁺
**¹H NMR:** EC17015-217-P1A (400 MHz DMSO) *δ* 8.98 (s, 1H), 8.29 (d, *J* = 7.0 Hz, 1H), 8.01 (d, *J* = 8.2 Hz, 1H), 7.77 (d, *J* = 7.6 Hz, 1H), 7.60 (d, *J =* 7.6 Hz, 1H), 7.41 - 7.47 (m, 3H), 7.38 (s, 3H), 7.30 - 7.35 (m, 2H), 7.26 (d, *J* = 12.6 Hz, 1H), 7.17 (d, *J =* 7.6 Hz, 1H), 7.05 - 7.12 (m, 1H), 6.79 (dd, *J =* 7.0, 2.0 Hz, 1H), 5.30 (s, 1H), 4.90 (s, 3H), 4.40 - 4.47 (m, 1H), 4.23 - 4.30 (m, 1H), 4.00 (s, 2H), 3.84 (d, *J* = 4.4 Hz, 2H), 3.56 - 3.63 (m, 3H), 3.05 (s, 2H), 2.96 (s, 2H), 2.78 - 2.86 (m, 2H), 2.72 (s, 2H), 2.45 (s, 6H), 2.29 - 2.37 (m, 3H), 2.05 - 2.15 (m, 3H), 1.91 - 2.04 (m, 4H), 1.75 - 1.84 (m, 1H), 1.50 - 1.68 (m, 3H), 1.37 (d, *J =* 7.0 Hz, 4H), 1.20 - 1.28 (m, 4H), 0.92 (s, 9H).

### b) Synthesis of APH6, APH7, APH8, and APH9

### Synthesis of APH6:

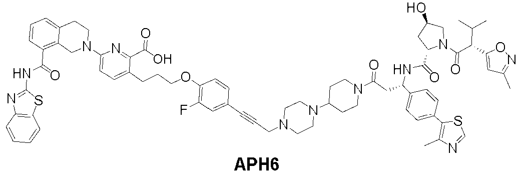

### Synthesis scheme:

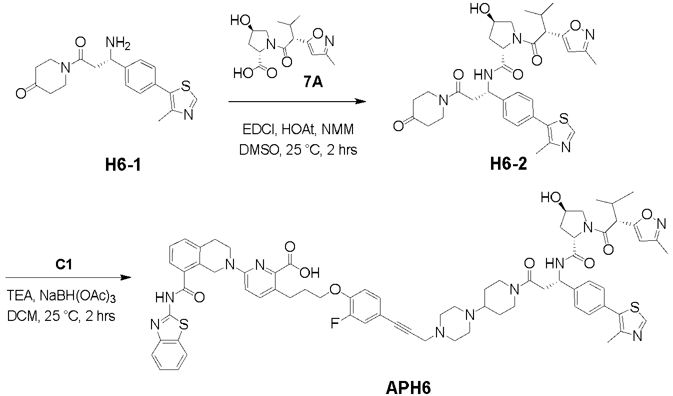

### 1. Synthesis of Compound H6-2

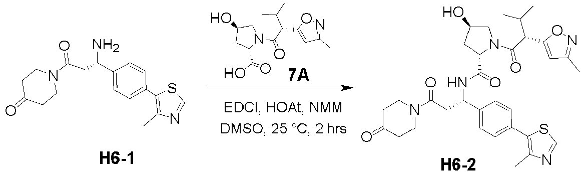

To a solution of Compound H6-1 (100 mg, 291 µmol, 1.0 eq.) in DMSO (1.00 mL), EDCI (83.7 mg, 436 µmol, 1.5 eq.), 1-hydroxy-7-azobenzotriazole (59.5 mg, 436 µmol, 61.10 µL, 1.5 eq.), NMM (88.3 mg, 873 µmol, 96.0 µL, 3.0 eq.) and Compound 7A (43.1 mg, 145 µmol, 0.5 eq.) were added, and the mixture was stirred and reacted at 25 °C for 2 hours. The reaction mixture was filtered and concentrated under reduced pressure to obtain a crude product, which was further purified by Prep-HPLC (FA conditions; chromatographic column: Phenomenex luna C18 150 * 25mm * 10µm; mobile phase: [water (FA)-ACN]; gradient: 16%-46% B, within 10 minutes) to provide the product H6-2 (80.0 mg, 129 µmol, 44.19% yield) as a white solid.

**LC-MS:** EC16331-96-P1A, product: RT = 0.328 min
**¹H NMR:** EC16331-96-P1A1 (400 MHz DMSO) δ ppm 8.95 - 9.03 (m, 1H), 8.43 - 8.53 (m, 1H), 7.29 - 7.58 (m, 4H), 6.09 - 6.28 (m, 1H), 5.21 - 5.35 (m, 1H), 5.09 (br s, 1H), 4.19 - 4.41 (m, 2H), 3.70 - 3.79 (m, 4H), 3.65 (br dd, J = 12.0, 5.6 Hz, 1H), 3.55 (br d, J = 10.0 Hz, 1H), 2.89 - 3.02 (m, 2H), 2.42 - 2.47 (m, 3H), 2.34 (br d, J = 5.6 Hz, 3H), 2.20 (s, 4H), 1.94 - 2.06 (m, 1H), 1.71 - 1.85 (m, 1H), 1.34 - 1.57 (m, 1H), 1.06 - 1.32 (m, 1H), 0.95 (br d, J = 5.6 Hz, 3H), 0.73 - 0.82 (m, 3H).

### 2. Synthesis of Compound APH6

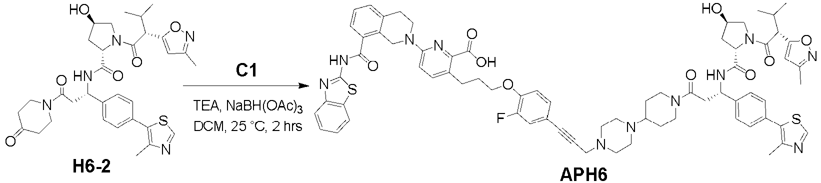

To a solution of Compound H6-2 (40.0 mg, 64.3 µmol, 1.0 eq.) in DCM (1.00 mL), TEA (65.1 mg, 643 µmol, 89.5 µL, 10.0 eq.), NaBH(OAc)₃ (40.9 mg, 193 µmol, 3.0 eq.) and Compound C1 (22.67 mg, 32.17 µmol, 0.5 eq.) were added, and the mixture was stirred and reacted at 25°C for 2 hours. The reaction mixture was extracted by adding water (5.00 mL) and ethyl acetate (5.00 mL * 3). The resulting organic layer was washed with saturated saline (10.0 mL * 2), dried over Na₂SO₄, filtered and concentrated under reduced pressure to obtain a crude product, which was further purified by Prep-HPLC (FA conditions; chromatographic column: Phenomenex luna C18 150 * 25mm * 10µm; mobile phase: [water (FA)-ACN]; gradient: 22%-52% B, within 8 minutes) to provide the product Compound APH6 (10.0 mg, 7.05 µmol, 10.96% yield, 92.44% purity) as a white solid.

**LC-MS:** EC16331-105-P1Y; product: RT = 0.445 min
**¹H NMR:** EC16331-105-P1E (400 MHz DMSO)δ ppm 8.92 - 8.99 (m, 1,H), 8.46 (br t*, J =* 8.4 Hz, 1H), 8.20 (s, 1H), 8.03 (d, *J* = 7.6 Hz, 1H), 7.79 (d, *J =* 8.0 Hz, 1H), 7.59 (br d, *J* = 7.6 Hz, 1H), 7.31 - 7.53 (m, 9H), 7.23 - 7.30 (m, 1H), 7.17 (br d, *J =* 8.0 Hz, 1H), 7.05 - 7.13 (m, 1H), 6.90 (br d, *J =* 8.8 Hz, 1H), 6.21 (s, 1H), 5.13 - 5.24 (m, 1H), 4.91 (s, 2H), 4.61 - 4.72 (m, 1H), 4.22 - 4.44 (m, 5H), 4.01 (br t, *J =* 6.2 Hz, 2H), 3.85 (br t, *J* = 5.6 Hz, 3H), 3.73 (br dd, *J =* 9.6, 4.8 Hz, 3H), 3.41 (br s, 1H), 3.38 - 3.38 (m, 1H), 3.38 (br s, 1H), 3.17 (s, 1H), 2.97 (br t, *J =* 5.6 Hz, 3H), 2.85 - 2.93 (m, 3H), 2.74 - 2.81 (m, 3H), 2.46 (br d, *J =* 2.8 Hz, 5H), 2.36 (br s, 3H), 2.19 (s, 2H), 2.17 (s, 1H), 1.90 - 2.06 (m, 4H), 1.73 - 1.83 (m, 1H), 1.61 - 1.72 (m, 2H), 1.23 (s, 2H), 0.96 (br d, *J =* 6.4 Hz, 3H), 0.78 (br s, 1H), 0.76 (d, *J =* 6.8 Hz, 2H).

### Synthesis of APH7:

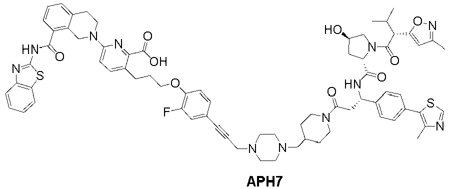

### Synthesis scheme:

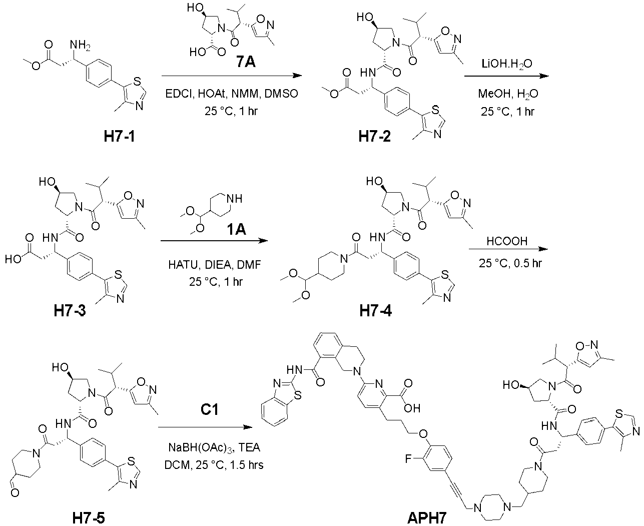

### 1. Synthesis of Compound H7-2

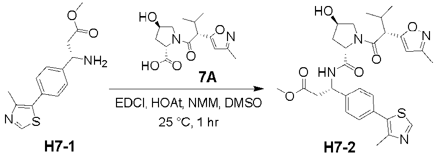

To a solution of Compound H7-1 (200 mg, 724 µmol, 1.00 eq.) and Compound 7A (193 mg, 651 µmol, 0.90 eq.) in DMSO (2.00 mL), EDCI (208 mg, 1.09 mmol, 1.50 eq.), HOAt (148 mg, 1.09 mmol, 152 µL, 1.50 eq.) and NMM (220 mg, 2.17 mmol, 239 µL, 3.00 eq.) were added, and the mixture was stirred and reacted at 25°C for 1 hour. The reaction mixture was diluted by adding water (10.0 mL), and extracted with DCM (10.0 mL * 6). The resulting organic layer was washed with saturated saline (10.0 mL), dried over Na₂SO₄, filtered and concentrated under reduced pressure to obtain a crude product, which was further purified by Prep-TLC (petroleum ether: ethyl acetate = 0:1, P1: R_{f}= 0.50) to provide the product Compound H7-2 (240 mg, 433 µmol, 59.8% yield) as a yellow oil.

**LC-MS:** EC17015-204-P1A, product: RT = 0.363 min, m/z = 555.2 [M+H]⁺.

### 2. Synthesis of Compound H7-3

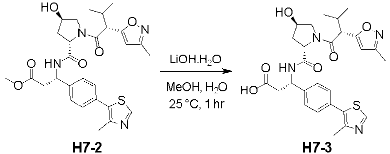

To a solution of Compound H7-2 (240 mg, 433 µmol, 1.00 eq.) in methanol (3.00 mL) and water (1.00 mL), LiOH.H₂O (54.5 mg, 1.30 mmol, 3.00 eq.) was added, and the mixture was stirred and reacted at 25 °C for 1 hour. LCMS (EC17015-206-P1A, P1: RT = 0.328 minutes) showed that the target product was generated, accounting for 96.0%. The reaction mixture was filtered and concentrated under reduced pressure to provide the crude product Compound H7-3 (230 mg) as a yellow solid, which was directly used in the next reaction without purification.

**LC-MS:** EC17015-206-P1A, product: RT = 0.328 min, m/z = 541.2 [M+H]⁺
**¹H NMR:** EC17015-206-P1A (400 MHz DMSO) *δ* ppm 8.93 (s, 1H), 7.17 - 7.47 (m, 4H), 6.12 - 6.35 (m, 1H), 4.78 - 5.00 (m, 1H), 4.13 - 4.41 (m, 1H), 3.65 - 3.88 (m, 2H), 3.50 (s, 2H), 2.41 (s, 3H), 2.25 (dt, *J* = 14.2, 6.8 Hz, 3H), 2.10 - 2.19 (m, 3H), 1.91 - 2.03 (m, 1H), 1.72 - 1.85 (m, 1H), 1.24 (d, *J* = 12.2 Hz, 1H), 0.93 (dd, *J* = 6.2*,* 2.8 Hz, 3H), 0.68 - 0.83 (m, 3H).

### 3. Synthesis of Compound H7-4

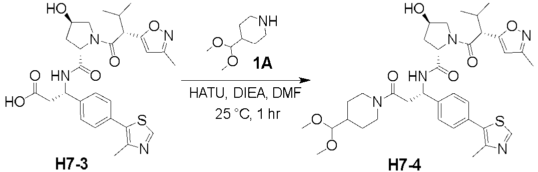

To a solution of Compound H7-3 (230 mg, 425 µmol, 1.00 eq.) and Compound 1A (102 mg, 638 µmol, 1.50 eq.) in DMF (2.00 mL), HATU (243 mg, 638 µmol, 1.50 eq.) and DIEA (330 mg, 2.55 mmol, 445 µL, 6.00 eq.) were added, and the mixture was stirred and reacted at 25°C for 1 hour. LCMS (EC17015-213-P1A, P1: RT = 0.356 minutes) showed that the target product was generated, accounting for 42.4%. To the reaction mixture, water (20.0 mL) was added, and extracted with ethyl acetate (15.0 mL * 3). The resulting organic layer was washed with saturated saline (20.0 mL), dried over Na₂SO₄, filtered and concentrated under reduced pressure to obtain a crude product, which was further purified by Prep-TLC (dichloromethane: methanol =10/1, P1: R_{f}= 0.45) to provide the product Compound H7-4 (100 mg, 147 µmol, 34.5% yield) as a colorless oil.

**LC-MS:** EC17015-213-P1A, product: RT = 0.356 min, m/z = 682.2 [M+H]⁺

### 4. Synthesis of Compound H7-5

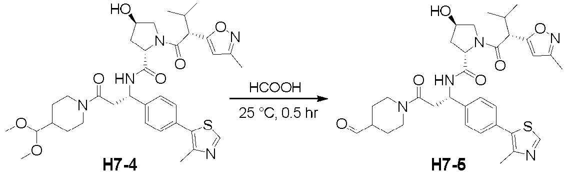

To Compound H7-4 (45.0 mg, 77.0 µmol, 1.00 eq.), formic acid (1.00 mL) was added, and the mixture was stirred and reacted at 25°C for 0.5 hours. LCMS (EC17015-224-P1A, P1: RT = 0.341 minutes) showed that the target product was generated, accounting for 91.5%. The reaction mixture was filtered and concentrated under reduced pressure to provide the product Compound H7-5 (42.0 mg, crude) as a yellow oil, which was directly used in the next reaction without purification.

LC-MS: EC17015-224-P1A, product: RT = 0.341 min, m/z = 636.2 [M+H]⁺.

### 5. Synthesis of Compound APH7

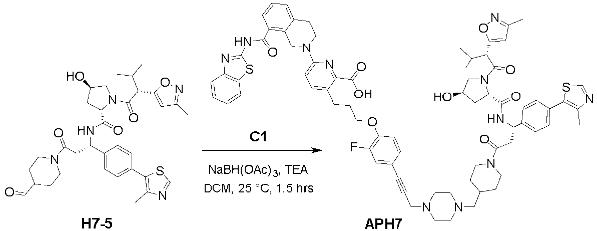

To a solution of Compound H7-5 (42.0 mg, 66.1 µmol, 1.00 eq.) in DCM (1.00 mL), TEA (66.8 mg, 661 µmol, 92.0 µL, 10.0 eq.) and Compound C1 (46.6 mg, 66.1 µmol, 1.00 eq.) were added, and the mixture was stirred and reacted at 25°C for 0.5 hours. Then, NaBH(OAc)₃ (42.0 mg, 198 µmol, 3.00 eq.) was added, and the mixture was further stirred and reacted at 25°C for 1 hour. LCMS (EC17015-225-P1B, P1: RT = 0.448 minutes) showed that the target product was generated, accounting for 52.0%. The reaction mixture was filtered and concentrated under reduced pressure to obtain a crude product, which was further purified by Prep-HPLC (chromatographic column: CD01-Phenomenex luna C18 150 * 25 * 10 µm; mobile phase: [water (FA)-ACN]; gradient: 30%-50% B, within 10 minutes) to provide the product Compound APH7 (12.0 mg, 8.71 µmol, 13.2% yield, 96.1% purity) as a white solid.

**LC-MS:** EC17015-225-P1B, product: RT = 0.448 min, m/z = 1324.6 [M+H]⁺
**¹H NMR:** EC17015-225-P1A (400 MHz DMSO) *δ* ppm 8.90 - 9.05 (m, 1H), 8.18 (s, 1H), 8.03 (d, *J* = 7.8 Hz, 1H), 7.78 (d, *J* = 8.0 Hz, 1H), 7.59 (d, *J* = 7.4 Hz, 1H), 7.48 - 7.54 (m, 1H), 7.46 (d, *J =* 8.2 Hz, 1H), 7.30 - 7.44 (m, 7H), 7.26 (d, *J =* 12.4 Hz, 1H), 7.14 - 7.21 (m, 1H), 7.05 - 7.13 (m, 1H), 6.91 (d, *J =* 8.8 Hz, 1H), 6.20 (s, 1H), 5.12 - 5.24 (m, 1H), 4.91 (s, 2H), 4.20 - 4.43 (m, 4H), 4.01 (t, *J* = 6.4 Hz, 3H), 3.85 (t, *J=* 5.6 Hz, 4H), 3.69 - 3.77 (m, 4H), 2.97 (t, *J =* 5.4 Hz, 4H), 2.74 - 2.81 (m, 3H), 2.45 (s, 6H), 2.23 - 2.34 (m, 4H), 2.14 - 2.21 (m, 4H), 1.86 - 2.07 (m, 6H), 1.50 - 1.70 (m, 4H), 0.95 (d, *J =* 6.4 Hz, 3H), 0.78 (dd, *J*= 14.2, 6.8 Hz, 5H).

### Synthesis of APH8:

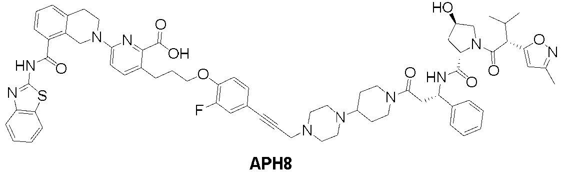

### Synthesis scheme:

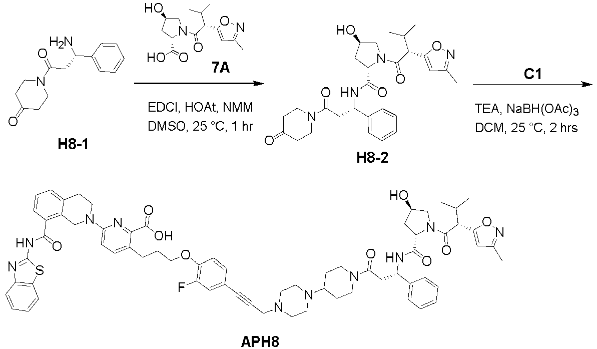

### 1. Synthesis of Compound H8-2

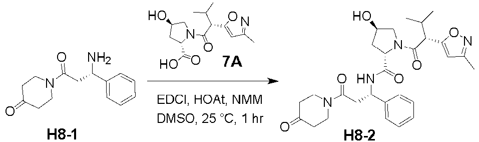

To a solution of Compound H8-1 (200 mg, 812 µmol, 1.0 eq.) in DMSO (2.00 mL), EDCI (233 mg, 1.22 mmol, 1.5 eq.), HOAt (166 mg, 1.22 mmol, 170 µL, 1.5 eq.), Compound 7A (120 mg, 406.00 µmol, 0.5 eq.) and NMM (246 mg, 2.44 mmol, 267.82 µL, 3.0 eq.) were added, and the mixture was stirred at 25°C for 1 hour. To the reaction mixture, water (15.0 mL) was added, and extracted with ethyl acetate (15.0 mL * 3). The resulting organic layer was washed with saturated saline (25.0 mL * 2), dried over Na₂SO₄, filtered and concentrated under reduced pressure to obtain a crude product, which was further purified by Prep-TLC (SiO₂, PE: EA=0:1, P1: R_{f}= 0.1) to provide the product Compound H8-2 (100 mg, 177 µmol, 21.74% yield, 92.6% purity) as a colorless oil.

**LC-MS:** EC16331-79-P1C2, product: RT = 0.317 min

### 2. Synthesis of Compound APH8

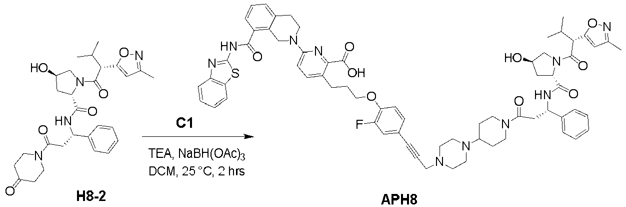

To a solution of Compound H8-2 (70.0 mg, 133 µmol, 1.0 eq.) in DCM (2.00 mL), TEA (135 mg, 1.33 mmol, 185.72 µL, 10.0 eq.), NaBH(OAc)₃ (84.8 mg, 400 µmol, 3.0 eq.) and Compound C1 (47.0 mg, 66.7 µmol, 0.5 eq.) were added, and the mixture was stirred and reacted at 25°C for 2 hours. The reaction mixture was filtered and concentrated under reduced pressure to obtain a crude product, which was further purified by Prep-HPLC (FA conditions; chromatographic column: Phenomenex luna C18 150 * 25mm * 10µm; mobile phase: [water (FA)-ACN]; gradient: 29%-59% B, within 10 minutes) to provide the product Compound APH8 (10.0 mg, 7.57 µmol, 5.67% yield, 91.86% purity) as a white solid.

**LC-MS:** EC16331-94-P1B1, product: RT = 0.440 min, 1/2 m/z = 607.5 [M+H]⁺;
**¹H NMR:** EC16331-94-P1H (400 MHz DMSO)δ ppm 8.39 (br d, *J* = 8.4 Hz, 1H), 8.32 (s, 1H), 7.98 - 8.05 (m, 1H), 7.77 (d, *J* =8.0 Hz, 1H), 7.59 (br d, *J =* 6.8 Hz, 1H), 7.45 (br t, *J* = 7.2 Hz, 1H), 7.34 - 7.42 (m, 3H), 7.33 (br dd, *J* = 7.2, 3.75 Hz, 2H), 7.25 - 7.30 (m, 4H), 7.24 (br s, 1H), 7.21 (br d, *J* = 4.4 Hz, 1H), 7.18 (br d, *J* =4.0 Hz, 1H), 7.15 (br s, 1H), 7.06 - 7.12 (m, 1H), 6.71 - 6.78 (m, 1H), 6.21 (s, 1H), 5.10 - 5.21 (m, 1H), 4.88 (br s, 2H), 4.21 - 4.36 (m, 5H), 3.95 - 4.02 (m, 4H), 3.81 (br d, *J* = 5.2 Hz, 5H), 3.71 (br d, *J* = 2.4 Hz, 6H), 2.95 (br s, 3H), 2.80 - 2.88 (m, 2H), 2.65 - 2.73 (m, 3H), 2.30 - 2.37 (m, 1H), 2.15 - 2.24 (m, 4H), 1.91 - 2.03 (m, 3H), 1.52 - 1.79 (m, 3H), 1.23 (br s, 2H), 1.05 (br t*, J =* 7.2 Hz, 1H), 0.92 - 1.01 (m, 3H), 0.71 - 0.82 (m, 3H).

### Synthesis of APH9:

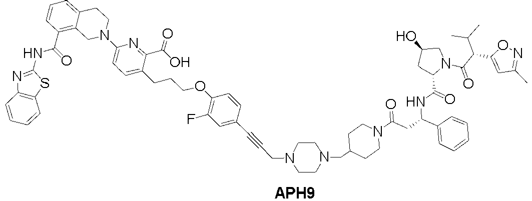

### Synthesis scheme:

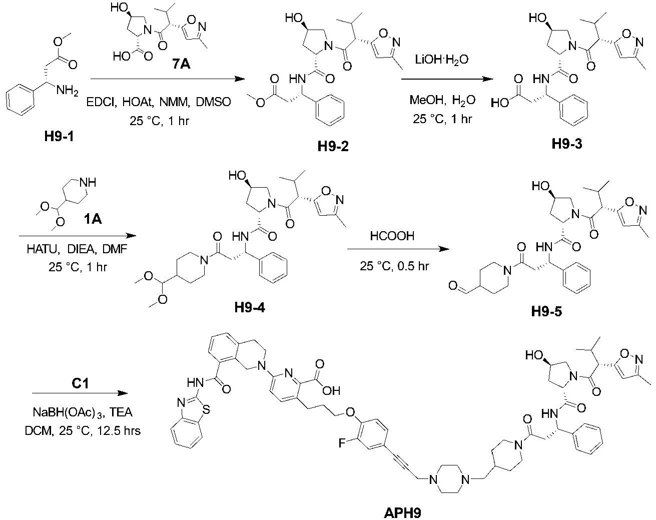

### 1. Synthesis of Compound H9-2

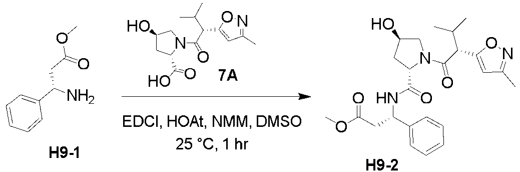

Compound H9-1 (500 mg, 2.79 mmol, 1.00 eq.), Compound 7A (413 mg, 1.39 mmol, 0.50 eq.), EDCI (802 mg, 4.18 mmol, 1.50 eq.), HOAt (570 mg, 4.18 mmol, 585 µL. 1.50 eq.) and NMM (846 mg, 8.37 mmol, 920 µL, 3.00 eq.) were mixed in DMSO (5.00 mL), and the mixture was stirred and reacted at 25°C for 1 hour. LCMS (EC17015-207-PA, P1: RT = 0.357 minutes) showed that the target product was generated, accounting for 99.4%. To the reaction mixture, water (10.0 mL) was added, and extracted with DCM (10.0 mL * 6). The resulting organic layer was washed with saturated saline (10.0 mL), dried over Na₂SO₄, filtered and concentrated under reduced pressure to obtain a crude product, which was further purified by Prep-TLC (petroleum ether: ethyl acetate = 0/1, P1: R_{f} = 0.50) to provide the product Compound H9-2 (700 mg, 1.53 mmol, 54.8% yield) as a colorless oil.

**LC-MS:** EC17015-207-P1A, product: RT = 0.357 min, m/z = 458.2 [M+H]⁺
**¹H NMR:** EC17015-207-P1A (400 MHz CDCl₃) *δ* ppm 7.16 - 7.31 (m, 5H), 5.96 - 6.12 (m, 1H), 5.31 (d, *J* = 8.2 Hz, 1H), 4.39 - 4.56 (m, 2H), 3.72 (dd, *J =* 10.4, 4.8 Hz, 1H), 3.44 - 3.64 (m, 5H), 2.63 - 2.93 (m, 3H), 2.25 - 2.41 (m, 2H), 2.10 - 2.22 (m, 3H), 1.85 - 2.04 (m, 2H), 0.97 (d, *J =* 6.4 Hz, 3H), 0.81 (d, *J =* 6.6 Hz, 3H).

### 2. Synthesis of Compound H9-3

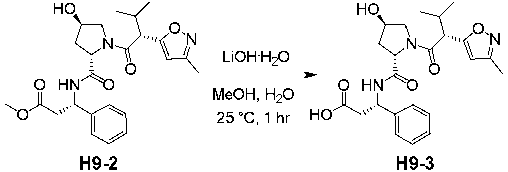

To a solution of Compound H9-2 (700 mg, 1.53 mmol, 1.00 eq.) in methanol (6.00 mL) and water (2.00 mL), LiOH.H₂O (193 mg, 4.59 mmol, 3.00 eq.) was added, and the mixture was stirred and reacted at 25°C for 1 hour. LCMS (EC17015-210-P1A, P1: RT= 0.327 minutes) showed that the target product was generated, accounting for 100%. The reaction mixture was filtered and concentrated under reduced pressure to provide the crude product H9-3 (700 mg) as a white solid, which was directly used in the next reaction without purification.

**LC-MS:** EC17015-210-P1A, product: RT = 0.327 min, m/z = 444.2 [M+H]⁺.

### 3. Synthesis of Compound H9-4

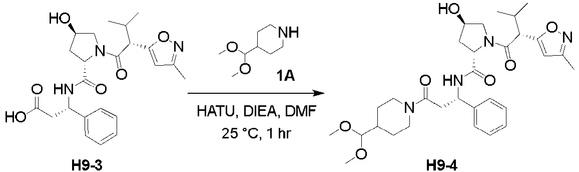

To a solution of Compound H9-3 (350 mg, 790 µmol, 1.00 eq.) and Compound 1A (188 mg, 1.18 mmol, 1.50 eq.) in DMF (4.00 mL), HATU (360 mg, 947 µmol, 1.20 eq.) and DIEA (612 mg, 4.74 mmol, 825 µL, 6.00 eq.) were added, and the mixture was stirred and reacted at 25°C for 1 hour. LCMS (EC17015-216-P1B, P1: RT = 0.343 minutes) showed that the target product was generated, accounting for 18.8%. The reaction mixture was filtered and concentrated under reduced pressure to obtain a crude product, which was further purified by reverse-phase HPLC (neutral conditions) to provide the product Compound H9-4 (140 mg, 239 µmol, 30.3% yield) as a yellow solid.

**LC-MS:** EC17015-216-P1B, product: RT = 0.343 min, m/z = 585.0 [M+H]⁺

### 4. Synthesis of Compound H9-5

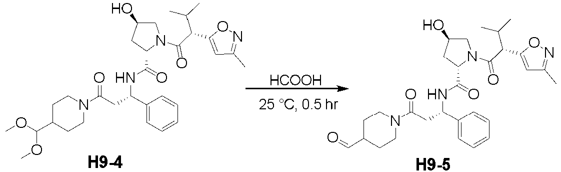

To Compound H9-4 (60.0 mg, 103 µmol, 1.00 eq.), formic acid (1.00 mL) was added, and the mixture was stirred and reacted at 25°C for 0.5 hours. LCMS (EC17015-230-P1A, P1: RT = 0.303 minutes) showed that the target product was generated, accounting for 87.8%. The reaction mixture was filtered and concentrated under reduced pressure to provide the crude product H9-5 (52.0 mg, 96.5 µmol, 94.1% yield) as a yellow oil.

**LC-MS:** EC17015-230-P1A, product: RT = 0.303 min, m/z = 539.1 [M+H]⁺

### 5. Synthesis of Compound APH9

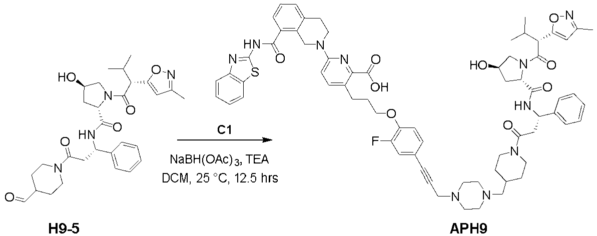

To a solution of Compound H9-5 (52.0 mg, 96.5 µmol, 1.00 eq.) in DCM (2.00 mL), TEA (97.7 mg, 965 µmol, 134 µL, 10.0 eq.) and Compound C1 (54.4 mg, 77.2 µmol, 0.80 eq.) were added, and the mixture was stirred and reacted at 25°C for 0.5 hours. Then, NaBH(OAc)₃ (61.4 mg, 290 µmol, 3.00 eq.) was added, and the mixture was further stirred and reacted at 25°C for 12 hours. LCMS (EC17015-231-P1A, P1: RT = 0.447 minutes) showed that the target product was generated, accounting for 63.3%. The reaction mixture was filtered and concentrated under reduced pressure to provide the crude product, which was further purified by Prep-HPLC (chromatographic column: CD02-Waters Xbidge BEH C18 150*25*10 µm; mobile phase: [water (NH₄HCO₃)-ACN]; gradient: 38%-58% B, within 10 minutes) to provide Compound APH9 (16.0 mg, 12.1 µmol, 12.5% yield, 92.8% purity) as a white solid.

**LC-MS:** EC17015-231-P1A, product: RT = 0.447 min, m/z = 1227.5 [M+H]⁺
**¹H NMR:** EC17015-231-P1A (400 MHz DMSO) *δ* ppm 8.03 (d, *J =* 7.8 Hz, 1H), 7.78 (d, *J =* 8.2 Hz, 1H), 7.60 (d, *J* = 7.2 Hz, 1H), 7.52 (d, *J =* 8.4 Hz, 1H), 7.47 (t, *J =* 7.6 Hz, 1H), 7.38 - 7.43 (m, 1H), 7.35 (d, *J* = 7.4 Hz, 2H), 7.30 - 7.33 (m, 1H), 7.29 (s, 4H), 7.15 - 7.22 (m, 2H), 7.05 - 7.13 (m, 1H), 6.91 (d, *J* = 9.0 Hz, 1H), 6.14 - 6.23 (m, 1H), 5.07 - 5.23 (m, 1H), 4.92 (s, 2H), 4.14 - 4.46 (m, 3H), 4.01 (t, *J =* 6.4 Hz, 2H), 3.85 (t, *J* = 5.6 Hz, 3H), 3.72 (d, *J*=9.8 Hz, 2H), 3.43 - 3.58 (m, 6H), 2.97 (t, *J =* 5.6 Hz, 2H), 2.74 - 2.87 (m, 5H), 2.64 - 2.73 (m, 2H), 2.28 - 2.36 (m, 3H), 2.14 - 2.21 (m, 4H), 2.07 (d, *J* = 5.2 Hz, 2H), 1.90 - 2.03 (m, 5H), 1.50 - 1.80 (m, 5H), 0.95 (d, *J =* 6.6 Hz, 3H), 0.68 - 0.86 (m, 5H).

### c) Synthesis of APH10, APH11, and APH12

### Synthesis of APH10:

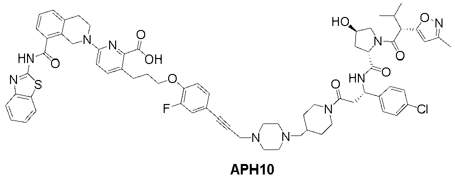

### Synthesis scheme:

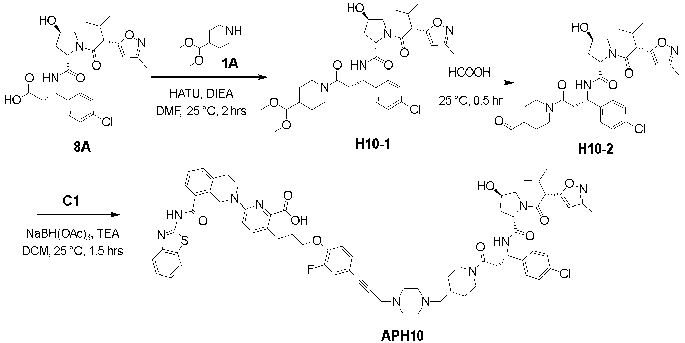

### 1. Synthesis of Compound H10-1

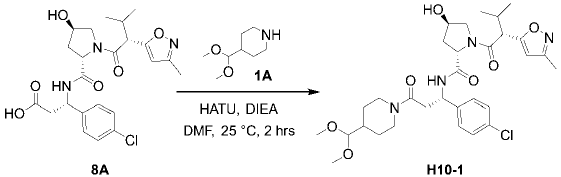

Compound 8A(200 mg, 418 µmol, 1.00 eq.), Compound 1A(100 mg, 628 µmol, 1.50 eq.), HATU (239 mg, 628 µmol, 1.50 eq.) and DIEA (324 mg, 2.51 mmol, 437 µL, 6.00 eq.) were mixed in DMF (3.00 mL), and the mixture was stirred and reacted at 25°C for 2 hours. LCMS (EC17015-293-P1B, P1: RT = 0.370 minutes) showed that the target product was generated, accounting for 20.1%. To the reaction mixture, water (20.0 mL) was added, and extracted with ethyl acetate (15.0 mL * 5). The resulting organic layer was washed with saturated saline (10.0 mL), dried over Na₂SO₄, filtered and concentrated under reduced pressure to obtain a crude product, which was further purified by Prep-HPLC (chromatographic column: CD07-Daisogel SP-100-8-ODS-PK 150*25*10 µm; mobile phase: [water (NH₄HCO₃)-ACN]; gradient: 24%-54% B, within 12 minutes) to provide the product Compound H10-1 (165 mg, 266 µmol, 63.7% yield) as a colorless oil.

**LC-MS:** EC17015-293-P1B, product: RT = 0.370 min, m/z = 619.1 [M+H]⁺
**¹H NMR:** EC17015-293-P1A (400 MHz CDCl₃) *δ* ppm 7.31 - 7.47 (m, 4H), 6.14 - 6.32 (m, 1H), 5.23 - 5.52 (m, 1H), 4.51 - 4.71 (m, 2H), 3.89 - 4.00 (m, 1H), 3.62 - 3.83 (m, 3H), 3.34 - 3.52 (m, 6H), 2.73 - 3.16 (m, 3H), 2.45 - 2.60 (m, 2H), 2.35 (d, *J* = 9.6 Hz, 4H), 2.16 - 2.26 (m, 1H), 2.15 - 2.15 (m, 1H), 1.92 - 2.13 (m, 2H), 1.71 - 1.87 (m, 3H), 1.70 - 1.71 (m, 1H), 1.09 - 1.22 (m, 3H), 0.84 - 0.89 (m, 1H), 0.98 (t, *J* = 6.2 Hz, 3H)

### 2. Synthesis of Compound H10-2

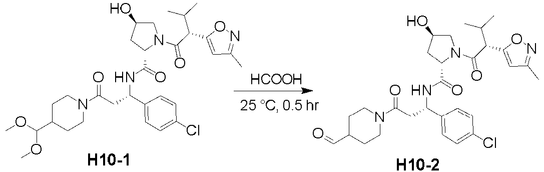

To Compound H10-1 (77.0 mg, 124 µmol, 1.00 eq.), formic acid (1.00 mL) was added, and the mixture was stirred and reacted at 25°C for 0.5 hours. LCMS (EC17015-297-P1A) showed that Compound H10-1 was completely reacted. The reaction mixture was filtered and concentrated under reduced pressure to provide the crude product H10-2 (71.0 mg) as a colorless oil.

LC-MS: EC17015-297-P1A, product: RT = 0.334 min, m/z = 573.1 [M+H]⁺

### 3. Synthesis of Compound APH10

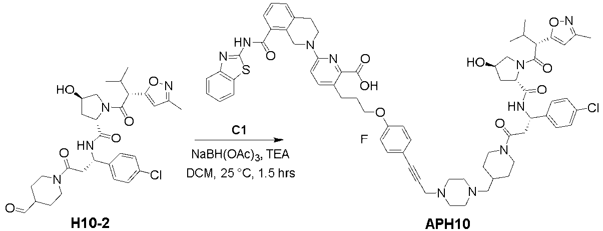

To a solution of Compound H10-2 (71.0 mg, 124 µmol, 1.00 eq.) in DCM (3.00 mL), TEA (125 mg, 1.24 mmol, 172 µL, 10.0 eq.) and Compound C1 (43.7 mg, 62.0 µmol, 0.50 eq.) were added, and the mixture was stirred at 25°C for 0.5 hours. Then, NaBH(OAc)₃ (78.8 mg, 372 µmol, 3.00 eq.) and TEA (125 mg, 1.24 mmol, 172 µL, 10.0 eq.) were added, and the mixture was stirred and reacted at 25°C for 1 hour. LCMS (EC17015-299-P1A, P1: RT = 0.610 minutes) showed that the target product was generated, accounting for 68.0%. The reaction mixture was filtered and concentrated under reduced pressure to obtain a crude product, which was further purified by Prep-HPLC (chromatographic column: CD02-Waters Xbidge BEH C18 150*25*10 µm; mobile phase: [water (NH₄HCO₃)-ACN]; gradient: 32%-62% B, within 13 minutes) to provide Compound APH10 (26.0 mg, 19.4 µmol, 15.7% yield, 94.2% purity) as a white solid.

**LC-MS:** EC17015-299-P1Q1, product: RT = 0.618 min, m/z = 1261.2 [M+H]⁺
**¹H NMR:** EC17015-299-P1A (400 MHz DMSO) *δ* ppm 8.04 (d, *J =* 7.6 Hz, 1H), 7.79 (d, *J =* 8.0 Hz, 1H), 7.60 (d, *J* = 7.6 Hz, 1H), 7.53 (d, *J =* 8.8 Hz, 1H), 7.48 (t, *J* = 7.6 Hz, 1H), 7.39 - 7.43 (m, 1H), 7.27 - 7.39 (m, 6H), 7.21 - 7.26 (m, 1H), 7.06 - 7.13 (m, 1H), 7.18 (d, *J* = 8.8 Hz, 1H), 6.93 (d, *J =* 8.4 Hz, 1H), 6.14 - 6.25 (m, 1H), 5.06 - 5.22 (m, 1H), 4.92 (s, 2H), 4.20 - 4.45 (m, 3H), 4.02 (t, *J* = 6.2 Hz, 2H), 3.86 (t, *J* = 5.6 Hz, 2H), 3.66 - 3.77 (m, 2H), 3.43 - 3.61 (m, 7H), 3.10 - 3.17 (m, 2H), 2.98 (t, *J* = 5.4 Hz, 2H), 2.86 - 2.92 (m, 1H), 2.79 (t, *J* = 7.4 Hz, 2H), 2.61 - 2.75 (m, 2H), 2.27 - 2.43 (m, 5H), 2.16 - 2.21 (m, 3H), 2.08 (s, 2H), 1.91 - 2.03 (m, 4H), 1.51 - 1.79 (m, 4H), 1.19 - 1.29 (m, 1H), 0.95 (d, *J* = 6.4 Hz, 3H), 0.68 - 0.83 (m, 4H).

### Synthesis of APH11:

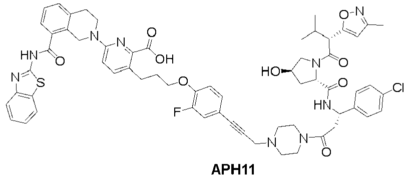

### Synthesis scheme:

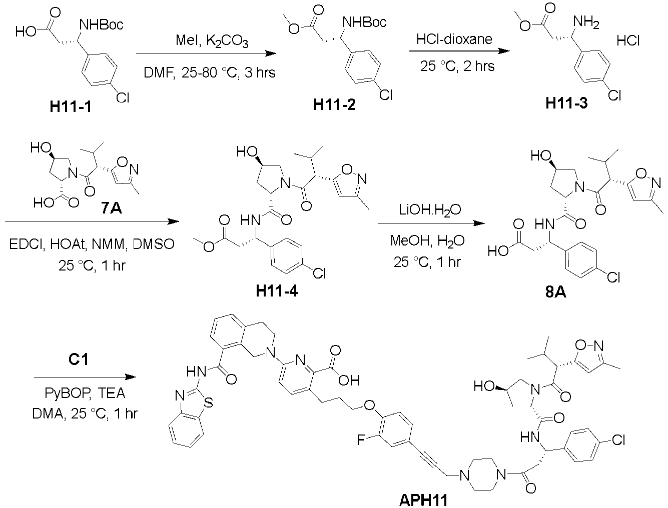

### 1. Synthesis of Compound H11-2

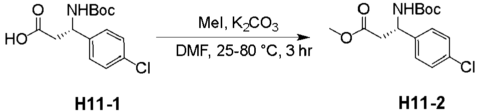

To a solution of Compound H11-1 (8.00 g, 26.7 mmol, 1.00 eq.) and K₂CO₃ (7.38 g, 53.4 mmol, 2.00 eq.) in DMF (120 mL), MeI (19.0 g, 133 mmol, 8.31 mL, 5.00 eq.) was added dropwise at 25 °C under N₂ atmosphere over 30 minutes, and the mixture was stirred and reacted at 80°C for 2.5 hours. To the reaction mixture, water (200 mL) was added, and extracted with ethyl acetate (120 mL * 3). The resulting organic layer was washed with saturated saline (120 mL * 2), dried over Na₂SO₄, filtered and concentrated under reduced pressure to provide the crude product Compound H11-2 (9.00 g) as a yellow solid, which was directly used in the next reaction without purification.

**LC-MS:** EC16331-109-P1A, product: RT = 0.439 min
**¹H NMR:** EC16331-109-P1B (400 MHz DMSO) *δ* ppm 7.51 (d, *J* = 8.4 Hz, 1H), 7.30 - 7.40 (m, 4H), 4.84 - 4.97 (m, 1H), 3.55 (s, 3H), 2.76 - 2.81 (m, 1H), 2.63 - 2.69 (m, 1H), 1.34 (s, 8H).

### 2. Synthesis of Compound H11-3

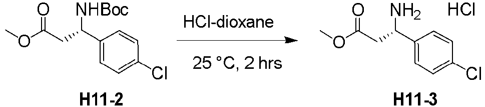

To Compound H11-2 (9.00 g, 28.7 mmol, 1.00 eq.), HCl-dioxane (2 M, 71.7 mL, 5.00 eq.) was added, and the mixture was stirred and reacted at 25 °C for 2 hours. The reaction mixture was filtered and concentrated under reduced pressure to provide the product Compound H11-3 (7.70 g, crude product, HCl salt) as a yellow solid, and this crude product was directly used in the next reaction without purification.

**¹H NMR:** EC16331-109-P1B (400 MHz DMSO) *δ* ppm 8.86 (s, 3H), 7.60 (d, *J =* 8.4 Hz, 2H), 7.48 (d, *J* = 8.4 Hz, 2H), 4.52 - 4.68 (m, 1H), 3.54 (s, 3H), 3.24 (dd, *J =* 16.4, 5.6 Hz, 1H), 3.02 (dd, *J =* 16.4, 8.8 Hz, 1H).

### 3. Synthesis of Compound H11-4

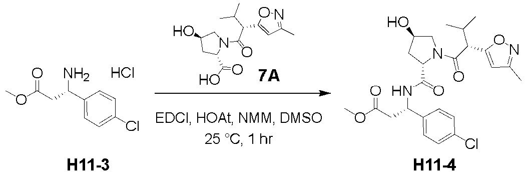

Compound H11-3 (1.80 g, 7.20 mmol, 1.00 eq., HCl), Compound 7A (1.07 g, 3.60 mmol, 0.50 eq.), EDCI (2.07 g, 10.8 mmol, 1.50 eq.), NMM (2.18 g, 21.6 mmol, 2.37 mL, 3.00 eq.) and HOAt (1.47 g, 10.8 mmol, 1.51 mL, 1.50 eq.) were mixed in DMSO (20.0 mL), and the mixture was stirred and reacted at 25 °C for 1 hour. LCMS (EC17015-237-P1A1, P1: RT = 0.353 minutes) showed that the target product was generated, accounting for 84.0%. The reaction mixture was filtered and concentrated under reduced pressure to obtain a crude product, which was further purified by reverse-phase HPLC (0.1 % FA conditions) to provide Compound H11-4 (1.60 g, 3.25 mmol, 45.2% yield) as a yellow solid.

**LC-MS:** EC17015-237-P1A1, product: RT = 0.353 min, m/z = 492.0 [M+H]⁺

### 4. Synthesis of Compound 8A

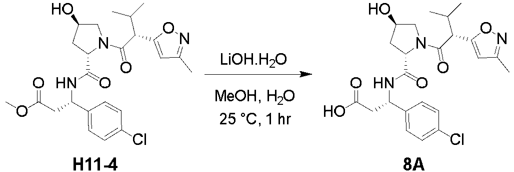

To a solution of Compound H11-4 (1.60 g, 3.25 mmol, 1.00 eq.) in methanol (12.0 mL) and water (4.00 mL), LiOH.H₂O (409 mg, 9.76 mmol, 3.00 eq.) was added, and the mixture was stirred and reacted at 25 °C for 1 hour. LCMS (EC17015-249-P1A, P1: RT = 0.326 minutes) showed that the target product was generated, accounting for 100%. The reaction mixture was filtered and concentrated under reduced pressure to provide the product Compound 8A (1.80 g, crude) as a yellow solid, and this crude product was directly used in the next reaction without purification.

**LC-MS:** EC17015-249-P1A, product: RT = 0.326 min, m/z = 478.0 [M+H]⁺

### 5. Synthesis of Compound APH11

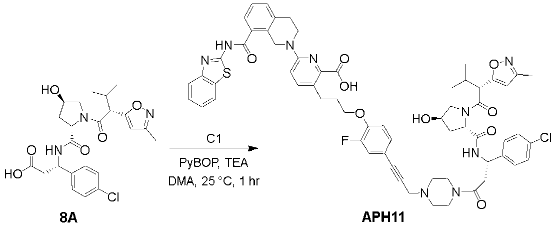

Compound 8A (10.0 mg, 20.9 µmol, 1.00 eq.), Compound C1 (10.3 mg, 14.6 µmol, 0.70 eq.), PyBOP (13.1 mg, 25.1 µmol, 1.20 eq.) and TEA (6.35 mg, 62.8 µmol, 8.74 µL, 3.00 eq.) were mixed in DMA (0.50 mL), and the mixture was stirred and reacted at 25 °C for 1 hour. LCMS (EC17015-294-P1E1, P1: RT = 0.423 minutes) showed that the target product was generated, accounting for 18.8%. The reaction mixture was filtered and concentrated under reduced pressure to obtain a crude product, which was further purified by Prep-HPLC (chromatographic column: CD02-Waters Xbidge BEH C18 150*25*10 µm; mobile phase: [water (NH₄HCO₃)-ACN]; gradient: 22%-52% B, within 10 minutes) to provide Compound APH11 (8.30 mg, 5.61 µmol, 2.68% yield, 93.4% purity) as a white solid.

**LC-MS:** EC17015-294-P1E1, product: RT = 0.423 min, m/z = 1164.0 [M+H]⁺
**¹H NMR:** EC17015-294-P1A (400 MHz DMSO) *δ* ppm 8.02 (d, *J* = 8.2 Hz, 1H), 7.78 (d, *J* = 8.4 Hz, 1H), 7.60 (d, *J* = 7.2 Hz, 1H), 7.43 - 7.51 (m, 2H), 7.38 - 7.42 (m, 1H), 7.23 - 7.37 (m, 7H), 7.17 (d, *J* = 8.4 Hz, 1H), 7.05 - 7.13 (m, 1H), 6.85 (d, *J* = 7.8 Hz, 1H), 6.09 - 6.28 (m, 1H), 5.07 - 5.22 (m, 1H), 4.91 (s, 2H), 4.18 - 4.40 (m, 2H), 4.01 (s, 2H), 3.80 - 3.87 (m, 2H), 3.68 - 3.75 (m, 2H), 3.47 (s, 4H), 2.95 - 2.99 (m, 2H), 2.72 - 2.79 (m, 4H), 2.30 - 2.45 (m, 5H), 2.18 (s, 3H), 1.88 - 2.05 (m, 4H), 1.67 - 1.78 (m, 1H), 1.23 (s, 2H), 0.94 (d, *J* = 6.0 Hz, 3H), 0.68 - 0.86 (m, 4H).

### Synthesis of APH12:

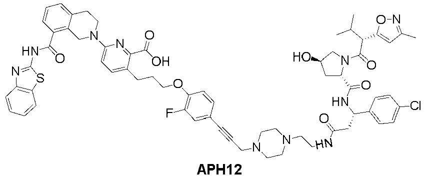

### Synthesis scheme:

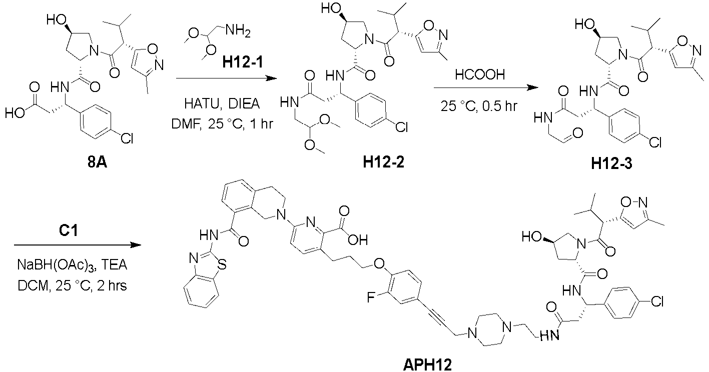

### 1. Synthesis of Compound H12-2

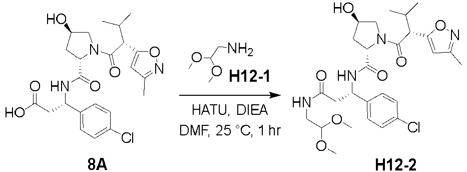

Compound 8A (200 mg, 418 µmol, 1.00 eq.), Compound H12-1 (66.0 mg, 628 µmol, 68.4 µL, 1.50 eq.), HATU (239 mg, 628 µmol, 1.50 eq.) and DIEA (324 mg, 2.51 mmol, 437 µL, 6.00 eq.) were mixed in DMF (3.00 mL), and the mixture was stirred and reacted at 25°C for 1 hour. LCMS (EC17015-279-P1B, P1: RT = 0.337 minutes) showed that the target product was generated, accounting for 99.7%. The reaction mixture was concentrated under reduced pressure to obtain a crude product, which was further purified by Prep-HPLC (neutral conditions; chromatographic column: CD07-Daisogel SP-100-8-ODS-PK 150*25*10 µm; mobile phase: [water (NH₄HCO₃)-ACN]; gradient: 22%-52% B, within 10 minutes) to provide the product Compound H12-2 (160 mg, crude) as a white solid.

**LC-MS:** EC17015-279-P1B, product: RT = 0.337 min

### 2. Synthesis of Compound H12-3

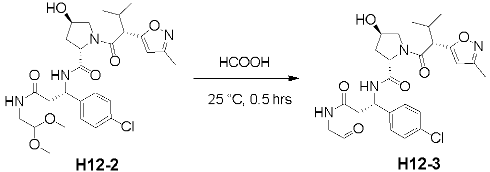

To Compound H12-2 (70.0 mg, 124 µmol, 1.00 eq.), formic acid (1.00 mL) was added, and the mixture was stirred and reacted at 25 °C for 0.5 hours. The reaction mixture was filtered and concentrated under reduced pressure to provide the product Compound H12-3 (60.0 mg, crude) as a yellow oil, and this crude product was directly used in the next reaction without purification.

**LC-MS:** EC16331-182-P1A, product: RT = 0.332 min.

### 3. Synthesis of Compound APH12

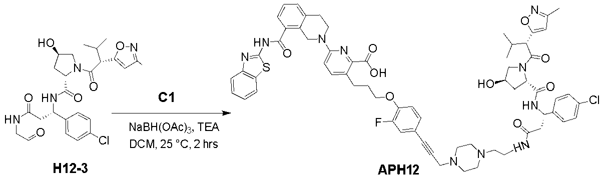

To a solution of Compound H12-3 (60.0 mg, 116 µmol, 1.00 eq.) and Compound C1 (40.7 mg, 57.8 µmol, 0.50 eq.) in DCM (2.00 mL), TEA (117 mg, 1.16 mmol, 161 µL, 10.0 eq.) and NaBH(OAc)₃ (73.5 mg, 347 µmol, 3.00 eq.) were added, and the mixture was stirred and reacted at 25 °C for 2 hours. The reaction mixture was filtered and concentrated under reduced pressure to obtain a crude product (60.0 mg), which was further purified by Prep-HPLC (chromatographic column: Phenomenex luna C18 150*25 mm* 10 µm; mobile phase: [water (HCl)-ACN]; gradient: 37%-67% B, within 14 minutes) to provide Compound APH12 (25.0 mg, 19.3 µmol, 16.7% yield, 96.2% purity, HCl) as a white solid.

**LC-MS:** EC16331-183-P1A1, product: RT = 1.853 min
**¹H NMR:** EC16331-183-P1R (400 MHz DMSO) *δ* ppm 8.26 - 8.34 (m, 1H), 8.04 (d, *J* = 7.6 Hz, 1H), 7.79 (d, *J* = 8.0 Hz, 1H), 7.55 - 7.63 (m, 2H), 7.44 - 7.51 (m, 2H), 7.40 - 7.43 (m, 1H), 7.28 - 7.39 (m, 7H), 7.24 (br d, *J* = 8.4 Hz, 1H), 7.15 (br t, *J* = 8.8 Hz, 1H), 6.98 (d, *J* =8.8 Hz, 1H), 6.20 - 6.24 (m, 1H), 5.14 (dt, *J* = 14.8, 7.6 Hz, 1H), 4.93 (s, 2H), 4.41 (br d, *J* = 7.6 Hz, 1H), 4.33 - 4.38 (m, 2H), 4.23 (br d, *J* = 3.2 Hz, 2H), 4.16 (br s, 2H), 4.04 (br t, *J =* 6.4 Hz, 3H), 3.86 (br t, *J =* 5.6 Hz, 2H), 3.70 - 3.75 (m, 2H), 3.44 - 3.55 (m, 5H), 3.34 - 3.43 (m, 4H), 3.11 - 3.21 (m, 2H), 2.98 (br t, *J* = 5.2 Hz, 2H), 2.81 (br t, *J* = 7.6 Hz, 2H), 2.65 (br dd, *J* = 14.8, 7.6 Hz, 1H), 2.56 (br d, *J* = 6.8 Hz, 1H), 2.25 (br d, *J* = 7.6 Hz, 1H), 2.18 (s, 3H), 1.92 - 2.02 (m, 3H), 1.64 - 1.73 (m, 1H), 1.20 - 1.27 (m, 1H), 0.91 - 1.00 (m, 3H), 0.70 - 0.82 (m, 4H).

### d) Synthesis of APH14, APH15, and APH16

### Synthesis scheme:

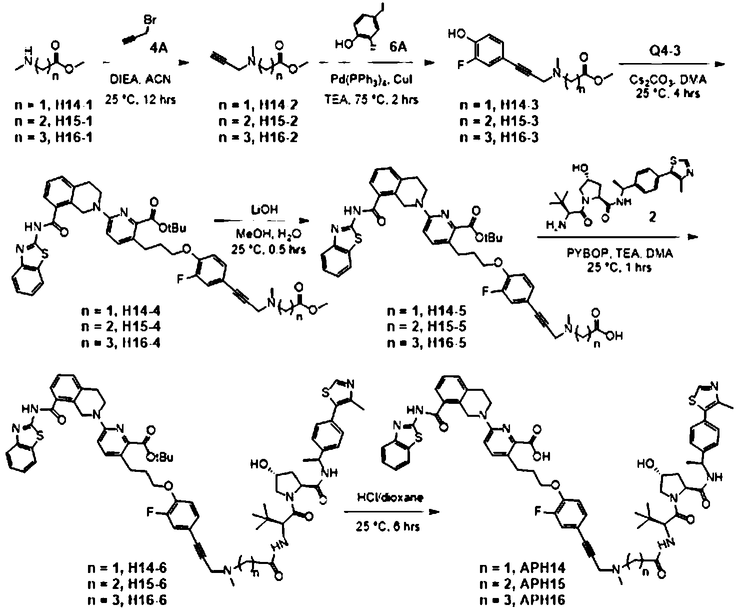

### Synthesis of APH14:

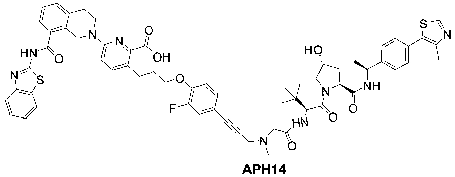

### 1. Synthesis of Compound H14-2

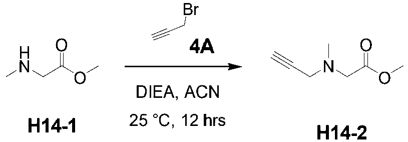

To a solution of Compound H14-1 (3.00 g, 21.5 mmol, 1.00 eq., HCl) in ACN (15.0 mL), DIEA (5.06 g, 39.1 mmol, 6.81 mL, 1.82 eq.) and Compound 4A (2.33 g, 19.6 mmol, 1.69 mL, 0.91 eq.) were added, and the mixture was stirred and reacted at 25°C for 12 hours. The reaction mixture was filtered and concentrated under reduced pressure to obtain a crude product, which was further purified by column chromatography (SiO₂, petroleum ether /ethyl acetate = 4/1 to 1/1) to provide the product H14-2 (2.25 g, 15.9 mmol, 74.2% yield) as a yellow oil.

**¹H NMR:** EC17015-223-P1A (400 MHz CDCl₃) *δ* ppm 3.71 (s, 3H), 3.47 (d, *J* = 2.0 Hz, 2H), 3.31 (s, 2H), 2.39 (s, 3H), 2.20 - 2.28 (m, 1H).

### 2. Synthesis of Compound H14-3

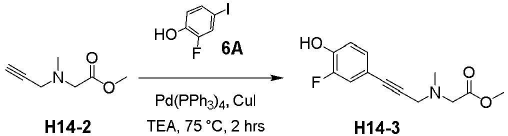

A solution of Compound 6A (200 mg, 840 µmol, 1.00 eq.), Compound H14-2 (178 mg, 1.26 mmol, 1.50 eq.), Pd(PPh₃)₂Cl₂ (59.0 mg, 84.0 µmol, 0.10 eq.) and CuI (8.00 mg, 42.0 µmol, 0.05 eq.) in TEA (2.00 mL) was stirred and reacted at 75°C under N₂ atmosphere for 2 hours. LCMS (EC17015-226-P1A, P1: RT = 0.341 minutes) showed that the target product was generated, accounting for 49.3%. The reaction mixture was concentrated under reduced pressure to obtain a crude product, which was further purified by column chromatography (SiO₂, petroleum ether /ethyl acetate =7/3 to 1/1) to provide the product Compound H14-3 (170 mg, 677 µmol, 80.5% yield) as a yellow oil.

**LC-MS:** EC17015-226-P1A, product: RT = 0.341 min, m/z = 250.0 [M+H]⁺
**¹H NMR:** EC17015-226-P1A (400 MHz CDCl₃) *δ* ppm 7.08 - 7.19 (m, 2H), 6.89 - 6.97 (m, 1H), 3.75 (s, 3H), 3.67 (s, 2H), 3.40 (s, 2H), 2.48 (s, 3H).

### 3. Synthesis of Compound H14-4

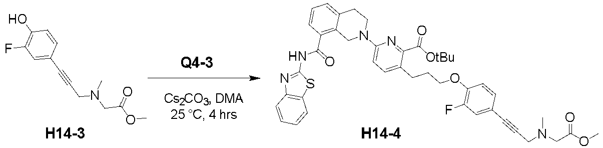

A solution of Compound Q4-3 (200 mg, 329 µmol, 1.00 eq.), Compound H14-3 (91.0 mg, 362 µmol, 1.10 eq.) and Cs₂CO₃ (322 mg, 988 µmol, 3.00 eq.) in DMA(4.00 mL) was stirred and reacted at 25°C under N₂ atmosphere for 4 hours. LCMS (EC17015-229-P1A, P1: RT = 0.469 minutes) showed that the target product was generated, accounting for 48.0%. To the reaction mixture, water (20.0 mL) was added, and extracted with ethyl acetate (15.0 mL * 3). The resulting organic layer was washed with saturated saline (10.0 mL), dried over Na₂SO₄, filtered and concentrated under reduced pressure to obtain a crude product, which was further purified by column chromatography (SiO₂, petroleum ether /ethyl acetate =3/2 to 1/1) to provide the product Compound H14-4 (228 mg, 293 µmol, 89.0% yield) as a yellow oil.

**LC-MS:** EC17015-229-P1A, product: RT = 0.469 min, m/z = 778.3 [M+H]⁺
**¹H NMR:** EC17015-229-P1A (400 MHz DMSO) *δ* ppm 12.84 (s, 1H), 8.03 (d, *J =* 7.8 Hz, 1H), 7.78 (d, *J* = 8.0 Hz, 1H), 7.55 (d, *J* = 7.2 Hz, 1H), 7.43 - 7.52 (m, 2H), 7.27 - 7.42 (m, 4H), 7.18 (d, *J* = 8.2 Hz, 1H), 7.05 - 7.13 (m, 1H), 6.91 (d, *J =* 8.8 Hz, 1H), 4.93 (s, 2H), 4.02 (t, *J =* 6.07 Hz, 2H), 3.78 (t, *J* = 6.0 Hz, 2H), 3.56 - 3.64 (m, 5H), 3.33 (s, 4H), 2.96 - 3.00 (m, 2H), 2.66 (t, *J =* 7.6 Hz, 2H), 2.33 (s, 3H), 1.29 (s, 9H).

### 4. Synthesis of Compound H14-5

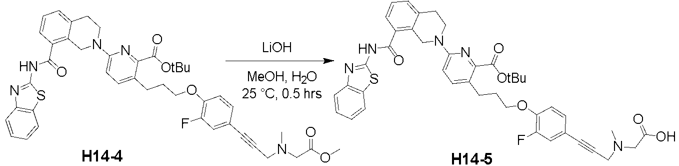

To a solution of Compound H14-4 (228 mg, 293 µmol, 1.00 eq.) in methanol (3.00 mL) and H₂O (1.00 mL), LiOH.H₂O (36.9 mg, 880 µmol, 3.00 eq.) was added, and the mixture was stirred and reacted at 25°C for 0.5 hours. LCMS (EC17015-232-P1W, P1: RT = 0.464 minutes) showed that the target product was generated, accounting for 68.5%. The reaction mixture was adjusted to pH 3 with 1 M hydrochloric acid, diluted by adding water (10.0 mL), and extracted with a solution of DCM and methanol (DCM: methanol =10:1, 15.0 mL * 3). The resulting organic layer was dried over Na₂SO₄, filtered and concentrated under reduced pressure to provide the crude product H14-5 (200 mg, 262 µmol, 89.3% yield) as a yellow solid, which was directly used in the next reaction without purification.

**LC-MS:** EC17015-232-P1W, product: RT = 0.464 min, m/z = 764.2 [M+H]⁺.

### 5. Synthesis of Compound H14-6

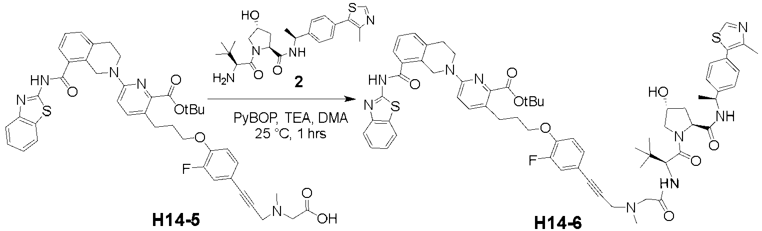

Compound H14-5 (120 mg, 157 µmol, 1.00 eq.), Compound 2 (69.8 mg, 157 µmol, 1.00 eq.), PyBOP (98.1 mg, 188 µmol, 1.20 eq.) and TEA (47.7 mg, 471 µmol, 65.6 µL, 3.00 eq.) were mixed in DMA (2.00 mL), and the mixture was stirred and reacted at 25°C for 1 hour. LCMS (EC17015-242-P1C, P1: RT = 0.859 minutes) showed that the target product was generated, accounting for 92.3%. The reaction mixture was diluted by adding water (20.0 mL), and extracted with DCM (15.0 mL * 3). The resulting organic layer was washed with saturated saline (10.0 mL), dried over Na₂SO₄, filtered and concentrated under reduced pressure to provide the crude product H14-6 (160 mg, crude product) as a yellow oil, which was directly used in the next reaction without purification.

**LC-MS:** EC17015-242-P1C, product: RT = 0.859 min, m/z = 1190.4 [M+H]⁺.

### 6. Synthesis of Compound APH14

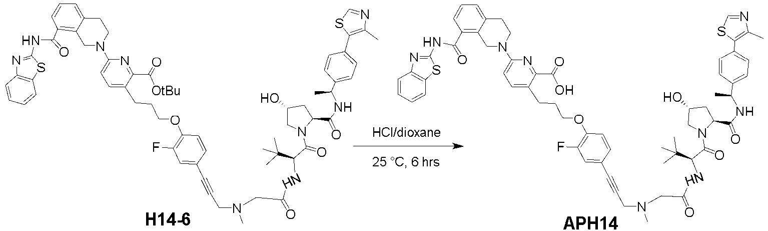

To Compound H14-6 (150 mg, 126 µmol, 1.00 eq.), HCl/dioxane (5.00 mL) was added, and the mixture was stirred and reacted at 25°C for 6 hours. LCMS (EC17015-250-P1B2, P1: RT = 0.607 minutes) showed that the target product was generated, accounting for 35.2%. The reaction mixture was concentrated under reduced pressure to obtain a crude product, which was further purified by Prep-HPLC (chromatographic column: CD02-Waters Xbidge BEH C18 150*25*10µm; mobile phase: [water (NH₄HCO₃)-ACN]; gradient: 36%-66% B, within 10 minutes) to provide Compound APH14 (20.0 mg, 17.6 µmol, 14.0% yield, 96.1% purity) as a white solid.

**LC-MS:** EC17015-250-P1B2, product: RT = 0.607 min, m/z = 1134.4 [M+H]⁺
**¹H NMR:** EC21184-4-P1A (400 MHz DMSO) *δ* ppm 8.93 - 9.02 (m, 1H), 8.44 (d, *J =* 7.6 Hz, 1H), 8.03 (d, *J* = 8.0 Hz, 1H), 7.76 - 7.81 (m, 1H), 7.69 (d, *J* =9.6 Hz, 1H), 7.60 (d, *J* = 7.6 Hz, 1H), 7.51 - 7.55 (m, 1H), 7.27 - 7.50 (m, 9H), 7.18 - 7.23 (m, 1H), 7.08 - 7.15 (m, 1H), 6.89 - 6.96 (m, 1H), 4.85 - 4.98 (m, 3H), 4.52 (d, *J* = 9.6 Hz, 1H), 4.39 - 4.48 (m, 1H), 4.29 (d, *J*=2.8 Hz, 1H), 4.02 (t, *J*=6.4 Hz, 2H), 3.85 (t, *J* = 5.8 Hz, 2H), 3.54 - 3.65 (m, 4H), 3.11 (d, *J =* 2.8 Hz, 2H), 2.94 - 3.03 (m, 2H), 2.75 - 2.85 (m, 2H), 2.41 - 2.46 (m, 5H), 2.34 (s, 2H), 1.92 - 2.09 (m, 4H), 1.70 - 1.83 (m, 1H), 1.37 (d, *J =* 6.8 Hz, 3H), 1.23 (s, 1H), 0.94 (s, 9H).

### Synthesis of APH15:

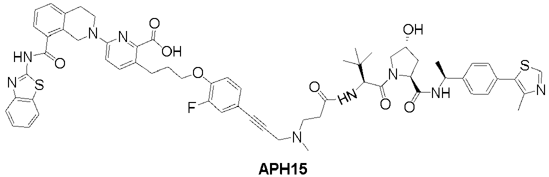

The synthesis procedure for the PROTAC molecule APH15 was similar to that for APH14, but the starting materials were replaced with Compounds H15-1 and 4A, and the target product APH15 (20.0 mg, 16.3 µmol, 6.55% yield, 93.6% purity) was obtained by purification after synthesis, as a white solid.

**¹H NMR:** EC16331-167-P1L (400 MHz DMSO)*δ* ppm 8.98 (s, 1H), 8.38 (d, *J =* 7.6 Hz, 1H), 8.16 (d, *J* = 9.2 Hz, 1H), 8.03 (d, *J* = 8.0 Hz, 1H), 7.79 (d, *J =* 8.0 Hz, 1H), 7.60 (d, *J=* 7.2 Hz, 1H), 7.53 (d, *J =* 8.8 Hz, 1H), 7.46 - 7.50 (m, 1H), 7.44 (s, 1H), 7.42 (s, 2H), 7.37 - 7.40 (m, 2H), 7.35 - 7.37 (m, 2H), 7.34 (s, 1H), 7.26 (d, *J =* 12.0 Hz, 1H), 7.17 (d, *J* = 8.8 Hz, 1H), 7.07 - 7.13 (m, 1H), 6.93 (d, *J* = 8.8 Hz, 1H), 4.92 (s, 3H), 4.50 (d, *J* = 9.2 Hz, 1H), 4.42 (t, *J* = 8.0 Hz, 1H), 4.28 (s, 1H), 4.02 (t, *J* = 6.0 Hz, 2H), 3.86 (t, *J* = 5.6 Hz, 2H), 3.60 (s, 2H), 3.53 (s, 3H), 2.79 (t, *J* = 7.2 Hz, 2H), 2.62 (dd, *J =* 16.8, 6.8 Hz, 2H), 2.45 (s, 4H), 2.31 (s, 1H), 2.26 (s, 3H), 1.93 - 2.01 (m, 3H), 1.77 - 1.82 (m, 1H), 1.37 (d, *J=* 6.8 Hz, 3H), 0.94 (s, 12H).

### Synthesis of APH16:

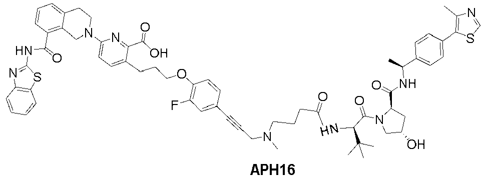

The synthesis procedure for the PROTAC molecule APH16 was similar to that for APH14, but the starting materials were replaced with Compounds H16-1 and 4A, and the target product APH16 (20.0 mg, 16.0 µmol, 6.49% yield, 92.9% purity) was obtained by purification after synthesis, as a white solid.

**¹H NMR:** EC16331-166-P1M (400 MHz DMSO)*δ* ppm 8.96 - 8.99 (m, 1H), 8.37 (d, *J* = 7.6 Hz, 1H), 8.04 (d, *J* = 7.6 Hz, 1H), 7.77 - 7.84 (m, 2H), 7.60 (d, *J* = 7.6 Hz, 1H), 7.54 (d, *J =* 8.8 Hz, 1H), 7.46 - 7.50 (m, 1H), 7.44 (s, 1H), 7.33 - 7.43 (m, 6H), 7.23 - 7.29 (m, 1H), 7.15 - 7.19 (m, 1H), 7.06 - 7.12 (m, 1H), 6.95 (d, *J* = 8.8 Hz, 1H), 5.04 - 5.16 (m, 1H), 4.88 - 4.94 (m, 3H), 4.51 (d, *J* = 9.2 Hz, 1H), 4.42 (t, *J* = 8.0 Hz, 1H), 4.27 (s, 1H), 4.02 (t, *J =* 6.4 Hz, 2H), 3.86 (t, *J =* 5.6 Hz, 2H), 3.60 (s, 2H), 3.48 (s, 3H), 2.98 (t, *J =* 5.2 Hz, 2H), 2.80 (t, *J* = 7.2 Hz, 2H), 2.42 - 2.47 (m, 3H), 2.36 (t, *J=* 7.2 Hz, 2H), 2.22 (s, 3H), 2.11 - 2.19 (m, 1H), 1.92 - 2.04 (m, 3H), 1.79 (tt, *J* = 8.4, 4.0 Hz, 1H), 1.59 - 1.68 (m, 2H), 1.37 (d, *J =* 6.8 Hz, 3H), 1.16 - 1.27 (m, 1H), 0.93 (s, 9H).

### e) Synthesis of APH17, APH18, APH19, APH20, and APH21

### Synthesis scheme:

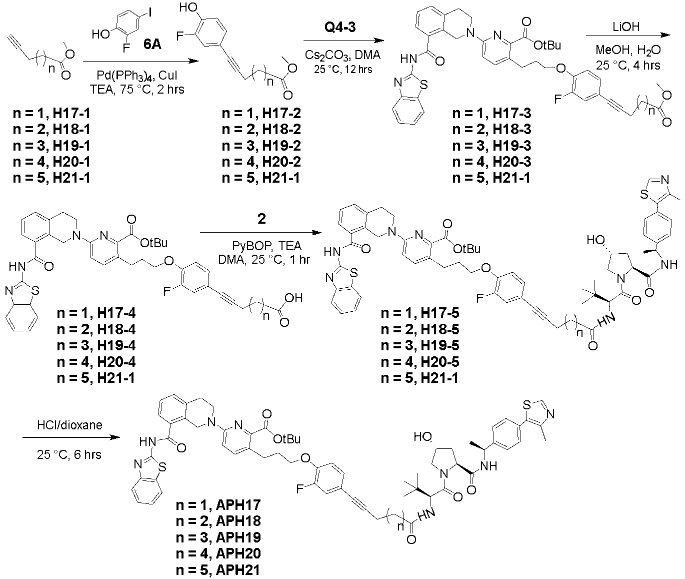

### Synthesis of APH17:

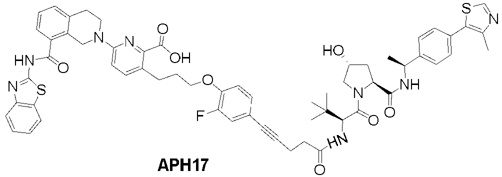

### 1. Synthesis of Compound H17-2

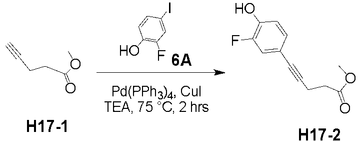

Compound 6A (300 mg, 1.26 mmol, 1.00 eq.), Compound H17-1 (212 mg, 1.89 mmol, 1.50 eq.), Pd(PPh₃)₂Cl₂ (88.5 mg, 126 µmol, 0.10 eq.) and CuI (12.0 mg, 63.0 µmol, 0.05 eq.) were mixed in TEA(5.00 mL), and the mixture was stirred and reacted at 75°C under N₂ atmosphere for 2 hours. LCMS (EC17015-233-P1A, P1: RT = 0.355 minutes) showed that the target product was generated, accounting for 62.9%. The reaction mixture was filtered and concentrated under reduced pressure to obtain a crude product, which was further purified by column chromatography (SiO₂, petroleum ether /ethyl acetate =7/3 to 1/1) to provide the product H17-2 (220 mg, 990 µmol, 78.5% yield) as a yellow oil.

**LC-MS:** EC17015-233-P1A, product: RT = 0.355 min, m/z = 223.0 [M+H]⁺
**¹H NMR:** EC17015-233-P1A (400 MHz DMSO) *δ* ppm 10.21 (s, 1H), 7.13 (dd, *J* = 12.0, 1.8 Hz, 1H), 7.01 (dd, *J* = 8.4, 1.0 Hz, 1H), 6.84 - 6.93 (m, 1H), 3.62 (s, 3H), 2.60 (dd, *J =* 9.6, 5.2 Hz, 4H).

### 2. Synthesis of Compound H17-3

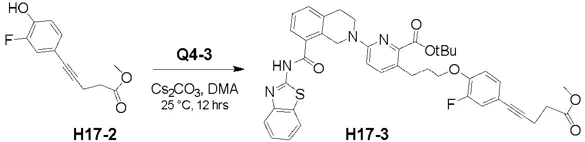

Compound Q4-3 (200 mg, 329 µmol, 1.00 eq.), Compound H17-2 (80.5 mg, 362 µmol, 1.10 eq.) and Cs₂CO₃ (322 mg, 988 µmol, 3.00 eq.) were mixed in DMA (3.00 mL), and the mixture was stirred and reacted at 25°C for 12 hours. LCMS (EC17015-247-P1A, P1: RT = 0.517 minutes) showed that the target product was generated, accounting for 68.2%. The reaction mixture was diluted by adding water (20.0 mL), and extracted with ethyl acetate (15.0 mL * 3). The resulting organic layer was washed with saturated saline (20.0 mL), dried over Na₂SO₄, filtered and concentrated under reduced pressure to obtain a crude product, which was further purified by column chromatography (SiO₂, petroleum ether /ethyl acetate =7/3 to 1/1) to provide the product H17-3 (155 mg, 207 µmol, 62.9% yield) as a yellow solid.

**LC-MS:** EC17015-247-P1A, product: RT = 0.517 min, m/z = 749.2 [M+H]⁺
**¹H NMR:** EC17015-247-P1A (400 MHz DMSO) *δ* ppm 12.82 (s, 1H), 8.03 (d, *J* = 7.8 Hz, 1H), 7.78 (d, *J* = 8.0 Hz, 1H), 7.55 (d, *J* = 6.8 Hz, 1H), 7.44 - 7.51 (m, 2H), 7.31 - 7.31 (m, 1H), 7.31 - 7.41 (m, 2H), 7.19 (dd, *J* = 11.8, 1.6 Hz, 1H), 7.04 - 7.13 (m, 2H), 6.91 (d, *J* = 8.8 Hz, 1H), 4.93 (s, 2H), 3.96 - 4.03 (m, 2H), 3.78 (t, *J =* 6.0 Hz, 2H), 3.62 (s, 3H), 2.98 (t, *J* = 5.8 Hz, 2H), 2.60 - 2.66 (m, 4H), 2.54 (s, 2H), 1.86 - 1.96 (m, 2H), 1.30 (s, 9H).

### 3. Synthesis of Compound H17-4

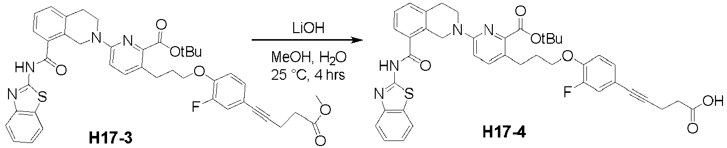

To a solution of Compound H17-3 (155 mg, 207 µmol, 1.00 eq.) in methanol (1.20 mL) and water (0.40 mL), LiOH.H₂O (26.1 mg, 621 µmol, 3.00 eq.) was added, and the mixture was stirred and reacted at 25°C for 4 hours. LCMS (EC17015-252-P1A2, P1: RT = 0.483 minutes) showed that the target product was generated, accounting for 92.2%. The reaction mixture was adjusted to pH 3 by 1 M hydrochloric acid, diluted by adding water (10.0 mL), and extracted with a solution of DCM and methanol (DCM: methanol =10:1, 15.0 mL * 3). The resulting organic layer was dried over Na₂SO₄, filtered and concentrated under reduced pressure to provide the product Compound H17-4 (150 mg, crude) as a yellow solid.

**LC-MS:** EC17015-252-P1A2, product: RT = 0.483 min, m/z = 735.1 [M+H]⁺

### 4. Synthesis of Compound H17-5

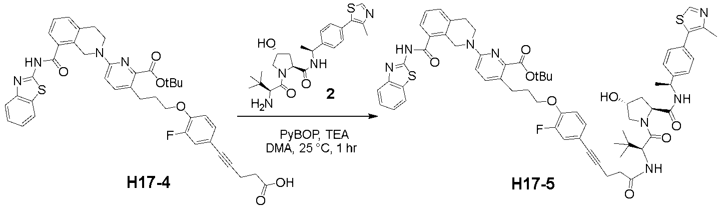

Compound H17-4 (150 mg, 204 µmol, 1.00 eq.), Compound 2 (90.8 mg, 204 µmol, 1.00 eq.), PyBOP (127 mg, 245 µmol, 1.20 eq.) and TEA (62.0 mg, 612 µmol, 85.2 µL, 3.00 eq.) were mixed in DMA (2.00 mL), and the mixture was stirred and reacted at 25°C for 1 hour. LCMS (EC17015-256-P1A, P1: RT = 0.548 minutes) showed that the target product was generated, accounting for 81.8%. The reaction mixture was diluted by adding water (20.0 mL), and extracted with DCM (15.0 mL * 3). The resulting organic layer was washed with saturated saline (10.0 mL), dried over Na₂SO₄, filtered and concentrated under reduced pressure to provide the crude product Compound H17-5 (200 mg) as a yellow oil, and this crude product was directly used in the next reaction without purification.

**LC-MS:** EC17015-256-P1A, product: RT = 0.548 min, m/z = 1161.4 [M+H]⁺

### 5. Synthesis of Compound APH17

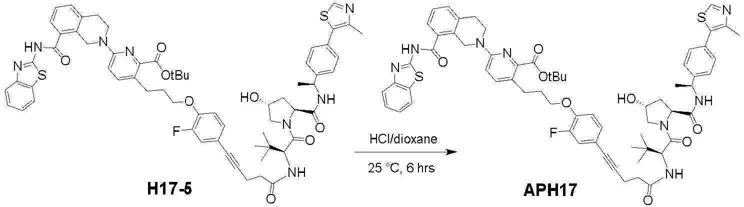

Compound H17-5 (190 mg, 164 µmol, 1.00 eq.) was dissolved in HCl/dioxane (4.00 mL), and the mixture was stirred and reacted at 25 °C for 6 hours. LCMS (EC17015-261-P1A1, P1: RT = 0.597 minutes) showed that the target product was generated, accounting for 30.0%. The reaction mixture was filtered and concentrated under reduced pressure to obtain a crude product, which was further purified by Prep-HPLC (chromatographic column: CD02-Waters Xbidge BEH C18 150*25*10µm; mobile phase: [water (NH₄HCO₃)-ACN]; gradient: 28%-58% B, within 13 minutes) to provide the product APH17 (24.0 mg, 21.2 µmol, 12.9% yield, 97.5% purity) as a white solid.

**LC-MS:** EC17015-261-P1A1, product: RT = 0.597 min, m/z = 1105.3 [M+H]⁺
**¹H NMR:** EC17015-261-P1A(400 MHz DMSO) *δ* ppm 8.98 (s, 1H), 8.38 *(d, J=* 7.8 Hz, 1H), 8.01 (dd, *J* = 19.2, 8.2 Hz, 2H), 7.79 (d, *J* = 7.8 Hz, 1H), 7.60 (d, *J =* 7.8 Hz, 1H), 7.53 (d, *J =* 8.4 Hz, 1H), 7.47 (s, 1H), 7.32 - 7.45 (m, 7H), 7.17 (d, *J* = 12.4 Hz, 1H), 7.06 - 7.11 (m, 1H), 6.93 (d, *J* = 8.6 Hz, 1H), 5.13 (s, 1H), 4.92 (s, 3H), 4.56 (d, *J =* 9.2 Hz, 1H), 4.42 (t, *J* = 8.2 Hz, 1H), 4.28 (s, 1H), 4.01 (t, *J =* 5.6 Hz, 2H), 3.85 (s, 2H), 3.54 - 3.69 (m, 3H), 2.98 (d, *J =* 5.8 Hz, 2H), 2.78 (t, *J* = 7.0 Hz, 2H), 2.58 (s, 3H), 2.45 (s, 3H), 2.35 - 2.42 (m, 1H), 1.89 - 2.08 (m, 3H), 1.70 - 1.85 (m, 1H) 1.37 (d, *J* = 6.8 Hz, 3H), 0.94 (s, 9H).

### Synthesis of APH18:

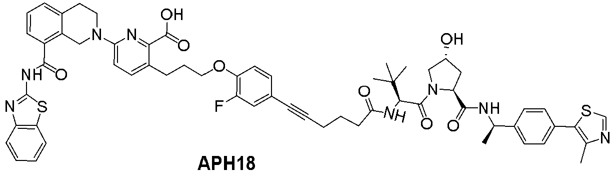

The synthesis procedure for the PROTAC molecule APH18 was similar to that for APH17, but the starting materials were replaced with Compounds H18-1 and 6A, and the target product APH18 (32.7 mg, 27.5 µmol, 18.6% yield, 93.9% purity) was obtained by purification after synthesis, as a white solid.

**LC-MS:** EC21184-23-p1aa, P1: RT = 0.602 min, m/z = 1119.5 [M+H]⁺
**¹H NMR:** EC21184-23-P1QC (400 MHz, DMSO) *δ* ppm 8.98 (s, 1H), 8.38 (d, *J* = 7.6 Hz, 1H), 8.03 (d, *J* = 7.6 Hz, 1H), 7.91 (d, *J* = 9.2 Hz, 1H), 7.79 (d, *J* = 7.6 Hz, 1 H), 7.60 *(d, J=* 7.2 Hz, 1H), 7.31 - 7.54 (m, 9H), 7.25 (dd, *J =* 12.0, 1.6 Hz, 1H), 7.12 - 7.17 (m, 1H), 7.04 - 7.11 (m, 1H), 6.91 (d, *J =* 8.8 Hz, 1H), 5.00 - 5.23 (m, 1H), 4.92 (s, 2H), 4.53 (d, J = 9.2 Hz, 1H), 4.42 *(t, J=* 8.00 Hz, 1H), 4.28 (d, *J* = 1.6 Hz, 1H), 4.01 (t, *J =* 6.4 Hz, 2H), 3.85 (t, *J* = 6.0 Hz, 2H), 3.61 (s, 2H), 2.94 - 3.04 (m, 3H), 2.78 (t, *J* = 7.6 Hz, 2H), 2.45 (s, 3H), 2.34 - 2.43 (m, 3H), 2.24 - 2.34 (m, 1H), 1.91 - 2.05 (m, 3H), 1.65 - 1.85 (m, 4H), 1.37 (d, *J* = 6.8 Hz, 3H), 0.91 - 0.98 (m, 9H).

### Synthesis of APH19:

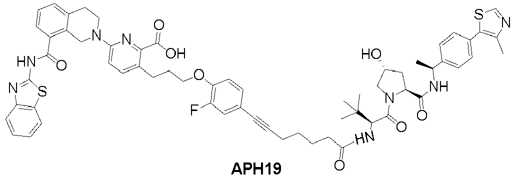

The synthesis procedure for the PROTAC molecule APH19 was similar to that for APH17, but the starting materials were replaced with Compounds H19-1 and 6A, and the target product APH19 (18.0 mg, 15.6 µmol, 10.0% yield, 98.5% purity) was obtained by purification after synthesis, as a white solid.

**LC-MS:** EC21184-25-p1b, product: RT = 0.612 min, m/z = 1133.5 [M+H]⁺
**¹H NMR:** EC21184-25-p1qc (400 MHz DMSO) *δ* ppm 8.94 - 9.02 (m, 1H), 8.37 (d, *J =* 7.6 Hz, 1H), 8.03 (d, *J* = 7.6 Hz, 1H), 7.81 (dd, *J* = 17.2, 8.8 Hz, 2H), 7.60 (d, *J* = 7.2 Hz, 1H), 7.29 - 7.56 (m, 10H), 7.20 (d, *J* = 12.0 Hz, 1H), 7.09 (dt, J = 16.4, 8.4 Hz, 2H), 6.8 (d, *J* = 8.8 Hz, 1 ), 5.02 - 5.22 (m, 1H), 4.87 - 4.98 (m, 3H), 4.52 (d, *J* = 9.2 Hz, 1H), 4.42 *(t, J=* 8.0 Hz, 1H), 4.28 (s, 1H), 4.00 *(t, J=* 6.0 Hz, 2H), 3.85 (s, 2H), 3.60 (s, 2H), 2.97 (s, 2H), 2.79 (t, *J =* 6.8 Hz, 2H), 2.42 - 2.47 (m, 3H), 2.39 (t, *J =* 6.4 Hz, 2H), 2.25 - 2.34 (m, 1H), 2.11 - 2.21 (m, 1H), 1.91 - 2.05 (m, 3H), 1.74 - 1.84 (m, 1H), 1.57 - 1.69 (m, 2H), 1.45 - 1.54 (m, 2H), 1.37 (d, *J =* 6.8 Hz, 3H), 0.94 (s, 9H).

### Synthesis of APH20:

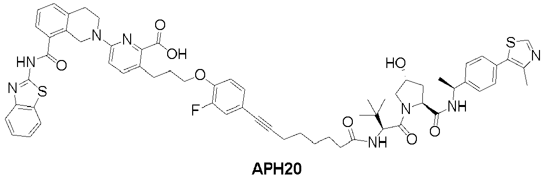

The synthesis procedure for the PROTAC molecule APH20 was similar to that for APH17, but the starting materials were replaced with Compounds H20-1 and 6A, and the target product APH20 (18.0 mg, 14.9 µmol, 5.96% yield, 94.7% purity) was obtained by purification after synthesis, as a white solid.

**LC-MS:** EC17015-336-P1B, product: RT = 0.629 min, m/z = 1147.6 [M+H]⁺
**¹H NMR:** EC17015-336-P1A (400 MHz DMSO) *δ* ppm 8.98 (s, 1H), 8.37 (d, *J* = 7.6 Hz, 1H), 8.02 (d, *J* = 7.6 Hz, 1H), 7.80 (dd, *J* = 12.8, 8.8 Hz, 2H), 7.60 (d, *J* = 6.8 Hz, 1H), 7.45 - 7.51 (m, 2H), 7.31 - 7.45 (m, 7H), 7.20 (dd, *J =* 12.0, 1.8 Hz, 1H), 7.03 - 7.13 (m, 2H), 6.87 (d, *J* = 8.8 Hz, 1H), 4.82 - 5.03 (m, 3H), 4.51 (d, *J* = 9.6 Hz, 1H), 4.42 (t, *J* = 8.0 Hz, 1H), 4.27 (s, 1H), 4.00 (t, *J =* 6.4 Hz, 2H), 3.85 (t, *J*=5.6 Hz, 2H), 3.60 (s, 2H), 2.97 (t, *J =* 5.6 Hz, 2H), 2.76 (t, *J =* 7.6 Hz, 2H), 2.43 - 2.47 (m, 3H), 2.36 (t, *J=* 6.8 Hz, 2H), 2.21 - 2.31 (m, 1H), 2.08 - 2.20 (m, 1H), 1.88 - 2.08 (m, 4H), 1.78 (ddd, *J* = 12.8, 8.4, 4.8 Hz, 1H), 1.44 - 1.57 (m, 4H), 1.37 (d, *J* = 7.2 Hz, 5H), 0.92 (s, 9H).

### Synthesis of APH21:

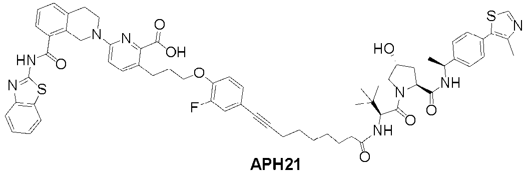

The synthesis procedure for the PROTAC molecule APH21 was similar to that for APH17, but the starting materials were replaced with Compounds H21-1 and 6A, and the target product APH21 (22.0 mg, 18.3 µmol, 7.43% yield, 96.6% purity) was obtained by purification after synthesis, as a white solid.

**LC-MS:** EC17015-342-P1A1, product: RT = 0.642 min, m/z = 1161.3 [M+H]⁺
**¹H NMR:** EC17015-342-P1A (400 MHz DMSO) *δ* ppm 8.98 (s, 1H), 8.37 (d, *J =* 7.6 Hz, 1H), 8.03 (d, *J* = 8.0 Hz, 1H), 7.79 (dd, *J* = 8.4, 5.6 Hz, 2H), 7.59 (d, *J* = 7.6 Hz, 1H), 7.52 (d, *J =* 8.8 Hz, 1H), 7.46 - 7.50 (m, 1H), 7.32 - 7.45 (m, 7H), 7.20 (d, *J =* 12.0 Hz, 1H), 7.03 - 7.15 (m, 2H), 6.92 (d, *J =* 8.4 Hz, 1H), 4.92 (s, 3H), 4.51 (d, *J* = 9.2 Hz, 1H), 4.41 (t, *J =* 8.0 Hz, 1H), 4.27 (s, 1H), 4.00 (t, *J =* 6.4 Hz, 2H), 3.85 (t, *J* = 5.6 Hz, 2H), 3.60 (s, 2H), 2.95 - 3.00 (m, 2H), 2.74 - 2.82 (m, 2H), 2.45 (s, 3H), 2.36 (t, *J* = 6.8 Hz, 2H), 2.17 - 2.30 (m, 2H), 2.12 (dt, *J =* 13.6, 7.2 Hz, 1H), 1.91 - 2.03 (m, 3H), 1.78 (ddd, *J* = 12.8, 8.4, 4.4 Hz, 1H), 1.44 - 1.54 (m, 4H), 1.37 (d, *J =* 7.2 Hz, 5H), 1.23 - 1.31 (m, 2H), 0.93 (s, 9H)

### f) Synthesis of APH101, APH102, APH103, APH104, APH105, and APH106

### Synthesis of APH101:

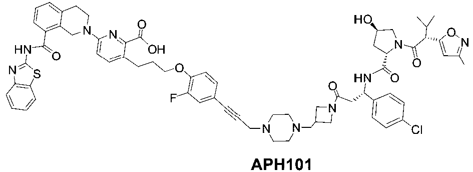

### Synthesis scheme:

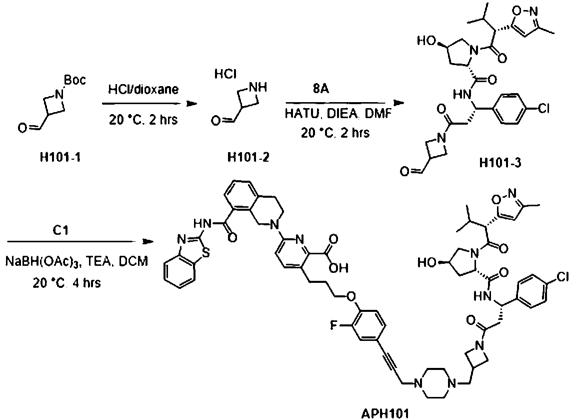

### 1. Synthesis of Compound H101-1

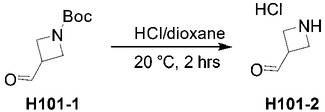

Compound H101-1 (1.00 g, 5.40 mmol, 1.00 eq.) was mixed in HCl/dioxane (1.00 mL), and the mixture was stirred and reacted at 20°C under N₂ atmosphere for 2 hours. The reaction mixture was filtered and concentrated under reduced pressure to provide the product Compound H101-2 (930 mg, crude product) as a white solid.

**¹H NMR:** EC21184-134-p1a (400 MHz DMSO) *δ* 9.37 (d, *J =* 1.2 Hz, 1H), 3.88 (d, *J =* 3.2 Hz, 4H), 2.99 - 3.20 (m, 1H).

### 2. Synthesis of Compound H101-3

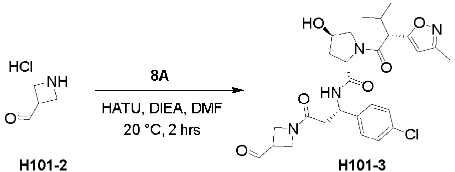

To a solution of Compound H101-2 (100 mg, 823 µmol, 1.00 eq., HCl) and Compound 8A (275 mg, 576 µmol, 0.70 eq.) in DMF (3.00 mL), HATU (469 mg, 1.23 mmol, 1.50 eq.) and DIEA (319 mg, 2.47 mmol, 430 µL, 3.00 eq.) were added, and the mixture was stirred and reacted at 20°C for 2 hours. LC-MS (EC21184-136-p1a, P1: RT = 0.344 minutes) showed that approximately 20.9% of the target product was generated. The reaction mixture was concentrated under reduced pressure to obtain a crude product, which was further purified by Prep-HPLC (FA conditions; chromatographic column: CD01-Phenomenex luna C18 150*25*10µm; mobile phase: [water (FA)-ACN]; gradient elution: 10%-40% B, within 10 minutes) to provide the product Compound H101-3 (25.0 mg, 35.2 µmol, 4.28% yield, 76.7% purity), as a light yellow solid.

**LC-MS:** EC21184-136-p1a, P1: RT = 0.344 min, m/z = 545.2 [M+H]⁺

### 3. Synthesis of Compound APH101

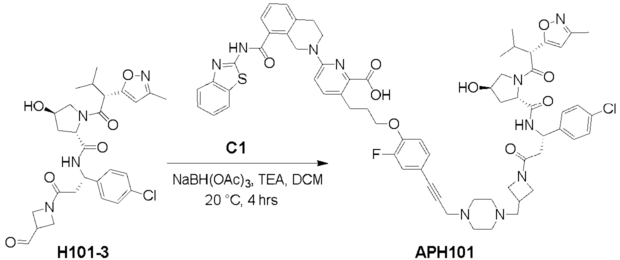

To a solution of Compound H101-3 (25.0 mg, 45.9 µmol, 1.00 eq.) and Compound C1 (22.6 mg, 32.1 µmol, 0.70 eq.) in DCM (1.00 mL), TEA (46.4 mg, 459 µmol, 63.8 µL, 10.0 eq.) and NaBH(OAc)₃ (29.2 mg, 138 µmol, 3.00 eq.) were added, and the mixture was stirred and reacted at 20°C for 4 hours. LC-MS (EC21184-144-p1b, P1: RT = 2.036 minutes) showed that approximately 58.8% of the target product was generated. The reaction mixture was concentrated under reduced pressure to obtain a crude product, which was purified by Prep-HPLC (neutral conditions; chromatographic column: CD02-Waters Xbidge BEH C18 150*25*10µm; mobile phase: [water (NH₄HCO₃)-ACN]; gradient elution: 26%-56% B, within 10 minutes) and then was further purified by Prep-HPLC (alkaline conditions; chromatographic column: CD02-Waters Xbidge BEH C18 150*25*10µm; mobile phase: [water (NH₃H₂O)-ACN]; gradient elution: 17%-47% B, within 10 minutes) to provide Compound APH101 (6.11 mg, 4.72 µmol, 10.3% yield, 95.3% purity) as a white solid.

**LC-MS:** EC21184-144-p1b, P1: RT = 2.036 min, m/z = 617.7 [M/2+H]⁺
**¹H NMR:** EC21184-144-p1b (400 MHz DMSO) *δ* 11.88 - 13.34 (m, 1H), 8.35 - 8.51 (m, 1H), 8.03 (d, *J* = 8.0 Hz, 1H), 7.79 (d, *J =* 8.0 Hz, 1H), 7.60 (d, *J =* 7.6 Hz, 1H), 7.44 - 7.55 (m, 2H), 7.32 - 7.43 (m, 5H), 7.23 - 7.31 (m, 3H), 7.15 - 7.19 (m, 1H), 7.06 - 7.12 (m, 1H), 6.92 (d, *J* = 8.8 Hz, 1H), 6.05 - 6.25 (m, 1H), 5.01 - 5.15 (m, 1H), 4.92 (s, 2H), 4.19 - 4.34 (m, 2H), 3.92 - 4.11 (m, 3H), 3.85 (t, *J* = 6.0 Hz, 2H), 3.76 - 3.83 (m, 1H), 3.68 - 3.75 (m, 2H), 3.44 (s, 3H), 2.97 (t, *J* = 5.6 Hz, 2H), 2.79 (t, *J* = 7.2 Hz, 2H), 2.60 - 2.68 (m, 1H), 2.39 - 2.48 (m, 5H), 2.21 - 2.39 (m, 6H), 2.11 - 2.21 (m, 4H), 1.92 - 2.01 (m, 3H), 1.66 - 1.76 (m, 1H), 1.18 - 1.34 (m, 2H), 0.95 (d, *J* = 6.4 Hz, 3H), 0.72 - 0.80 (m, 3H).

### Synthesis of APH102:

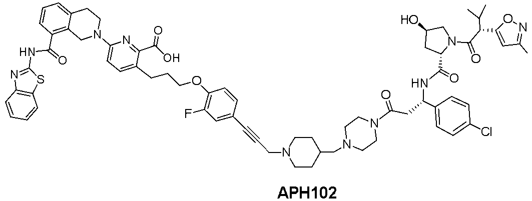

### Synthesis scheme:

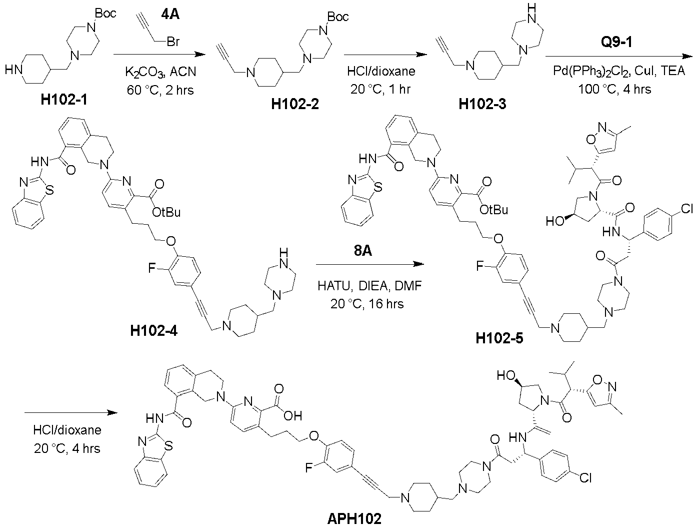

### 1. Synthesis of Compound H102-2

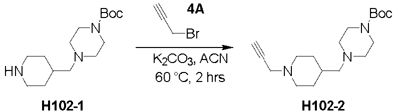

To a solution of Compound H102-1 (1.00 g, 3.53 mmol, 1.00 eq.) and Compound 4A (630 mg, 4.23 mmol, 456 µL, 1.20 eq.) in ACN (10.0 mL), K₂CO₃ (1.46 g, 10.6 mmol, 3.00 eq.) was added, and the mixture was stirred and reacted at 60°C for 2 hours. The reaction mixture was filtered and concentrated under reduced pressure to obtain a crude product, which was further purified by column chromatography (SiO2, DCM/methanol =100/0 to 85/15) to provide the product Compound H102-2 (530 mg, crude) as a brown solid.

**¹H NMR:** EC21184-58-P1A (400 MHz CDCl₃) *δ* 3.37 - 3.46 (m, 4H), 3.32 (d, *J =* 2.4 Hz, 2H), 2.90 (d, *J* = 11.2 Hz, 2H), 2.29 - 2.39 (m, 4H), 2.14 - 2.28 (m, 5H), 1.79 (d, *J =* 13.2 Hz, 2H), 1.49 - 1.56 (m, 1H), 1.46 (s, 9H), 1.21 - 1.33 (m, 2H)

### 2. Synthesis of Compound H102-3

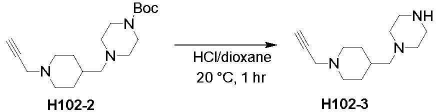

Compound H102-2 (430 mg, 1.34 mmol, 1.00 eq.) was mixed in HCl/dioxane (20.0 mL), and the mixture was stirred and reacted at 20°C for 1 hour. The reaction mixture was filtered and concentrated under reduced pressure to provide the product Compound H102-3 (300 mg, crude) as a white solid.

### 3. Synthesis of Compound H102-4

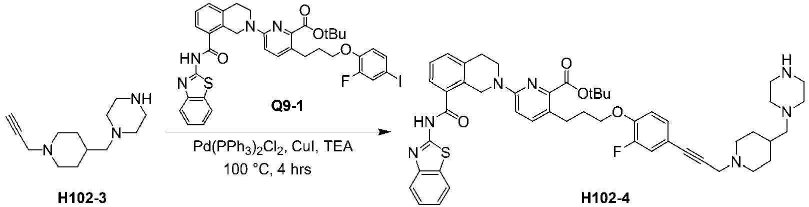

Compound H102-3 (300 mg, 1.36 mmol, 1.00 eq.), Compound Q9-1 (518 mg, 678 µmol, 0.50 eq.), CuI (12.9 mg, 67.8 µmol, 0.05 eq.) and Pd(PPh₃)₂Cl₂ (95.1 mg, 135 µmol, 0.10 eq.) were mixed in a solution of TEA (5.00 mL) and DMF (1.00 mL), and the mixture was stirred and reacted at 20 °C under N₂ atmosphere for 4 hours. To the reaction mixture, water (50 mL) was added, and extracted with ethyl acetate (20.0 mL * 3). The resulting organic layer was washed with saturated saline (30.0 mL * 2), dried over Na₂SO₄, filtered and concentrated under reduced pressure to obtain a crude product, which was further purified by column chromatography (SiO₂, petroleum ether /ethyl acetate =100/0 to 0/100) to provide the product Compound H102-4 (300 mg, crude) as a brown solid.

**LC-MS:** EC21184-69-p1a, P1: RT = 0.398 min, m/z = 429.9 [M/2+H]⁺
**¹H NMR:** EC21184-69-p1aa (400 MHz DMSO) *δ* 8.03 (d, *J =* 7.6 Hz, 1H), 7.79 (d, *J =* 8.0 Hz, 1H), 7.56 (d, *J* = 7.2 Hz, 1H), 7.44 - 7.53 (m, 2H), 7.39 - 7.43 (m, 1H), 7.35 (td, *J=* 7.2, 4.0 Hz, 2H), 7.27 (dd, *J* = 12.0, 1.6 Hz, 1H), 7.14 - 7.20 (m, 1H), 7.06 - 7.13 (m, 1H), 6.92 (d, *J =* 8.8 Hz, 1H), 4.93 (s, 2H), 4.08 - 4.19 (m, 1H), 3.97 - 4.05 (m, 2H), 3.79 (t, *J =* 6.0 Hz, 2H), 3.47 (s, 2H), 3.17 (s, 2H), 2.97 - 2.99 (m, 1H), 2.78 - 2.86 (m, 2H), 2.62 - 2.72 (m, 3H), 2.17 (d, *J =* 6.4 Hz, 4H), 1.92 - 1.98 (m, 2H), 1.69 (d, *J* = 10.8 Hz, 2H), 1.40 - 1.54 (m, 2H), 1.31 (s, 9H), 1.16 - 1.23 (m, 6H), 1.11 (d, *J* = 10.0 Hz, 1H)

### 4. Synthesis of Compound H102-5

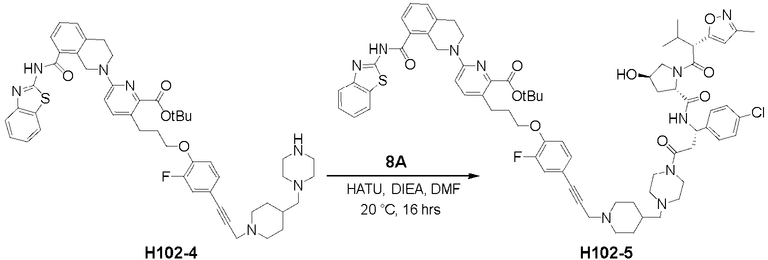

To a solution of Compound H102-4 (200 mg, 233 µmol, 1.00 eq.) in DMF (2.00 mL), Compound 8A (111 mg, 233 µmol, 1.00 eq.), HATU (133 mg, 350 µmol, 1.50 eq.) and DIEA (90.3 mg, 699 µmol, 122 µL, 3.00 eq.) were added, and the mixture was stirred and reacted at 20°C for 16 hours. LC-MS (EC21184-132-P1A, P1: RT = 0.425 minutes) showed that approximately 55.6% of the target product was generated. The reaction mixture was filtered and concentrated under reduced pressure to obtain a crude product, which was further purified by Prep-HPLC (HCl conditions; chromatographic column: CD02-Waters Xbidge BEH C18 150*25*10µm; mobile phase: [water (HCl)-ACN]; gradient elution: 30%-60% B, within 10 minutes) to provide the product Compound H102-5 (25.0 mg, 18.2 µmol, 7.83% yield, 96.1% purity) as a white solid.

**LC-MS:** EC21184-132-P1A, P1: RT = 0.425 min, m/z = 659.9 [M/2+H]⁺

### 5. Synthesis of Compound APH102

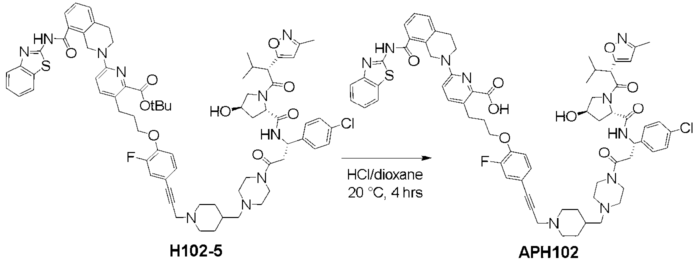

Compound H102-5 (25.0 mg, 19.0 µmol, 1.00 eq.) was mixed in HCl/dioxane (10.0 mL), and the mixture was stirred and reacted at 20 °C under N₂ atmosphere for 4 hours. LC-MS (EC21184-142-p1c, P1: RT = 2.008 minutes) showed that approximately 79.5% of the target product was generated. The reaction mixture was filtered and concentrated under reduced pressure to obtain a crude product, which was further purified by Prep-HPLC (neutral conditions; chromatographic column: CD02-Waters Xbidge BEH C18 150*25*10µm; mobile phase: [water (NH₄HCO₃)-ACN]; gradient elution: 32%-62% B, within 10 minutes) to provide the product Compound APH102 (7.36 mg, 5.73 µmol, 30.2% yield, 98.1% purity) as a white solid.

**LC-MS:** EC21184-142-p1c, P1: RT = 2.008 min, m/z = 631.8 [M/2+H]⁺
**¹H NMR:** EC21184-142-P1ZB (400 MHz DMSO) *δ* 12.32 - 13.04 (m, 1H), 8.37 - 8.76 (m, 1H), 8.04 (d, *J* = 7.6 Hz, 1H), 7.79 (d, *J =* 8.0 Hz, 1H), 7.60 (d, *J* = 7.2 Hz, 1H), 7.53 (d, *J =* 8.8 Hz, 1H), 7.44 - 7.50 (m, 1H), 7.39 - 7.43 (m, 1H), 7.23 - 7.38 (m, 8H), 7.13 - 7.20 (m, 1H), 7.06 - 7.12 (m, 1H), 6.94 (d, *J =* 8.8 Hz, 1H), 6.09 - 6.24 (m, 1H), 5.08 - 5.17 (m, 1H), 4.92 (s, 2H), 4.20 - 4.32 (m, 2H), 4.02 (br t, *J =* 6.4 Hz, 2H), 3.86 (t, *J =* 6.0 Hz, 2H), 3.66 - 3.76 (m, 2H), 3.48 (s, 1H), 3.44 (s, 3H), 2.98 (t, *J =* 5.2 Hz, 2H), 2.75 - 2.83 (m, 6H), 2.09 - 2.30 (m, 12H), 2.06 (d, *J* = 6.4 Hz, 3H), 1.92 - 1.99 (m, 3H), 1.64 - 1.76 (m, 3H), 1.38 - 1.48 (m, 1H), 1.04 - 1.13 (m, 2H), 0.94 (d, *J =* 6.63 Hz, 3H), 0.73 - 0.80 (m, 3H).

### Synthesis of APH103:

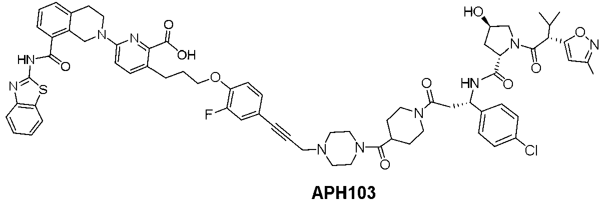

### Synthesis scheme:

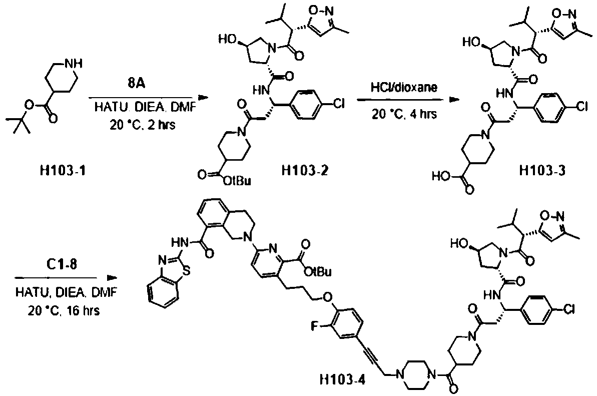

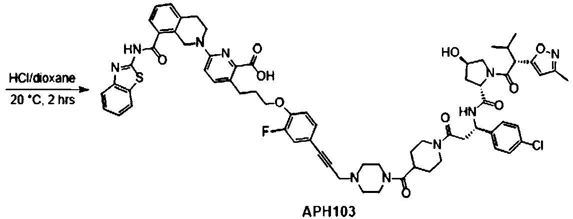

### 1. Synthesis of Compound H103-2

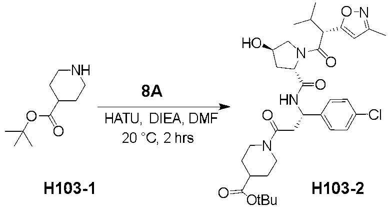

To a solution of Compound H103-1 (100 mg, 540 µmol, 1.00 eq.) in DMF (3.00 mL), Compound 8A (258 mg, 540 µmol, 1.00 eq.), HATU (246 mg, 648 µmol, 1.20 eq.) and DIEA (209 mg, 1.62 mmol, 282 µL, 3.00 eq.) were added, and the mixture was stirred and reacted at 20°C for 2 hours. LC-MS (EC21184-116-P1A, P1: RT = 0.437 minutes) showed that approximately 73.1% of the target product was generated. The reaction mixture was filtered and concentrated under reduced pressure to obtain a crude product, which was further purified by Prep-HPLC (FA conditions; chromatographic column: CD19-Daisogel SP-100-8-ODS-PK 200*50*10µm; mobile phase: [water (NH₄HCO₃)-ACN]; gradient elution: 47%-77% B, within 10 minutes) to provide the product Compound H103-2 (150 mg, 232 µmol, 43.0% yield, 100% purity) as a white solid.

**LC-MS:** EC21184-116-P1A, P1: RT = 0.437 min, m/z = 645.2 [M+H]⁺
**¹H NMR**: EC21184-116-p1cc (400 MHz DMSO) *δ* 8.31 - 8.74 (m, 1H), 7.25 - 7.40 (m, 4H), 6.10 - 6.25 (m, 1H), 5.09 - 5.18 (m, 1H), 4.96 - 5.08 (m, 1H), 4.20 - 4.33 (m, 2H), 4.05 - 4.19 (m, 1H), 3.72 (d, *J* = 9.6 Hz, 2H), 3.38 (d, *J =* 11.2 Hz, 1H), 2.94 - 3.10 (m, 1H), 2.81 - 2.93 (m, 1H), 2.78 (d, *J =* 6.4 Hz, 1H), 2.61 - 2.70 (m, 1H), 2.34 - 2.46 (m, 1H), 2.21 - 2.30 (m, 1H), 2.15 - 2.21 (m, 3H), 1.95 (t, *J= 9.2* Hz, 1H), 1.65 - 1.79 (m, 3H), 1.38 (d, *J =* 1.2 Hz, 9H), 1.04 - 1.37 (m, 3H), 0.95 (d, *J=* 6.4 Hz, 3H), 0.73 - 0.80 (m, 3H).

### 2. Synthesis of Compound H103-3

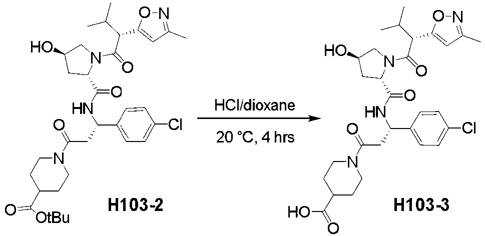

Compound H103-2 (150 mg, 232 µmol, 1.00 eq.) was mixed in HCl/dioxane (5.00 mL), and the mixture was stirred and reacted at 20°C for 4 hours. LC-MS (EC21184-128-P1B, P1: RT = 0.344 minutes) showed that approximately 91.8% of the target product was generated. The reaction mixture was filtered and concentrated under reduced pressure to provide the product Compound H103-3 (150 mg, crude) as a colorless oil.

**LC-MS:** EC21184-128-P1B, P1: RT = 0.344 min, m/z = 589.2 [M+H]⁺

### 3. Synthesis of Compound H103-4

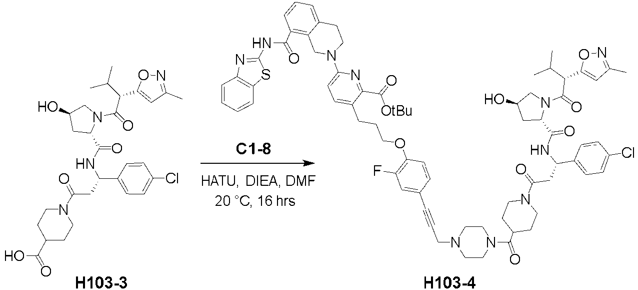

To a solution of Compound C1-8 (103 mg, 136 µmol, 1.00 eq.) and Compound H103-3 (80.0 mg, 136 µmol, 1.00 eq.) in DMF (1.00 mL), HATU (77.5 mg, 204 µmol, 1.50 eq.) and DIEA (52.6 mg, 407 µmol, 70.9 µL, 3.00 eq.) were added, and the mixture was stirred and reacted at 20°C for 16 hours. LC-MS (EC21184-131-P1A, P1: RT = 0.460 minutes) showed that approximately 42.8% of the target product was generated. The reaction mixture was filtered and concentrated under reduced pressure to obtain a crude product, which was further purified by Prep-HPLC (FA conditions; chromatographic column: CD03-Welch Xtimate C18 150*25*5µm; mobile phase: [water (FA)-ACN]; gradient elution: 47%-77% B, within 10 minutes) to provide the product Compound H103-4 (50.0 mg, 34.7 µmol, 25.5% yield, 92.4% purity) as a white solid.

**LC-MS:** EC21184-131-P1A, P1: RT = 0.460 min, m/z = 666.5 [M/2+H]⁺.

### 4. Synthesis of Compound APH103

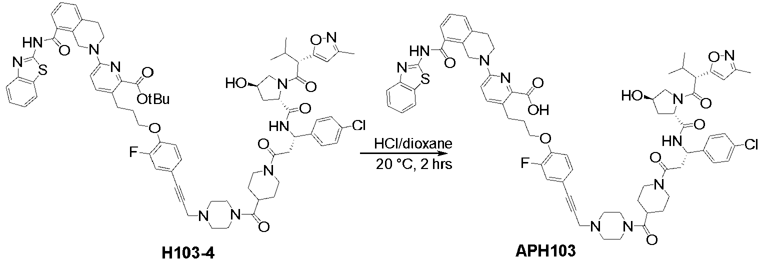

Compound H103-4 (50.0 mg, 37.5 µmol, 1.00 eq.) was mixed in HCl/dioxane (2.00 mL), and the mixture was stirred and reacted at 20 °C under N₂ atmosphere for 2 hours. LC-MS (EC21184-137-p1b, P1: RT = 1.873 min, R1: RT = 2.703 minutes) showed that approximately 36.5% of the target product was generated. The reaction mixture was filtered and concentrated under reduced pressure to obtain a crude product, which was further purified by Prep-HPLC (neutral conditions; chromatographic column: CD02-Waters Xbidge BEH C18 150*25*10µm; mobile phase: [water (NH₄HCO₃)-ACN]; gradient elution: 32%-62% B, within 10 minutes) to provide the product Compound APH103 (16.0 mg, 11.3 µmol, 30.1% yield, 90.2% purity) as a white solid.

**LC-MS:** EC21184-137-p1b, P1: RT = 1.873 min, m/z = 638.7 [M/2+H]⁺
**¹H NMR:** EC21184-137-p1a (400 MHz DMSO) *δ* 8.34 - 8.75 (m, 1H), 8.03 (d, *J =* 7.6 Hz, 1H), 7.79 (d, *J* = 8.0 Hz, 1H), 7.60 (d, *J =* 7.6 Hz, 1H), 7.52 (d, *J =* 8.8 Hz, 1H), 7.47 (t, *J =* 7.6 Hz, 1H), 7.38 - 7.42 (m, 1H), 7.24 - 7.38 (m, 7H), 7.14 - 7.20 (m, 1H), 7.06 - 7.13 (m, 1H), 6.92 (d, *J* = 8.8 Hz, 1H), 6.21 (s, 1H), 5.08 - 5.19 (m, 1H), 4.92 (s, 2H), 4.13 - 4.38 (m, 3H), 4.01 (t, *J =* 6.0 Hz, 2H), 3.85 (t, *J =* 5.6 Hz, 3H), 3.69 - 3.74 (m, 1H), 3.42 - 3.61 (m, 7H), 3.36 - 3.42 (m, 4H), 2.97 (t, *J* = 5.6 Hz, 3H), 2.71 - 2.90 (m, 5H), 2.40 - 2.46 (m, 3H), 2.21 - 2.29 (m, 1H), 2.15 - 2.20 (m, 3H), 1.91 - 2.01 (m, 3H), 1.68 - 1.79 (m, 1H), 1.53 - 1.64 (m, 2H), 1.18 - 1.36 (m, 2H), 0.94 (d, *J =* 6.4 Hz, 3H), 0.70 - 0.81 (m, 3H).

### Synthesis of APH104:

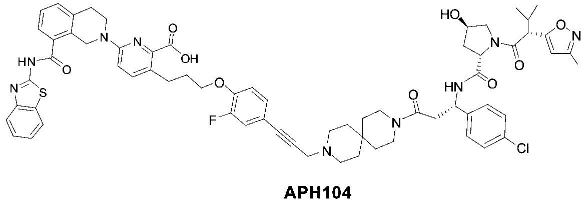

### Synthesis scheme:

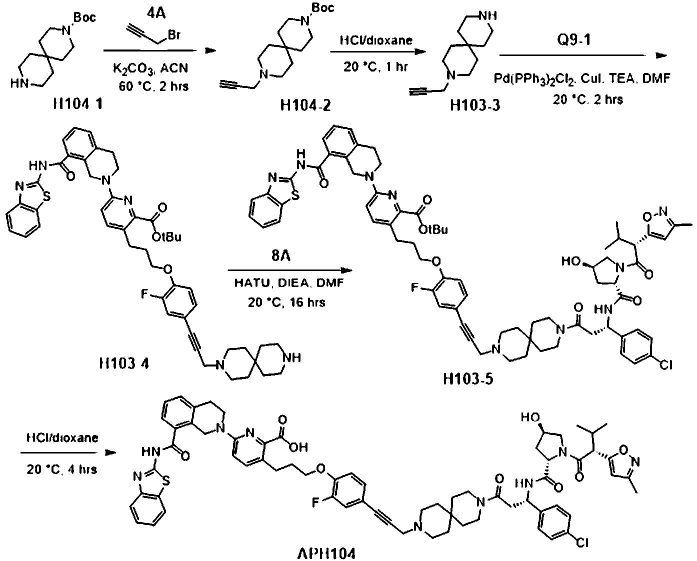

### 1. Synthesis of Compound H104-2

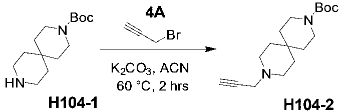

To a solution of Compound H104-1 (1.20 g, 4.72 mmol, 1.00 eq.) in ACN (12.0 mL), Compound 4A (842 mg, 5.66 mmol, 610 µL, 1.20 eq.) and K₂CO₃ (1.96 g, 14.2 mmol, 3.00 eq.) were added, and the mixture was stirred and reacted at 60°C for 2 hours. The reaction mixture was filtered and concentrated under reduced pressure to obtain a crude product, which further purified by column chromatography (SiO₂, DCM/methanol =100/0 to 88/12) to provide the product Compound H104-2 (900 mg, crude) as a brown gel.

¹H NMR: EC21184-78-P1GZ (400 MHz chloroform) *δ* 3.34 - 3.40 (m, 6H), 2.60 (s, 4H), 2.29 (s, 1H), 1.61 (s, 4H), 1.46 (s, 13H)

### 2. Synthesis of Compound H104-3

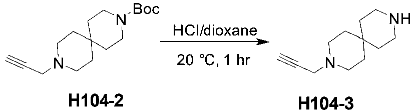

Compound H104-2 (900 mg, 3.08 mmol, 1.00 eq.) was mixed in HCl/dioxane (10.0 mL), and the mixture was stirred and reacted at 20 °C under N₂ atmosphere for 1 hour. The reaction mixture was concentrated under reduced pressure to provide the product Compound H104-3 (600 mg, crude) as a white solid.

### 3. Synthesis of Compound H104-4

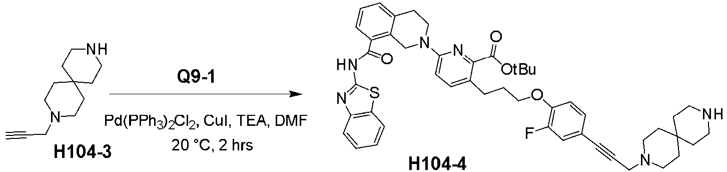

Compound H104-3 (600 mg, 3.12 mmol, 1.00 eq.), Compound Q9-1 (716 mg, 936 µmol, 0.30 eq.), Pd(PPh₃)₂Cl₂ (219 mg, 312 µmol, 0.10 eq.) and CuI (29.7 mg, 156 µmol, 0.05 eq.) were mixed in TEA (6.00 mL) and DMF (2.00 mL), and the mixture was stirred and reacted at 20 °C under N₂ atmosphere for 2 hours. The reaction mixture was filtered and concentrated under reduced pressure to obtain a crude product, which was further purified by Prep-HPLC (HCl conditions; chromatographic column: CD05-Phenomenex luna C18 150*40*10µm; mobile phase: [water (HCl)-ACN]; gradient elution: 21%-51% B, within 10 minutes) to provide the product Compound H104-4 (320 mg, crude) as a white solid.

LC-MS: EC21184-86-p1a, P1: RT = 0.410 min, m/z = 829.4 [M+H]⁺.

### 4. Synthesis of Compound H104-5

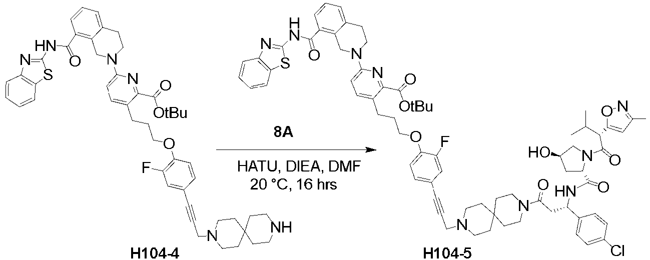

To a solution of Compound H104-4 (200 mg, 241 µmol, 1.00 eq.) in DMF (2.00 mL), Compound 8A (115 mg, 241 µmol, 1.00 eq.), HATU (138 mg, 362 µmol, 1.50 eq.) and DIEA (93.5 mg, 724 µmol, 126 µL, 3.00 eq.) were added, and the mixture was stirred and reacted at 20°C for 16 hours. The reaction mixture was concentrated under reduced pressure to obtain a crude product, which was further purified by Prep-HPLC (HCl conditions; chromatographic column: CD02-Waters Xbidge BEH C18 150*25*10µm; mobile phase: [water (HCl)-ACN]; gradient elution: 35%-65% B, within 10 minutes) to provide the product Compound H104-5 (25.0 mg, 18.4 µmol, 7.61% yield, 94.7% purity) as a white solid.

**LC-MS:** EC21184-133-P1A, P1: RT = 0.458 min, m/z = 645.3 [M/2+H]⁺.

### 5. Synthesis of Compound APH104

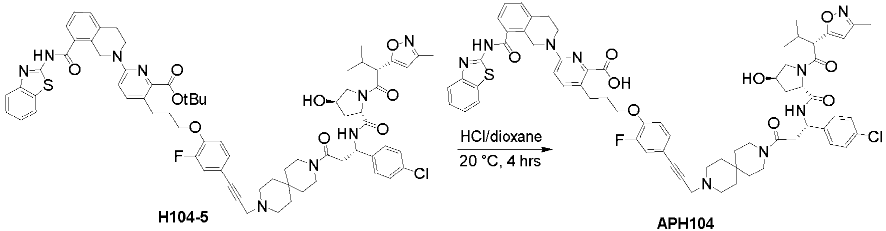

Compound H104-5 (25.0 mg, 19.4 µmol, 1.00 eq.) was mixed in HCl/dioxane (10.0 mL), and the mixture was stirred and reacted at 20 °C under N₂ atmosphere for 4 hours. The reaction mixture was concentrated under reduced pressure to obtain a crude product, which was further purified by Prep-HPLC (neutral conditions; chromatographic column: CD02-Waters Xbidge BEH C18 150*25*10µm; mobile phase: [water (NH₄HCO₃)-ACN]; gradient elution: 32%-62% B, within 10 minutes) to provide the product Compound APH104 (10.8 mg, 8.39 µmol, 43.2% yield, 95.1% purity) as a white solid.

**LC-MS:** EC21184-143-p1c, P1: RT = 2.013 min, m/z = 617.3 [M/2+H]⁺
**¹H NMR:** EC21184-143-P1ZB (400 MHz DMSO) *δ* 12.25 - 13.14 (m, 1H), 8.37 - 8.76 (m, 1H), 8.03 (d, *J* = 8.0 Hz, 1H), 7.79 (d, *J =* 8.0 Hz, 1H), 7.60 (d, *J* = 7.2 Hz, 1H), 7.53 (d, *J* = 8.8 Hz, 1H), 7.47 (t, *J* = 7.6 Hz, 1H), 7.39 - 7.43 (m, 1H), 7.28 - 7.37 (m, 6H), 7.23 - 7.27 (m, 1H), 7.14 - 7.18 (m, 1H), 7.06 - 7.13 (m, 1H), 6.93 (d, *J =* 8.8 Hz, 1H), 6.10 - 6.22 (m, 1H), 5.10 - 5.18 (m, 1H), 4.92 (s, 2H), 4.20 - 4.32 (m, 2H), 4.02 (t, *J =* 6.4 Hz, 2H), 3.86 (t, *J* = 5.6 Hz, 2H), 3.66 - 3.76 (m, 2H), 3.42 (d, *J* = 16.0 Hz, 6H), 2.98 (t, *J* = 5.2 Hz, 2H), 2.73 - 2.83 (m, 4H), 2.44 (s, 6H), 2.13 - 2.28 (m, 4H), 1.91 - 2.01 (m, 3H), 1.68 - 1.77 (m, 1H), 1.36 - 1.49 (m, 4H), 1.16 - 1.31 (m, 4H), 1.01 - 1.11 (m, 1H), 0.94 *(d, J=* 6.4 Hz, 3H), 0.72 - 0.80 (m, 3H).

### Synthesis of APH105:

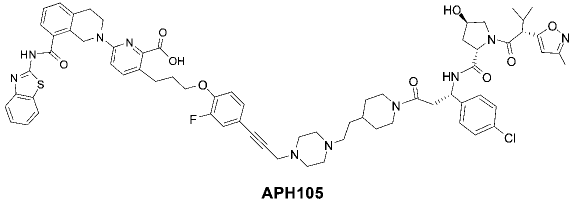

### Synthesis scheme:

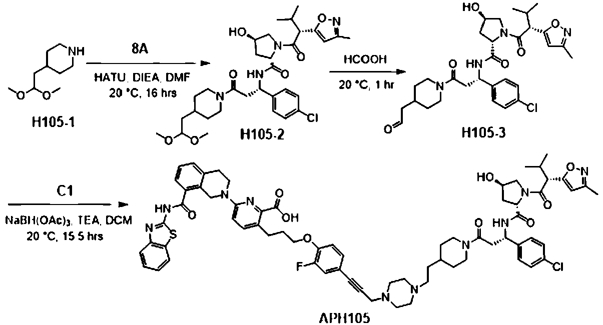

### 1. Synthesis of Compound H105-2

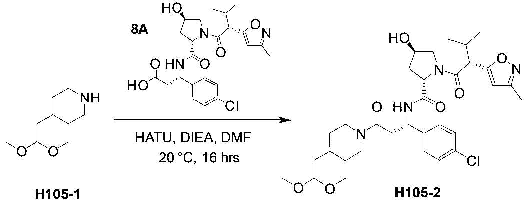

To a solution of Compound H105-1 (100 mg, 577 µmol, 1.00 eq.) in DMF (3.00 mL), Compound 8A (276 mg, 577 µmol, 1.00 eq.), HATU (329 mg, 865 µmol, 1.50 eq.) and DIEA (224 mg, 1.73 mmol, 302 µL, 3.00 eq.) were added, and the mixture was stirred and reacted at 20°C for 16 hours. The reaction mixture was concentrated under reduced pressure to obtain a crude product, which was further purified by Prep-HPLC (chromatographic column: CD02-Waters Xbidge BEH C18 150*25*10µm; mobile phase: [water (NH₄HCO₃)-ACN]; gradient elution: 26%-56% B, within 10 minutes) to provide the product Compound H105-2 (250 mg, 382 µmol, 66.1% yield, 96.6% purity) as a white solid.

**LC-MS:** EC21184-157-p1b, P1: RT = 0.584 min, m/z = 633.4 [M+H]⁺

### 2. Synthesis of Compound H105-3

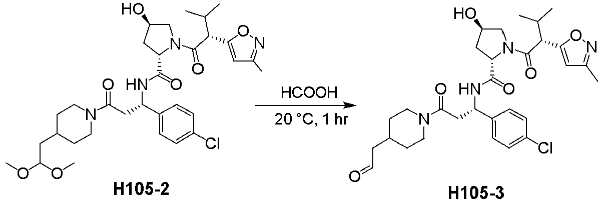

HCOOH (5.00 mL) was added to Compound H105-2 (250 mg, 395 µmol, 1.00 eq.), and the mixture was stirred and reacted at 20°C for 1 hour. LC-MS (EC21184-167-p1a, R1: RT = 0.404 min, P1: RT = 0.377 minutes) showed that approximately 77.8% of the target product was generated. The reaction mixture was concentrated under reduced pressure to provide the product Compound H105-3 (230 mg, crude) as a colorless oil.

**LC-MS**: EC21184-167-p1a, P1: RT = 0.377 min, m/z = 587.2 [M+H]⁺

### 3. Synthesis of Compound APH105

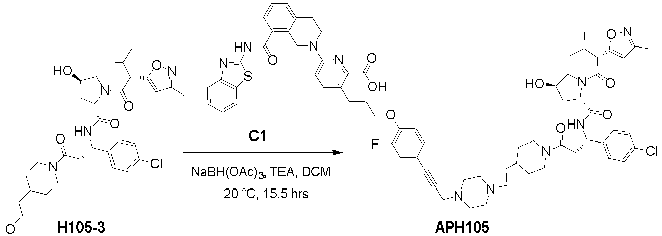

To a solution of Compound H105-3 (230 mg, 392 µmol, 1.00 eq.) and Compound C1 (221 mg, 313 µmol, 0.80 eq.) in DCM (3.00 mL), TEA (396 mg, 3.92 mmol, 545 µL, 10.0 eq.) was added, and the mixture was stirred and reacted at 20°C for 0.5 hour. Then, NaBH(OAc)₃ (249 mg, 1.18 mmol, 3.00 eq.) was added, and the mixture was stirred and reacted at 20°C for 15.5 hours. LCMS (EC21184-170-p1a, P1: RT = 0.453 minutes) showed that approximately 71.7% of the target product was generated. To the reaction mixture, water (2.00 mL) was added, and then concentrated under reduced pressure to obtain a crude product, which was further purified by Prep-HPLC (neutral conditions; chromatographic column: CD02-Waters Xbidge BEH C18 150*25*10µm; mobile phase: [water (NH₄HCO₃)-ACN]; gradient elution: 34%-54% B, within 13 minutes) to provide the product Compound APH105 (130 mg, 99.5 µmol, 25.4% yield, 97.3% purity) as a white solid.

**LC-MS:** EC21184-170-p1a, P1: RT = 0.453 min, m/z = 1276.6 [M+H]⁺
**¹H NMR:** EC21184-170-p1a (400 MHz DMSO) *δ* 12.17 - 13.16 (m, 1H), 8.40 (d, *J =* 7.2 Hz, 1H), 8.03 (d, *J* = 8.0 Hz, 1H), 7.79 (d, *J =* 7.6 Hz, 1H), 7.60 (d, *J =* 6.8 Hz, 1H), 7.53 (d, *J* = 8.8 Hz, 1H), 7.44 - 7.49 (m, 1H), 7.39 - 7.43 (m, 1H), 7.21 - 7.38 (m, 8H), 7.14 - 7.20 (m, 1H), 7.04 - 7.13 (m, 1H), 6.93 (d, *J =* 8.8 Hz, 1H), 6.20 (s, 1H), 5.06 - 5.18 (m, 1H), 4.92 (s, 2H), 4.17 - 4.34 (m, 3H), 4.02 (t, *J* = 6.7 Hz, 2H), 3.86 (t, *J* = 6.0 Hz, 2H), 3.68 - 3.75 (m, 2H), 3.48 - 3.60 (m, 1H), 3.44 (s, 2H), 3.36 - 3.41 (m, 2H), 2.98 (t, *J =* 5.6 Hz, 2H), 2.63 - 2.92 (m, 6H), 2.31 - 2.44 (m, 7H), 2.21 - 2.30 (m, 4H), 2.14 - 2.20 (m, 4H), 1.92 - 2.00 (m, 3H), 1.68 - 1.76 (m, 1H), 1.53 - 1.63 (m, 2H), 1.40 - 1.47 (m, 1H), 1.20 - 1.36 (m, 3H), 0.95 (d, *J=* 6.4 Hz, 3H), 0.76 (d, *J =* 6.4 Hz, 3H).

### Synthesis of APH106:

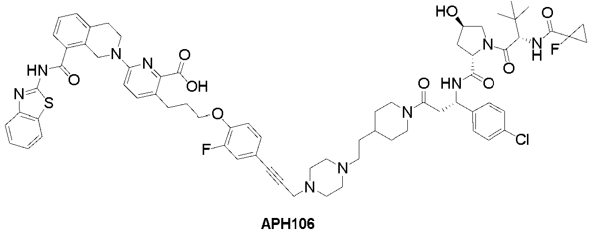

### Synthesis scheme:

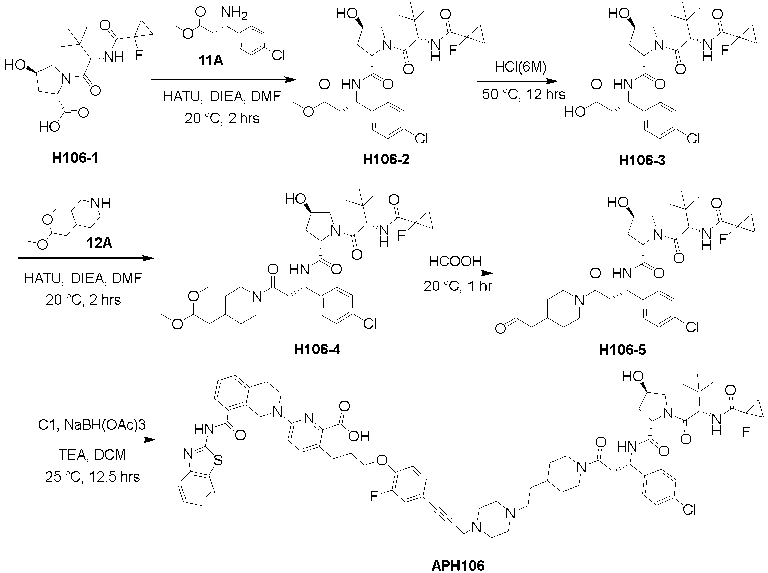

### 1. Synthesis of Compound H106-2

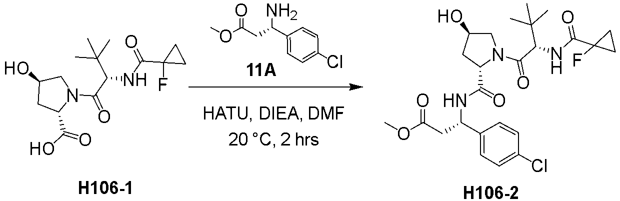

Compound H106-1 (400 mg, 1.21 mmol, 1.00 eq.), Compound 11A (259 mg, 1.21 mmol, 1.00 eq.), HATU (690 mg, 1.82 mmol, 1.50 eq.) and DIEA (469 mg, 3.63 mmol, 632 µL, 3.00 eq.) were mixed in DMF (6.00 mL), and the mixture was stirred and reacted at 20°C for 2 hours. LCMS (EC17015-495-P1A, P1: RT = 0.392 minutes) showed that 71.4% of the target product was generated. The reaction mixture was filtered and concentrated under reduced pressure to obtain a crude product. The crude product obtained by combining several batches was purified by reverse-phase HPLC (0.1% FA conditions) to provide the product Compound H106-2 (570 mg, 71.6% yield) as a white solid.

**LC-MS:** EC17015-495-P1A, product: RT = 0.392 min, m/z = 526.2 [M+H]⁺
**¹H NMR:** EC17015-495-P1A (400 MHz DMSO) *δ* ppm 8.56 (d, *J* = 8.0 Hz, 1H), 7.29 - 7.39 (m, 4H), 7.27 (dd, *J* = 9.2, 2.4 Hz, 1H), 5.09 - 5.18 (m, 2H), 4.56 (d, *J= 9.2* Hz, 1H), 4.39 (t, *J=* 8.4 Hz, 1H), 4.25 (s, 1H), 3.51 - 3.63 (m, 4H), 2.71 - 2.89 (m, 2H), 1.99 (dd, *J =* 12.0, 7.6 Hz, 1H), 1.68 (ddd, *J =* 12.8, 8.8, 4.4 Hz, 1H), 1.29 - 1.43 (m, 2H), 1.21 (dd, *J =* 8.4, 2.8 Hz, 2H), 0.88 - 1.04 (m, 9H).

### 2. Synthesis of Compound H106-3

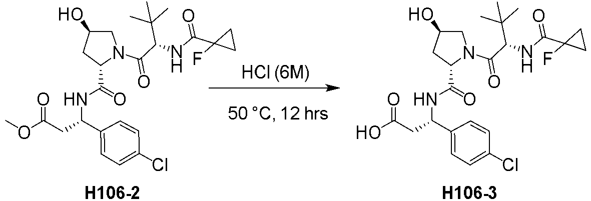

Compound H106-2 (200 mg, 380 µmol, 1.00 eq.) was mixed in HCl (1.00 mL) and H₂O (1.00 mL), and the mixture was stirred and reacted at 50°C for 12 hours. LCMS (EC17015-502-P1A, P1: RT = 0.355 minutes) showed that 60.3% of the target product was generated. The reaction mixture was filtered and concentrated under reduced pressure to obtain a crude product, which was further purified by Prep-HPLC (chromatographic column: CD06-Waters Xbidge C18 150*40*10 µm; mobile phase: [water (HCl)-ACN]; gradient elution: 20%-50% B, within 10 minutes) to provide the product Compound H106-3 (96.0 mg, 188 µmol, 49.3% yield) as a white solid.

**LC-MS:** EC17015-502-P1A, product: RT = 0.355 min, m/z = 512.2 [M+H]⁺
**¹H NMR:** EC17015-502-P1A (400 MHz DMSO) *δ* ppm 11.94 - 12.59 (m, 1H), 8.51 (d, *J* = 7.6 Hz, 1H), 6.99 - 7.47 (m, 5H), 5.06 (q, *J* = 7.6 Hz, 1H), 4.56 (d, *J* = 9.2 Hz, 1H), 4.40 (t, *J =* 8.4 Hz, 1H), 4.15 - 4.29 (m, 1H), 3.50 - 3.69 (m, 3H), 2.73 - 2.82 (m, 1H), 2.63 (dd, *J =* 15.6, 8.0 Hz, 1H), 1.94 - 2.05 (m, 1H), 1.67 (ddd, *J* = 12.8, 8.8, 4.4 Hz, 1H), 1.29 - 1.43 (m, 2H), 1.15 - 1.26 (m, 2H), 0.73 - 1.03 (m, 9H).

### 3. Synthesis of Compound H106-4

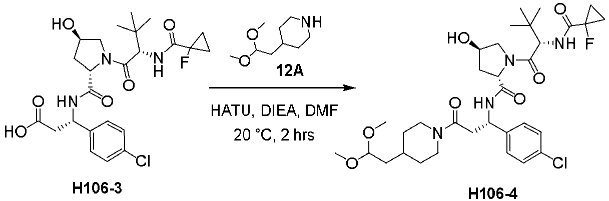

Compound H106-3 (85.0 mg, 166 µmol, 1.00 eq.), Compound 12A (43.2 mg, 249 µmol, 1.50 eq.), HATU (94.7 mg, 249 µmol, 1.50 eq.) and DIEA (64.4 mg, 498 µmol, 86.8 µL, 3.00 eq.) were mixed in DMF (1.00 mL), and the mixture was stirred and reacted at 20°C for 2 hours. LCMS (EC17015-513-P1A, P1: RT = 0.433 minutes) showed that approximately 54.0% of the target product was generated. The reaction mixture was filtered and concentrated under reduced pressure to obtain a crude product, which was further purified by Prep-HPLC (chromatographic column: CD01-Phenomenex luna C18 150*25*10 µm; mobile phase: [water (FA)-ACN]; gradient elution: 34%-64% B, within 8 minutes) to provide the product Compound H106-4 (80.0 mg) as a white solid.

**LC-MS:** EC17015-513-P1A, product: RT = 0.433 min, m/z = 667.4 [M+H]⁺.

### 4. Synthesis of Compound H106-5

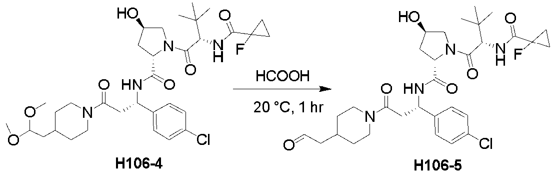

Compound H106-4 (65.0 mg, 97.4 µmol, 1.00 eq.) was mixed in HCOOH (1.00 mL), and the mixture was stirred and reacted at 20°C for 1 hour. LCMS (EC17015-526-P1A, P1: RT = 0.403 minutes) showed that approximately 65.6% of the target product was generated. The reaction mixture was filtered and concentrated under reduced pressure to provide the product Compound H106-5 (60.0 mg, crude) as a colorless oil, which was directly used in the next reaction without purification.

**LC-MS:** EC17015-526-P1A, product: RT = 0.403 min, m/z = 621.3 [M+H]⁺.

### 5. Synthesis of Compound APH106

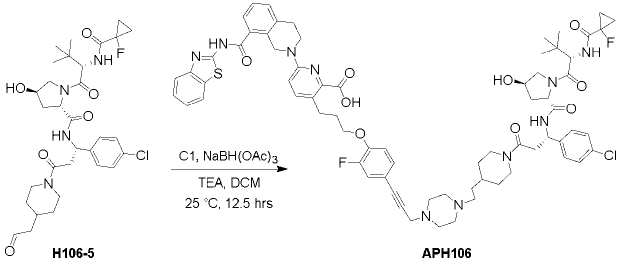

To a solution of Compound C1 (54.5 mg, 77.3 µmol, 0.80 eq.) in DCM (2.00 mL), TEA (97.8 mg, 966 µmol, 134 µL, 10.0 eq.) and Compound H106-5 (60.0 mg, 96.6 µmol, 1.00 eq.) were added, and the mixture was stirred and reacted at 25°C for 0.5 hours. Then, NaBH(OAc)₃ (61.4 mg, 290 µmol, 3.00 eq.) was added, and the mixture was stirred and reacted at 25 °C for 12 hours. LCMS (EC17015-527-P1B, P1: RT = 1.510 minutes) showed that approximately 41.3% of the target product was generated. The reaction mixture was diluted by adding a saturated NaHCO₃ solution (10.0 mL), and extracted with DCM (20.0 mL * 3). The resulting organic layer was washed with saturated saline (20.0 mL), dried over Na₂SO₄, filtered and concentrated under reduced pressure to obtain a crude product, which was further purified by Prep-HPLC (chromatographic column: CD02-Waters Xbidge BEH C18 150*25*10 µm; mobile phase: [water (NH₄HCO₃)-ACN]; gradient elution: 25%-55% B, within 10 minutes) to provide the product Compound APH106 (46.0 mg, 34.9 µmol, 36.1% yield, 99.3% purity) as a white solid.

**LC-MS:** EC17015-527-P1B, product: RT = 1.510 min, m/z = 1309.9 [M+H]⁺
**¹H NMR:** EC17015-527-P1A (400 MHz DMSO) *δ* ppm 8.48 (t, *J =* 7.6 Hz, 1H), 8.04 (d, *J* = 7.6 Hz, 1H), 7.79 (d, *J* = 8.0 Hz, 1H), 7.61 (d, *J* = 7.2 Hz, 1H), 7.52 (d, *J* = 8.8 Hz, 1H), 7.48 *(t, J=* 7.6 Hz, 1H), 7.39 - 7.43 (m, 1H), 7.31 - 7.37 (m, 4H), 7.27 (dd, *J =* 11.6, 9.2 Hz, 2H), 7.17 (d, *J =* 8.8 Hz, 1H), 7.06 - 7.12 (m, 1H), 6.92 (d, *J* = 8.8 Hz, 1H), 5.04 - 5.21 (m, 1H), 4.92 (s, 2H), 4.57 (d, *J* = 9.2 Hz, 1H), 4.38 - 4.48 (m, 1H), 4.27 (s, 2H), 4.02 (t, *J* = 6.4 Hz, 2H), 3.86 *(t, J=* 5.6 Hz, 2H), 3.66 - 3.81 (m, 2H), 3.51 - 3.66 (m, 3H), 3.45 (s, 2H), 3.17 - 3.41 (m, 3H,) 2.98 (t, *J =* 5.2 Hz, 2H), 2.64 - 2.93 (m, 6H), 2.53 - 2.61 (m, 2H), 2.34 - 2.45 (m, 3H), 2.27 (dd, *J =* 11.6, 6.0 Hz, 2H), 2.08 (s, 1H), 1.91 - 2.05 (m, 3H), 1.67 - 1.79 (m, 1H), 1.51 - 1.65 (m, 2H), 1.16 - 1.47 (m, 7H), 0.85 - 1.07 (m, 9H).

### Example 8. Biological activity test

### Cell culture and amplification

Molt-4 cells were purchased from Procell (CL-0160) and cultured in a RPMI-1640 medium containing 10% fetal bovine serum (FBS, Ausbian) and 1% penicillin/streptomycin (Gibco). HEF cells were derived from the isolation and amplification of human primary cells, which was approved by the Institute of Review Board in Peking University (IRB 00001052-15087) and conducted according to the approved protocol. Samples were collected from informed consent donors according to the ethical approval procedures of the China-Japanese Friendship Hospital. Human embryonic skin fibroblasts (HEF) and human embryonic lung fibroblasts (HELF) were obtained as previously described (Xie, B. et al. Cell Res. 29, 696-710 (2019)). Briefly, human embryonic skin tissue and fetal lung tissue obtained from aborted tissues of informed consent patients were minced with forceps and incubated in 1 mg/mL collagenase IV at 37°C for 1-2 hours. After enzymatic treatment, cells were collected by centrifugation and resuspended in HEF medium (Dulbecco's Modified Eagle Medium (DMEM, Gibco)) containing 10% fetal bovine serum (FBS, Ausbian), 1% GlutaMAX (Gibco), 1% non-essential amino acids (NEAA, Gibco) and 1% penicillin/streptomycin (Gibco). Cells were plated on 10 cm tissue culture dishes and grown in 10% FBS DMEM. MRC5 and BJ cells were purchased from ATCC and cultured in DMEM medium containing 10% fetal bovine serum (FBS, Ausbian) and 1% penicillin/streptomycin (Gibco).

### Molt-4 cell killing activity test

Molt-4 cells were cultured and amplified in 10 cm dishes by using RPMI-1640 medium supplemented with 10% fetal bovine serum. Actively growing and healthy Molt-4 cells were collected, centrifuged at 1000 rpm for 5 minutes, and after removing the supernatant, the cells were resuspended in fresh RPMI-1640 medium containing 10% fetal bovine serum. Cell counting was then performed. The cell density was adjusted to 120,000 cells per milliliter, and 100 µL of the cell suspension was added to each well of a 96-well transparent cell culture plate. It was noted that the edge wells of the 96-well plate were not used for testing; and 300 µL of PBS alone was added to these wells to reduce medium evaporation.

The small molecule compounds to be tested for activity were prepared. A series of small molecule concentration gradients (0.001, 0.003, 0.01, 0.03, 0.1, 0.3, 1, and 3 µM) were pre-set, and a series of 2× drug dilutions for the above concentrations were prepared in RPMI-1640 medium containing 10% fetal bovine serum. 100 µL of the above medium containing the gradient concentrations of the drug was added to each well of a 96-well plate pre-seeded with cells, so that the final drug concentration in each well was the pre-set concentration gradient. Three replicate wells were used for each tested drug concentration. The 96-well plate was placed back in a 37°C incubator, and the cell viability was measured after three days.

The plate was taken out three days after adding the drugs, and 100 µL of pre-prepared CCK-8 working solution was added to each well. The CCK-8 working solution was obtained through diluting a CCK-8 stock solution (C0041, Beyotime) by 10-fold volumn with a serum-free RPMI-1640 medium. The 96-well plates with the added CCK-8 working solution were placed back in the 37°C incubator and incubated for 0.5-2 hours. The plates were then taken out, and the absorbance value (OD value) of each well was measured at a wavelength of 450 nm, and the cell viability in each experimental group was calculated according to the following formula: Relative cell viability (%) = (Blank control group - Experimental group) / Blank control group × 100%.

Table 1 shows the activity test results of each compound for killing Molt-4 cells. As can be seen from Table 1, as the compounds of the present invention adopting different targeting ligands, linkers, and E3 ligase ligands, multiple PROTAC molecules have excellent activity for killing Bcl-xL-positive Molt-4 cells. Among them, the targeting ligand C1 exhibited a cellular activity between 10-100 nM, which was higher than that of the commercially available control molecule ABT-263, but lower than those of A-1331852 and A-1155463. Several PROTAC molecules designed based on C1 showed a significantly improved activity compared to the ligand C1, which was higher than those of ABT-263 and the known PROTAC molecule DT-2216, and achieved a good killing activity similar to that of A-1331852 and below 10 nM, including APQ2, APQ5a, APQ7a, APQ9, APQ10, APQ11, APQ10-2, APQ10-3, APQ902, APQ902a, APQ904, APH105, and the like. It is indicated that these structurally designed molecules can better exert the target protein degradation function of PROTACs, and thus possess the advantage in killing activity. Some other molecules showed an activity similar to that of C1, such as APQ3, BF3, BF4, APQ5b, APQ6b, APQ7b, APQ8, APL-1, APH1, APH7, APH106, and the like. Some of the designed molecules showed a lower killing activity or loss the function, such as APQ1, APQ4, AF3, AF4, APQ9-1, APQ10-1, and the like. It can be seen that several PROTAC molecules based on the A-1331852 targeting ligand exhibited a good cellular activity similar to those of the targeting ligand per se, such as APF2, APF3, APF4, APR1, APR2, APR3, APR4, and the site-specifically deuterated PROTAC molecule A13D6-PF3. Overall, multiple PROTAC molecules of the present invention achieved relatively ideal cell killing activity (below 10 nM), and exhibited a highly efficient killing ability for Bcl-xL-positive tumor cells.

**Table 1. Activity comparison of compounds for killing Molt-4 cells**

| **Compound Name** | **EC₅₀ (Molt-4)** | **Compound Name** | **EC₅₀ (Molt-4)** |
|---|---|---|---|
| APQ1 | NA | APF1 | ++ |
| APQ2 | +++ | APF2 | +++ |
| APQ3 | ++ | APF3 | +++ |
| APQ4 | NA | APF4 | +++ |
| AF3 | NA | APR1 | +++ |
| AF4 | NA | APR2 | +++ |
| BF3 | ++ | APR3 | +++ |
| BF4 | ++ | APR4 | +++ |
| APQ5a | +++ | APF1A | ++ |
| APQ5b | ++ | A13D6-PF3 | +++ |
| APQ6a | ++ | C1 | ++ |
| APQ6b | ++ | C4 | ++ |
| APQ7a | +++ | A-1331852 | +++ |
| APQ7b | ++ | A-1155463 | +++ |
| APQ8 | ++ | ABT-263 | + |
| APQ9 | +++ | DT-2216 | ++ |
| APQ10 | +++ | APL-1 | ++ |
| APQ11 | +++ | APL-2 | + |
| APH1 | ++ | APL-3 | NA |
| APH2 | ++ | APL-4 | NA |
| APH3 | + | APL-5 | NA |
| APH4 | + | APL-6 | + |
| APH5 | + | APQ9-1 | NA |
| APH6 | + | APQ9-2 | ++ |
| APH7 | ++ | APQ10-1 | NA |
| APH8 | + | APQ10-2 | +++ |
| APH9 | + | APQ10-3 | +++ |
| APH10 | ++ | APQ901 | +++ |
| APH11 | + | APQ902 | +++ |
| APH12 | NA | APQ902a | +++ |
| APH14 | NA | APQ903 | + |
| APH15 | NA | APQ904 | +++ |
| APH16 | + | APH101 | + |
| APH17 | NA | APH102 | ++ |
| APH18 | NA | APH103 | ++ |
| APH19 | + | APH104 | ++ |
| APH20 | NA | APH105 | +++ |
| APH21 | NA | APH106 | ++ |

| | | | |
|---|---|---|---|
| Cellular activity: +++ (EC₅₀ <10 nM), ++ (EC₅₀ of 10-100 nM), + (EC₅₀ of 100-1000 nM), NA (EC₅₀>1000 nM). | | | |

Noted: A-1331852, A-1155463, ABT-263, and DT-2216 are known compounds with the same target, in which DT-2216 is a PROTAC molecule that uses ABT-263 as a targeting ligand and recruits the VHL-E3 enzyme. All of the above molecules were purchased from MCE company. Information on the known commercially available control molecules is shown below:

| Compound | Structural Formula |
|---|---|
| A-1331852 | 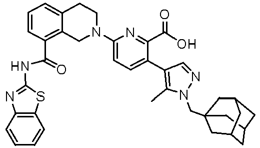 |
| A-1155463 | 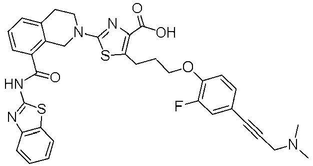 |
| ABT-263 | 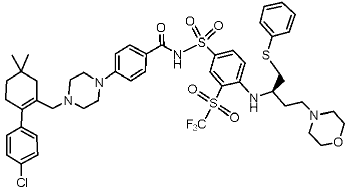 |
| DT-2216 | 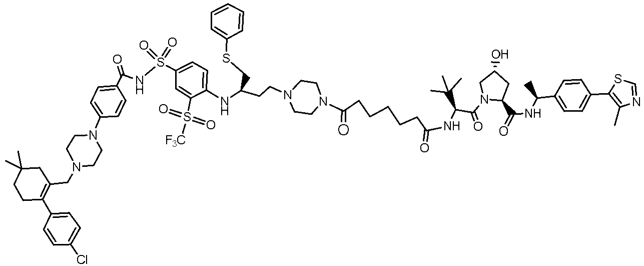 |

Figure 1 shows graphs of some compounds for killing Molt-4 cells. The experimental results show that, at an equal concentration, Compound C1 exhibited a higher percentage for killing Molt-4 cells than those of ABT-263 and DT-2216, but lower than those of A-1331852 and A-1155463 (Figure 1A). In this invention, A-1331852, which showed the highest cellular activity in the test, was used as a positive control molecule, and the cellular activity of PROTAC molecules with different linkers and E3 ligase ligands was compared with that of the positive control molecule. According to Figures 1B, 1C, 1D, 1G, and 1H, at different concentrations, the PROTAC molecules APQ2, APQ11, APQ9, APQ7a, APQ10-2, APQ10-3, APQ902, and APQ904 showed an activity for killing Molt-4 cells comparable to A-1331852, all of them are higher than those of the ligand molecule C1 as well as the commercially available molecules A-1151463, ABT-263, and DT-2216.

### Detection for the E3 enzyme-dependence of PROTAC molecules

Actively growing and healthy Molt-4 cells were collected, centrifuged at 1000 rpm for 5 minutes, and after removing the supernatant, the cells were resuspended in fresh RPMI-1640 medium containing 10% fetal bovine serum. Cell counting was then performed. The cell density was adjusted to 120,000 cells per milliliter, and 100 µL of the cell suspension was added to each well of a 96-well transparent cell culture plate. It was noted that the edge wells of the 96-well plate were not used for testing, and 300 µL of PBS alone was added to these wells to reduce medium evaporation.

Molt-4 cells were plated in duplicate according to the number of the tested small molecules, so as to test the effects with and without the addition of pomalidomide (a competitive ligand specific for E3 ligase), in which one half of the parallel cells was added 10 µM pomalidomide. A series of small molecule concentration gradients (0.001, 0.003, 0.01, 0.03, 0.1, 0.3, 1, and 3 µM) were pre-set, and a series of 2× drug dilutions for the above concentrated were prepared in DMEM medium containing 10% fetal bovine serum. 100 µL of the above DMEM medium containing the gradient concentrations of the drug was added to 96-well plates that have been pre-plated with cells, so that the final drug concentration in each well was the pre-set series of concentrations respectively. Three replicate wells were used for each test drug concentration. The 96-well plates were placed back in a 37°C incubator, and the cell viability was detected after three days.

Cell viability after drug treatment was detected by using the CCK-8 method: three days after adding the drugs, the plates were taken out, and the supernatant was discarded. Then, 100 µL of pre-prepared CCK-8 working solution was added to each well. The CCK-8 working solution was obtained through diluting a CCK-8 stock solution (C0041, Beyotime) by 10-fold volumn with a serum-free DMEM culture medium. The 96-well plates with the added CCK-8 working solution were placed back in the 37°C incubator and incubated for 0.5-2 hours. The plates were then taken out, and the absorbance value (OD value) of each well was measured at a wavelength of 450 nm. The cell viability in each experimental group was calculated according to the following formula: Relative cell viability (%) = (Blank control group - Experimental group) / Blank control group × 100%.

Figure 2 shows the experimental results. It can be seen that, the PROTAC molecules APQ2, BF3, BF4, APQ9, APQ10, APQ10-2, APQ902, and APQ904 showed significantly reduced cell killing activity after the addition of the CRBN-E3 ligase competitive binding ligand pomalidomide (+poma), compared to that without adding pomalidomide (-poma). In particular, at concentrations of 0.01, 0.03, 0.1, and 0.3 µM, with pomalidomide, the cell killing activity of these PROTAC molecules was reduced to almost completely loss. In contrast, the activity of the control molecules C1, A1331852, and ABT-263 having a non-PROTAC structure was only slightly affected by the addition of pomalidomide (+poma), and the cell killing activity of the PROTAC molecule DT-2216 that recruits the VHL-E3 enzyme was also almost unaffected by pomalidomide. Such results indicate that the designed and synthesized PROTAC molecules require recruitment of the CRBN-E3 enzyme and accordance to the action mechanism of PROTACs to exert the target protein degradation and cell killing functions.

### Cellular senescence induction

To avoid replication of the induced senescent cells, low-passage proliferating HEF cells (<10 passages), MRC5 cells (<6 passages), and BJ cells (<10 passages) were used as normal controls. For chemical drug induced senescence, HEF cells were treated with 10 µM etoposide (Selleck, S1225) for 36-48 hours, MRC5 cells were treated with 5 µM bleomycin (Selleck, S1225) for 72 hours, and BJ cells were treated with 10 µg/mL bleomycin for 72 hours. Three days after the treatment, the cells were subcultured in fresh culture medium at a 1:3 ratio for 72 hours before further analysis and use.

### Senescent cell killing activity test

Non-senescent and induced-senescent HEF cells, non-senescent and induced-senescent MRC5 cells, and non-senescent and induced-senescent BJ cells were respectively digested with trypsin, and centrifuged at 1000 rpm for 5 minutes. Then, the supernatant was removed, and the cells were resuspended in fresh DMEM culture medium containing 10% fetal bovine serum for counting. The cell density was adjusted so that the non-senescent cell density was 4000 cells/100 µL and the senescent cell density was 7000 cells/100 µL. 100 µL of the cell suspension was added to each well of a 96-well transparent cell culture plate. It was noted that the edge wells of the 96-well plate were not used as testing wells, and 300 µL of PBS alone was added to these wells to reduce evaporation of the culture medium. The 96-well plates with the well-plated cells were placed back in a 37°C incubator for overnight culture until the cells adhered. On the next day, the small molecule compounds to be tested were prepared and added after the cells had adhered.

A series of small molecule concentration gradients (0.01, 0.03, 0.1, 0.3, 1, 3, 10, and 25 µM) were pre-set, and a series of 2× drug dilutions for the above concentrations were prepared in DMEM culture medium containing 10% fetal bovine serum. 100 µL of the above DMEM culture medium containing the gradient concentrations of the drug was added to each well of the 96-well plate that had been well-plated with cells, so that the final drug concentration in each well was the pre-set series of concentrations. Three replicate wells were used for each test drug concentration. The 96-well plates were placed back in a 37°C incubator, and cell viability was measured after three days.

Three days after adding the drug, the plates were taken out, and the supernatant was discarded. 100 µL of pre-prepared CCK-8 working solution was added to each well. The CCK-8 working solution was obtained through diluting a CCK-8 stock solution (C0041, Beyotime) with a serum-free DMEM culture medium by 10-fold volumn. The 96-well plates containing the CCK-8 working solution were placed back in the 37°C incubator and incubated for 0.5-2 hours. The plates were then taken out, and the absorbance value (OD value) of each well was measured at a wavelength of 450 nm. The cell viability in each experimental group was calculated according to the following formula: Relative cell viability (%) = (Blank control group - Experimental group) / Blank control group × 100%.

Figure 3 shows the experimental results of some compounds for killing non-senescent and senescent HEF cells, non-senescent and senescent MRC5 cells, and non-senescent and senescent BJ cells. As can be seen from Figure 3, the PROTAC molecules APQ2, BF4, APQ9, and APQ10 could efficiently and selectively kill senescent HEF cells, and exhibited significant advantages in both activity and selectivity for killing senescent cells compared to the commercially available control molecules. In terms of the activity for killing senescent cells, the above PROTAC molecules were generally superior to the control molecule ABT-263 and similar to A-1331852. Among them, APQ2, APQ9, and APQ10 even showed a better activity for killing senescent cells at lower concentrations (0.01 µM, and 0.03 µM) than A-1331852, as shown in Figures 3A-3F. In terms of the selectivity for senescent cells, the control molecules ABT-263 and A-1331852 both showed toxic killing effects on normal non-senescent HEF cells at a higher concentration (25 µM). In contrast, the above PROTAC molecules showed a better selectivity for senescent cells, and exhibited no toxicity on normal non-senescent HEF cells at a higher concentration (25 µM), as shown in Figures 3A-3F. In addition, cellular activity tests of the selected PROTAC molecules were also conducted in a human embryonic lung fibroblast (MRC5) cell model, and similar results were observed, where the PROTAC molecules such as APQ9, APQ10, and APQ902 showed a better selectivity for senescent cells, and their activity for killing senescent cells at lower concentrations (0.01 µM, and 0.03 µM) was even superior to that of A-1331852, as shown in Figures 3G-3L. The killing activity of some selected PROTAC molecules such as APQ10, APQ902, and APQ10-3 were also tested in more senescent cell models. For example, in a senescent skin-derived BJ cell model, these three molecules exhibited highly selective and potent killing effects for senescent cells, without significant killing effect for the normal early-passage BJ cells, as shown in Figures 3M-3O, suggesting that these molecules possess good biological activity and safety. It was observed that the PROTAC molecules exhibited superior activity at lower concentrations, which was hypothetically mainly due to the catalytic degradation function of the PROTAC molecules. Unlike conventional small molecule inhibitors (ABT-263, and A-1331852) where a single molecule can only act on one target protein molecule, each PROTAC molecule could cyclically promote the formation of a complex by the target protein and E3 ligase to degrade many protein molecules. Thus, only a small amount of PROTAC molecules was needed in the cells to maintain the target protein degradation effect. Overall, the PROTAC molecules included in this invention demonstrated significant advantages in terms of the activity and selectivity for killing senescent cells.

### Degradation experiment of Bcl-xL target protein

Actively growing and healthy Molt-4 cells were subcultured into a fresh RPMI-1640 medium containing 10% fetal bovine serum, and each PROTAC molecule was added thereto at a final concentration of 100 nM. After drug treatment for the specified times (0, 2, 6, and 16 hours), the cells were collected, the supernatant was discarded by centrifugation, and the cells were washed twice with pre-cooled PBS buffer. Total protein was extracted with a lysis buffer (Servicebio, G2002-100ML), and the protein concentration was normalized by using a BCA protein quantification kit (Servicebio, G2026). Protein samples were mixed with a protein-loading buffer and incubated at 95°C for 5 minutes. Western blotting experiments were performed by using the following antibodies: Bcl-xL (Abcam, catalog number ab32370), diluted at 1:500; GAPDH antibody (Servicebio, catalog number GB15004), diluted at 1:1000; and horseradish peroxidase-conjugated goat anti-rabbit secondary antibody (Servicebio, catalog number GB23303), diluted at 1:5000.

Figure 4 shows the degradation results of the tested molecules for the target protein Bcl-xL. It can be seen that the PROTAC molecules APQ2, BF4, APQ9, APQ10, APQ10-2, APH1, APH7, APQ902, APQ904, APH105, and APH106 all had the ability to degrade the Bcl-xL protein, and the degradation degree of the target protein was increased over time. A significant decrease in the Bcl-xL protein level was observed earliest at 2 hours after adding the drug (see Figures 4A-4K), indicating that the tested PROTAC molecules effectively exerted their degradation function for the Bcl-xL target protein.

### In vivo platelet toxicity test in mice

The in vivo platelet toxicity test was performed by using 5-6 week old female C57BL/6J mice. The C57BL/6J mice were purchased from Beijing Vital River Laboratory Animal Technology Co., Ltd. and housed under specific pathogen-free (SPF) conditions, with a 12-hour light/12-hour dark cycle, and free access to food and water. The vehicle used for dissolving the drugs for administering was comprised of 85% physiological saline, 5% DMSO (Sigma, D8418), and 10% Solutol HS 15 (Sigma, 42966). Different doses of the molecules to be tested (e.g., 0.1, 0.5, 2.5 mg/kg or 2, 5, 20 mg/kg) were prepared in advance with the aforementioned vehicle, and administered to mice via intraperitoneal injection at a volume of 200 µL for each mouse. After administration was completed, approximately 50 µL of blood was collected from each mouse at different time points via the intra-canthus sampling method into EDTA anticoagulant tubes (RAM Scientific), then a routine analysis of blood was performed by using a hematology analyzer, and the platelets was counted.

Figure 5 shows the results of the in vivo platelet toxicity test. As can be seen from Figure 5A, no significant decrease in platelet number was observed in the three tested dose groups (0.1, 0.5, and 2.5 mg/kg) for APQ2, while the control molecule A-1331852 showed severe toxicity on platelets even at a dose of 0.5 mg/kg. The effects of the PROTAC molecules APQ9 and APQ10 as well as A-1331852 on peripheral blood platelets at a higher dose (5 mg/kg) were further tested and compared. The results showed that the administration of A-1331852 significantly decreased the platelet number in peripheral blood (decreased up to below 100 × 10⁹/L), while APQ9 and APQ10 only showed a slightly decrease in platelet number at the same dose (see Figure 5B). Next, the effect of APQ10 on platelets was tested at doses of 2, 5, and 20 mg/kg. The results showed that, compared to the A-1331852 group administered at equivalent doses, APQ10 at all tested doses exhibited a lower platelet toxicity, where a significant decrease in the platelet number was observed only at the highest dose (20 mg/kg), but the decrease level was lower than that observed for the A-1331852 group at a dose of 2 mg/kg (see Figure 5C). The above results indicated that, compared to the equally active Bcl-xL inhibitor A-1331852, the PROTAC molecules APQ2, APQ9, and APQ10 exhibited significantly lower platelet toxicity, demonstrating that the PROTAC molecules designed and synthesized in this invention had the effect of reducing the platelet toxicity of Bcl-xL inhibitors.

### Metabolic Stability Assay for Compounds in Liver Microsome

The metabolic reaction of compounds in liver microsome was performed in a system with a total volume of 400 µL, and the following steps below were sequentially performed for the reaction and detection: 1) 200 µL of phosphate buffer (200 mM), 106 µL of sterile ultrapure water, and 40 µL of magnesium chloride solution (50 mM) were added to each detection tube to prepare a reaction working solution. 2) Two reaction systems with or without adding NADPH were prepared for each compound to be tested. For the reaction system with adding NADPH: 10 µL of liver microsomes (20 mg/mL) and 40 µL of NADPH (10 mM) were added to the above working solution; for the reaction system without adding NADPH: 10 µL of liver microsomes (20 mg/mL) and 40 µL of sterile ultrapure water were added to the above working solution. The final concentration of liver microsomes in both reaction systems was 0.5 mg/mL. 3) 4 µL of the compound to be tested (100 mM) was added to each reaction tube, resulting in a final concentration of 1 µM for the compound in the reaction system. The detection tubes were placed at 37°C for reaction. 4) 50 µL samples were taken from each detection tube at 0, 15, 30, 45, and 60 minutes after adding the compound. The reaction was terminated by adding cold acetonitrile containing IS (100 nM alprazolam, 200 nM labetalol, 200 nM caffeine, and 2 µM ketoprofen) in a 4× volume. The samples were centrifuged at 3220 g for 40 minutes, 100 µL of the supernatant was mixed with 100 µL of ultrapure water, and a sample was taken for LC-MS/MS analysis.

Calculation of in vitro liver microsomal metabolic half-life and in vitro metabolic intrinsic clearance rate: The LC-MS detection results were analyzed to obtain the percentage of the remaining amount of the tested compound relative to the initial amount at each time point. The natural logarithm of the remaining percentage was linearly fitted against the incubation time. The slope k was calculated according to the fitting result, and the half-life is calculated according to the following formula: ${t}_{1 / 2} = \frac{0.693}{k}$

The in vitro metabolic intrinsic clearance rate was calculated according to the following formula: ${CL}_{int} = \frac{0.693}{{t}_{1 / 2}} \times \frac{Total volue for incubation \left(μL\right)}{Microsome content \left(mg\right)}$

Table 2 shows the experimental results for metabolic stability of the compounds in liver microsomes. The experimental results showed that, compared to the shorter half-life (26.12 minutes in humans and 75.21 minutes in mice) and the higher clearance rate (53.07 µL/min/mg in humans and 18.43 µL/min/mg in mice) for A-1331852 in in vitro liver microsome metabolism, Compound C1 and the PROTAC molecule APQ2 based on the C1 ligand of this invention exhibited a more superior metabolic stability in liver microsomes. The metabolic half-lives of C1 and APQ2 were significantly longer, and their clearance rates were lower: C1 had metabolic half-lives of 188.38 minutes in human and 282.82 minutes in mice respectively, and metabolic clearance rates of 7.36 µL/min/mg in human and 4.90 µL/min/mg in mice respectively, both being superior to those of A-1331852. APQ2 had metabolic half-lives of 500.6 minutes in human and 322.26 minutes in mice respectively, and metabolic clearance rates of 2.77 µL/min/mg in human and 4.30 µL/min/mg in mice respectively, both being superior to those of A-1331852. In addition, by comparing A-1331852 and its modified molecules with deuteration at different sites, the results showed that the deuteration-modified molecule A13D-6 had a better metabolic stability in liver microsomes, suggesting that deuterated modification at this site may be took as an effective method for further improving the in vivo metabolic stability of similar molecules (including the PROTAC molecules of the present invention).

**Table 2. The metabolic stability of synthesized compounds in human and mouse liver microsomes**

| **Compounds** | **Species** | **T_{1/2} (minutes)** | **Cl**_{**in**t} **(µL/min/mg protein)** |
|---|---|---|---|
| Verapamil | human | 10.73 | 129.20 |
| | mouse | 5.61 | 247.04 |
| A-1331852 | human | 26.12 | 53.07 |
| | mouse | 75.21 | 18.43 |
| C1 | human | 188.38 | 7.36 |
| | mouse | 282.82 | 4.90 |
| A13D-1 | human | 5.88 | 235.82 |
| | mouse | 5.19 | 266.89 |
| A13D-2 | human | 25.06 | 55.31 |
| | mouse | 76.36 | 18.15 |
| A13D-5 | human | 25.88 | 53.56 |
| | mouse | 78.69 | 17.61 |
| A13D-6 | human | 27.62 | 50.18 |
| | mouse | 98.52 | 14.07 |
| APQ2 | human | 500.60 | 2.77 |
| | mouse | 322.26 | 4.30 |

## Claims

1. A compound of formula I, or pharmaceutically acceptable salts, esters, prodrugs, solvates, stereoisomers or deuterated compounds thereof:
A-L-E (I)
wherein moiety A is a small molecule ligand moiety targeting the Bcl-xL protein, moiety E is a small molecule ligand moiety targeting an E3 ubiquitin ligase complex, and moiety L is a linker,
moiety A has the structure of formula 1 or 2: 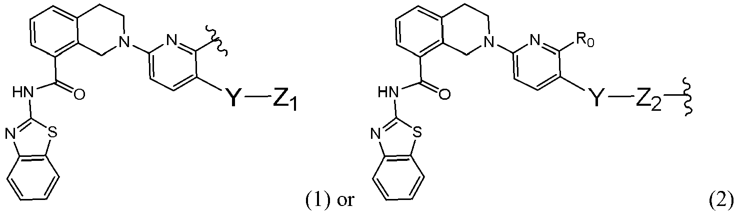
wherein R₀ is absent or selected from: -COOR^{a}, -CONR^{a}R^{b}, -SO₃R^{a}, -SO₂NR^{a}R^{b}, -SO₂NR^{a}COR^{b}, - CONR^{a}SO₂R^{b}, -COSO₂NR^{a}R^{b}, 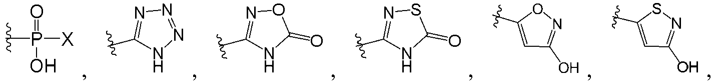 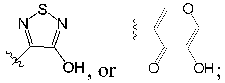
X is selected from hydrogen, hydroxyl, amino, or CH(OR^{a})₂;
R^{a} and R^{b} are independently selected from hydrogen, cyano, substituted or unsubstituted alkyl, or substituted or unsubstituted alkoxy;
Y is absent or selected from substituted or unsubstituted alkylene, substituted or unsubstituted alkyleneoxy, substituted or unsubstituted alkenylene, substituted or unsubstituted alkenyleneoxy, substituted or unsubstituted arylene, substituted or unsubstituted heteroarylene, substituted or unsubstituted cycloalkylene, or substituted or unsubstituted heterocyclylene;
Z₁ and Z₂ are independently absent or selected from substituted or unsubstituted -alkylene-cyclic group, substituted or unsubstituted cycloalkyl, substituted or unsubstituted heterocyclyl, substituted or unsubstituted aryl, or substituted or unsubstituted heteroaryl;
moiety E is selected from the following structures: 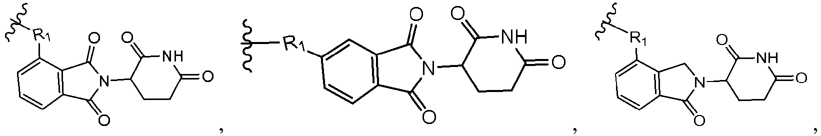 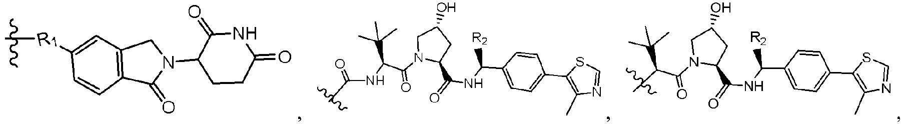 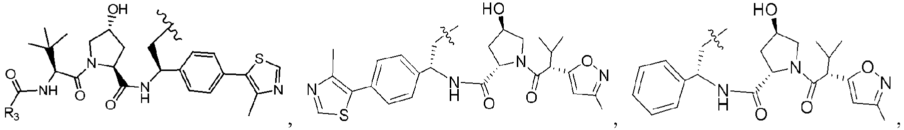 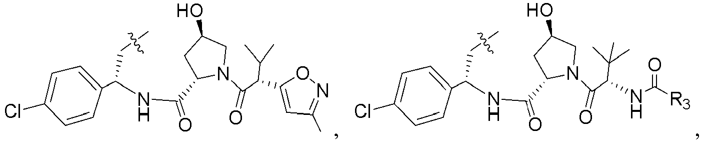 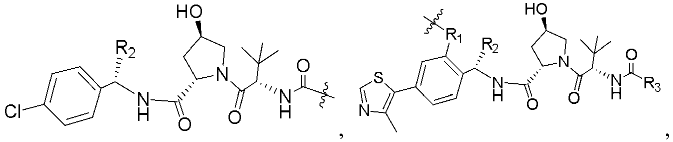 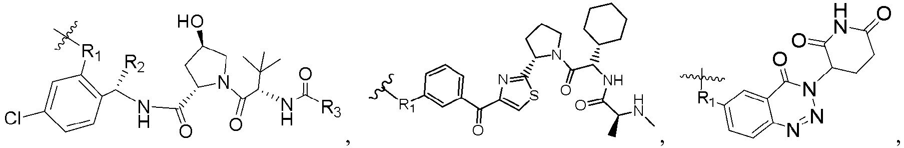 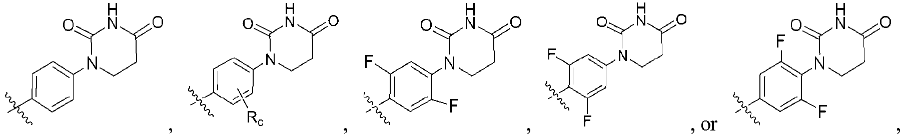
wherein R₁ is O, NH or absent; R₂ is H or methyl; R₃ is 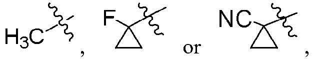 and R_{c} is F, methoxy, Cl, or cyano;
moiety L has the following structure: 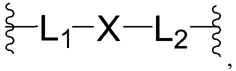
wherein the moiety L is linked to the moiety A at L₁, and is linked to the moiety E at L₂,
L₁ and L₂ are independently absent or selected from: 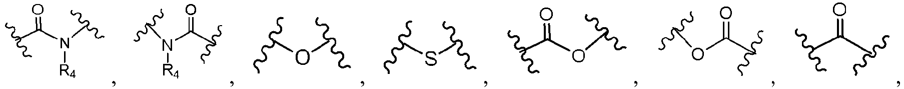 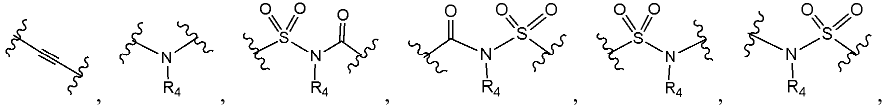 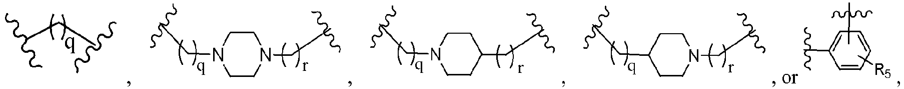
X is absent or has the following structure: 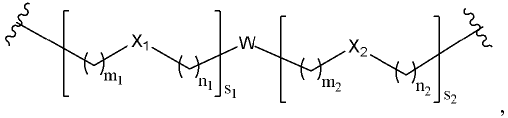
X₁ and X₂ are independently absent or selected from: 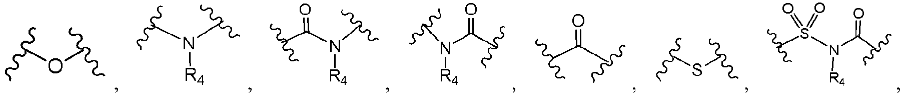 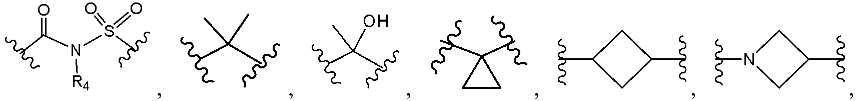 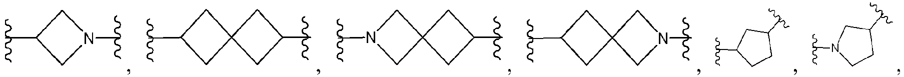 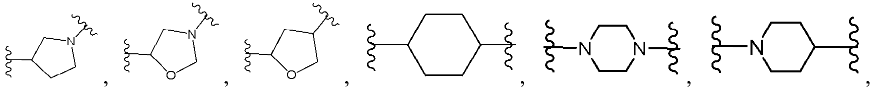 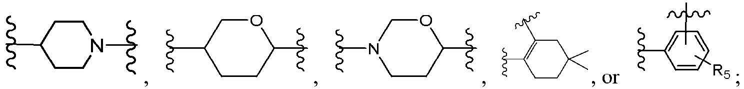
W is absent or selected from: 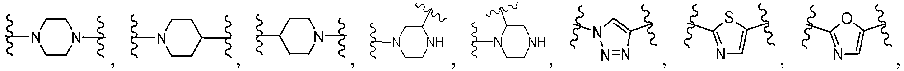 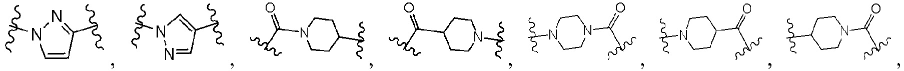 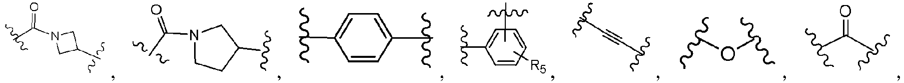 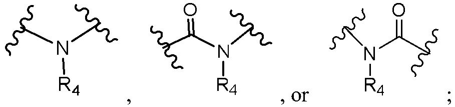
wherein,
R₄ is independently selected from hydrogen, substituted or unsubstituted alkyl, substituted or unsubstituted cycloalkyl, or substituted or unsubstituted heterocyclyl;
R₅ is independently selected from hydrogen, nitro, cyano, isocyano, amino, hydroxyl, thiol, halogen, substituted or unsubstituted alkyl, substituted or unsubstituted alkoxy, alkylamino, dialkylamino, carboxyl, carbonyl, amido, sulfonyl, sulfonic acid group, phosphoryl, or phosphonyl;
q and r are independently selected from integers from 0 to 10;
m₁, m₂, n₁, n₂, s₁ and s₂ are independently selected from integers from 0 to 10.

2. The compound of formula I according to claim 1, wherein the moiety A is a deuterated moiety having the structure of formula 1' or 2': 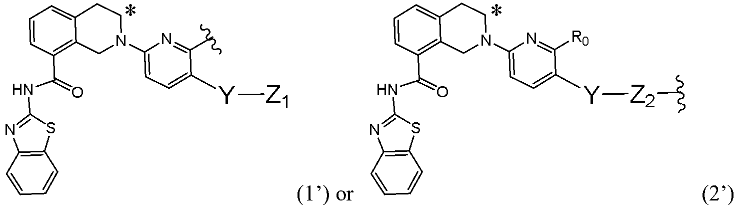
wherein one or more hydrogen atoms at position * are replaced by deuterium,
preferably, two hydrogen atoms at position * are replaced by deuterium.

3. The compound of formula I according to claim 1 or 2, wherein R^{a} and R^{b} are independently selected from hydrogen, cyano, alkyl, alkoxy, halogen-substituted alkyl, or halogen-substituted alkoxy.

4. The compound of formula I according to claim 1 or 2, wherein R₀ is absent or selected from -COOR^{a}, - CONHR^{b}, -SO₃R^{a}, -SO₂NHR^{b}, -SO₂NHCOR^{b}, -CONHSO₂R^{b}, -COSO₂NHR^{b}, wherein R^{a} and R^{b} are independently selected from hydrogen, cyano, or alkyl,
preferably, R₀ is selected from:

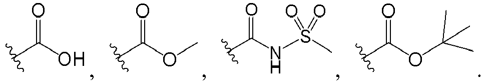

5. The compound of formula I according to claim 1, wherein Y is absent or selected from alkylene, alkyleneoxy, alkenylene, alkenyleneoxy, arylene, heteroarylene, cycloalkylene, or heterocyclylene, which are optionally substituted with one or more substituents selected from halogen or alkyl.

6. The compound of formula I according to claim 1, wherein Y is selected from alkylene, alkyleneoxy, alkenylene, or alkenyleneoxy,
preferably, Y is selected from 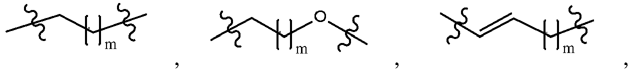 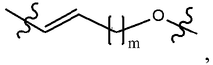 wherein m is 0, 1, 2 or 3.

7. The compound of formula I according to claim 1, wherein Y is selected from arylene or 5- or 6-membered heteroarylene, which are optionally substituted with one or more substituents selected from halogen or alkyl,
preferably, Y is selected from phenylene or pyrazolylene, which are optionally substituted with one or more substituents independently selected from halogen or alkyl,
more preferably, Y is selected from 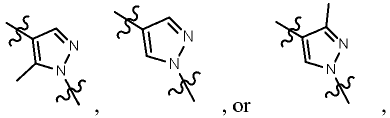 wherein Y is linked to Z₁ or Z₂ at the nitrogen atom indicated in the ring.

8. The compound of formula I according to claim 1, wherein Y is selected from the following structures: 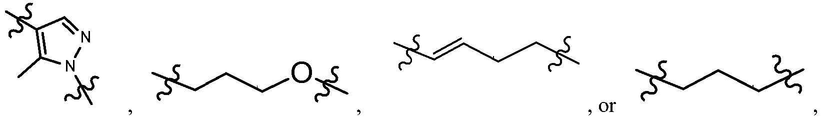 wherein Y is linked to Z₁ or Z₂ at the nitrogen atom indicated in the ring.

9. The compound of formula I according to claim 1, wherein Z₁ and Z₂ are independently selected from - alkylene-cyclic group, cycloalkyl-(R₂')ₚ, heterocyclyl-(R₂')ₚ, aryl-(R₂')ₚ, heteroaryl-(R₂')ₚ, aryl-propargylene, heteroaryl-propargylene, aryl-propargylene-N(R₂')₂, heteroaryl-propargylene-N(R₂')₂, aryl-propargylene-cycloalkyl-(R₂')ₚ, heteroaryl-propargylene-cycloalkyl-(R₂')ₚ, aryl-propargylene-heterocyclyl-(R₂')ₚ or heteroaryl-propargylene-heterocyclyl-(R₂')ₚ, which are optionally substituted with one or more substituent R_{1'},
wherein R_{1'} is selected from deuterium, halogen, amino, hydroxyl, nitro, thiol, cyano, isocyano, substituted or unsubstituted alkyl (e.g., alkyl substituted with halogen, amino, hydroxyl, nitro, and/or carboxyl), substituted or unsubstituted alkoxy (e.g., alkoxy substituted with halogen, amino, hydroxyl, nitro, and/or carboxyl), alkylamino, dialkylamino, carboxyl, carbonyl, amido, sulfonyl, sulfonic acid group, phosphoryl, or phosphonyl,
R₂' is hydrogen, alkyl, aryl, heteroaryl, cycloalkyl, or heterocyclyl, and
p is 0, 1, 2, 3, or 4.

10. The compound of formula I according to claim 9, wherein for the groups Z₁ and Z₂, the cyclic group is 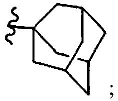
the cycloalkyl is cyclopentyl, cyclohexyl, cycloheptyl, or cyclooctyl;
the heterocyclyl is a 5- or 6-membered heterocyclyl containing one or two heteroatoms independently selected from nitrogen, oxygen, or sulfur, preferably the heterocyclyl is piperidinyl, piperazinyl, morpholinyl, or 3,9-diazaspiro[5.5]undecyl;
the aryl is phenyl; and/or
the heteroaryl is a 5- or 6-membered heteroaryl containing one or two heteroatoms independently selected from nitrogen, oxygen, or sulfur, preferably the heteroaryl is pyridinyl.

11. The compound of formula I according to claim 9, wherein Z₁ is selected from the following groups: 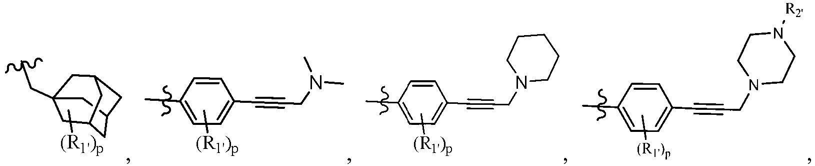 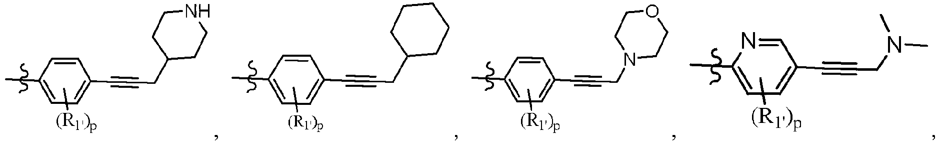 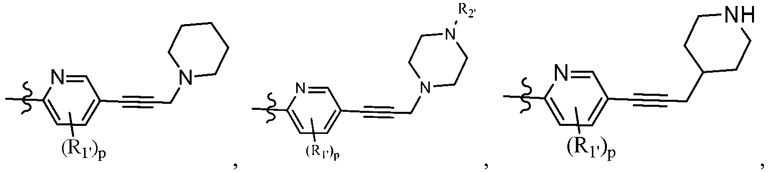 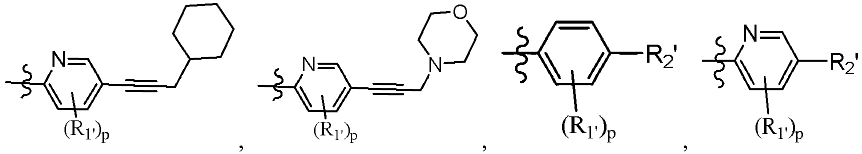 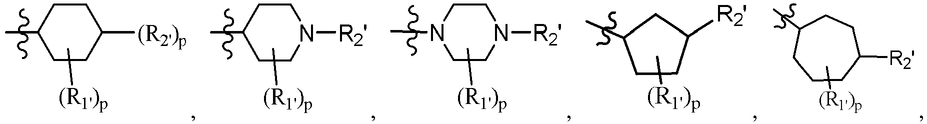 or 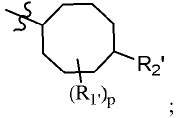 preferably, Z₁ is selected from the following groups:

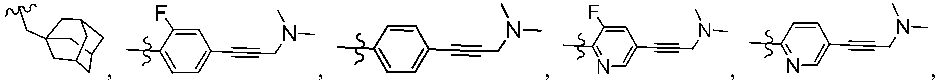

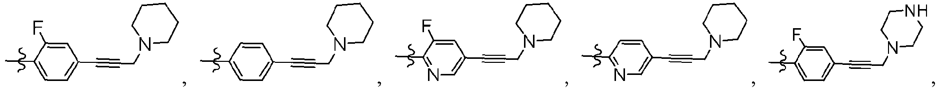

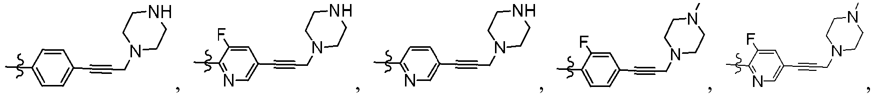

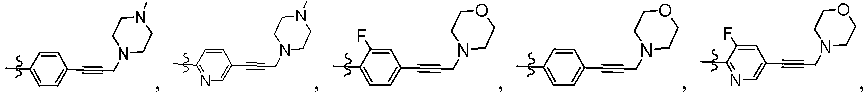

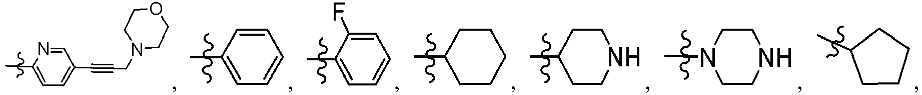

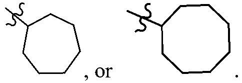

12. The compound of formula I according to claim 9, wherein Z₂ is selected from the following groups: 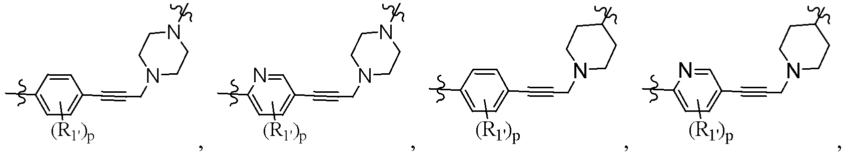 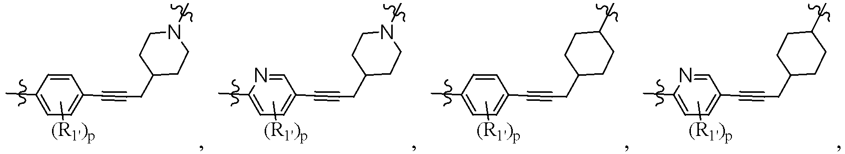 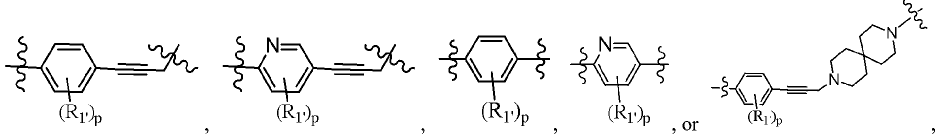 preferably, Z₂ is selected from the following groups:

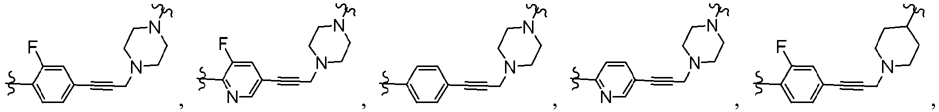

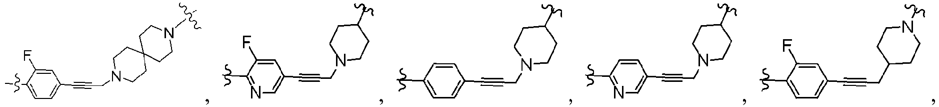

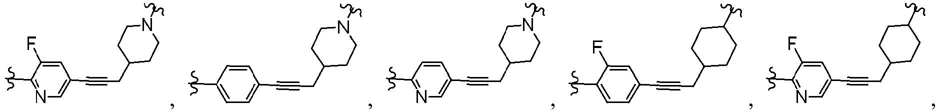

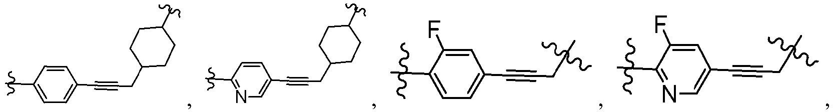

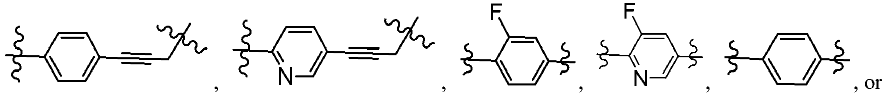

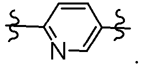

13. The compound of formula I according to claim 1 or 2, wherein the moiety A is selected from the following structures:

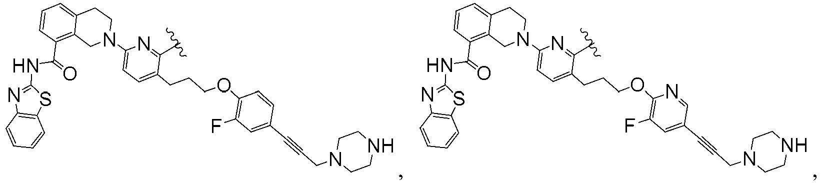

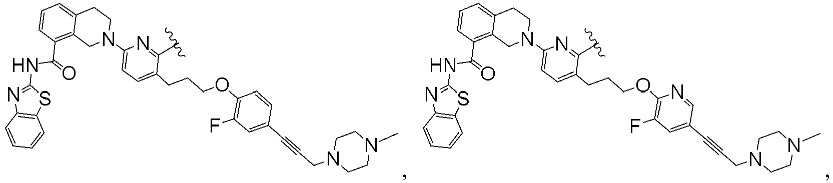

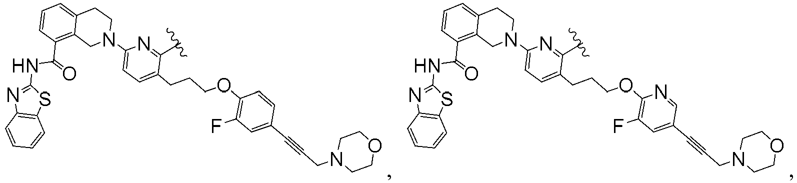

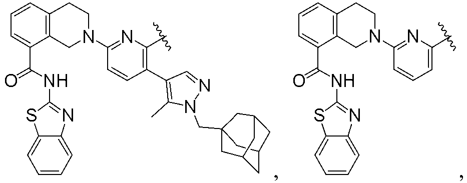

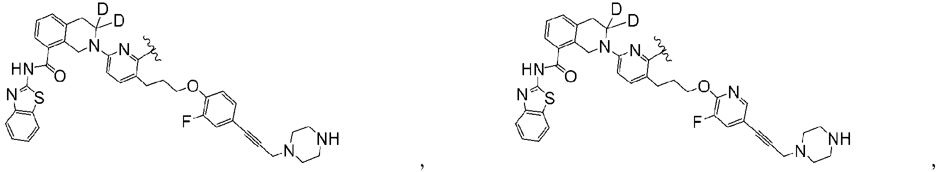

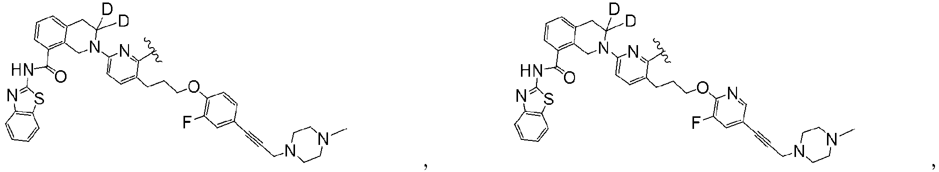

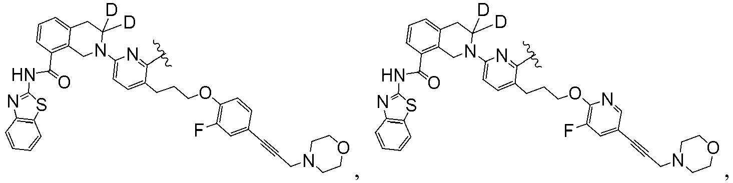

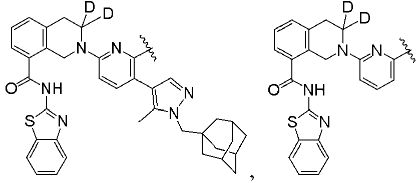

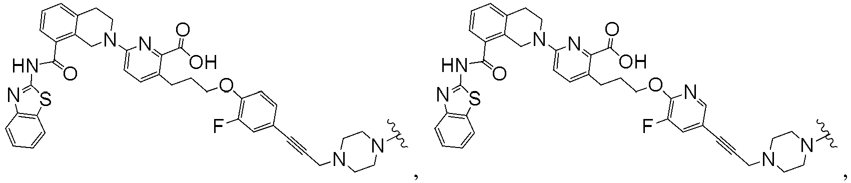

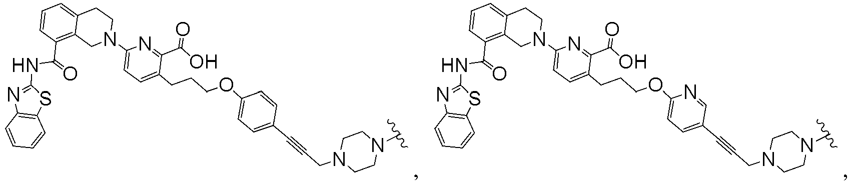

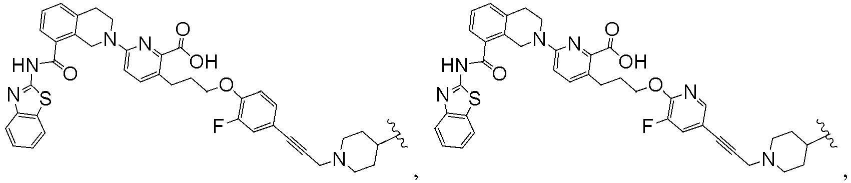

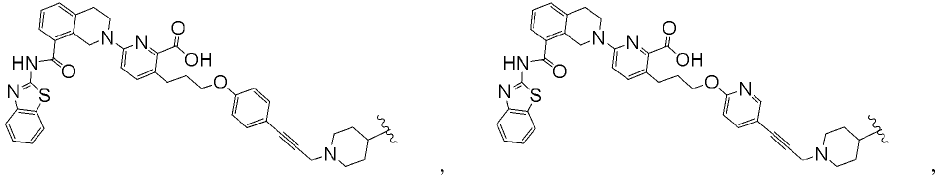

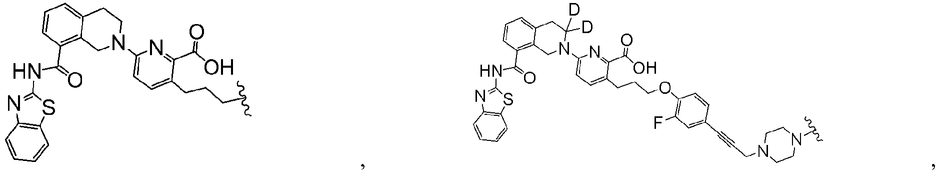

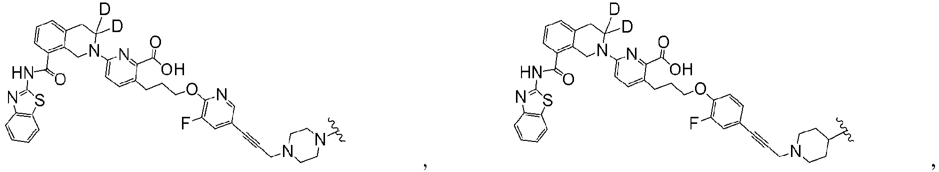

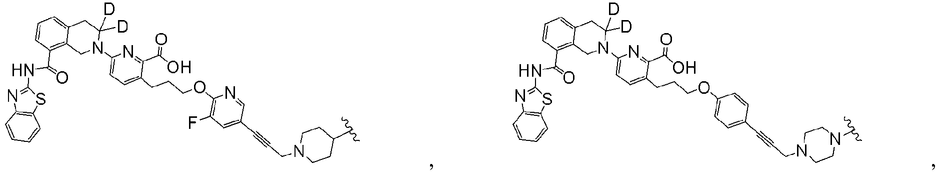

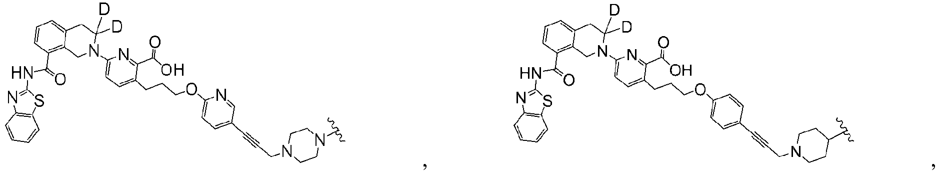

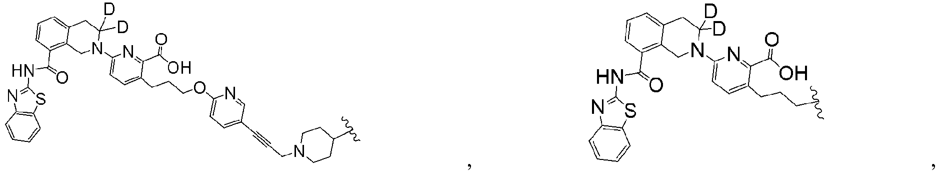

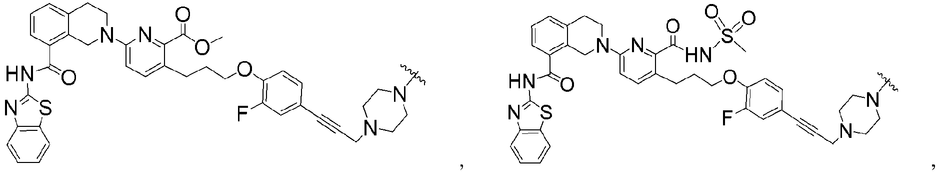

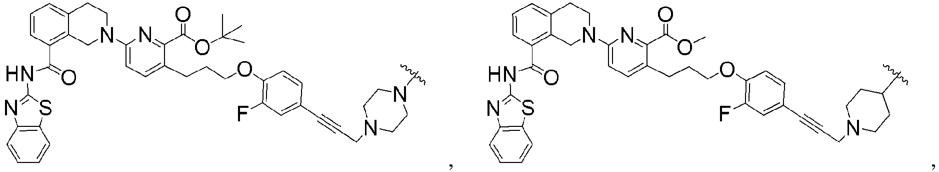

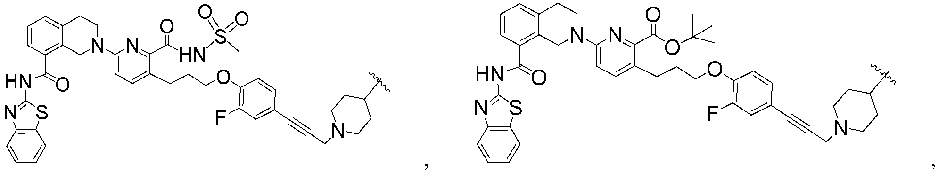

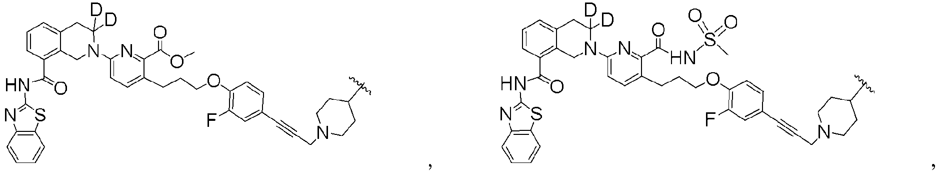

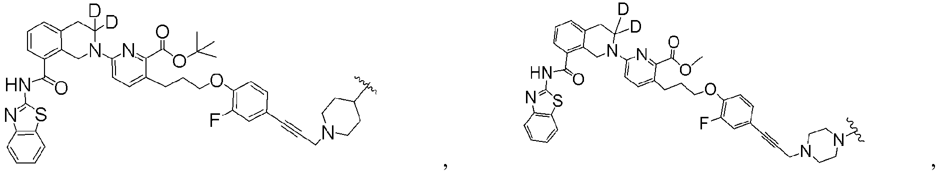

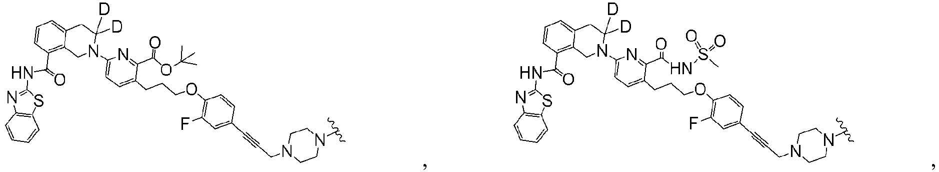

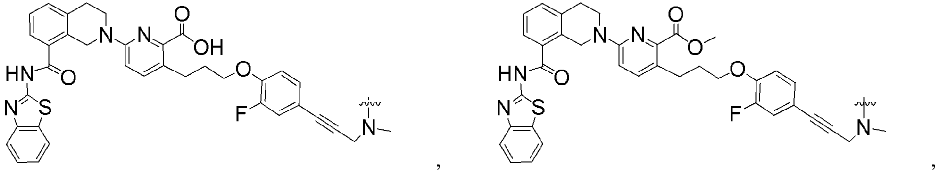

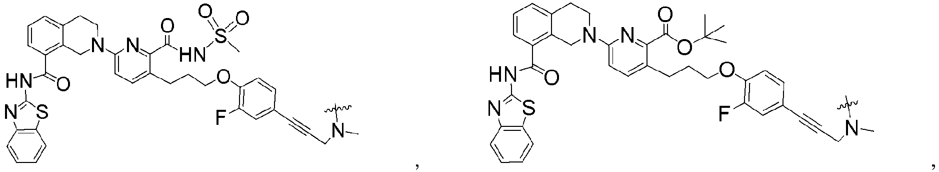

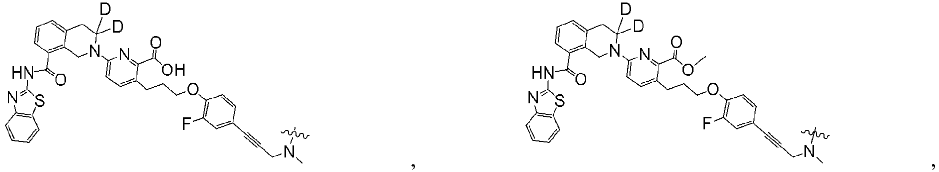

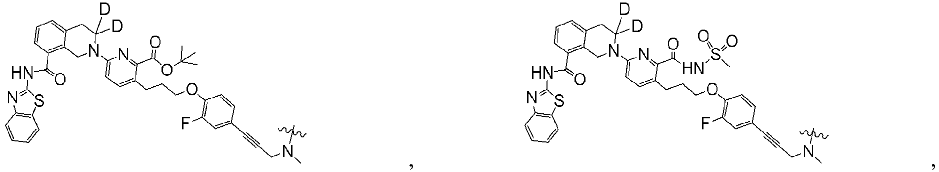

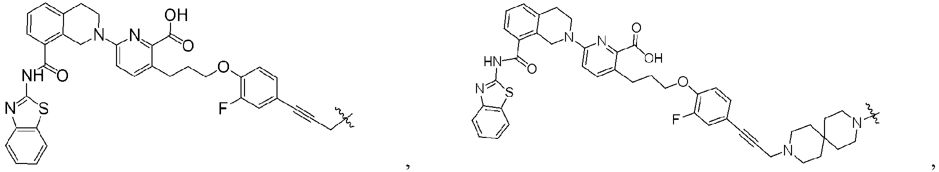

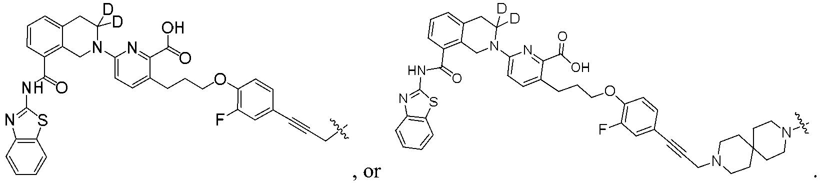

14. The compound of formula I according to claim 1, wherein the moiety E is selected from the following structures:

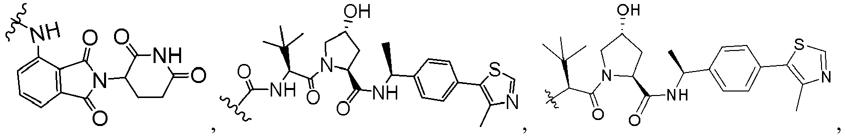

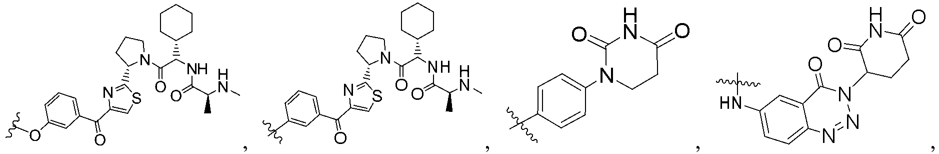

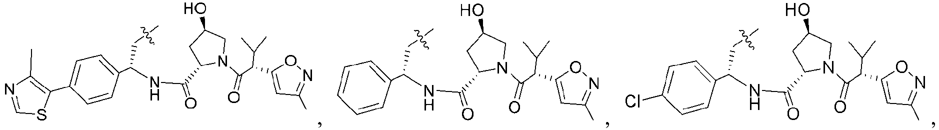

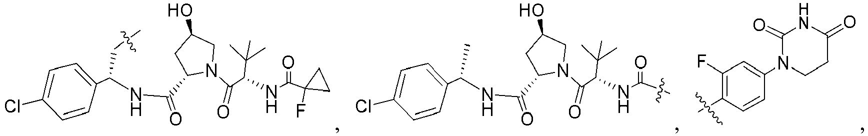

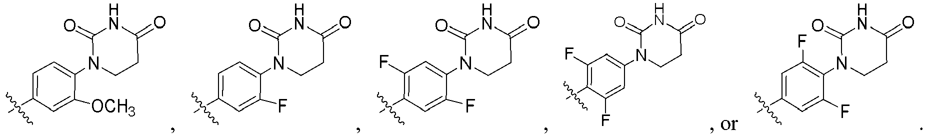

15. The compound of formula I according to claim 1, wherein L₁ is absent or selected from

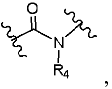

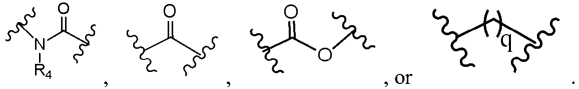

16. The compound of formula I according to claim 1, wherein L₂ is absent or selected from

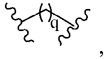

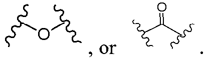

17. The compound of formula I according to claim 1, wherein the group X is absent or selected from: 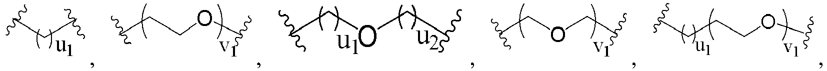 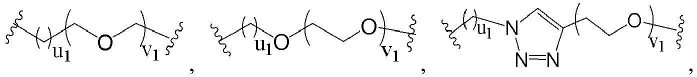 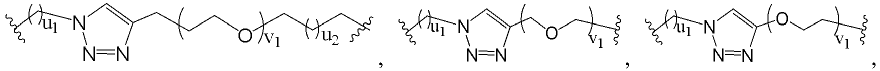 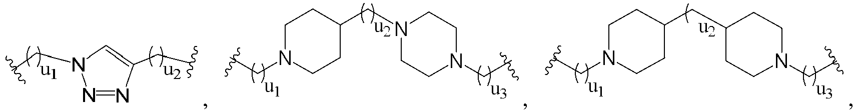 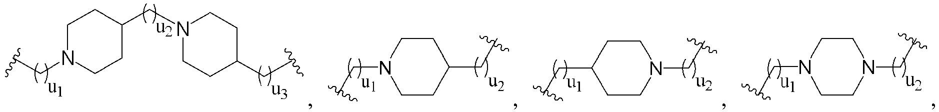 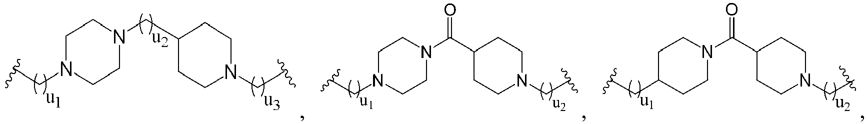 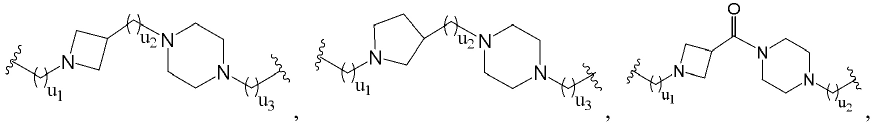 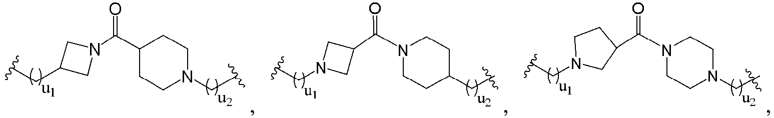 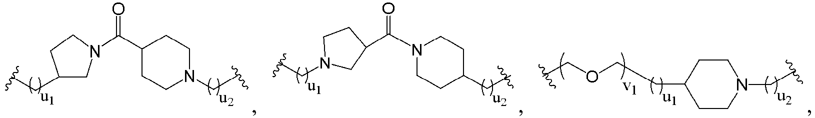 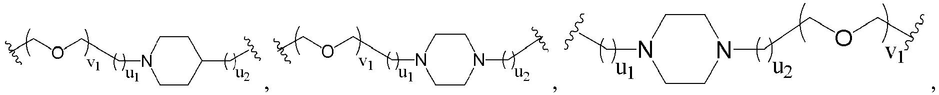 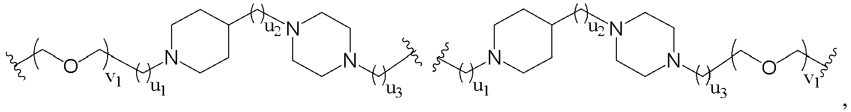 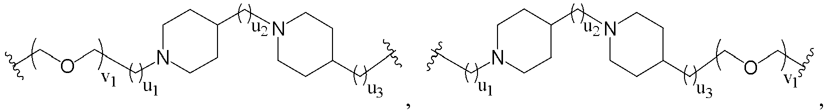 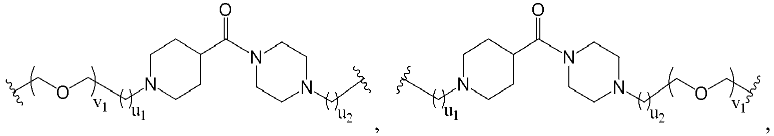 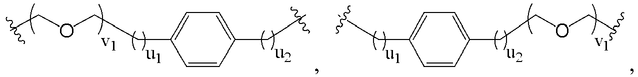 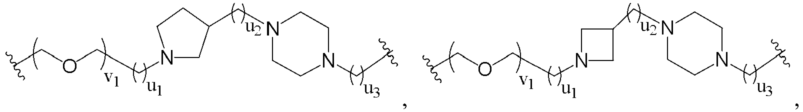 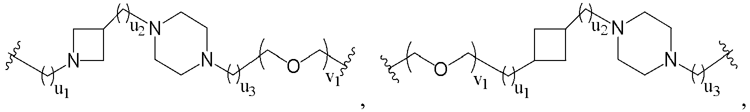 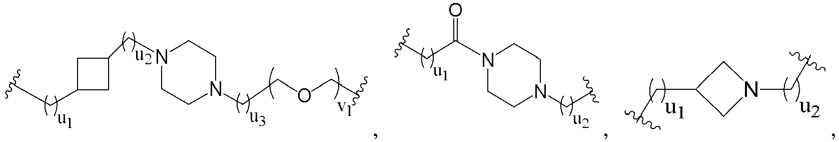 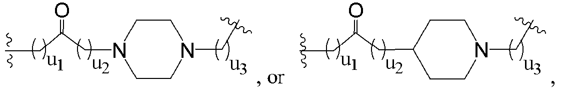 wherein u₁, u₂, u₃ and v₁ are independently selected from integers from 0 to 10.

18. The compound of formula I according to claim 1, wherein the moiety L is absent or selected from the following structures: 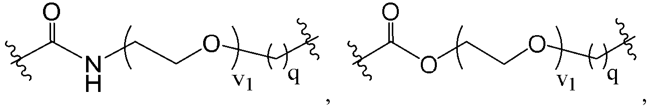 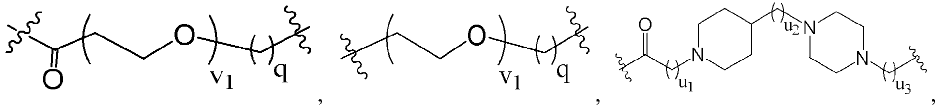 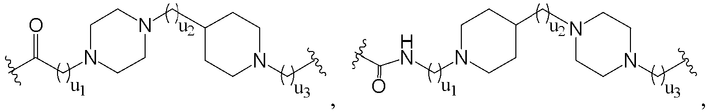 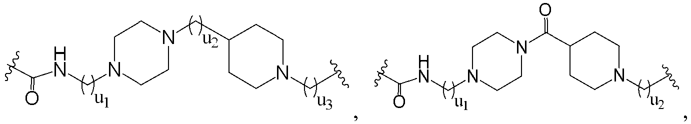 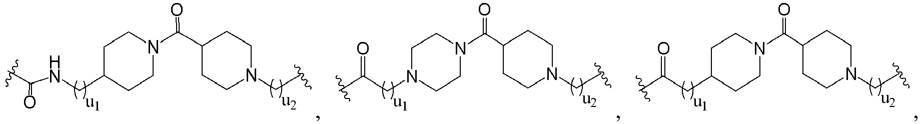 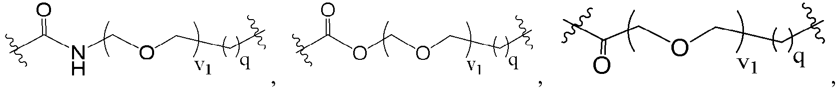 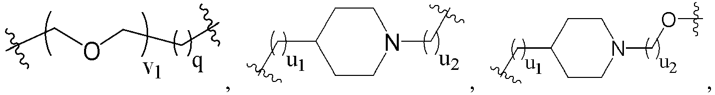 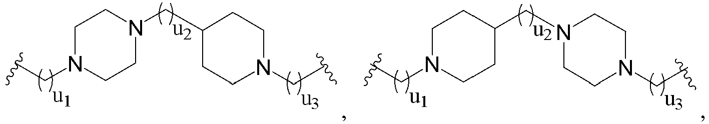 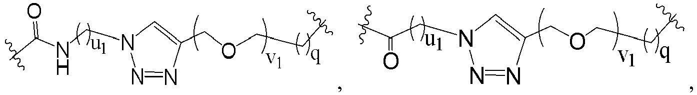 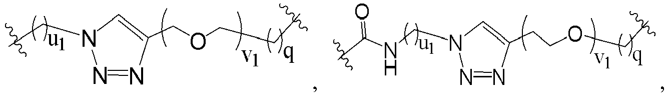 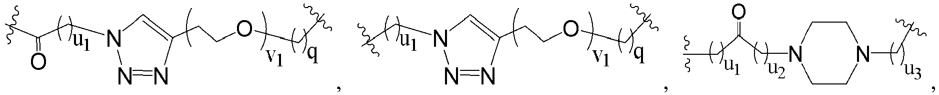 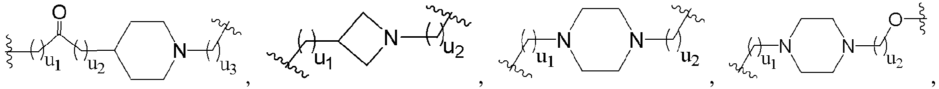 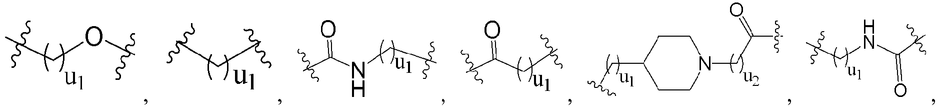 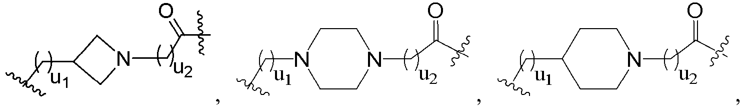 or 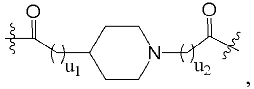 wherein q, u₁, u₂, u₃ and v₁ are independently selected from integers from 0 to 10.

19. The compound of formula I according to claim 1, wherein the moiety L is absent or selected from the following structures:

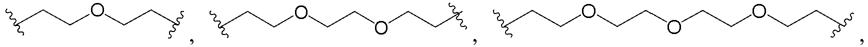

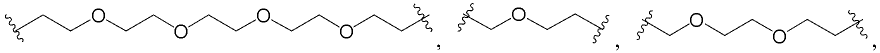

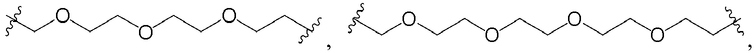

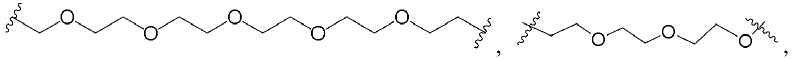

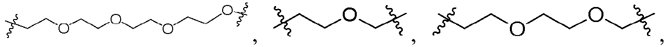

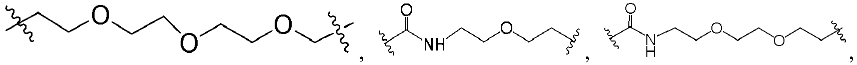

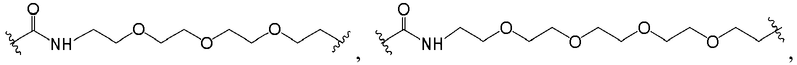

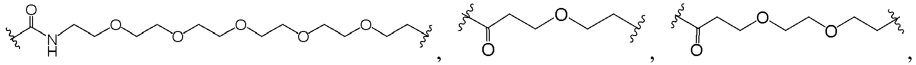

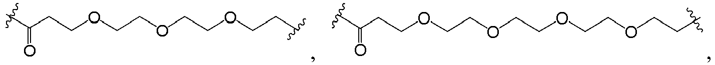

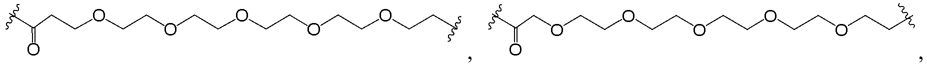

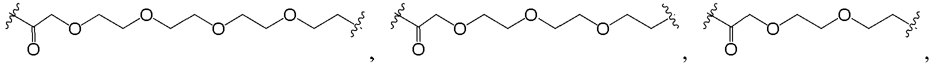

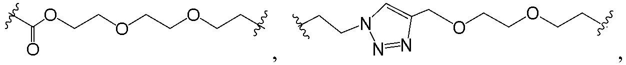

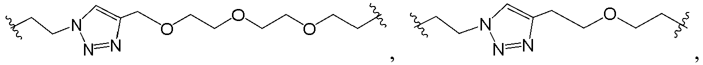

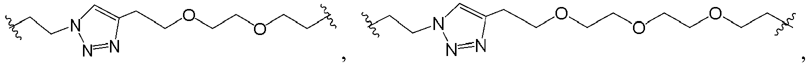

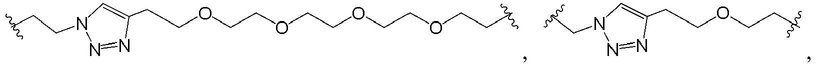

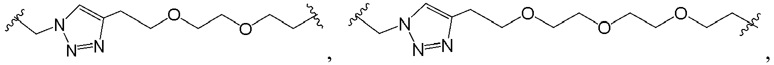

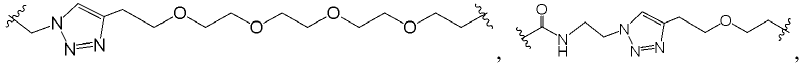

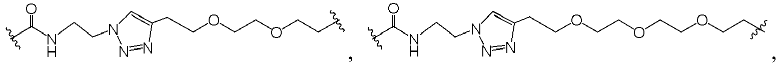

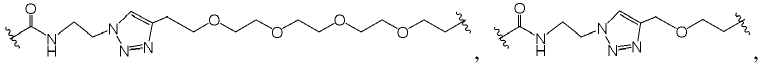

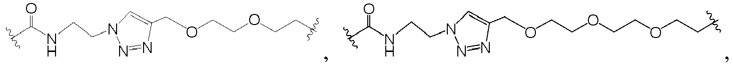

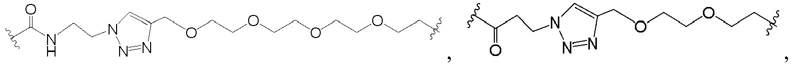

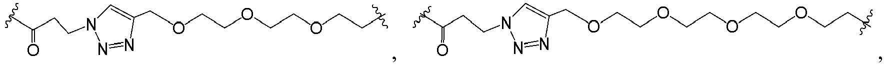

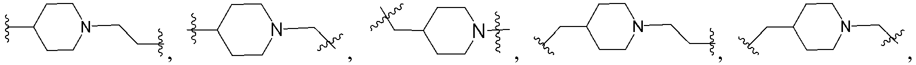

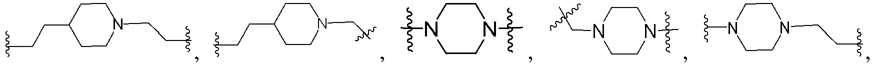

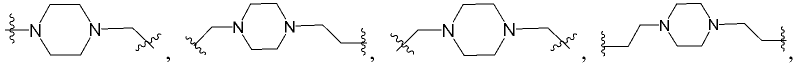

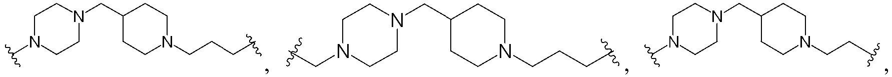

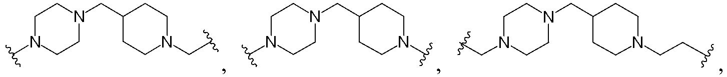

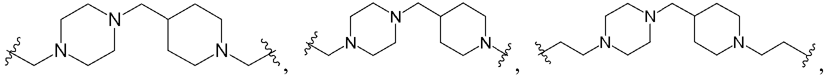

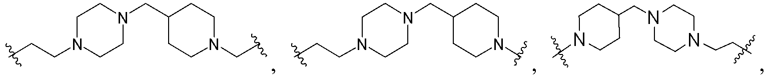

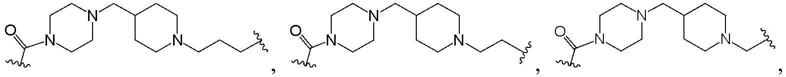

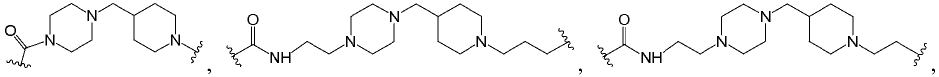

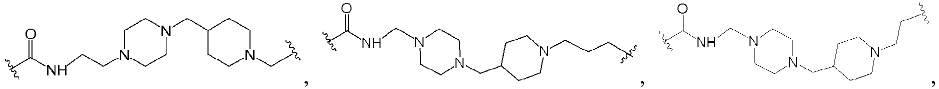

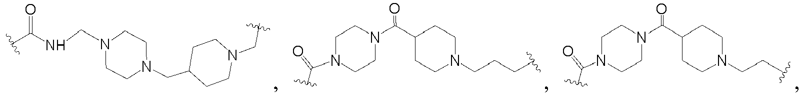

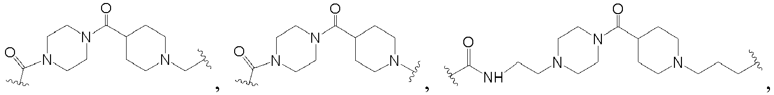

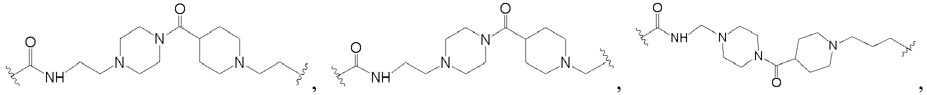

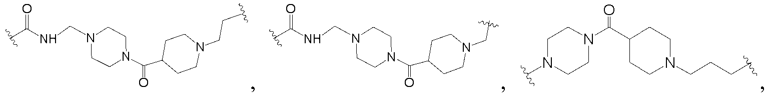

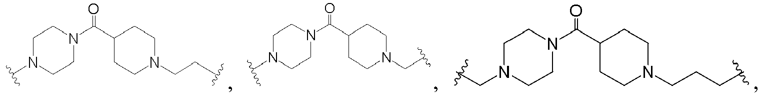

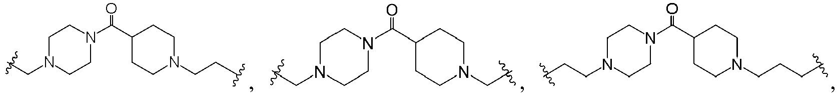

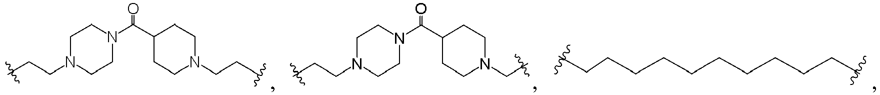

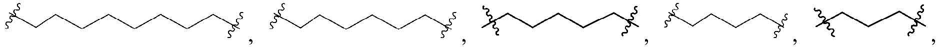

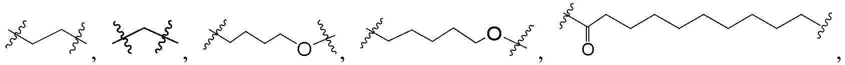

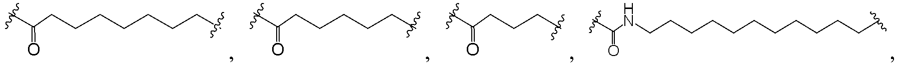

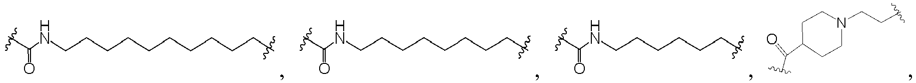

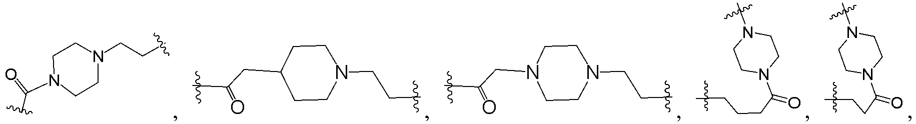

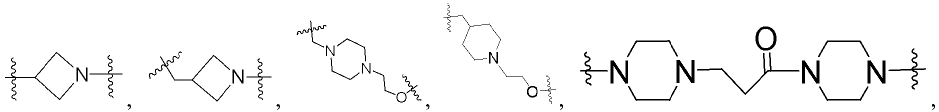

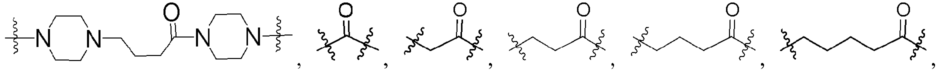

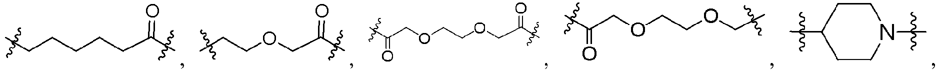

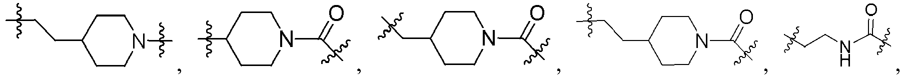

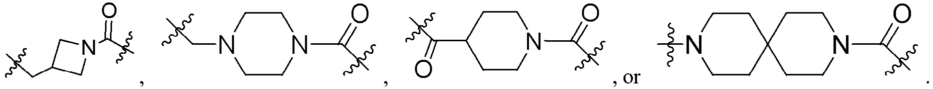

20. A composition comprising an effective amount of the compound of formula (I) according to any of claims 1-19 and a pharmaceutically acceptable carrier.

21. A method for selectively killing one or more senescent cells or treating an aging-related disease or disorder in a subject, comprising administering to the subject a therapeutically effective amount of the compound according to any of claims 1-19 or the composition according to claim 20.

22. Use of the compound according to any of claims 1-19 or the composition according to claim 20 in the manufacture of a medicament for selectively killing one or more senescent cells or treating an aging-related disease or disorder in a subject.

23. The method or use according to claim 21 or 22, wherein the aging-related disease or disorder is a metabolic disease, an inflammatory disease or disorder, a pulmonary disease or disorder, a neurological disease or disorder, a proliferative disorder, a renal disorder or disease, an ocular disease or disorder, or a dermatological disorder or disease.

24. The method or use according to claim 21 or 22, wherein the aging-related disease or disorder is selected from:
(i) an inflammatory or autoimmune disease or disorder selected from oral mucositis, inflammatory bowel disease, kyphosis, or herniated disc;
(ii) a neurological disease or disorder selected from Alzheimer's disease, Parkinson's disease, Huntington's disease, dementia, mild cognitive impairment, macular degeneration, or motor neuron dysfunction;
(iii) a metabolic disease selected from diabetic ulcer, metabolic syndrome, or obesity;
(iv) a pulmonary disease selected from pulmonary fibrosis, chronic obstructive pulmonary disease, asthma, cystic fibrosis, emphysema, bronchiectasis, or age-related loss of lung function;
(v) an ocular disease or disorder selected from macular degeneration, glaucoma, cataracts, presbyopia, or vision loss;
(vi) an age-related disorder selected from renal failure, frailty, hearing loss, muscle fatigue, skin conditions, skin wound healing, liver fibrosis, pancreatic fibrosis, oral submucous fibrosis, or sarcopenia; or
(vii) a skin disease or disorder selected from eczema, psoriasis, hyperpigmentation, moles, rashes, atopic dermatitis, urticaria, diseases and disorders associated with photosensitivity or photoaging, wrinkles, pruritus, hypoesthesia, eczematous rashes, eosinophilic dermatoses, reactive neutrophilic dermatoses, pemphigus, pemphigoid, immunobullous skin diseases, cutaneous fibrohistiocytosis, cutaneous lymphoma, or cutaneous lupus.

25. The method or use according to claim 21 or 22, wherein the subject has been diagnosed with cancer, and optionally is undergoing a cancer treatment selected from chemotherapy or radiotherapy.

26. The method or use according to claim 21 or 22, wherein the compound or composition
(i) reduces cells that have been pushed towards senescence or cancer cells;
(ii) reduces one or more side effects produced by senescent cells, wherein the side effects include inflammation, promotion of cancer growth, or promotion of metastasis;
(iii) reduces one or more side effects of chemotherapy; or
(iv) reduces one or more side effects of radiotherapy.

27. The method or use according to claim 21 or 22, wherein the subject has a viral infection, optionally wherein the viral infection causes the overproduction of activated macrophages in the subject.

28. The method or use according to claim 27, wherein the viral infection is a coronavirus infection caused by a virus selected from coronavirus, severe acute respiratory syndrome (SARS) coronavirus (CoV), SARS-CoV-2 (which causes COVID-19 (Coronavirus Disease 2019)), or Middle East respiratory syndrome (MERS)-CoV.

29. The method or use according to claim 27, wherein the subject has a SARS-CoV-2 infection and has been diagnosed with Coronavirus Disease 2019.

30. The method according to claim 21, wherein a compound having the following structure is obtained in an effective amount for killing one or more senescent cells after administrating the compound or composition in vivo: 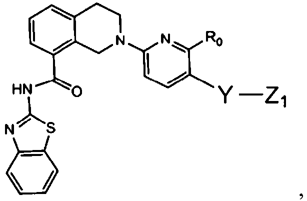 wherein R₀, Y and Z₁ are defined as in any of claims 1-19.
